# EUROPEAN PATENT APPLICATION

(11) **EP 4 851 157 A1**
(43) Date of publication of application: **09.12.2026**
(21) Application number: 25181261.6
(22) Date of filing: 06.06.2025
(51) Int. Cl.: C07D 413/14, A01N 43/88

(54) **DIAZINONE COMPOUNDS FOR THE CONTROL OF INVERTEBRATE PESTS**

(71) Applicant: BASF Agricultural Solutions Deutschland GmbH, 67117 Limburgerhof (DE)
(72) Inventor: SCHISSLER, Christoph, 67056 Ludwigshafen am Rhein (DE); POHLMAN, Matthias, 67056 Ludwigshafen am Rhein (DE); CHAUDHURI, Rupsha, 67056 Ludwigshafen am Rhein (DE); WAKEHAM, Matthew Charles Linford, 67056 Ludwigshafen am Rhein (DE)
(74) Representative: AP-IP

(57) **Abstract**

The invention relates to compounds of formula I 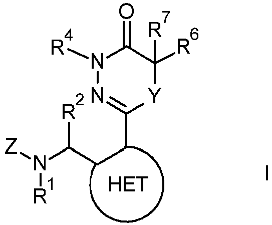 wherein the variables have the meanings as defined in the specification, to compositions comprising them, to active compound combinations comprising them, and to their use for protecting growing plants and animals from attack or infestation by invertebrate pests, furthermore, to seed comprising such compounds.

## Description

The invention relates to compounds of formula I 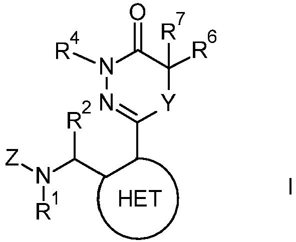 wherein
- R¹: is H, OR^{10a}, NR¹²R¹³, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₆-alkoxy, C₂-C₆-alkenyl, C₂-C₆-alkynyl, C₁-C₆-alkyl-C₁-C₆-alkoxy, C₁-C₆-alkoxy-C₃-C₆-cycloalkyl, C₃-C₆-cycloalkyl-C₁-C₆-alkoxy, C₂-C₆-alkenyl-C₃-C₆-cycloalkyl, C₂-C₆-alkynyl-C₃-C₆-cycloalkyl, C₃-C₆-cycloalkyl-C₂-C₆-alkenyl, C₃-C₆-cycloalkyl-C₂-C₆-alkynyl, wherein the alkyl, alkoxy, alkenyl, alkynyl, and cycloalkyl moieties are unsubstituted or substituted with R¹¹; or C(O)R^{11a};
- R²: is H, C₁-C₃-alkyl, C₂-C₃-alkenyl, C₃-C₆-cycloalkyl, or C₂-C₃-alkynyl, wherein the alkyl, alkenyl, alkynyl and cycloalkyl moieties are unsubstituted or substituted with one or more halogen;
- R³: is halogen, CN, NO₂, SF₅, C₁-C₆-alkyl, C₁-C₆-alkoxy, C₃-C₆-cycloalkyl, C₂-C₆-alkenyl, C₂-C₆-alkynyl, C₃-C₆-cycloalkyl-C₁-C₆-alkyl, C₁-C₆-alkyl-C₃-C₆-cycloalkyl, 3- to 6-membered heterocyclyl, wherein the alkyl, alkenyl, alkynyl, alkoxy, heterocyclyl and cycloalkyl moieties are unsubstituted or substituted with R¹¹; OR^{10a}, NR¹²R¹³, C(O)NR¹²R¹³, C(O)OR¹⁰, C(O)R¹⁴, OS(O)₂-R¹⁴, S(O)ₘ-R¹⁴, -N=S(O)R^{12a}R^{13a};
- R⁴: H, C₁-C₃-alkyl, C₂-C₄-alkenyl, C₂-C₄-alkynyl, C₃-C₆-cycloalkyl-C₁-C₂-alkyl, C₁-C₂-alkyl-C₃-C₆-cycloalky, C₃-C₆-cycloalkyl, wherein the alkyl, alkenyl, alkynyl or cycloalkyl moieties are unsubstituted or substituted with one or more halogen, CN, or C(O)NH₂; phenyl or 5- or 6-membered hetaryl, wherein the phenyl or hetaryl moiety are unsubstituted or substituted with one or more halogen, CN, C(O)NH₂, C₁-C₃-alkyl, C₁-C₃-haloalkyl, C₁-C₃-alkoxy, or C₁-C₃-haloalkoxy;
- HET: is a group HA or HB 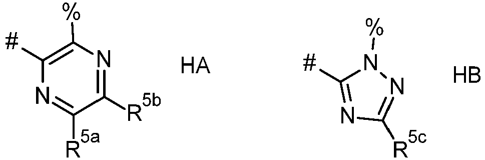 wherein # is the bond to the CH(R²)N(R¹) spacer, and % is the bond to the diazinone ring;
- R^{5a}, R^{5b}: are independently from each other H, halogen, CN, C₁-C₃-alkyl, C₁-C₃-haloalkyl, C₃-C₆-cycloalkyl, C₃-C₆-cycloalkyl-C₁-C₃-alkyl, C₁-C₃-alkyl-C₃-C₆-cycloalkyl, C₁-C₃-alkoxy, C₁-C₃-haloalkoxy, C₂-C₃-alkenyl, or C₂-C₃-alkynyl;
- R^{5c} is H,: halogen, CN, OR^{10a}, NR¹²R¹³, C(O)NR¹²R¹³, C(O)OR^{10a}, C(O)R¹⁴, S(O)ₘ-R¹⁴, C₁-C₃-alkyl, C₃-C₆-cycloalkyl, C₃-C₆-cycloalkyl-C₁-C₃-alkyl, C₁-C₃-alkyl-C₃-C₆-cycloalkyl, C₁-C₃-alkoxy, C₂-C₃-alkenyl, C₂-C₃-alkynyl, -C(=NOC₁-C₄-alkyl)H, or -C(=NOC₁-C₄-alkyl)-C₁-C₄-alkyl, wherein alkyl, alkenyl, alkynyl, cycloalkyl and alkoxy are unsubstituted or substituted with one or more halogen and/or CN;
- R⁶, R⁷: are independently from each other H, halogen, CN, C(O)NH₂, C₁-C₃-alkyl, C₁-C₃-haloalkyl, or C₃-C₆-cycloalkyl; or
R⁶ and R⁷ together with the carbon atom to which they are bound, form a 3-, 4-, 5-, or 6-membered saturated or partially unsaturated carbocycle, wherein the carbocycle is unsubstituted or substituted with one or more halogen, cyano, C₁-C₃-alkyl, or C₁-C₃-haloalkyl; or form a 3-, 4-, 5-, or 6-membered saturated or partially unsaturated heterocycle, which may contain 1 or 2 heteroatoms or heteroatom-containing groups selected from N, O, S(O)ₘ, and optionally one or two C(O) groups as ring members, wherein the heterocycle is unsubstituted or substituted with one or more halogen, cyano, C₁-C₃-alkyl, C₁-C₃-haloalkyl;
- R¹⁰: is H, C₁-C₄-alkyl, C₁-C₄-haloalkyl, C₃-C₆-cycloalkyl, C₃-C₆-halocycloalkyl, C₃-C₄-cycloalkyl-C₁-C₂-alkyl, C₃-C₄-halocycloalkyl-C₁-C₂-alkyl, C(O)-C₁-C₄-alkyl, C(O)-C₁-C₄-haloalkyl, C(O)-C₃-C₄-cycloalkyl, C(O)-C₃-C₄-halocycloalkyl, SOₘ-C₁-C₄-alkyl, SOₘ-C₁-C₄-haloalkyl, SOₘ₋C₃-C₆-cycloalkyl, or phenyl which is unsubstituted or partially or fully substituted with R^{a};
- R^{10a}: is H, C₃-C₆-cycloalkyl, C₃-C₆-halocycloalkyl, C₃-C₄-cycloalkyl-C₁-C₂-alkyl, C₃-C₄-halocycloalkyl-C₁-C₂-alkyl, C(O)-C₁-C₄-alkyl, C(O)-C₁-C₄-haloalkyl, C(O)-C₃-C₄-cycloalkyl, C(O)-C₃-C₄-halocycloalkyl, SOₘ-C₁-C₄-alkyl, SOₘ-C₁-C₄-haloalkyl, SOₘ-C₃-C₆-cycloalkyl, or phenyl which is unsubstituted or partially or fully substituted with R^{a};
- R¹¹: is halogen, CN, NO₂, NR¹²R¹³, C(O)NH₂, C(S)NH₂, C(O)OH, OR¹⁰; 3- to 6-membered heterocyclyl, 5- or 6-membered hetaryl, or phenyl, wherein the heterocyclyl, hetaryl and phenyl moieties are unsubstituted or substituted with one or more halogen, C₁-C₃-haloalkyl, and/or CN;
- R^{11a}: is NR¹²R¹³, C₁-C₆-alkyl, C₁-C₆-alkoxy, C₂-C₆-alkenyl, C₂-C₆-alkynyl, C₃-C₄-cycloalkyl-C₁-C₂-alkyl, wherein the alkyl, alkoxy, alkenyl, alkynyl or cycloalkyl moiety is unsubstituted or substituted with one or more halogen; or 3- to 6-membered heterocyclyl, wherein the heterocyclyl moiety is unsubstituted or substituted with one or more halogen, C₁-C₃-haloalkyl, and/or CN;
- R¹², R¹³: are independently from each other H, C₁-C₄-alkyl, C₁-C₄-alkoxy, C₁-C₄-haloalkoxy, C₁-C₄-haloalkyl, C₃-C₆-cycloalkyl, C(O)-C₁-C₄-alkyl, C(O)-C₁-C₄-haloalkyl, C(O)-C₃-C₄-cycloalkyl, C(O)-C₃-C₄-halocycloalkyl, C(O)NH-C₁-C₄-alkyl, C(O)NH-C₁-C₄-haloalkyl, C(O)N(C₁-C₄-alkyl)-C₁-C₄-alkyl, C(O)N(C₁-C₄-haloalkyl)-C₁-C₄-alkyl, C(O)N(C₁-C₄-haloalkyl)-C₁-C₄-haloalkyl, C(O)NH-C₁-C₄-alkoxy, C(O)NH-C₁-C₄-haloalkoxy, C(O)NH-C₁-C₄-alkoxy-C₁-C₄-alkyl, C(O)NH-C₁-C₄-alkoxy-C₁-C₄-haloalkyl; C(O)NH-3-6-membered heterocyclyl, C(O)NH-phenyl, C(O)NH-5-6-membered hetaryl, C(O)NH-C₁-C₄-alkyl-3-6-membered heterocyclyl, C(O)NH-C₁-C₄-alkylphenyl, C(O)NH-C₁-C₄-alkyl-5-6-membered hetaryl, wherein the phenyl, heterocyclyl and hetaryl moieties are unsubstituted or substituted with halogen, C₁-C₃-haloalkyl, and/or CN; S(O)ₘ-C₁-C₄-haloalkyl, S(O)ₘC₃-C₄-cycloalkyl, S(O)ₘ-C₃-C₄-halocycloalkyl; 3- to 6-membered heterocyclyl, 5- or 6-membered hetaryl, or phenyl, wherein the phenyl, heterocyclyl and hetaryl moieties are unsubstituted or substituted with halogen, C₁-C₃-haloalkyl, and/or CN; or
R¹² and R¹³ together with the atom/atoms to which they are bound, form a 3-, 4-, 5-, 6-, or 7-membered saturated or partially unsaturated heterocycle, which heterocycle may additionally contain 1 or 2 heteroatoms or heteroatom-containing groups selected from N, O, S(O)ₘ, and optionally one or two groups C(O) as ring members, and wherein the heterocycle moiety is unsubstituted or substituted with one or more R^{a};
- R^{12a}, R^{13a}: are independently from each other C₁-C₄-alkyl, C₁-C₄-haloalkyl, C₃-C₆-cycloalkyl, unsubstituted or substituted with halogen, C₁-C₃-haloalkyl, and/or CN; or R^{12a} and R^{13a} together with the atom to which they are bound, form a 3-, 4-, 5-, 6-, or 7-membered saturated or partially unsaturated heterocycle, wherein the heterocycle moiety may additionally contain 1 or 2 heteroatoms or heteroatom-containing groups selected from N, O, S(O)ₘ, and optionally one or two groups C(O) as ring members, and wherein the heterocycle moiety is unsubstituted or substituted with one or more R^{a};
- R¹⁴: is H, C₁-C₆-alkyl, C₃-C₆-cycloalkyl, wherein the alkyl and cycloalkyl moieties are unsubstituted or partially or fully substituted with R¹¹; or 3- to 6-membered heterocyclyl, 5- or 6-membered hetaryl, or phenyl, wherein the phenyl, heterocyclyl and hetaryl moieties are unsubstituted or substituted with R^{a};
- R^{a}: is halogen, CN, NO₂, OH, C₁-C₄-alkyl, C₁-C₄-alkoxy, C₁-C₄-haloalkyl, C₁-C₄-alkyl-C₁-C₄-alkoxy, C₁-C₄-haloalkoxy, C₃-C₄-cycloalkyl, C₃-C₄-halocycloalkyl, S(O)ₘ-C₁-C₄-alkyl, S(O)ₘ-C₁-C₄-haloalkyl, S(O)ₘ-C₃-C₄-cycloalkyl, or S(O)ₘ-C₃-C₄-halocycloalkyl;
- m: is 0, 1, or 2;
- n: is 0, 1, 2, 3 or 4;
- Y: is O, S, or N-R^{N};
- Z: R^{N} is as defined for R⁴;
is a 9- or 10-membered saturated, partially or fully unsaturated bicyclic heterocyclic ring which contains 1-4 heteroatoms or heteroatom-containing groups selected from N, O, S(O)ₘ, wherein the bicyclic heterocyclic ring is unsubstituted or substituted with 1-4 R³;
and the N-oxides, stereoisomers, tautomers, and agriculturally or veterinarily acceptable salts thereof.

The invention also provides agricultural compositions comprising at least one compound of formula I, a stereoisomer thereof and/or an agriculturally acceptable salt thereof and at least one liquid and/or solid carrier, especially at least one inert liquid and/or solid agriculturally acceptable carrier.

The invention also provides a veterinary composition comprising at least one compound of formula I, a stereoisomer thereof and/or a veterinarily acceptable salt thereof and at least one liquid and/or solid carrier, especially at least one inert veterinarily liquid and/or solid acceptable carrier.

The invention also provides a method for controlling invertebrate pests which method comprises treating the pests, their food supply, their habitat or their breeding ground or a cultivated plant, plant propagation materials (such as seed), soil, area, material or environment in which the pests are growing or may grow, or the materials, cultivated plants, plant propagation materials (such as seed), soils, surfaces or spaces to be protected from pest attack or infestation with a pesticidally effective amount of a compound of formula I or a salt thereof as defined herein.

The invention also relates to plant propagation material, in particular seed, comprising at least one compound of formula I and/or an agriculturally acceptable salt thereof.

The invention further relates to a method for treating or protecting an animal from infestation or infection by parasites which comprises bringing the animal in contact with a parasiticidally effective amount of a compound of formula I or a veterinarily acceptable salt thereof. Bringing the animal in contact with the compound I, its salt or the veterinary composition of the invention means applying or administering it to the animal.

WO2023104714, WO2023/072849, WO2021148639, WO2021/083936, WO2021/177160 and WO2020/212235 describe structurally closely related active compounds, that feature different moieties in the position of the diazinone ring. These compounds are mentioned to be useful for combating invertebrate pests.

Nevertheless, there remains a need for highly effective and versatile agents for combating invertebrate pests. It is therefore an object of the invention to provide compounds having a good pesticidal activity and showing a broad activity spectrum against a large number of different invertebrate pests, especially against difficult to control pests, such as insects.

It has been found that these objects can be achieved by compounds of formula I as depicted and defined below, and by their stereoisomers, salts, tautomers and N-oxides, in particular their agriculturally acceptable salts.

Formula I correspond to formulae I.A and I.B.

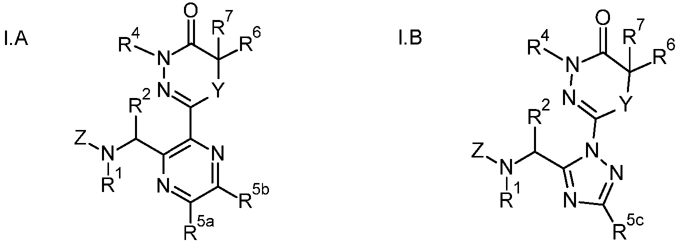

The amine intermediates I.A.I or I.B.I and the bicyclic compound BC1-Cl, BC2-Cl and BC3-Cl can undergo a nucleophilic substitution reaction to yield the final compound I.A or I.B.

To obtain the final product I.A or I.B, an amine building block I.A.I or I.B.I and a halogenated bicyclic compound (e.g., BC1-Cl, BC2-Cl or BC3-Cl; commercially availabe or known in the literature, e.g. WO2024110554, WO2021148639, WO2021110891, WO2022101265) can undergo an nucleophilic substitution reaction, e.g. base-catalyzed by, e.g., triethylamine as reported in, e.g. WO2024110554, yield I.A or I.B.

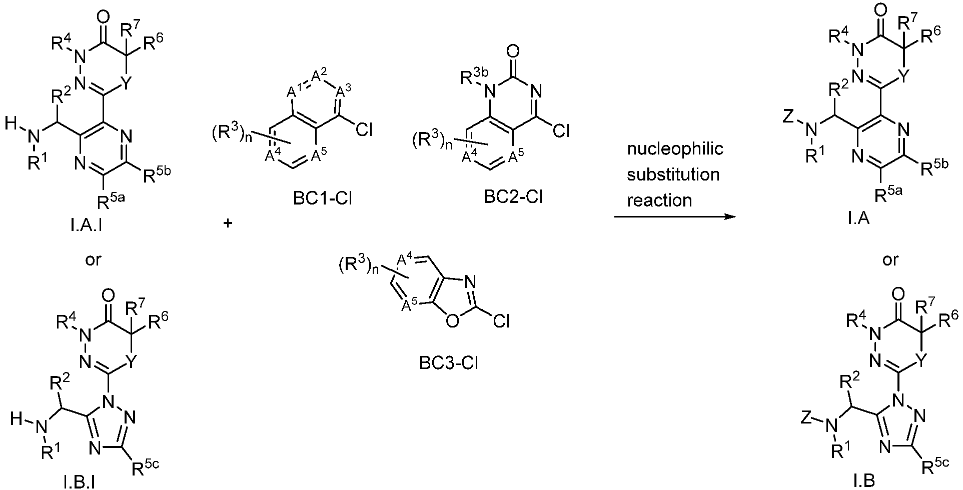

The amine intermediates I.A.I and I.B.I:

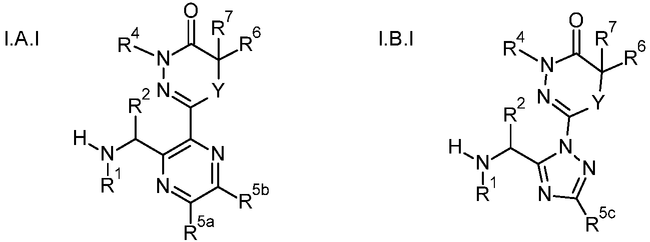

The amine intermediate I.A.I with Y=O (formula I.A.IO) can be prepared via an reductive amination reaction from I.A.II with NR¹H₂ in the presence of reducing agent such as NaBH₄ or NaCNBH₃, in the presence of a metal-catalyst such as, e.g., Ti(IV)iso-propoxide in an organic solvent. This transformation is usually carried out at temperatures of from 0°C to 100°C, preferably at 25°C, in an inert solvent. Suitable solvents are alcohols such as methanol (MeOH), ethanol (EtOH), n-propanol, isopropanol, n-butanol, and tert.-butanol or ethers such as diethylether, diisopropylether, tert.-butylmethylether (MTBE), dioxane, anisole, and tetrahydrofurane (THF), preferably MeOH or THF.

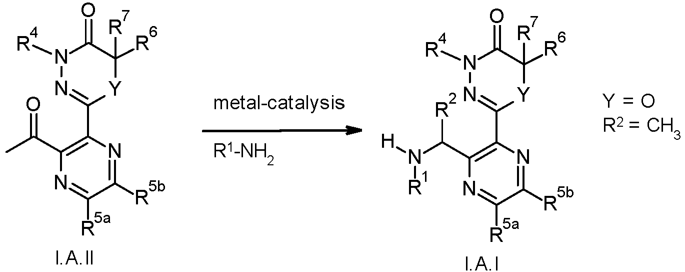

The methyl keton intermediate I.A.II with Y=O can be prepared from I.A.III via an Pd-catalyzed cross-coupling reaction, using, e.g., Pd(PPh₃)Cl₂ with a metal M¹ substituted alkoxy-enol regent (M¹ can be e.g. SnBu₃) to obtain the enol derivative intermediate. This transformation is usually carried out at temperatures of from 0°C to 150°C, preferably from 80°C to 110°C, in an inert solvent. Suitable solvents are aromatic hydrocarbons such as toluene, o-, m-, and p-xylene, preferably toluene. The reaction mixture can be worked up under acidic conditions to yield the methyl-ketone derivative I.A.II. Suitable acids and acidic catalysts are in general inorganic acids such as hydrofluoric acid, hydrochloric acid, hydrobromic acid, sulphuric acid und perchloric acid, preferably hydrochloric acid.

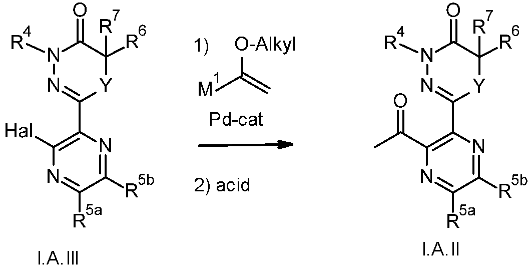

Compounds I.A.III with Y=O can be prepared via an intramolecular O-alkylation of the amide compound II with suitable leaving groups X". In formula II R¹, R², and R³ have the meaning as in formula I, and X" is a nucleophilic leaving group, such as a halide, preferably Br or Cl. The alkylation can be carried out under conditions known from literature.

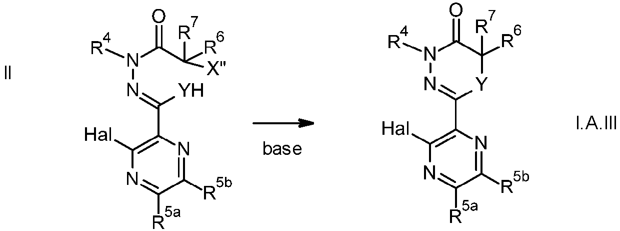

This transformation is usually carried out at temperatures of from 0°C to 100°C, preferably from 50°C to the boiling point of the solvent, in an inert solvent, in the presence of a base [cf. European Journal of Med. Chemistry 1988, Vol.23. p.441-451; WO2020157201].

Suitable solvents are alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably EtOH and DMF. It is also possible to use mixtures of the solvents mentioned. Suitable bases are, in general, alkali metal and alkaline earth metal carbonates, such as Li₂CO₃, Na₂CO₃, K₂CO₃, and CaCO₃, and also alkali metal bicarbonates, such as NaHCO₃. Particular preferences are given to NaHCO₃ and K₂CO₃. The bases are generally employed in equimolar amounts or in excess.

Compounds II can be obtained from Compounds III via an acylation reaction. Suitable acylating reagents Illa are acyl halides (preferably acyl chlorides) or anhydrides. In formula Illa Y" can be chloride or an alkoxy residue.

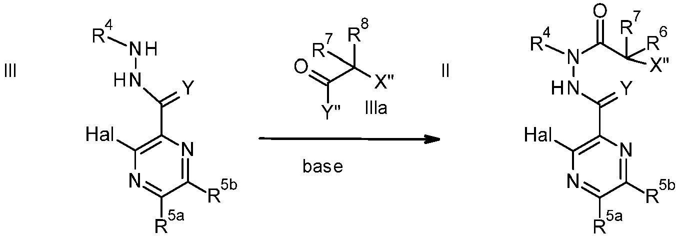

This transformation is usually carried out at temperatures of from 30°C to 50°C, preferably from 40°C to 45°C, in an inert solvent, in the presence of a base [WO2006045587, Journal für Praktische Chemie (Leipzig) (1985), 327(1), 109-16].

Suitable solvents are halogenated hydrocarbons such as methylene chloride, chloroform, and chlorobenzene, or ethers such as diethylether, diisopropylether, tert.-butylmethylether, dioxane, anisole, and tetrahydrofuran (THF), preferably methylene chloride or THF. Suitable bases are, in general, organic bases, for example tertiary amines, such as trimethylamine (TMA), triethylamine (TEA), diisopropylethylamine and N-methylpiperidine, pyridine, substituted pyridines, such as collidine, lutidine and 4-dimethylaminopyridine, and also bicyclic amines. Particular preference is given to TEA. The bases are generally employed in catalytic amounts; however, they can also be used in equimolar amounts, in excess or, if appropriate, as solvent. The starting materials are generally reacted with one another in equimolar amounts. In terms of yield, it may be advantageous to employ an excess of Illa, based on III.

Compounds III can be obtained from compounds IV via activation of the carbocyclic acid as acid chloride or via conventional amide coupling reagents.

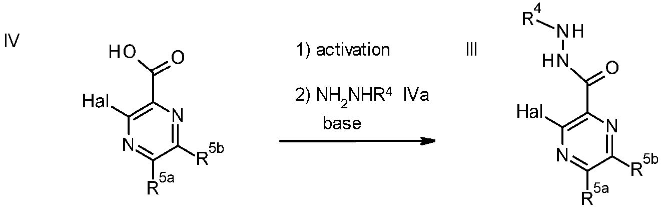

The activation is usually carried out at temperatures of from 0°C to 100°C, preferably from 0°C to 25°C, in DMF or methylene chloride, in the presence of SOCl₂ or oxalyl chloride [Journal of Catalysis (2023), 423, 19-25, Advanced Synthesis & Catalysis (2023), 365(1), 43-52].

The activation with amide coupling reagents is usually carried out at temperatures of from 0°C to 100°C, preferably at about 25°C, in DMF in the presence of 1-[bis(dimethylamin)methylen]-1H-1,2,3-triazol[4,5-b]pyridinium-3-oxid-hexafluorophosphate [WO2021163302].

The reaction with IVa is usually carried out at temperatures of from 0°C to 30°C, preferably at about 0°C in DMF, in the presence of a base [Organic Letters (2021), 23(3), 974-978].

Suitable solvents are halogenated hydrocarbons such as methylene chloride, chloroform, and chlorobenzene, or ethers such as diethylether, diisopropylether, tert.-butylmethylether, dioxane, anisole, and tetrahydrofuran (THF), preferably methylene chloride or THF. It is also possible to use mixtures of the solvents mentioned. Suitable bases are, in general, organic bases, for example tertiary amines, such as trimethylamine (TMA), triethylamine (TEA), diisopropylethylamine and N-methylpiperidine, pyridine, substituted pyridines, such as collidine, lutidine and 4-dimethylaminopyridine, and also bicyclic amines. Particular preferences are given to TEA and pyridine. The bases are generally employed in equimolar amounts, in excess or, if appropriate, as solvent. The starting materials are generally reacted with one another in equimolar amounts. In terms of yield, it may be advantageous to employ an excess of IVa, based on IV.

Alternatively, compounds IV can be converted directly to compounds II with a suitable, substituted acyl hydrazine Illb under similar reaction conditions as described above.

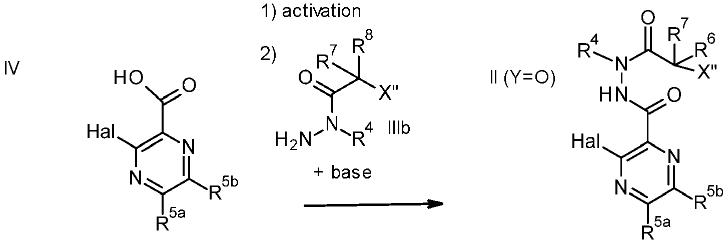

Compounds I.A with Y=S can be obtained via an acylation reaction and a subsequent intramolecular S-alkylation analogously to what is described for the O-derivative above. Therefore, the hydrazide carbonyl group in III is transformed to a thiocarbonyl group in compound V.

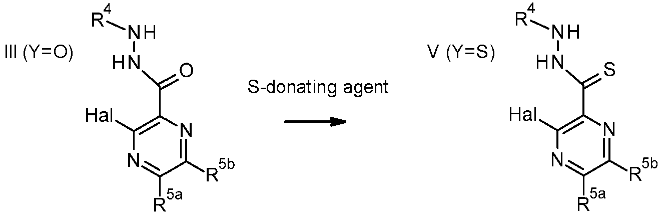

Compounds V can be obtained from compounds III via an O to S-exchange. This transformation is usually carried out at temperatures of from 45°C to 150°C, preferably from 80°C to 120°C, in an inert solvent, in the presence of sulfur-donating agents such as 2,4-bis(4-methoxyphenyl)-1,3,2,4-dithiadiphosphetan-2,4-disulfid, P₂S₅ [WO2005077124, Journal of Heterocyclic Chemistry

(1986), 23(2), 417-19]. Suitable solvents are aromatic hydrocarbons such as toluene, o-, m-, and p-xylene and ethers such as diethylether, diisopropylether, tert.-butylmethylether, dioxane, anisole, and THF. It is also possible to use mixtures of the solvents mentioned.

Alternatively, compounds VII can be converted directly to compounds V with an unsubstituted or substituted methyl 2-sulfanylacetate Va via previous in situ bromination of VI. The subsequent substitution reaction yields the cyclized product I.A (Y=S).

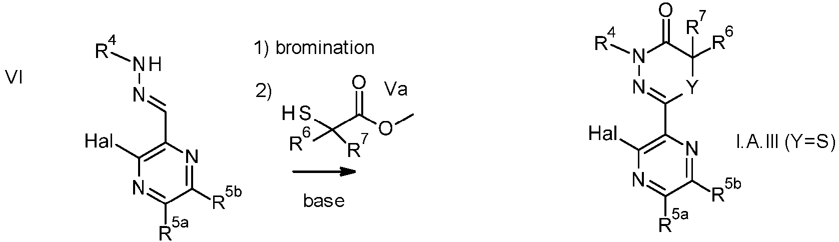

Compounds I.A with Y=N can be obtained via an in situ bromination of VI with subsequent substitution reactions to obtain the cyclized product I.A with Y=N. Optionally, established protecting group chemistry can be applied to improve the yield of the reaction sequence.

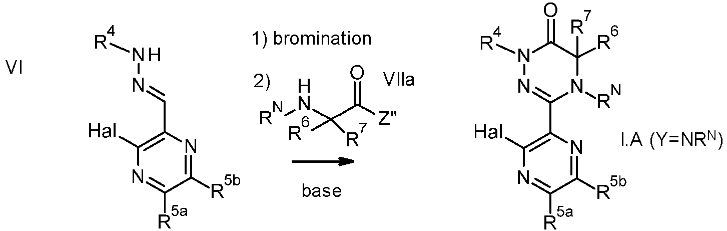

Compounds I.A (Y=NR^{N}) can be obtained from compounds VI via an in situ two step procedure. In the first step an alpha-bromination of the hydrazone derivative VI is conducted. This transformation is usually carried out at temperatures of from -78°C to 30°C, preferably from 0°C to 30°C, in an inert solvent, in the presence of bromination regents such as N-bromosuccinimde (NBS) or Br₂ in presence of an organic acid [WO2021222017; Chemische Berichte (1984), 117(3), 1194-214]. Suitable solvents are halogenated hydrocarbons such as methylene chloride, chloroform, and chlorobenzene, ethers such as diethylether, diisopropylether, tert.-butylmethylether, dioxane, anisole, and THF, moreover dimethyl sulphoxide (DMSO), dimethyl formamide (DMF), and dimethylacetamide (DMA), preferably THF and DMF. Suitable acids and acidic catalysts are in general organic acids such as formic acid, acetic acid, propionic acid, oxalic acid, toluene sulphonic acid, benzene sulphonic acid, camphor sulphonic acid, citric acid, and trifluoro acetic acid (TFA).

In the second step the cyclisation to obtain compound I.An via nucleophilic substitution is conducted. This transformation is usually carried out at temperatures of from 0°C to 30°C, in an inert solvent, in the presence of an alpha amino carbonyl derivative bearing R^{N} and a leaving group Z" such as an alkoxy residue or a halide and a base [Australian Journal of Chemistry (1996), 49(4), 463-468; Phosphorus, Sulfur and Silicon and the Related Elements (2011), 186(9), 1876-1884]. Suitable solvents are alcohols such as methanol (MeOH), ethanol (EtOH), n-propanol, isopropanol, n-butanol, and tert.-butanol, ethers such as diethylether, diisopropylether, tert.-butylmethylether, dioxane, anisole, and THF, preferably THF. Suitable bases are, in general, organic bases, e.g. tertiary amines, such as TMA, TEA, diisopropylethylamine and N-methylpiperidine, pyridine, substituted pyridines, such as collidine, lutidine and 4-dimethylaminopyridine, and also bicyclic amines. Particular preference is given to MeOH and TEA. It is also possible to use mixtures of the solvents mentioned. The bases are generally employed in equimolar amounts or in excess, if appropriate, as solvent.

Compounds VI can be obtained via a condensation reaction with a hydrazine derivative VIIIa.

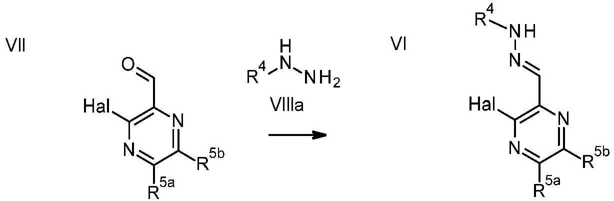

This transformation is usually carried out at temperatures of from 0°C to 80°C, preferably at about 30°C in an inert solvent, in the presence of a hydrazine derivative VIIIa. [Bioorganic & Medicinal Chemistry Letters (2006), 16(21), 5488-5492; Journal of Medicinal Chemistry (2015), 58(13), 5355-5360]. Suitable solvents are alcohols such as MeOH, EtOH, n-propanol, isopropanol, n-butanol, and tert.-butanol, preferably MeOH or EtOH. It is also possible to use mixtures of the solvents mentioned.

Compounds VII can be obtained from compounds VIII via ozonolysis.

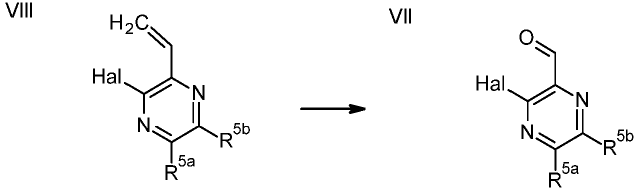

This transformation is usually carried out at temperatures of -78°C, in an inert solvent, in the presence of O₃ [Chemistry - A European Journal (2017), 23(57)]. Suitable solvents are alcohols such as MeOH, EtOH, n-propanol, isopropanol, n-butanol, and tert.-butanol, preferably MeOH. It is also possible to use mixtures of the solvents mentioned.

The starting materials required for preparing the compounds I are commercially available or can be prepared according to cited literature WO2021170881 and WO2023025602.

The halogenated bicyclic compounds e.g. BC1-Cl, BC2-Cl or BC3-Cl, which correspond to the Z moiety in the compound of formula I can be prepared via different synthetic routes, e.g., as described in the following.

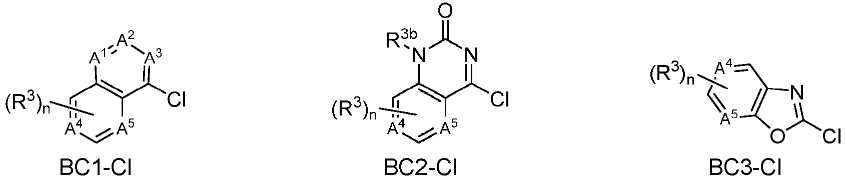

BC1-Cl and similar derivatives can be synthesized as described in, e.g., WO2024110554.

BC2-Cl and similar derivatives can be synthesized as described in, e.g., WO2021148639.

BC3-Cl and similar derivatives can be synthesized as described in, e.g., WO2022101265.

The starting materials required for preparing the compounds I are commercially available or known from literature [WO2023072849, WO2023104714, WO2025022007, WO2024110554, WO2021148639, WO2021110891 and WO2022101265.

The reaction mixtures are worked up in a customary manner, for example by mixing with water, extracting with an appropriate organic solvent, separating the phases and, if appropriate, chromatographic purification of the crude products. Some of the intermediates and end products are obtained in the form of colourless or slightly brownish viscous oils which are purified or freed from volatile components under reduced pressure and at moderately elevated temperature. If the intermediates and end products are obtained as solids, purification can also be carried out by recrystallization or digestion.

If individual compounds I cannot be obtained by the routes described above, they can be prepared by derivatization of other compounds I.

However, if the synthesis yields mixtures of isomers, a separation is generally not necessarily required since in some cases the individual isomers can be interconverted during work-up for use or during application (for example under the action of light, acids or bases). Such conversions may also take place after use, for example in the treatment of plants in the treated plant, or in the pest to be controlled.

The organic moieties groups mentioned in the above definitions of the variables are - like the term halogen - collective terms for individual listings of the individual group members. The prefix Cₙ-Cₘ indicates in each case the possible number of carbon atoms in the group.

The term "partially or fully substituted" by a radical means that in general the group is substituted with same or different radicals.

The term "halogen" denotes in each case fluorine, bromine, chlorine, or iodine, in particular fluorine, chlorine, or bromine.

The term "alkyl" as used herein and in the alkyl moieties of alkylamino, alkylcarbonyl, alkylthio, alkylsulfinyl, alkylsulfonyl and alkoxyalkyl denotes in each case a straight-chain or branched alkyl group having usually from 1 to 10 carbon atoms, frequently from 1 to 6 carbon atoms, preferably 1 to 4 carbon atoms, more preferably from 1 to 3 carbon atoms. Examples of an alkyl group are methyl (Me), ethyl (Et), n-propyl (n-Pr), iso-propyl, n-butyl, 2-butyl, iso-butyl, tert-butyl, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, and 1-ethyl-2-methylpropyl.

The term "haloalkyl" as used herein and in the haloalkyl moieties of haloalkylcarbonyl, haloalkoxycarbonyl, haloalkylthio, haloalkylsulfonyl, haloalkylsulfinyl, haloalkoxy and haloalkoxyalkyl, denotes in each case a straight-chain or branched alkyl group having usually from 1 to 10 carbon atoms, frequently from 1 to 6 carbon atoms, preferably from 1 to 4 carbon atoms, wherein the hydrogen atoms of this group are partially or totally replaced with halogen atoms. Preferred haloalkyl moieties are selected from C₁-C₄-haloalkyl, more preferably from C₁-C₃-haloalkyl or C₁-C₂-haloalkyl, in particular from C₁-C₂-fluoroalkyl such as fluoromethyl, difluoromethyl, trifluoromethyl, 1-fluoroethyl, 2-fluoroethyl, 2,2-difluoroethyl, 2,2,2-trifluoroethyl, pentafluoroethyl, and the like.

The term "alkoxy" as used herein denotes in each case a straight-chain or branched alkyl group which is bonded via an oxygen atom and has usually from 1 to 10 carbon atoms, frequently from 1 to 6 carbon atoms, preferably 1 to 4 carbon atoms. Examples of an alkoxy group are methoxy, ethoxy, n-propoxy, iso-propoxy, n-butyloxy, 2-butyloxy, iso-butyloxy, tert.-butyloxy, and the like.

The term "alkoxyalkyl" as used herein refers to alkyl usually comprising 1 to 10, frequently 1 to 4, preferably 1 to 2 carbon atoms, wherein 1 carbon atom carries an alkoxy radical usually comprising 1 to 4, preferably 1 or 2 carbon atoms as defined above. Examples are CH₂OCH₃, CH₂-OC₂H₅, 2-(methoxy)ethyl, and 2-(ethoxy)ethyl.

The term "haloalkoxy" as used herein denotes in each case a straight-chain or branched alkoxy group having from 1 to 10 carbon atoms, frequently from 1 to 6 carbon atoms, preferably 1 to 4 carbon atoms, wherein the hydrogen atoms of this group are partially or totally replaced with halogen atoms, in particular fluorine atoms. Preferred haloalkoxy moieties include C₁-C₄-haloalkoxy, in particular C₁-C₂-fluoroalkoxy, such as fluoromethoxy, difluoromethoxy, trifluoromethoxy, 1-fluoroethoxy, 2-fluoroethoxy, 2,2-difluoroethoxy, 2,2,2-trifluoroethoxy, 2-chloro-2-fluoroethoxy, 2-chloro-2,2-difluoro-ethoxy, 2,2dichloro-2-fluorethoxy, 2,2,2-trichloroethoxy, pentafluoroethoxy and the like.

The term "alkylthio "(alkylsulfanyl: S-alkyl)" as used herein refers to a straight-chain or branched saturated alkyl group having 1 to 10 carbon atoms, preferably 1 to 4 carbon atoms (= C₁-C₄-alkylthio), more preferably 1 to 3 carbon atoms, which is attached via a sulfur atom.

The term "haloalkylthio" as used herein refers to an alkylthio group as mentioned above wherein the hydrogen atoms are partially or fully substituted by fluorine, chlorine, bromine and/or iodine.

The term "alkylsulfinyl" (alkylsulfoxyl: S(=O)-alkyl), as used herein refers to a straight-chain or branched saturated alkyl group (as mentioned above) having 1 to 10 carbon atoms, preferably 1 to 4 carbon atoms (= C₁-C₄-alkylsulfinyl), more preferably 1 to 3 carbon atoms bonded through the sulfur atom of the sulfinyl group at any position in the alkyl group.

The term "haloalkylsulfinyl" as used herein refers to an alkylsulfinyl group as mentioned above wherein the hydrogen atoms are partially or fully substituted by fluorine, chlorine, bromine and/or iodine.

The term "alkylsulfonyl" (S(=O)₂-alkyl) as used herein refers to a straight-chain or branched saturated alkyl group having 1 to 10 carbon atoms, preferably 1 to 4 carbon atoms (= C₁-C₄-alkylsulfonyl), preferably 1 to 3 carbon atoms, which is bonded via the sulfur atom of the sulfonyl group at any position in the alkyl group.

The term "haloalkylsulfonyl" as used herein refers to an alkylsulfonyl group as mentioned above wherein the hydrogen atoms are partially or fully substituted by fluorine, chlorine, bromine and/or iodine.

The term "alkylcarbonyl" refers to an alkyl group as defined above, which is bonded via the carbon atom of a carbonyl group (C=O) to the remainder of the molecule.

The term "haloalkylcarbonyl" refers to an alkylcarbonyl group as mentioned above, wherein the hydrogen atoms are partially or fully substituted by fluorine, chlorine, bromine and/or iodine.

The term "alkoxycarbonyl" refers to an alkylcarbonyl group as defined above, which is bonded via an oxygen atom to the remainder of the molecule.

The term "haloalkoxycarbonyl" refers to an alkoxycarbonyl group as mentioned above, wherein the hydrogen atoms are partially or fully substituted by fluorine, chlorine, bromine and/or iodine.

The term "alkenyl" as used herein denotes in each case a singly unsaturated hydrocarbon radical having usually 2 to 10, frequently 2 to 6, preferably 2 to 4 carbon atoms, e.g. vinyl, allyl (2-propen-1-yl), 1-propen-1-yl, 2-propen-2-yl, methallyl (2-methylprop-2-en-1-yl), 2-buten-1-yl, 3-buten-1-yl, 2-penten-1-yl, 3-penten-1-yl, 4-penten-1-yl, 1-methylbut-2-en-1-yl, 2-ethylprop-2-en-1-yl and the like.

The term "haloalkenyl" as used herein refers to an alkenyl group as defined above, wherein the hydrogen atoms are partially or totally replaced with halogen atoms.

The term "alkynyl" as used herein denotes in each case a singly unsaturated hydrocarbon radical having usually 2 to 10, frequently 2 to 6, preferably 2 to 4 carbon atoms, e.g. ethynyl, propargyl (2-propyn-1-yl), 1-propyn-1-yl, 1-methylprop-2-yn-1-yl), 2-butyn-1-yl, 3-butyn-1-yl, 1-pentyn-1-yl, 3-pentyn-1-yl, 4-pentyn-1-yl, 1-methylbut-2-yn-1-yl, 1-ethylprop-2-yn-1-yl and the like.

The term "haloalkynyl" as used herein refers to an alkynyl group as defined above, wherein the hydrogen atoms are partially or totally replaced with halogen atoms.

The term "cycloalkyl" as used herein and in the cycloalkyl moieties of cycloalkoxy and cyclo-alkylthio denotes in each case a monocyclic cycloaliphatic radical having usually from 3 to 10 or from 3 to 6 carbon atoms, such as cyclopropyl (cC₃H₅), cyclobutyl (cC₄H₇), cyclopentyl (cC₅H₉), cyclohexyl (cC₆H₁₁), cycloheptyl, cyclooctyl, cyclononyl and cyclodecyl or cyclopropyl, cyclobutyl, cyclopentyl and cyclohexyl.

The term "cycloalkylalkyl" refers to a cycloalkyl group as defined above which is bonded via an alkylene group, such as a C₁-C₅-alkyl group or a C₁-C₄-alkyl group, in particular a methylene group CH₂ (=cycloalkylmethyl), to the remainder of the molecule.

The term "halocycloalkyl" as used herein and in the halocycloalkyl moieties of halocycloalkoxy and halocycloalkylthio denotes in each case a monocyclic cycloaliphatic radical having usually from 3 to 10 C atoms or 3 to 6 C atoms, wherein at least one, e.g. 1, 2, 3, 4 or 5 of the hydrogen atoms, are replaced by halogen, in particular by fluorine or chlorine. Examples are 1- and 2-fluorocyclopropyl, 1,2-, 2,2- and 2,3-difluorocyclopropyl, 1,2,2-trifluorocyclopropyl, 2,2,3,3-tetrafluorocyclpropyl, 1- and 2-chlorocyclopropyl, 1,2-, 2,2- and 2,3-dichlorocyclopropyl, 1,2,2-trichlorocyclopropyl, 2,2,3,3-tetrachlorocyclpropyl, 1-,2- and 3-fluorocyclopentyl, 1,2-, 2,2-, 2,3-, 3,3-, 3,4-, 2,5-difluorocyclopentyl, 1-,2- and 3-chlorocyclopentyl, 1,2-, 2,2-, 2,3-, 3,3-, 3,4-, 2,5-dichlorocyclopentyl and the like.

The term "halocycloalkenyl" as used herein and in the halocycloalkenyl moieties of halocycloalkenyloxy and halocycloalkenylthio denotes in each case a monocyclic singly unsaturated non-aromatic radical having usually from 3 to 10, e.g. 3 or 4 or from 5 to 10 carbon atoms, preferably from 3- to 8 carbon atoms, wherein at least one, e.g. 1, 2, 3, 4 or 5 of the hydrogen atoms, are replaced by halogen, in particular by fluorine or chlorine. Examples are 3,3-difluorocyclopropen-1-yl and 3,3-dichlorocyclopropen-1-yl.

The term "cycloalkenylalkyl" refers to a cycloalkenyl group as defined above which is bonded via an alkyl group, such as a C₁-C₅-alkyl group or a C₁-C₄-alkyl group, in particular a methylene group (= cycloalkenylmethyl), to the remainder of the molecule.

The term "carbocycle" or "carbocyclyl" includes in general a 3- to 12-membered, preferably a 3-to 8-membered or a 5- to 8-membered, more preferably a 5- or 6-membered mono-cyclic, non-aromatic ring comprising 3 to 12, preferably 3 to 8 or 5 to 8, more preferably 5 or 6 carbon atoms. Preferably, the term "carbocycle" covers cycloalkyl and cycloalkenyl groups as defined above.

The term "heterocycle" or "heterocyclyl" includes in general 3- to 12-membered, preferably 3-to 6-membered, in particular 6-membered monocyclic heterocyclic non-aromatic radicals. The heterocyclic non-aromatic radicals usually comprise 1, 2, 3, 4 or 5, preferably 1, 2 or 3 heteroatoms selected from N, O, and S, wherein S-atoms as ring members may be present as S, SO, or SO₂, and optionally one or two groups C(O) as ring members. Examples of 5- or 6-membered heterocyclic radicals comprise saturated or unsaturated, non-aromatic heterocyclic rings, such as oxiranyl, oxetanyl, thietanyl, thietanyl-S-oxid (S-oxothietanyl), thietanyl-S-dioxid (S-dioxothiethanyl), pyrrolidinyl, pyrrolinyl, pyrazolinyl, tetrahydrofuranyl, dihydrofuranyl, 1,3-dioxolanyl, thiolanyl, S-oxothiolanyl, S-dioxothiolanyl, dihydrothienyl, S-oxodihydrothienyl, S-dioxodihydrothienyl, oxazolidinyl, oxazolinyl, thiazolinyl, oxathiolanyl, piperidinyl, piperazinyl, pyranyl, dihydropyranyl, tetrahydropyranyl, 1,3- and 1,4-dioxanyl, thiopyranyl, S.oxothiopyranyl, S-dioxothiopyranyl, dihydrothiopyranyl, S-oxodihydrothiopyranyl, S-dioxodihydrothiopyranyl, tetrahydrothiopyranyl, S-oxotetrahydrothiopyranyl, S-dioxotetrahydrothiopyranyl, morpholinyl, thiomorpholinyl, S-oxothiomorpholinyl, S-dioxothiomorpholinyl, thiazinyl and the like. Examples for heterocyclic ring also comprising 1 or 2 carbonyl groups as ring members comprise pyrrolidin-2-onyl, pyrrolidin-2,5-dionyl, imidazolidin-2-onyl, oxazolidin-2-onyl, thiazolidin-2-onyl, and the like.

The term "hetaryl" includes monocyclic 5- or 6-membered heteroaromatic radicals comprising as ring members 1, 2, 3 or 4 heteroatoms selected from N, O, and S. Examples of 5- or 6-membered heteroaromatic radicals include pyridyl, i.e. 2-, 3-, or 4-pyridyl, pyrimidinyl, i.e. 2-, 4- or 5-pyrimidinyl, pyrazinyl, pyridazinyl, i.e. 3- or 4-pyridazinyl, thienyl, i.e. 2- or 3-thienyl, furyl, i.e. 2-or 3-furyl, pyrrolyl, i.e. 2- or 3-pyrrolyl, oxazolyl, i.e. 2-, 3- or 5-oxazolyl, isoxazolyl, i.e. 3-, 4- or 5-isoxazolyl, thiazolyl, i.e. 2-, 3- or 5-thiazolyl, isothiazolyl, i.e. 3-, 4- or 5-isothiazolyl, pyrazolyl, i.e. 1-, 3-, 4- or 5-pyrazolyl, i.e. 1-, 2-, 4- or 5-imidazolyl, oxadiazolyl, e.g. 2- or 5-[1,3,4]oxadiazolyl, 4- or 5-(1,2,3-oxadiazol)yl, 3- or 5-(1,2,4-oxadiazol)yl, 2- or 5-(1,3,4-thiadiazol)yl, thiadiazolyl, e.g. 2- or 5-(1,3,4-thiadiazol)yl, 4- or 5-(1,2,3-thiadiazol)yl, 3- or 5-(1,2,4-thiadiazol)yl, triazolyl, e.g. 1H-, 2H- or 3H-1,2,3-triazol-4-yl, 2H-triazol-3-yl, 1H-, 2H-, or 4H-1,2,4-triazolyl and tetrazolyl, i.e. 1H- or 2H-tetrazolyl. The term "hetaryl" also includes bicyclic 8 to 10-membered heteroaromatic radicals comprising as ring members 1, 2 or 3 heteroatoms selected from N, O, and S, wherein a 5- or 6-membered heteroaromatic ring is fused to a phenyl ring or to a 5- or 6-membered heteroaromatic radical. Examples of a 5- or 6-membered heteroaromatic ring fused to a phenyl ring or to a 5- or 6-membered heteroaromatic radical include benzofuranyl, benzothienyl, indolyl, indazolyl, benzimidazolyl, benzoxathiazolyl, benzoxadiazolyl, benzothiadiazolyl, benzoxazinyl, chinolinyl, isochinolinyl, purinyl, 1,8-naphthyridyl, pteridyl, pyrido[3,2-d]pyrimidyl or pyridoimidazolyl and the like. These fused hetaryl radicals may be bonded to the remainder of the molecule via any ring atom of 5- or 6-membered heteroaromatic ring or via a carbon atom of the fused phenyl moiety.

The terms "heterocyclylalkyl" and "hetarylalkyl" refer to heterocyclyl or hetaryl, respectively, as defined above which are bonded via a C₁-C₅-alkyl group or a C₁-C₄-alkyl group, in particular a methylene group (= heterocyclylmethyl or hetarylmethyl, resp.), to the remainder of the molecule.

The term "arylalkyl" and "phenylalkyl" refer to aryl as defined above and phenyl, respectively, which are bonded via C₁-C₅-alkyl group or a C₁-C₄-alkyl group, in particular a methyl group (= arylmethyl or phenylmethyl), to the remainder of the molecule, examples including benzyl, 1-phenylethyl, 2-phenylethyl, 2-phenoxyethyl etc.

The terms "alkylene", "cycloalkylene", "heterocycloalkylene", "alkenylene", "cycloalkenylene", "heterocycloalkenylene" and "alkynylene" refer to alkyl, cycloalkyl, heterocycloalkyl, alkenyl, cycloalkenyl, heterocycloalkenyl and alkynyl as defined above, resp., which are bonded to the remainder of the molecule, via two atoms, preferably via two carbon atoms, of the respective group, so that they represent a linker between two moieties of the molecule.

In a particular embodiment, the variables of the compounds of the formula I have the following meanings, these meanings, both on their own and in combination with one another, being particular embodiments of the compounds of the formula I.

Embodiments and preferred compounds of the invention for use in pesticidal methods and for insecticidal application purposes are outlined in the following paragraphs.

With respect to the variables, the particularly preferred embodiments of the intermediates correspond to those of the compounds of the formula I.

In a preferred embodiment, the compounds I are present in form of a mixture of compounds I.S and I.R, wherein compound I.S with S-configuration of the carbon atom neighboring the nitrogen is present in an amount of more than 50% by weight, in particular of at least 70% by weight, more particularly of at least 85% by weight, more particularly of at least 90% by weight, more particularly of at least 95% by weight, specifically of at least 99% by weight, based on the total weight of compounds I.S and I.R. Compounds of formula I.S are a particularly preferred embodiment of the invention.

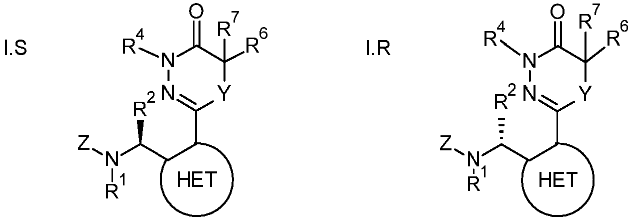

In one particularly preferred embodiment of the invention, the method comprises the step of contacting the plant, parts of it, its propagation material, the pests, their food supply, habitat or breeding grounds with a pesticidally effective amount of a compound of formula I.S.

R¹ is H, OR¹⁰, NR¹²R¹³, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₃-haloalkyl, C₁-C₆-alkoxy, C₁-C₃-haloalkoxy, C₂-C₆-alkenyl, C₂-C₆-alkynyl, C₁-C₆-alkoxy-C₁-C₆-alkyl, C₁-C₆-alkyl-C₁-C₆-alkoxy, C₁-C₆-alkoxy-C₃-C₆-cycloalkyl, C₃-C₆-cycloalkyl-C₁-C₆-alkoxy, C₂-C₆-alkenyl-C₃-C₆-cycloalkyl, C₂-C₆-alkynyl-C₃-C₆-cycloalkyl, C₃-C₆-cycloalkyl-C₂-C₆-alkenyl, C₃-C₆-cycloalkyl-C₂-C₆-alkynyl,, C(=N-R¹³)R¹², or C(O)R^{11a}. In these groups, alkyl, alkoxy, alkenyl, alkynyl, and cycloalkyl moieties are unsubstituted or substituted with R¹¹. In various embodiments, R¹ is H, C₁-C₆-alkyl, for example methyl, or C₁-C₆-alkyl-C₃-C₆-cycloalkyl-, for example CH₂-cyclopropyl.

In various embodiments, R¹ is H, OH, NR¹²R¹³; C₁-C₆-alkyl, C₃-C₆-cycloalkyl, C₁-C₅-alkoxy, C₃-C₆-cycloalkyl-C₁-C₄-alkyl, C₁-C₄-alkyl-C₃-C₆-cycloalkyl, C₃-C₆-halocycloalkyl-C₁-C₄-alkyl, C₂-C₆-alkenyl, C₂-C₆-alkynyl, which groups are unsubstituted, or partially or fully substituted with R¹¹; or C(O)R^{11a}, preferably C₁-C₆-alkyl, C₁-C₆-haloalkyl, C₃-C₆-cycloalkyl, C₃-C₆-halocycloalkyl, C₃-C₆-cycloalkyl-C₁-C₄-alkyl, or C₁-C₄-alkyl-C₃-C₆-cycloalkyl; more preferably H, CH₃, CH₂-CH₃, or CH₂C≡CH, CH₂-O-CH₃, CH(CH₃)₂, or CH₂-cC₃H₅.

In various embodiments, R¹ is H, C₁-C₃-alkyl, C₂-C₆-alkenyl, C₂-C₆-alkynyl, C₃-C₆-cycloalkyl, or C₃-C₆-cycloalkyl-C₁-C₄-alkyl, preferably H, CH₃, C₂H₅, CH₂cC₃H₅, CH₂CH=CH₂, or CH₂C≡CH, more preferably H, CH₃ or CH₂cC₃H₅. Particularly for compounds I.A is R¹ preferably H, C₁-C₃-alkyl, C₂-C₆-alkenyl, C₂-C₆-alkynyl, C₃-C₆-cycloalkyl, or C₃-C₆-cycloalkyl-C₁-C₄-alkyl, more preferably H, CH₃, C₂H₅, CH₂-O-CH₃, CH₂cC₃H₅, particularly H, CH₃, or CH₂C≡CH, or CH₂cC₃H₅.

In various embodiments, R¹ is H, C₁-C₆-alkyl, C₁-C₆-haloalkyl, C₃-C₆-cycloalkyl, C₃-C₆-halocycloalkyl, C₃-C₆-cycloalkyl-C₁-C₄-alkyl, C₁-C₄-alkyl-C₃-C₆-cycloalkyl, C₂-C₆-alkenyl, C₂-C₆-alkynyl, or C₁-C₆-alkyl-C₁-C₆-alkoxy.

In various embodiments, R² is H or C₁-C₃-alkyl, preferably CH₃.

In various embodiments, Z is selected form BC1, BC2 or BC3, wherein A¹, A², A³, A⁴ and A⁵ are independently selected from N, CH or CR³; n is 0, 1, 2, 3 or 4; and R^{3b} is H, C₁-C₃-alkyl, C₁-C₃-haloalkyl.

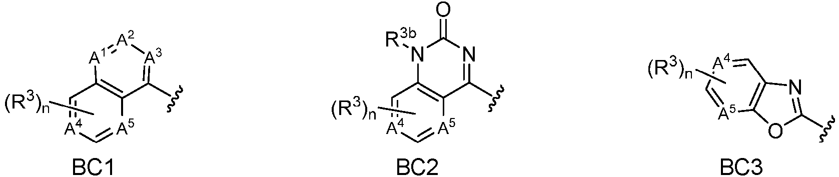

The wavy line denotes the bond to the remainder of the molecule.

In various embodiments, Z is selected form BC1a, BC2a, BC3a or BC3b.

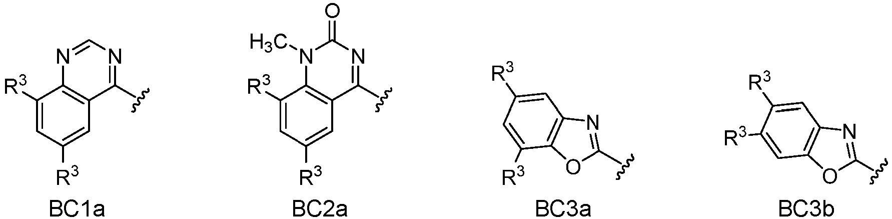

The wavy line denotes the bond to the remainder of the molecule.

In various embodiments, Z is selected form

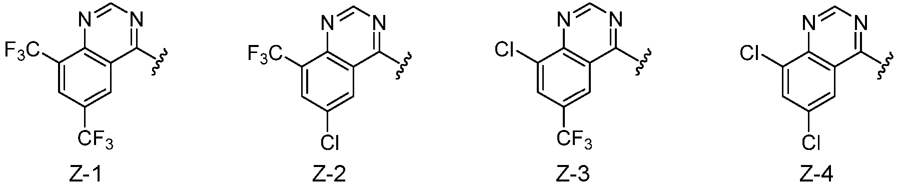

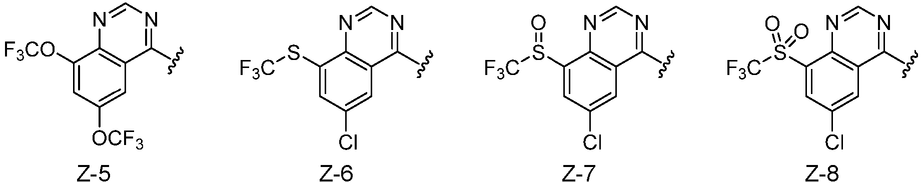

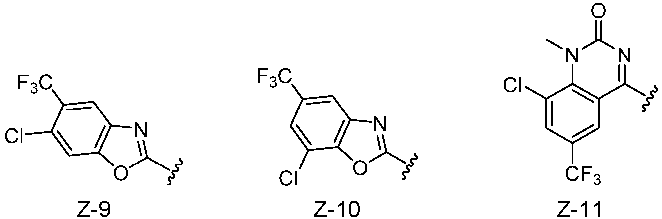

The wavy line denotes the bond to the remainder of the molecule.

In various embodiments, n is 2.

In various embodiments, each R³ is independently selected from halogen, CN, C₁-C₄-alkyl, wherein alkyl is unsubstituted or substituted with one or more CN, C₁-C₄-haloalkyl, C₁-C₄-haloalkoxy, C₃-C₄-cycloalkyl, wherein cycloalkyl is unsubstituted or substituted with one or more CN or halogen, S(O)ₘ-C₁-C₄-alkyl, S(O)ₘ-C₁-C₄-haloalkyl, OS(O)ₘ-C₁-C₄-alkyl, OS(O)ₘ-C₁-C₄-haloalkyl, S(O)ₘ-phenyl, wherein phenyl is unsubstituted or substituted with one or more halogen.

In various embodiments, R⁴ is H, C₁-C₃-alkyl, C₂-C₄-alkenyl, C₂-C₄-alkynyl, C₃-C₆-cycloalkyl-C₁-C₂-alkyl, C₁-C₂-alkyl-C₃-C₆-cycloalky, C₃-C₆-cycloalkyl, which are unsubstituted or partially or fully substituted with halogen, CN, or C(O)NH₂; preferably R⁴ is CH₃, CH₂CH₃, CH(CH₃)₂, CH₂CH₂CH₃, cC₃H₅, CH₂CN, CH₂C≡CH or CH₂OCH₃, CHF₂, CH₂CF₃, 1-CN-cC₃H₄, CH₂cC₃H₅, C(H)=CH₂, CH₂CH₂CN, more preferably H, CH₃ or CH₂C≡CH .

In various embodiments, R^{5a}, R^{5b} and R^{5c} are independently selected from H, halogen, C₁-C₄-alkyl, or C₃-C₆-cycloalkyl, preferably H, F, Cl, CH₃, or cC₃H₅, more preferably H or cC₃H₅. preferred.

In various embodiments, R^{5a}, R^{5b} and R^{5c} are H.

In various embodiments, R⁶ and R⁷ are independently from each other H, CH₃, CH₂CH₃, CH(CH₃)₂ or CH₂CH₂CH₃, preferably H or CH₃. In various embodiment, R⁶ and R⁷ are both H; or R⁶ is H and R⁷ is CH₃; or R⁶ is CH₃ and R⁷ is CH₃.

In various embodiments, R⁶ and R⁷ form together with the atom to which they are bound a carbocyclic ring, preferably a cyclopropyl ring or cyclobutyl ring.

In various embodiments, Y is S or N-R^{N}, when HET is HB; and Y is O, S, or N-R^{N}, when HET is HA. In various embodiments, Y is S, when HET is HB; and Y is O or S, preferably O, when HET is HA.

In various embodiments, HET is HB; and Y is S or N-R^{N}; preferably Y is S.

In various embodiments, HET is HA; and Y is O, S, or N-R^{N}; preferably Y is S or O; further preferably Y is O.

In various embodiments, the compound of formula I is selected from 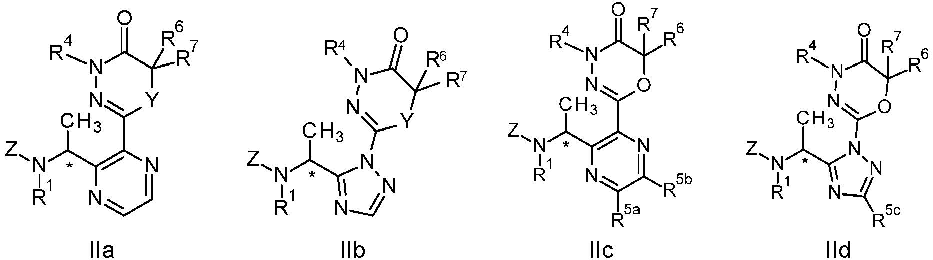 or the N-oxides, stereoisomers, tautomers, and agriculturally or veterinarily acceptable salts thereof.

In various embodiments, the compound of formula I is selected from the compounds of Table I or the N-oxides, stereoisomers, tautomers, and agriculturally or veterinarily acceptable salts thereof.

In various embodiments, the compound of formula I is selected from the compounds of Table A:

**Table A**

| No. | Formula | R¹ | Z | R⁴ | R⁶ | R⁷ | Y |
|---|---|---|---|---|---|---|---|
| A-1 | Ila | H | Z-2 | CH₃ | H | H | O |
| A-2 | Ila | H | Z-3 | CH₃ | H | H | O |
| A-3 | Ila | H | Z-10 | CH₃ | H | H | O |
| A-4 | Ila | H | Z-9 | CH₃ | H | H | O |
| A-5 | Ila | H | Z-1 | CH₃ | H | H | O |
| A-6 | Ila | H | Z-8 | CH₃ | H | H | O |
| A-7 | Ila | H | Z-6 | CH₃ | H | H | O |
| A-8 | Ila | H | Z-7 | CH₃ | H | H | O |
| A-9 | Ila | H | Z-5 | CH₃ | H | H | O |
| A-10 | Ila | H | Z-4 | CH₃ | H | H | O |
| A-11 | Ila | H | Z-1 | H | H | H | O |
| A-12 | Ila | H | Z-11 | CH₃ | H | H | O |
| A-13 | Ila | H | Z-1 | H | CH₃ | CH₃ | O |
| A-14 | Ila | H | Z-1 | CH₃ | CH₃ | CH₃ | O |
| A-15 | Ila | H | Z-1 | H | CH₂-CH₂ | | O |

or the N-oxides, stereoisomers, tautomers, and agriculturally or veterinarily acceptable salts thereof.

In various embodiments, the invention relates to compounds of Table I.

As used herein, the term "compound(s) of the invention" or "compound(s) according to the invention" refers to the compound(s) of formula (I) as defined above, which are also referred to as "compound(s) of formula I" or "compound(s) I" or "formula I compound(s)", and includes their salts, tautomers, stereoisomers, and N-oxides.

### Mixtures

The invention also relates to a mixture of at least one compound of the invention with at least one mixing partner. Preferred are binary mixtures of one compound of the invention as component I with one mixing partner herein as component II. Preferred weight ratios for such binary mixtures are from 5000:1 to 1:5000, preferably from 1000:1 to 1:1000, more preferably from 100:1 to 1:100, particularly from 10:1 to 1:10. In such binary mixtures, components I and II may be used in equal amounts, or an excess of component I, or an excess of component II may be used.

Mixing partners can be selected from pesticides, in particular insecticides, nematicides, and acaricides, fungicides, herbicides, plant growth regulators, fertilizers. Preferred mixing partners are insecticides, nematicides, and fungicides.

The following list M of pesticides, grouped according to the Mode of Action Classification of the Insecticide Resistance Action Committee (IRAC), together with which the compounds of the invention can be used and with which potential synergistic effects might be produced, illustrates the possible combinations:
M.1 AChE inhibitors: aldicarb, alanycarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, trimethacarb, XMC, xylylcarb, triazamate; acephate, azamethiphos, azinphos-ethyl, azinphosmethyl, cadusafos, chlorethoxyfos, chlorfenvinphos, chlormephos, chlorpyrifos, chlorpyrifosmethyl, coumaphos, cyanophos, demeton-S-methyl, diazinon, dichlorvos/ DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O-(methoxyaminothio-phosphoryl) salicylate, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion;
M.2. GABA-gated chloride channel antagonists: cyclodiene organochlorine compounds: endosulfan, chlordane; phenylpyrazoles: ethiprole, fipronil, flufiprole, pyrafluprole, pyriprole;
M.3 Sodium channel modulators: pyrethroids: acrinathrin, allethrin, d-cis-trans allethrin, d-trans allethrin, bifenthrin, kappa-bifenthrin, bioallethrin, bioallethrin S-cylclopentenyl, bio-resmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin, deltamethrin, empenthrin, esfenvalerate, etofenprox, fenpropathrin, fenvalerate, flucythrinate, flumethrin, tau-fluvalinate, halfenprox, heptafluthrin, imiprothrin, meperfluthrin,metofluthrin, momfluorothrin, epsilon-momfluorothrin, permethrin, phenothrin, prallethrin, profluthrin, pyrethrin (pyrethrum), resmethrin, silafluofen, tefluthrin, kappa-tefluthrin, tetramethylfluthrin, tetramethrin, tralomethrin, transfluthrin; sodium channel modulators, e.g.: DDT, methoxychlor;
M.4 nAChR agonists: neonicotinoids: acetamiprid, clothianidin, cycloxaprid, dinotefuran, imidacloprid, nitenpyram, thiacloprid, thiamethoxam; 4,5-dihydro-N-nitro-1-(2-oxiranylmethyl)-1H-imidazol-2-amine, (2E-)-1-[(6-Chloropyridin-3-yl)methyl]-N'-nitro-2-pentylidenehydrazinecarboximidamide; 1-[(6-Chloropyridin-3-yl)methyl]-7-methyl-8-nitro-5-propoxy-1,2,3,5,6,7-hexahydroimidazo[1,2-a]pyridine; nicotine; sulfoxaflor; flupyradifurone; triflumezopyrim, fenmezoditiaz, flupyrimin, 1-[(2-chlorothiazol-5-yl)methyl]-3-(3,5-dimethylisoxazol-4-yl)pyrido[1,2-a]pyrimidine-2,4-dione;
M.5 Nicotinic acetylcholine receptor allosteric activators:spinosyns, e.g. spinosad or spineto-ram;
M.6 Chloride channel activators from the class of avermectins and milbemycins, e.g. abamectin, emamectin benzoate, ivermectin, lepimectin, or milbemectin;
M.7 Juvenile hormone mimics, such as hydroprene, kino-prene, methoprene; fenoxycarb, or pyriproxyfen;
M.8 miscellaneous multi-site inhibitors: CH₃Br, other alkyl halides, chloropicrin, sulfuryl fluoride, borax, tartar emetic;
M.9 Chordotonal organ TRPV channel modulators: afidopyropen, pymetrozine; pyrifluquinazon;
M.10 Mite growth inhibitors: clofentezine, hexythiazox, diflovidazin, etoxazole;
M.11 Microbial disruptors of insect midgut membranes: *bacillus thuringiensis*, *bacillus sphaericus*, and insecticdal proteins they produce e.g.: *bacillus thuringiensis* subsp. *israelensis*, *bacillus sphaericus*, *bacillus thuringiensis* subsp. *aizawai*, *bacillus thuringiensis* subsp. *kurstaki*, *bacillus thuringiensis* subsp. *tenebrionis*, Bt crop proteins: Cry1Ab, Cry1Ac, Cry1Fa, Cry2Ab, mCry3A, Cry3Ab, Cry3Bb, Cry34/35Ab1;
M.12 Inhibitors of mitochondrial ATP synthase: diafenthiuron, organotin miticides, e.g.: azocyclotin, cyhexatin, fenbutatin oxide, propargite, tetradifon;
M.13 Uncouplers of oxidative phosphorylation via disruption of the proton gradient: chlorfenapyr, DNOC, sulfluramid;
M.14 nAChR channel blockers: nereistoxin analogues bensultap, cartap hydrochloride, thiocyclam, thiosultap-sodium;
M.15 Inhibitors of the chitin biosynthesis type 0, e.g.: bistrifluron, chlorfluazuron, difluben-zuron, flucycloxuron, flufenoxuron, hexaflumuron, lufenuron, novaluron, noviflumuron, teflubenzuron, triflumuron;
M.16 Inhibitors of the chitin biosynthesis type 1: buprofezin;
M.17 Moulting disruptors: Dipteran, cyromazine;
M.18 Ecdyson receptor agonists, e.g.: methoxyfenozide, tebufenozide, halofenozide, fufeno-zide, chromafenozide;
M.19 Octopamin receptor agonists: amitraz;
M.20 Mitochondrial complex III electron transport inhibitors: hydramethylnon, acequinocyl, fluacrypyrim; bifenazate;
M.21 METI acaricides and insecticides, e.g.: fenazaquin, fenpyroximate, pyrimidifen, pyrida-ben, tebufenpyrad, tolfenpyrad, rotenone;
M.22 Voltage-dependent sodium channel blockers: indoxacarb, metaflumizone, N-(3-chloro-2-methyl-phenyl)-2-[(4-chlorophenyl)[4-[methyl(methylsulfonyl)amino]phenyl]-methylene]-hydrazinecarboxamide, N-[4-chloro-2-[[(1,1-dimethylethyl)amino]carbonyl]-6-methylphenyl]-1-(3-chloro-2-pyridinyl)-3-(fluoromethoxy)-1H-pyrazole-5-carboxamide, 2-[2-(4-cyanophenyl)-1-[3-(trifluoromethyl)phenyl]ethylidene]-N-[4-(difluoromethoxy)phenyl]-hydrazinecarboxamide;
M.23 Inhibitors of the of acetyl CoA carboxylase, e.g.: spirodiclofen, spiromesifen, spirotetramat; spiropidion; spirobudifen, 11-(4-chloro-2,6-dimethylphenyl)-12-hydroxy-1,4-dioxa-9-azadispiro[4.2.4.2]tetradec-11-en-10-one, spidoxamat;
M.24 Mitochondrial complex IV electron transport inhibitors: e.g. aluminium phosphide, calcium phosphide, zinc phosphide, cyanide;
M.25 Mitochondrial complex II electron transport inhibitors, e.g.: cyenopyrafen, cyflumetofen, cyetpyrafen, pyflubumide;
M.28 Ryanodine receptor-modulators: chlorantraniliprole, cyantraniliprole, cyclaniliprole, flubendiamide, fluchlordiniliprole, (R)-3-chloro-N1-{2-methyl-4-[1,2,2,2-tetrafluoro-1-(trifluoromethyl)ethyl]phenyl}-N2-(1-methyl-2-methylsulfonylethyl)phthalamid, (S)-3-chloro-N1-{2-methyl-4-[1,2,2,2-tetrafluoro-1-(trifluoromethyl)ethyl]phenyl}-N2-(1-methyl-2-methylsulfonylethyl)phthalamide, methyl-2-[3,5-dibromo-2-({[3-bromo-1-(3-chlorpyridin-2-yl)-1H-pyrazol-5-yl]carbonyl}amino)benzoyl]-1,2-dimethylhydrazine-carboxylate; N-[2-(5-amino-1,3,4-thiadiazol-2-yl)-4-chloro-6-methyl-phenyl]-3-bromo-1-(3-chloro-2-pyridinyl)-1H-pyrazole-5-carboxamide; 3-chloro-1-(3-chloro-2-pyridinyl)-N-[2,4-dichloro-6-[[(1-cyano-1-methylethyl)amino]carbonyl]phenyl]-1H-pyrazole-5-carboxamide; tetrachlorantraniliprole; tetraniliprole; tiorantraniliprole; N-[4-chloro-2-[[(1,1-dimethylethyl)amino]carbonyl]-6-methyl-phenyl]-1-(3-chloro-2-pyridinyl)-3-(fluoromethoxy)-1H-pyrazole-5-carboxamide; cyhalodiamide; N-[2-(5-amino-1,3,4-thiadiazol-2-yl)-4-chloro-6-methylphenyl]-3-bromo-1-(3-chloro-2-pyridinyl)-1H-pyrazole-5-carboxamide, pioxaniliprole;
M.29: Chordotonal organ Modulators: flonicamid, flumetnicam;
M.30: broflanilide; fluxametamide, isocycloseram, piperflanilide;
M.33 acynonapyr;
M.UN. Unknown mode of action: afoxolaner, azadirachtin, amidoflumet, ben-zoximate, bromopropylate, chinomethionat, cryolite, cyproflanilid, dicloromezotiaz, dicofol, dimpropyridaz, flufenerim, flometoquin, fluensulfone, fluhexafon, fluopyram, fluralaner, metaldehyde, metoxadiazone, mivorilaner, modoflaner, piperonyl butoxide, pyridalyl, tioxazafen, trifluenfuronate, umifoxolaner, 11-(4-chloro-2,6-dimethylphenyl)-12-hydroxy-1,4-dioxa-9-azadispiro[4.2.4.2]-tetradec-11-en-10-one, 3-(4'-fluoro-2,4-dimethylbiphenyl-3-yl)-4-hydroxy-8-oxa-1-azaspiro[4.5]dec-3-en-2-one, 1-[2-fluoro-4-methyl-5-[(2,2,2-trifluoroethyl)sulfinyl]phenyl]-3-(trifluoromethyl)-1H-1,2,4-triazole-5-amine, actives on basis of *bacillus firmus* (Votivo, I-1582); fluazaindolizine; N-[5-[[2-bromo-6-chloro-4-[1,2,2,3,3,3-hexafluoro-1-(trifluoromethyl)-propyl]phenyl]carbamoyl]-2-cyano-phenyl]-4-cyano-2-methyl-benzamide; 4-cyano-N-[2-cyano-5-[[2,6-dichloro-4-[1,2,2,3,3,3-hexafluoro-1-(trifluoromethyl)-propyl]phenyl]carbamoyl]phenyl]-2-methyl-benzamide; 4-cyano-N-[2-cyano-5-[[2,6-dichloro-4-[1,2,2,2-tetrafluoro-1-(trifluoromethyl)ethyl]phenyl]carbamoyl]phenyl]-2-methyl-benzamide; N-[5-[[2-bromo-6-chloro-4-[1,2,2,2-tetrafluoro-1-(trifluoromethyl)ethyl]phenyl]carbamoyl]-2-cyano-phenyl]-4-cyano-2-methyl-benzamide;
1-[(6-chloro-3-pyridinyl)methyl]-1,2,3,5,6,7-hexahydro-5-methoxy-7-methyl-8-nitro-imidazo[1,2-a]pyridine; 1-[(6-chloropyridin-3-yl)methyl]-7-methyl-8-nitro-1,2,3,5,6,7-hexahydroimidazo[1,2-a]pyridin-5-ol; 1-[(6-chloro-3-pyridinyl)methyl]-1,2,3,5,6,7-hexahydro-5-methoxy-7-methyl-8-nitro-imidazo[1,2-a]pyridine; 2-(3-pyridinyl)-N-(2-pyrimidinylmethyl )-2H-indazole-5-carboxamide; tyclopyrazoflor; sarolaner, lotilaner; N-[4-chloro-3-[[(phenylmethyl)amino]carbonyl]phenyl]-1-methyl-3-(1,1,2,2,2-pentafluoroethyl)-4-(trifluoromethyl)-1H-pyrazole-5-carboxamide; N-[4-chloro-3-[[(phenylmethyl)amino]carbonyl]phenyl]-1-methyl-3-(1,1,2,2,2-pentafluoroethyl)-4-(trifluoromethyl)-1H-pyrazole-5-carboxamide; 2-(3-ethylsulfonyl-2-pyridyl)-3-methyl-6-(trifluoromethyl)imidazo[4,5-b]pyridine, 2-[3-ethylsulfonyl-5-(trifluoromethyl)-2-pyridyl]-3-methyl-6-(trifluoromethyl)imidazo[4,5-b]pyridine; N-[4-chloro-3-(cyclopropylcarbamoyl)phenyl]-2-methyl-5-(1,1,2,2,2-pentafluoroethyl)-4-(trifluoromethyl)pyrazole-3-carboxamide, N-[4-chloro-3-[(1-cyanocyclopropyl)carbamoyl]phenyl]-2-methyl-5-(1,1,2,2,2-pentafluoroethyl)-4-(trifluoromethyl)pyrazole-3-carboxamide; benzpyrimoxan; tigolaner; oxazosulfyl; [(2S,3R,4R,5S,6S)-3,5-dimethoxy-6-methyl-4-propoxy-tetrahydropyran-2-yl] N-[4-[1-[4-(trifluoromethoxy)phenyl]-1,2,4-triazol-3-yl]phenyl]carbamate; [(2S,3R,4R,5S,6S)-3,4,5-trimethoxy-6-methyl-tetrahydropyran-2-yl] N-[4-[1-[4-(trifluoromethoxy)phenyl]-1,2,4-triazol-3-yl]phenyl]carbamate; [(2S,3R,4R,5S,6S)-3,5-dimethoxy-6-methyl-4-propoxy-tetrahydropyran-2-yl] N-[4-[1-[4-(1,1,2,2,2-pentafluoroethoxy)phenyl]-1,2,4-triazol-3-yl]phenyl]carbamate; [(2S,3R,4R,5S,6S)-3,4,5-trimethoxy-6-methyl-tetrahydropyran-2-yl] N-[4-[1-[4-(1,1,2,2,2-pentafluoroethoxy)phenyl]-1,2,4-triazol-3-yl]phenyl]carbamate; (2Z)-3-(2-isopropylphenyl)-2-[(E)-[4-[1-[4-(trifluoromethoxy)phenyl]-1,2,4-triazol-3-yl]phenyl]methylenehydrazono]thiazolidin-4-one, (2Z)-3-(2-isopropylphenyl)-2-[(E)-[4-[1-[4-(1,1,2,2,2-pentafluoroethoxy)phenyl]-1,2,4-triazol-3-yl]phenyl]methylenehydrazono]thiazolidin-4-one, (2Z)-3-(2-isopropylphenyl)-2-[(E)-[4-[1-[4-(1,1,2,2,2-pentafluoroethoxy)phenyl]-1,2,4-triazol-3-yl]phenyl]methylenehydrazono]thiazolidin-4-one; 2-(6-chloro-3-ethylsulfonyl-imidazo[1,2-a]pyridin-2-yl)-3-methyl-6-(trifluoromethyl)imidazo[4,5-b]pyridine, 2-(6-bromo-3-ethylsulfonyl-imidazo[1,2-a]pyridin-2-yl)-3-methyl-6-(trifluoromethyl)imidazo[4,5-b]pyridine, 2-(3-ethylsulfonyl-6-iodo-imidazo[1,2-a]pyridin-2-yl)-3-methyl-6-(trifluoromethyl)imidazo[4,5-b]pyridine, 2-(7-chloro-3-ethylsulfonyl-imidazo[1,2-a]pyridin-2-yl)-3-methyl-6-(trifluoromethyl)imidazo[4,5-b]pyridine, 2-(7-chloro-3-ethylsulfonyl-imidazo[1,2-a]pyridin-2-yl)-3-methyl-6-(trifluoromethyl)imidazo[4,5-b]pyridine, 2-(3-ethylsulfonyl-7-iodo-imidazo[1,2-a]pyridin-2-yl)-3-methyl-6-(trifluoromethyl)imidazo[4,5-b]pyridine, 3-ethylsulfonyl-6-iodo-2-[3-methyl-6-(trifluoromethyl)imidazo[4,5-b]pyridin-2-yl]imidazo[1,2-a]pyridine-8-carbonitrile, 2-[3-ethylsulfonyl-8-fluoro-6-(trifluoromethyl)imidazo[1,2-a]pyridin-2-yl]-3-methyl-6-(trifluoromethyl)imidazo[4,5-b]pyridine, 2-[3-ethylsulfonyl-7-(trifluoromethyl)imidazo[1,2-a]pyridin-2-yl]-3-methyl-6-(trifluoromethylsulfinyl)imidazo[4,5-b]pyridine, 2-[3-ethylsulfonyl-7-(trifluoromethyl)imidazo[1,2-a]pyridin-2-yl]-3-methyl-6-(trifluoromethyl)imidazo[4,5-c]pyridine, 2-(6-bromo-3-ethylsulfonyl-imidazo[1,2-a]pyridin-2-yl)-6-(trifluoromethyl)pyrazolo[4,3-c]pyridine; N-[[2-fluoro-4-[(2S,3S)-2-hydroxy-3-(3,4,5-trichlorophenyl)-3-(trifluoromethyl)pyrrolidin-1-yl]phenyl]methyl]cyclopropanecarboxamide; sulfiflumin; flupentiofenox, N-[3-chloro-1-(3-pyridyl)pyrazol-4-yl]-2-methylsulfonyl-propanamide, cyclobutrifluram; N-[4-chloro-3-[(1-cyanocyclopropyl)carbamoyl]phenyl]-2-methyl-4-methylsulfonyl-5-(1,1,2,2,2-pentafluoroethyl)pyrazole-3-carboxamide, cyproflanilide, nicofluprole; 1,4-dimethyl-2-[2-(pyridin-3-yl)-2h-indazol-5-yl]-1,2,4-triazolidine-3,5-dione, indazapyroxamet, tiapyrachlor, N-cyclopropyl-5-[(5S)-5-(3,5-dichloro-4-fluoro-phenyl)-5-(trifluoromethyl)-4H-isoxazol-3-yl]isoquinoline-8-carboxamide, 5-[(5S)-5-(3,5-dichloro-4-fluoro-phenyl)-5-(trifluoromethyl)-4H-isoxazol-3-yl]-N-(pyrimidin-2-ylmethyl)isoquinoline-8-carboxamide, N-[1-(2,6-difluorophenyl)pyrazol-3-yl]-2-(trifluoromethyl)benzamide, 5-((1R,3R)-3-(3,5-Bis(trifluoromethyl)phenyl)-2,2-dichlorocyclopropane-1-carboxamido)-2-chloro-N-(3-(2,2-difluoroacetamido)-2,4-difluorophenyl)benzamide, 1-[6-(2,2-difluoro-7-methyl-[1,3]dioxolo[4,5-f]benzimidazol-6-yl)-5-ethylsulfonyl-3-pyridyl]cyclopropanecarbonitrile, 6-(5-cyclopropyl-3-ethylsulfonyl-2-pyridyl)-2,2-difluoro-7-methyl-[1,3]dioxolo[4,5-f]benzimidazole, Ledprona. Flupyroxystrobin, 3,5-bis(trifluoromethyl)-N-[(1S)-1-[1-[6-(trifluoromethyl)-4-pyrimidinyl]-1H-1,2,4-triazol-5-yl]ethyl]-benzamide, 2-(3-ethylsulfonyl-2-pyridyl)-5-(2,2,3,3,3-pentafluoropropoxy)pyrazine, 2-[3-ethylsulfonyl-6-(trifluoromethyl)pyrazolo[1,5-a]pyridin-2-yl]-3-methyl-6-(trifluoromethyl)imidazo[4,5-b]pyridine, 9-(methoxymethyl)-5-(3-pyridyl)-2-oxa-5,6,9,14-tetrazatricyclo[8.4.0.0^{3,7}]tetradeca-1(10),3,6,11,13-pentaen-8-one, bisulfufen, 2-[5-[(E)-2-chloro-3,3,3-trifluoro-prop-1-enyl]-1-methyl-imidazol-2-yl]-5-cyclopropyl-3-ethylsulfonyl-pyridine, cybenzoxasulfyl, isoflualanam;
Bentioflumin, Vadescana, (3Z)-1-[2-fluoro-4-[1-[4-(trifluoromethoxy)phenyl]-1,2,4-triazol-3-yl]phenyl]-3-[3-[5-methyl-2-(2,2,2-trifluoroethoxymethyl)phenyl]-4-oxo-thiazolidin-2-ylidene]urea, 1-[6-(2,2-difluoro-8-oxo-[1,3]dioxolo[4,5-g]chromen-7-yl)-5-ethylsulfonyl-3-pyridyl]cyclopropanecarbonitrile, 2-chloro-N-[(1S)-2-ethyl-1-methyl-butyl]furan-3-carboxamide, galquin, [5-cyclopropyl-2-[3-methyl-6-(trifluoromethyl)imidazo[4,5-b]pyridin-2-yl]-3-pyridyl]-ethyl-hydroxy-oxo-λ⁶-sulfane, 4-[5-(3,5-dichloro-4-fluoro-phenyl)-5-(trifluoromethyl)-4H-isoxazol-3-yl]-2-methyl-N-[1-(2,2,2-trifluoroethylcarbamoyl)cyclopropyl]benzamide, 3-[3-ethylsulfonyl-7-(trifluoromethyl)imidazo[1,2-a]pyridin-2-yl]-7-(trifluoromethyl)chromen-4-one,

The commercially available compounds M listed above may be found in The Pesticide Man-ual, 18th Edition, C. MacBean, British Crop Protection Council (2018), or http://bcpcdata.com/pesticide-manual.html, http://www.alanwood.net/pesticides.

The active compounds described by IUPAC nomenclature are known from CN103814937; WO2013/003977, WO2007/101369, WO2018/177970, CN10171577, CN102126994, WO2007/101540, WO2007/043677, WO2011/085575, WO2008/134969, WO2012/034403, WO2006/089633, WO2008/067911, WO2006/043635, WO2009/124707, WO2013/050317, WO2010/060379, WO2010/127926, WO2010/006713, WO2012/000896, WO2007/101369, WO2012/143317, WO2015/038503, EP2910126, WO2015/059039, WO2015/190316, WO2012/126766, WO2009/102736, WO2013/116053, WO2018/052136, WO2015150252, WO2020055955, WO2021158455, WO2013092350, WO201811111, EP3608311, WO2019236274, WO2013092350, WO 2018052136, WO2009102736, WO2016174049, WO2012126766, CN106554335, WO2017054524, CN105153113, WO2022072650; WO2018071327, WO2022101502, WO2012158396, WO2007079162, WO2020013147, WO2020097414, EP242081, WO2023058748, WO2017065228, EP3428166, WO2021029308, WO2022009058, WO2017067500, WO2020090585, WO2017146226, WO2021043115, WO2023016278, WO2021011722, WO2024126388, WO2024137594, EP4389739, WO2016096584, WO2024120137, WO2024188755, CN115925576, WO2024087613.

The following list of fungicides, in conjunction with which the compounds of the invention can be used, illustrates the possible combinations:
A) Respiration (C)
   - complex III at Qₒ site (Qol, C3): azoxystrobin (A.1.1), bifemetstrobin (A.1.24), bifujunzhi (A.1.37), coumethoxystrobin (A.1.2), coumoxystrobin (A.1.3), dimoxystrobin (A.1.4), enestroburin (A.1.5), famoxadone (A.1.21), fenamidone (A.1.23), fenaminstrobin (A.1.6), flufenoxystrobin (A.1.7), fluoxastrobin (A.1.8), kresoxim-methyl (A.1.9), mandestrobin (A.1.10), metominostrobin (A.1.11), metyltetraprole (A.1.25; member of MoA subgroup A), orysastrobin (A.1.12), picoxy-strobin (A.1.13), pyraclostrobin (A.1.14), pyrametostrobin (A.1.15), pyraoxystrobin (A.1.16), pyribencarb (A.1.19), pyriminostrobin (A.1.36), triclopyricarb (A.1.20), trifloxystrobin (A.1.17), 2-(2-(3-(2,6-dichlorophenyl)-1-methyl-allylideneaminooxymethyl)-phenyl)-2-methoxyimino-*N*-methyl-acetamide (A.1.18), methyl-*N*-[2-[(1,4-dimethyl-5-phenyl-pyrazol-3-yl)oxylmethyl]-phenyl]-*N*-methoxy-carbamate (A.1.22), (*Z*,2*E*)-5-[1-(2,4-dichlorophenyl)pyrazol-3-yl]-oxy-2-methoxyimino-*N*,3-dimethyl-pent-3-enamide (A.1.34), (*Z*,2*E*)-5-[1-(4-chlorophenyl)pyrazol-3-yl]oxy-2-methoxyimino-*N*,3-dimethyl-pent-3-enamide (A.1.35), 2-(ortho-((2,5-dimethylphenyloxymethylen)phenyl)-3-methoxy-acrylic acid methylester (A.1.38), methyl (*Z*)-3-methoxy-2-[2-methyl-5-(3-propylpyrazol-1-yl)phenoxy]prop-2-enoate, methyl (*Z*)-2-[5-(3-isopropylpyrazol-1-yl)-2-methyl-phenoxy]-3-methoxy-prop-2-enoate, methyl (*Z*)-3-methoxy-2-[2-methyl-5-[3-(trifluoromethyl)pyrazol-1-yl]phenoxy]prop-2-enoate, methyl (*Z*)-3-methoxy-2-[2-methyl-5-(4-propyltriazol-2-yl)phenoxy]prop-2-enoate, methyl (*Z*)-3-methoxy-2-[2-methyl-5-[4-(trifluoromethyl)triazol-2-yl]phenoxy]prop-2-enoate, methyl (*Z*)-2-[5-(4-isopropyltriazol-2-yl)-2-methyl-phenoxy]-3-methoxy-prop-2-enoate, methyl (*Z*)-2-(5-cyclobutyl-2-methyl-phenoxy)-3-methoxy-prop-2-enoate, methyl (*Z*)-2-(5-cyclopentyl-2-methyl-phenoxy)-3-methoxy-prop-2-enoate, methyl (*Z*)-2-(5-cyclopropyl-2-methyl-phenoxy)-3-methoxy-prop-2-enoate, methyl (*Z*)-2-(5-cyclohexyl-2-methyl-phenoxy)-3-methoxy-prop-2-enoate, methyl (*E*)-3-methoxy-2-[(2-methyl-5-phenyl-phenyl)methyl]prop-2-enoate, methyl (*E*)-3-methoxy-2-[[5-[(*E*)-*N*-methoxy-*C*-methyl-carbonimidoyl]-2,4-dimethyl-phenyl]methyl]prop-2-enoate; methyl (*E*)-2-[[5-(2-cyclopropylethynyl)-2,4-dimethyl-phenyl]methyl]-3-methoxy-prop-2-enoate; methyl (*E*)-3-methoxy-2-(2-phenyl-1,3-benzoxazol-4-yl)prop-2-enoate; methyl (*E*)-3-methoxy-2-(2-phenyl-1,3-benzothiazol-4-yl)prop-2-enoate; methyl (*E*)-3-methoxy-2-(2-phenyl-1,3-benzoxazol-7-yl)prop-2-enoate; methyl (*Z*)-2-[6-(2-cyclopropylethynyl)benzimidazol-1-yl]-3-methoxy-prop-2-enoate; methyl (*Z*)-3-methoxy-2-(6-phenylbenzimidazol-1-yl)prop-2-enoate; methyl (*Z*)-2-(3-chloro-6-phenyl-indol-1-yl)-3-methoxy-prop-2-enoate; methyl (*Z*)-2-(2,3-dichloro-6-phenyl-indol-1-yl)-3-methoxy-prop-2-enoate; methyl (*Z*)-3-methoxy-2-[6-[(*E*)-methoxyiminomethyl]indol-1-yl]prop-2-enoate; methyl (*Z*)-3-methoxy-2-[6-[(*E*)-*N*-methoxy-*C*-methyl-carbonimidoyl]indol-1-yl]prop-2-enoate, methyl *N*-[[5-[1-(2,6-difluoro-4-isopropyl-phenyl)pyrazol-3-yl]-2-methyl-phenyl]methyl]carbamate, methyl *N*-[[5-[1-(4-cyclopropyl-2,6-difluoro-phenyl)-pyrazol-3-yl]-2-methyl-phenyl]methyl]carbamate, methyl *N*-[[5-[1-(4-chloro-2,6-difluorophenyl)pyrazol-3-yl]-2-methyl-phenyl]methyl]carbamate, methyl *N*-[[5-[1-[2,6-difluoro-4-(trifluoromethyl)phenyl]pyrazol-3-yl]-2-methyl-phenyl]methyl]carbamate;
   - complex III at Qᵢ site (Qil, C4): cyazofamid (A.2.1), amisulbrom (A.2.2), [(6S,7R,8R)-8-benzyl-3-[(3-hydroxy-4-methoxy-pyridine-2-carbonyl)amino]-6-methyl-4,9-dioxo-1,5-dioxonan-7-yl]-2-methylpropanoate (A.2.3), fenpicoxamid (A.2.4), florylpicoxamid (A.2.5), metarylpicoxamid (A.2.6);
   - complex II (SDHI, C2): benodanil (A.3.1), benzovindiflupyr (A.3.2), bixafen (A.3.3), boscalid (A.3.4), carboxin (A.3.5), cyclobutrifluram (A.3.24), fenfuram (A.3.6), fluopyram (A.3.7), flutolanil (A.3.8), fluxapyroxad (A.3.9), furametpyr (A.3.10), inpyrfluxam (A.3.22), isofetamid (A.3.11), isoflucypram (A.3.31), isopyrazam (A.3.12), mepronil (A.3.13), oxycarboxin (A.3.14), penflufen (A.3.15), penthiopyrad (A.3.16), pydiflumetofen (A.3.17), pyrapropoyne (A.3.23), pyraziflumid (A.3.18), sedaxane (A.3.19), tecloftalam (A.3.20), thifluzamide (A.3.21), fluindapyr (A.3.28), *N*-[2-[2-chloro-4-(trifluoromethyl)phenoxy]phenyl]-3-(difluoromethyl)-5-fluoro-1-methyl-pyrazole-4-carboxamide (A.3.29), methyl (*E*)-2-[2-[(5-cyano-2-methyl-phenoxy)methyl]phenyl]-3-methoxy-prop-2-enoate (A.3.30), 2-(difluoromethyl)-*N*-(1,1,3-trimethyl-indan-4-yl)pyridine-3-carboxamide (A.3.32), 2-(difluoromethyl)-*N*-[(3*R*)-1,1,3-trimethylindan-4-yl]pyridine-3-carboxamide (A.3.33), 2-(difluoromethyl)-*N*-(3-ethyl-1,1-dimethyl-indan-4-yl)pyridine-3-carboxamide (A.3.34), 2-(difluoromethyl)-*N*-[(3*R*)-3-ethyl-1,1-dimethyl-indan-4-yl]pyridine-3-carboxamide (A.3.35), 2-(difluoromethyl)-*N*-(1,1-dimethyl-3-propyl-indan-4-yl)pyridine-3-carboxamide (A.3.36), 2-(difluoromethyl)-*N*-[(3*R*)-1,1-dimethyl-3-propyl-indan-4-yl]pyridine-3-carboxamide (A.3.37), 2-(difluoromethyl)-*N*-(3-isobutyl-1,1-dimethyl-indan-4-yl)pyridine-3-carboxamide (A. 3. 38), 2-(difluoromethyl)-*N*-[(3*R*)-3-isobutyl-1,1-dimethyl-indan-4-yl]pyridine-3-carboxamide (A.3.39);
   - complex I NADH oxido-reductase (C1): diflumetorim (A.4.1);
   - uncouplers (C5): binapacryl (A.4.2), dinobuton (A.4.3), dinocap (A.4.4), fluazinam (A.4.5), meptyldinocap (A.4.6), ferimzone (A.4.7);
   - inhibitors of ox. phosphorylation (C6): fentin salts, e.g. fentin-acetate (A.4.8), fentin chloride (A.4.9) or fentin hydroxide (A.4.10);
   - ATP transport: silthiofam (A.4.11);
   - quinone inside and outside inhibitor stigmatellin binding type (QioSI; C8): ametoctradin (A.5.1);
B) Sterol biosynthesis (G)
   - C14 demethylase (DMI, G1): triazoles: azaconazole (B.1.1), bitertanol (B.1.2), bromuconazole (B.1.3), cyproconazole (B.1.4), difenoconazole (B.1.5), diniconazole (B.1.6), diniconazole-M (B.1.7), epoxiconazole (B.1.8), fenbuconazole (B.1.9), fluoxytioconazole (B.1.33), fluquinconazole (B.1.10), flusilazole (B.1.11), flutriafol (B.1.12), hexaconazole (B.1.13), imibenconazole (B.1.14), ipconazole (B.1.15), ipfentrifluconazole (B.1.37), mefentrifluconazole (B.1.38), metconazole (B.1.17), myclobutanil (B.1.18), oxpoconazole (B.1.19), paclobutrazole (B.1.20), penconazole (B.1.21), propiconazole (B.1.22), prothioconazole (B.1.23), simeconazole (B.1.24), tebuconazole (B.1.25), tetraconazole (B.1.26), triadimefon (B.1.27), triadimenol (B.1.28), triticonazole (B.1.29), uniconazole (B.1.30), 2-(2,4-difluorophenyl)-1,1-difluoro-3-(tetrazol-1-yl)-1-[5-[4-(2,2,2-trifluoroethoxy)phenyl]-2-pyridyl]propan-2-ol (B.1.31), 2-(2,4-difluorophenyl)-1,1-difluoro-3-(tetrazol-1-yl)-1-[5-[4-(trifluoromethoxy)phenyl]-2-pyridyl]propan-2-ol (B.1.32), 2-(chloromethyl)-2-methyl-5-(p-tolylmethyl)-1-(1,2,4-triazol-1-ylmethyl)cyclopentanol (B.1.43), 4-[[6-[2-(2,4-difluorophenyl)-1,1-difluoro-2-hydroxy-3-(1,2,4-triazol-1-yl)propyl]-3-pyridyl]oxy]benzonitrile (B.1.53), 2-[6-(4-bromophenoxy)-2-(trifluoromethyl)-3-pyridyl]-1-(1,2,4-triazol-1-yl)propan-2-ol (B.1.54), 2-[6-(4-chlorophenoxy)-2-(trifluoromethyl)-3-pyridyl]-1-(1,2,4-triazol-1-yl)propan-2-ol (B.1.55), (2R)-2-[4-(4-chlorophenoxy)-2-(trifluoromethyl)phenyl]-1-(1,2,4-triazol-1-yl)propan-2-ol, (2S)-2-[4-(4-chlorophenoxy)-2-(trifluoromethyl)phenyl]-1-(1,2,4-triazol-1-yl)propan-2-ol, methyl 2-[2-chloro-4-(4-chlorophenoxy)phenyl]-2-hydroxy-3-(1,2,4-triazol-1-yl)propanoate (B.1.56), 2-[2-chloro-4-(4-chlorophenoxy)phenyl]-2-hydroxy-3-(1,2,4-triazol-1-yl)propanoic acid (B.1.57); imidazoles: imazalil (B.1.44), pefurazoate (B.1.45), prochloraz (B.1.46), triflumizole (B.1.47); pyrimidines, pyridines, piperazines: fenarimol (B.1.49), pyrifenox (B.1.50), triforine (B.1.51), [3-(4-chloro-2-fluoro-phenyl)-5-(2,4-difluorophenyl)isoxazol-4-yl]-(3-pyridyl)methanol (B.1.52);
   - delta14-reductase (G2): aldimorph (B.2.1), dodemorph (B.2.2), dodemorph-acetate (B.2.3), fenpropidin (B.2.6), fenpropimorph (B.2.4), piperalin (B.2.7), spiroxamine (B.2.8), tridemorph (B.2.5);
   - 3-keto reductase (G3): fenhexamid (B.3.1), fenpyrazamine (B.3.2);
   - other: chlorphenomizole (B.4.1);
C) Nucleic acids metabolism (A)
   - RNA polymerase I (A1): benalaxyl (C.1.1), benalaxyl-M (C.1.2), kiralaxyl (C.1.3), metalaxyl (C.1.4), metalaxyl-M (C.1.5), ofurace (C.1.6), oxadixyl (C.1.7);
   - adenosine deaminase (A2): bupirimate (C.2.4), 5-fluoro-2-(p-tolylmethoxy)pyrimidin-4-amine (C.2.6), 5-fluoro-2-(4-fluorophenylmethoxy)pyrimidin-4-amine (C.2.7), 5-fluoro-2-(4-chlorophenylmethoxy)pyrimidin-4-amine (C.2.8);
   - DNA/RNA synthesis (A3): 5-fluorocytosine (C.2.5), hymexazole (C.2.1), octhilinone (C.2.2),
   - gyrase (A4): oxolinic acid (C.2.3);
   - dihydroorotate dehydrogenase (DHODH; A5): ipflufenoquin (C.5.1), quinofumelin (C.5.2), feneptamidoquin (C.5.3);
D) Cytoskeleton and motor protein (B)
   - tubulin polymerization (MBC; B1): benomyl (D.1.1), carbendazim (D.1.2), fuberidazole (D.1.3), pyridachlometyl (D.1.6), thiabendazole (D.1.4), thiophanate-methyl (D.1.5), N-ethyl-2-[(3-ethynyl-8-methyl-6-quinolyl)oxy]butanamide (D.1.8), *N*-ethyl-2-[(3-ethynyl-8-methyl-6-quinolyl)oxy]-2-methylsulfanyl-acetamide (D.1.9), 2-[(3-ethynyl-8-methyl-6-quinolyl)oxy]-*N*-(2-fluoroethyl)butanamide (D.1.10), 2-[(3-ethynyl-8-methyl-6-quinolyl)oxy]-*N*-(2-fluoroethyl)-2-methoxy-acetamide (D.1.11), 2-[(3-ethynyl-8-methyl-6-quinolyl)oxy]-*N*-propyl-butanamide (D.1.12), 2-[(3-ethynyl-8-methyl-6-quinolyl)oxy]-2-methoxy-*N*-propyl-acetamide (D.1.13), 2-[(3-ethynyl-8-methyl-6-quinolyl)oxy]-2-methylsulfanyl-*N*-propyl-acetamide (D.1.14), 2-[(3-ethynyl-8-methyl-6-quinolyl)oxy]-*N*-(2-fluoroethyl)-2-methylsulfanyl-acetamide (D.1.15), 4-(2-bromo-4-fluoro-phenyl)-*N*-(2-chloro-6-fluoro-phenyl)-2,5-dimethyl-pyrazol-3-amine (D.1.16), 4-(2-bromo-4-fluorophenyl)-*N*-(2-fluoro-6-nitrophenyl)-1,3-dimethyl-1*H*-pyrazol-5-amine, 4-(2-chloro-4,6-difluorophenyl)-*N*-(2-fluoro-6-nitrophenyl)-1,3-dimethyl-1*H*-pyrazol-5-amine, 4-(2-chloro-4-fluorophenyl)-*N*-(2-fluoro-6-nitrophenyl)-3-ethyl-1-methyl-1*H*-pyrazol-5-amine, 4-(2-chloro-4-fluorophenyl)-*N*-(2-fluoro-4-methyl-6-nitrophenyl)-1,3-dimethyl-1*H*-pyrazol-5-amine, 4-(2-chloro-4-fluorophenyl)-*N*-(2-fluoro-6-nitrophenyl)-1,3-dimethyl-1*H*-pyrazol-5-amine, 4-(2,4-difluorophenyl)-*N*-(2-fluoro-6-nitrophenyl)-1,3-dimethyl-1*H*-pyrazol-5-amine;
   - tubulin polymerization (B2): diethofencarb (D.2.1),
   - tubulin polymersation (B3): ethaboxam (D.2.2), zoxamide (D.2.5);
   - cell division (B4): pencycuron (D.2.3);
   - spectrin-like proteins (B5): fluopicolide (D.2.4), fluopimomide (D.2.9);
   - actin/myosin/fimbrin function (B6): metrafenone (D.2.6), phenamacril (D.2.8), pyriofenone (D.2.7);
E) Amino acids and protein synthesis (D)
   - methionine synthesis (D1): cyprodinil (E.1.1), mepanipyrim (E.1.2), pyrimethanil (E.1.3);
   - ribosome, termination step (D2): blasticidin-S (E.2.1);
   - ribosome initiation step (D3): kasugamycin (E.2.2), kasugamycin hydrochloride-hydrate (E.2.3);
   - ribosome initiation step (D4): streptomycin (E.2.5);
   - ribosome elongation step (D5): mildiomycin (E.2.4), oxytetracyclin (E.2.6);
F) Signal transduction
   - mechanism unknown (E1): proquinazid (F.2.2), quinoxyfen (F.2.1);
   - MAP/histidine kinase os-2 (E2): fludioxonil (F.1.5);
   - MAP/histidine kinase os-1 (E3): iprodione (F.1.2), procymidone (F.1.3), vinclozolin (F.1.4);
G) Lipid synthesis or transport / membrane (F)
   - methyl transferase (F2): edifenphos (G.1.1), iprobenfos (G.1.2), isoprothiolane (G.1.4); pyrazophos (G.1.3);
   - cell peroxidation (F3): biphenyl (G.2.5), chloroneb (G.2.6), dicloran (G.2.1), etridiazole (G.2.7), quintozene (G.2.2), tecnazene (G.2.3), tolclofos-methyl (G.2.4);
   - cell membrane permeability (F4): propamocarb (G.4.1);
   - ergosterol binding (F8): natamycin;
   - oxysterol binding protein (F9): fluoxapiprolin (G.5.3), oxathiapiprolin (G.5.1), 4-[1-[2-[3-(difluoromethyl)-5-methyl-pyrazol-1-yl]acetyl]-4-piperidyl]-N-tetralin-1-yl-pyridine-2-carboxamide (G.5.4), 4-[1-[2-[3,5-bis(difluoromethyl)pyrazol-1-yl]acetyl]-4-piperidyl]-*N*-tetralin-1-yl-pyridine-2-carboxamide (G.5.5), 4-[1-[2-[3-(difluoromethyl)-5-(trifluoromethyl)pyrazol-1-yl]acetyl]-4-piperidyl]-*N*-tetralin-1-yl-pyridine-2-carboxamide (G.5.6), 4-[1-[2-[5-cyclopropyl-3-(difluoromethyl)pyrazol-1-yl]acetyl]-4-piperidyl]-*N*-tetralin-1-yl-pyridine-2-carboxamide (G.5.7), 4-[1-[2-[5-methyl-3-(trifluoromethyl)pyrazol-1-yl]acetyl]-4-piperidyl]-*N*-tetralin-1-yl-pyridine-2-carboxamide (G.5.8), 4-[1-[2-[5-(difluoromethyl)-3-(trifluoromethyl)pyrazol-1-yl]acetyl]-4-piperidyl]-*N*-tetralin-1-yl-pyridine-2-carboxamide (G.5.9), 4-[1-[2-[3,5-bis(trifluoromethyl)pyrazol-1-yl]acetyl]-4-piperidyl]-*N*-tetralin-1-yl-pyridine-2-carboxamide (G.5.10), (4-[1-[2-[5-cyclopropyl-3-(trifluoromethyl)pyrazol-1-yl]acetyl]-4-piperidyl]-*N*-tetralin-1-yl-pyridine-2-carboxamide (G.5.11), (1-(4-(4-(5-(2,6-dichlorophenyl)-4,5-dihydroisoxazol-3-yl)thiazol-2-yl)piperidin-1-yl)-2-((3-trifluoromethyl)pyrazin-2-yl)oxy)ethan-1-one, 1-(4-(4-(5-(2-chloro-6-fluorophenyl)-4,5-dihydroisoxazol-3-yl)thiazol-2-yl)piperidin-1-yl)-2-((3-trifluoromethyl)pyridin-2-yl)oxy)ethan-1-one, *tert*-butyl 4-(4-(5-(2-bromo-6-fluorophenyl)-4,5-dihydroisoxazol-3-yl)thiazol-2-yl)piperidin-1-carboxylate, ((2-(3-(2-(1-(2-(3,5-bis(trifluoromethyl)-1*H*-pyrazol-1-yl)acetyl)piperidin-4-yl)thiazol-4-yl)-4,5-dihydroisoxazo-5-yl)-3-fluorophenyl)imino)dimethyl-λ⁶-sulfanone, ((2-(3-(2-(1-(2-(3,5-bis(difluoromethyl)-1*H*-pyrazol-1-yl)acetyl)piperidin-4-yl)thiazol-4-yl)-4,5-dihydroisoxazo-5-yl)-3-fluorophenyl)imino)dimethyl-λ⁶-sulfanone, ((2-(3-(2-(1-(2-(3,5-bis(difluoromethyl)-1*H*-pyrazol-1-yl)acetyl)piperidin-4-yl)thiazol-4-yl)-4,5-dihydroisoxazo-5-yl)-3-chorophenyl)imino)(isopropyl)(methyl)-λ⁶-sulfanone, ((2-(3-(2-(1-(2-(3,5-bis(trifluoromethyl)-1*H*-pyrazol-1-yl)acetyl)piperidin-4-yl)thiazol-4-yl)-4,5-dihydroisoxazo-5-yl)-3-fluorophenyl)imino)(isopropyl)(methyl)-λ⁶-sulfanone, ((2-(3-(2-(1-(2-(3,5-bis(difluoromethyl)-1*H*-pyrazol-1-yl)acetyl)piperidin-4-yl)thiazol-4-yl)-4,5-dihydroisoxazo-5-yl)-3-(trifluoromethyl)phenyl)imino)dimethyl-λ⁶-sulfanone, ((3-fluoro-2-(3-(2-(1-(2-(5-methyl-3-(trifluoromethyl)-1*H*-pyrazol-1-yl)acetyl)piperidin-4-yl)thiazol-4-yl)-4,5-dihydroisoxazo-5-yl)-phenyl)imino)dimethyl-λ⁶-sulfanone;
H) Multi Site Activity (M)
   - inorganics (M01): Bordeaux mixture (H.1.1), copper (H.1.2), copper acetate (H.1.3), copper hydroxide (H.1.4), copper oxychloride (H.1.5), basic copper sulfate (H.1.6), sulfur (H.1.7);
   - dithiocarbamates and relatives: ferbam (H.2.1), mancozeb (H.2.2), maneb (H.2.3), metam (H.2.4), metiram (H.2.5), propineb (H.2.6), thiram (H.2.7), zineb (H.2.8), ziram (H.2.9), zinc thiazole (H.2.10);
   - organochlorine compounds (M04, M05, M06, M08): anilazine (H.3.1), captafol (H.3.3), captan (H.3.4), chlorothalonil (H.3.2), dichlofluanid (H.3.6), dichlorophen (H.3.7), folpet (H.3.5), hexachlorobenzene (H.3.8), pentachlorphenole (H.3.9) and its salts, phthalide (H.3.10), tolylfluanid (H.3.11);
   - guanidines, quinones, quinoxalines, maleimides, thiocarbamates (M07, M09, M10, M11, M12): chinomethionat (H.4.13), dithianon (H.4.9), fluoroimide (H.4.11), guanidine (H.4.1), guazatine (H.4.4), guazatine-acetate (H.4.5), iminoctadine (H.4.6), iminoctadine-triacetate (H.4.7), iminoctadine-tris(albesilate) (H.4.8), methasulfocarb (H.4.12), 2,6-dimethyl-1*H*,5*H*-[1,4]dithiino[2,3-c:5,6-c']dipyrrole-1,3,5,7(2*H*,6*H*)-tetraone (H.4.10),
I) Cell wall biosynthesis (H) and melanin synthesis in cell wall (I)
   - chitin synthase (H4): polyoxin B (I.1.2);
   - cellulose synthase (H5): benthiavalicarb (I.3.5), dimethomorph (I.3.1), flumorph (I.3.2), iprovalicarb (I.3.6), mandipropamid (I.3.3), pyrimorph (I.3.4), valifenalate (I.3.7);
   - reductase in melanin synthesis (MBI-R; I1) pyroquilon (I.2.1), tricyclazole (I.2.2);
   - dehydratase in melanin synthesis (MBI-D, I2); carpropamid (I.2.3), dicyclomet (I.2.4), fenoxanil (I.2.5);
   - polyketide synthase in melanin synthesis (MBI-P, I3): tolprocarb (I.2.6);
J) Plant defence induction (P1 to P8)
   - salicylate-related (P01-P03, P08): acibenzolar-S-methyl (J.1.1), probenazole (J.1.2), isotianil (J.1.3), tiadinil (J.1.4), dichlobentiazox (J.1.13); phosphonates (P07): fosetyl (J.1.6), fosetyl-aluminum (J.1.7), phosphorous acid and its salts (J.1.8), calcium phosphonate (J.1.11), potassium phosphonate (J.1.12); others: potassium or sodium bicarbonate (J.1.9), 4-cyclopropyl-*N*-(2,4-di¬methoxy¬phenyl)thiadiazole-5-carboxamide (J.1.10);
K) Unknown mode of action (U)
   - aminopyrifen (K.1.54), benziothiazolinone (K.1.48), bromothalonil (K.1.49), bronopol (K.1.1), cyflufenamid (K.1.3), cymoxanil (K.1.4), dazomet (K.1.5), debacarb (K.1.6), diclomezine (K.1.8), difenzoquat (K.1.9), difenzoquat-methylsulfate (K.1.10), diphenylamin (K.1.11), dodine, dodine free base (K.1.18), fenitropan (K.1.12), flufenoxadiazam (K.1.58) [MoA proposed: class II histone deacetylase inhibitor], flumetover (K.1.14), flumetylsulforim (K.1.60), flusulfamide (K.1.15), flutianil (K.1.16), harpin (K.1.17), nitrapyrin (K.1.19), nitrothal-isopropyl (K.1.20), oxine-copper (K.1.22), picarbutrazox (K.1.41), pyrisoxazole (K.1.37), seboctylamine (K.1.61), tebufloquin (K.1.24), tecloftalam (K.1.25), triazoxide (K.1.26), validamycin (K.1.2); *N'*-(4-(4-chloro-3-trifluoromethyl-phenoxy)-2,5-dimethyl-phenyl)-*N*-ethyl-*N*-methyl formamidine (K.1.27), *N'*-(4-(4-fluoro-3-trifluoromethyl-phenoxy)-2,5-dimethyl-phenyl)-*N*-ethyl-*N*-methyl formamidine (K.1.28), *N'*-[4-[[3-[(4-chlorophenyl)methyl]-1,2,4-thiadiazol-5-yl]oxy]-2,5-dimethyl-phenyl]-*N*-ethyl-*N*-methyl-formamidine (K.1.29), *N'*-(5-bromo-6-indan-2-yloxy-2-methyl-3-pyridyl)-*N*-ethyl-*N*-methyl-formamidine (K.1.30), *N'*-[5-bromo-6-[1-(3,5-difluorophenyl)ethoxy]-2-methyl-3-pyridyl]-*N*-ethyl-*N*-methyl-formamidine (K.1.31), *N'*-[5-bromo-6-(4-isopropylcyclohexoxy)-2-methyl-3-pyridyl]-*N*-ethyl-*N*-methyl-formamidine (K.1.32), *N'*-[5-bromo-2-methyl-6-(1-phenylethoxy)-3-pyridyl]-*N*-ethyl-*N*-methyl-formamidine (K.1.33), *N'*-(2-methyl-5-trifluoromethyl-4-(3-trimethylsilanyl-propoxy)-phenyl)-*N*-ethyl-*N*-methyl formamidine (K.1.34), *N'*-(5-difluoromethyl-2-methyl-4-(3-trimethylsilanyl-propoxy)-phenyl)-*N*-ethyl-*N*-methyl formamidine (K.1.35), 2-(4-chloro-phenyl)-*N*-[4-(3,4-dimethoxy-phenyl)-isoxazol-5-yl]-2-prop-2-ynyloxy-acetamide (K.1.36), 3-[5-(4-methylphenyl)-2,3-dimethyl-isoxazolidin-3-yl]-pyridine (K.1.38), 5-chloro-1-(4,6-dimethoxy-pyrimidin-2-yl)-2-methyl-1*H*-benzoimidazole (K.1.39), ethyl (*Z*)-3-amino-2-cyano-3-phenyl-prop-2-enoate (K.1.40), pentyl *N*-[6-[[(*Z*)-[(1-methyltetrazol-5-yl)-phenyl-methylene]amino]oxymethyl]-2-pyridyl]carbamate (K.1.42), but-3-ynyl *N*-[6-[[(*Z*)-[(1-methyltetrazol-5-yl)-phenyl-methylene]amino]oxymethyl]-2-pyridyl]carbamate (K.1.43), 2-(6-benzyl-2-pyridyl)quinazoline (K.1.50), 2-[6-(3-fluoro-4-methoxy-phenyl)-5-methyl-2-pyridyl]quinazoline (K.1.51), *N'*-(2,5-dimethyl-4-phenoxy-phenyl)-*N*-ethyl-*N*-methyl-formamidine (K.1.53), *N*'-[5-bromo-2-methyl-6-(1-methyl-2-propoxy-ethoxy)-3-pyridyl]-*N*-ethyl-*N*-methyl-formamidine (K.1.56), *N'*-[4-(4,5-dichlorothiazol-2-yl)oxy-2,5-dimethyl-phenyl]-*N*-ethyl-*N*-methyl-formamidine (K.1.57), *N*-methyl-4-[5-(trifluoromethyl)-1,2,4-oxadiazol-3-yl]benzenecarbothioamide (K.1.59), *N*-methoxy-*N*-[[4-[5-(trifluoromethyl)-1,2,4-oxadiazol-3-yl]phenyl]methyl]cyclopropanecarboxamide (K.1.61), *N*-((4-[5-(trifluoromethyl)-1,2,4-oxadiazol-3-yl]phenyl)methyl)propanamide (K.1.62), 3,3,3-trifluoro-*N*-[[3-fluoro-4-[5-(trifluoromethyl)-1,2,4-oxadiazol-3-yl]phenyl]methyl]propanamide (K.1.63), 3,3,3-trifluoro-*N*-[[2-fluoro-4-[5-(trifluoromethyl)-1,2,4-oxadiazol-3-yl]phenyl]methyl]propanamide (K.1.64), *N*-[2,3-difluoro-4-[5-(trifluoromethyl)-1,2,4-oxadiazol-3-yl]benzyl]butanamide (K.1.65), *N*-[[2,3-difluoro-4-[5-(trifluoromethyl)-1,2,4-oxadiazol-3-yl]phenyl]methyl]-3,3,3-trifluoro-propanamide (K.1.66), 1-methoxy-1-methyl-3-[[4-[5-(trifluoromethyl)-1,2,4-oxadiazol-3-yl]phenyl]methyl]urea (K.1.67), 1,1-diethyl-3-[[4-[5-[trifluoromethyl]-1,2,4-oxadiazol-3-yl]phenyl]methyl]urea (K.1.68), *N*,2-dimethoxy-*N*-[[4-[5-(trifluoromethyl)-1,2,4-oxadiazol-3-yl]phenyl]methyl]propanamide (K.1.69), *N*-ethyl-2-methyl-*N*-[[4-[5-(trifluoromethyl)-1,2,4-oxadiazol-3-yl]phenyl]methyl]propanamide (K.1.70), 1-methoxy-3-methyl-1-[[4-[5-(trifluoromethyl)-1,2,4-oxadiazol-3-yl]-phenyl]methyl]urea (K.1.71), 1-[[4-[5-(trifluoromethyl)-1,2,4-oxadiazol-3-yl]phenyl]methyl]pyrrolidin-2-one (K.1.72), 1-[[4-[5-(trifluoromethyl)-1,2,4-oxadiazol-3-yl]phenyl]methyl]piperidin-2-one (K.1.73), 4-[[4-[5-(trifluoromethyl)-1,2,4-oxadiazol-3-yl]phenyl]methyl]morpholin-3-one (K.1.74), 4,4-dimethyl-2-[[4-[5-(trifluoromethyl)-1,2,4-oxadiazol-3-yl]phenyl]methyl]isoxazolidin-3-one (K.1.75), 2-[[4-[5-(trifluoromethyl)-1,2,4-oxadiazol-3-yl]phenyl]methyl]isoxazolidin-3-one (K.1.76), 5,5-dimethyl-2-[[4-[5-(trifluoromethyl)-1,2,4-oxadiazol-3-yl]phenyl]methyl]isoxazolidin-3-one (K.1.77), 3,3-dimethyl-1-[[4-[5-(trifluoromethyl)-1,2,4-oxadiazol-3-yl]phenyl]methyl]piperidin-2-one (K.1.78), 2-[[4-[5-(trifluoromethyl)-1,2,4-oxadiazol-3-yl]phenyl]methyl]oxazinan-3-one (K.1.79), 1-[[3-fluoro-4-[5-(trifluoromethyl)-1,2,4-oxadiazol-3-yl]phenyl]-methyl]azepan-2-one (K.1.80), 4,4-dimethyl-1-[[4-[5-(trifluoromethyl)-1,2,4-oxadiazol-3-yl]-phenyl]methyl]pyrrolidin-2-one (K.1.81), 5-methyl-1-[[4-[5-(trifluoromethyl)-1,2,4-oxadiazol-3-yl]phenyl]methyl]pyrrolidin-2-one (K.1.82), ethyl 1-[[4-[5-(trifluoromethyl)-1,2,4-oxadiazol-3-yl]phenyl]methyl]pyrazole-4-carboxylate (K.1.83), *N*-methyl-1-[[4-[5-(trifluoromethyl)-1,2,4-oxadiazol-3-yl]phenyl]methyl]pyrazole-4-carboxamide (K.1.84), *N*,*N*-dimethyl-1-[4-[5-(trifluoromethyl)-1,2,4-oxadiazol-3-yl]benzyl]-1*H*-1,2,4-triazol-3-amine (K.1.85), *N*-methoxy-*N*-methyl-1-[[4-[5-(trifluoromethyl)-1,2,4-oxadiazol-3-yl]phenyl]methyl]pyrazole-4-carboxamide (K.1.86), propyl-1-[[4-[5-(trifluoromethyl)-1,2,4-oxadiazol-3-yl]phenyl]methyl]-pyrazole-4-carboxamide (K.1.87), *N*-methoxy-1-[[4-[5-(trifluoromethyl)-1,2,4-oxadiazol-3-yl]phenyl]methyl]pyrazole-4-carboxamide (K.1.88), *N*-allyl-*N*-[[4-[5-(trifluoromethyl)-1,2,4-oxadiazol-3-yl]phenyl]methyl]propanamide (K.1.89), 3-ethyl-1-methoxy-1-[[4-[5-(trifluoromethyl)-1,2,4-oxadiazol-3-yl]phenyl]methyl]urea (K.1.90), 1,3-dimethoxy-1-[[4-[5-(trifluoromethyl)-1,2,4-oxadiazol-3-yl]phenyl]methyl]urea (K.1.91), *N*-allyl-*N*-[[4-[5-(trifluoromethyl)-1,2,4-oxadiazol-3-yl]phenyl]methyl]acetamide (K.1.92), *N*-[4-[5-(trifluoromethyl)-1,2,4-oxadiazol-3-yl]benzyl]cyclopropanecarboxamide (K.1.93), 1-methyl-3-[[4-[5-(trifluoromethyl)-1,2,4-oxadiazol-3-yl]phenyl]methyl]urea (K.1.94), *N'*-[2-chloro-4-(2-fluorophenoxy)-5-methyl-phenyl]-*N*-ethyl-*N*-methyl-formamidine (K.1.95), *N'*-[2-chloro-4-[(4-methoxy-phenyl)methyl]-5-methyl-phenyl]-*N*-ethyl-*N*-methyl-formamidine (K.1.96), *N'*-[2-chloro-4-[(4-cyano-phenyl)methyl]-5-methylphenyl]-*N*-ethyl-*N*-methyl-formamidine (K.1.97), *N'*-[2,5-dimethyl-4-(*o*-tolylmethyl)phenyl]-*N*-ethyl-*N*-methyl-formamidine (K.1.98), 6-chloro-3-(3-cyclopropyl-2-fluoro-phenoxy)-*N*-[2-(2,4-dimethylphenyl)-2,2-difluoro-ethyl]-5-methyl-pyridazine-4-carboxamide (K.1.99), 3-(3-bromo-2-fluoro-phenoxy)-6-chloro-*N*-[2-(2-chloro-4-methyl-phenyl)-2,2-difluoro-ethyl]-5-methyl-pyridazine-4-carboxamide (K.1.100), 6-chloro-N-[2-(2-chloro-4-methyl-phenyl)-2,2-difluoro-ethyl]-3-(3-cyclopropyl-2-fluoro-phenoxy)-5-methyl-pyridazine-4-carboxamide (K.1.101), 6-chloro-3-(3-cyclopropyl-2-fluoro-phenoxy)-*N*-[2-(3,4-dimethylphenyl)-2,2-difluoroethyl]-5-methyl-pyridazine-4-carboxamide (K.1.102), 6-chloro-3-(3-chloro-2-fluoro-phenoxy)-*N*-[2-(2,4-dimethylphenyl)-2,2-difluoro-ethyl]-5-methyl-pyridazine-4-carboxamide (K.1.103), *N*-[2-(2-bromo-4-methyl-phenyl)-2,2-difluoro-ethyl]-6-chloro-3-(3-cyclopropyl-2-fluoro-phenoxy)-5-methyl-pyridazine-4-carboxamide (K.1.104), 2-[cyano-(2,6-difluoro-4-pyridyl)amino]-5-methyl-*N*-spiro[3.4]octan-3-yl-thiazole-4-carboxamide, 2-[acetyl-(2,6-difluoro-4-pyridyl)amino]-*N*-(2,2-dimethylcyclobutyl)-5-methyl-thiazole-4-carboxamide, 2-[(2,6-difluoro-4-pyridyl)-(2-methoxyacetyl)amino]-*N*-(2,2-dimethylcyclobutyl)-5-methyl-thiazole-4-carboxamide, 2-[cyano-(2,6-difluoro-4-pyridyl)amino]-*N*-(2,2-dimethylcyclobutyl)-5-methyl-thiazole-4-carboxamide, *N*-[1-[[3-[2-(5-fluoro-2-methoxy-phenyl)-2-hydroxy-ethyl]-5-[(*Z*)-*N*-isopropoxy-*C*-methyl-carbonimidoyl]-2,6-dioxo-pyrimidin-1-yl]methyl]-2-methyl-propyl]-2-methyl-propanamide, *N*-[1-[[3-[2-(5-fluoro-2-methoxy-phenyl)-2-hydroxy-ethyl]-5-[(*Z*)-*N*-isopropoxy-*C*-methyl-carbonimidoyl]-2,6-dioxo-pyrimidin-1-yl]methyl]-2-methyl-propyl]-2,2-dimethyl-propanamide, *N*-[2-[3-[2-(5-fluoro-2-methoxy-phenyl)-2-hydroxy-ethyl]-5-[(*Z*)-*N*-isopropoxy-*C*-methyl-carbonimidoyl]-2,6-dioxo-pyrimidin-1-yl]-1-methyl-ethyl]-2-methyl-propanamide, *N*-[2-[3-[2-hydroxy-2-(2-methoxyphenyl)ethyl]-5-[(*Z*)-*N*-isopropoxy-*C*-methyl-carbonimidoyl]-2,6-dioxo-pyrimidin-1-yl]-1-methyl-ethyl]-2-methyl-propanamide, *N*-[2-[3-[2-(2-cyanoethoxy)-2-(5-fluoro-2-methoxy-phenyl)ethyl]-5-[(*Z*)-*N*-isopropoxy-*C*-methyl-carbonimidoyl]-2,6-dioxo-pyrimidin-1-yl]-1-methyl-ethyl]-2-methyl-propanamide, *rac*-3-[3-(3-chloro-2-fluoro-phenoxy)-6-methyl-pyridazin-4-yl]-5-[(2-chloro-4-methyl-phenyl)methyl]-5,6-dihydro-4*H*-1,2,4-oxadiazine, (5*S*)-3-[3-(3-chloro-2-fluoro-phenoxy)-6-methyl-pyridazin-4-yl]-5-[(2-chloro-4-methylphenyl)methyl]-5,6-dihydro-4*H*-1,2,4-oxadiazine, (5*R*)-3-[3-(3-chloro-2-fluoro-phenoxy)-6-methyl-pyridazin-4-yl]-5-[(2-chloro-4-methyl-phenyl)methyl]-5,6-dihydro-4*H*-1,2,4-oxadiazine, 2-(4-fluorophenoxy)-1-[4-[5-(trifluoromethyl)-1,2,4-oxadiazol-3-yl]phenyl]ethanone, 2-[(6-fluoro-3-pyridyl)oxy]-1-[4-[5-(trifluoromethyl)-1,2,4-oxadiazol-3-yl]phenyl]ethanone, 2-(4-fluoroanilino)-1-[4-[5-(trifluoromethyl)-1,2,4-oxadiazol-3-yl]phenyl]ethenone, ethyl 1-[[4-[[2-(trifluoromethyl)-1,3-dioxolan-2-yl]methoxy]phenyl]methyl]-1*H*-pyrazole-4-carboxylate, ethyl 1-[[4-[[(1*Z*)-2-ethoxy-3,3,3-trifluoro-1-propen-1-yl]oxy]phenyl]methyl]-1*H*-pyrazole-4-carboxylate;

The fungicides described by common names, their preparation and their activity e.g. against harmful fungi is known (cf.: http://www.alanwood.net/pesticides/); these substances are commercially available.

The active substances, their preparation and their activity e.g. against fungi is known (www.bcpcpesticidecompendium.bcpc.org/); many of them are commercially available. Compounds defined by IUPAC nomenclature, their preparation and pesticidal activity are also known (e.g. Can. J. Plant Sci. 48(6), 587-94, 1968; EP-141 317; EP-152 031; EP-226 917; EP-243 970; EP-256 503; EP-428 941; EP-532 022; EP-1 028 125; EP-1 035 122; EP-1 201 648; EP-1 122 244, JP2002316902; DE19650197; DE10021412; DE102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 10/139271, WO 11/028657, WO 12/168188, WO 07/006670, WO 11/77514; WO 13/047749, WO 10/069882, WO 13/047441, WO 03/16303, WO 09/90181, WO 13/007767, WO 13/010862, WO 13/127704, WO 13/024009, WO 13/24010, WO 13/047441, WO 13/162072, WO 13/092224, WO 11/135833, CN 1907024, CN 1456054, CN 103387541, CN 1309897, WO 12/84812, CN 1907024, WO 09094442, WO 14/60177, WO 13/116251, WO 08/013622, WO 15/65922, WO 94/01546, EP 2865265, WO 07/129454, WO 12/165511, WO 11/081174, WO 13/47441, WO 16/156241, WO 16/162265, WO 23/99460, WO 21/244950, WO 21/244951, WO 22/130188, WO 22/243810, WO 21/255070, WO 21/176057, WO 21/153754, JP2023064110, JP2022153603, JP2022046550, JP2022031355, WO 22/249074, WO 23/046861, WO 18/177894, WO 20/212513, WO 20/097012, US 2023/0069915.

Suitable mixing partners for the compounds of the invention also include biopesticides.

Biopesticides have been defined as a form of pesticides based on micro-organisms (bacteria, fungi, viruses, nematodes, etc.) or natural products (compounds, e.g. metabolites, proteins, or extracts from biological or other natural sources) (U.S. Environmental Protection Agency: http://www.epa.gov/pesticides/biopesticides/). Biopesticides fall into two major classes, microbial and biochemical pesticides:
(1) Microbial pesticides consist of bacteria, fungi or viruses (and often include the metabolites that bacteria and fungi produce). Entomopathogenic nematodes are also classified as microbial pesticides, even though they are multi-cellular.
(2) Biochemical pesticides are naturally occurring substances or or structurally-similar and functionally identical to a naturally-occurring substance and extracts from biological sources that control pests or provide other crop protection uses as defined below, but have non-toxic mode of actions (e.g. growth or developmental regulation, attractants, repellents or defence activators (e.g. induced resistance) and are relatively non-toxic to mammals.

The following list of biopesticides, in conjunction with which the compounds of the invention can be used, illustrates the possible combinations:
L) Biopesticides
L1) Microbial pesticides with fungicidal, bactericidal, viricidal and/or plant defense activator activity: *Ampelomyces quisqualis*, *Aspergillus flavus*, *Aureobasidium pullulans*, *Bacillus altitudinis, B. amyloliquefaciens*, *B. amyloliquefaciens* ssp. *plantarum* (also referred to as B. *velezensis), B. megaterium, B. mojavensis, B. mycoides, B. pumilus, B. simplex, B. solisalsi, B. subtilis*, *B. subtilis* var. *amyloliquefaciens*, *B. velezensis*, *Candida oleophila*, *C. saitoana*, *Clavibacter michiganensis* (bacteriophages), *Coniothyrium minitans*, *Cryphonectria parasitica*, *Cryptococcus albidus*, *Dilophosphora alopecuri*, *Fusarium oxysporum*, *Clonostachys rosea* f. *catenulate* (also named *Gliocladium catenulatum*), *Gliocladium roseum*, *Lysobacter antibioticus*, *L. enzymogenes, Metschnikowia fructicola, Microdochium dimerum, Microsphaeropsis ochracea, Muscodor albus, Paenibacillus alvei, Paenibacillus epiphyticus*, *P. polymyxa, Pantoea vagans, Penicillium bilaiae*, *Phlebiopsis gigantea*, *Pseudomonas* sp., *Pseudomonas chloraphis*, *Pseudozyma flocculosa, Pichia anomala, Pythium oligandrum*, *Sphaerodes mycoparasitica, Streptomyces griseoviridis, S. Iydicus*, *S. violaceusniger, Talaromyces flavus, Trichoderma asperelloides, T. asperellum, T. atroviride, T. fertile, T. gamsii, T. harmatum, T. harzianum, T. polysporum, T. stromaticum, T. virens, T. viride, Typhula phacorrhiza, Ulocladium oudemansii, Verticillium dahliae*, zucchini yellow mosaic virus (avirulent strain);
L2) Biochemical pesticides with fungicidal, bactericidal, viricidal and/or plant defense activator activity: harpin protein, plant oils (BM3): tea tree oil, orange oil (L.2.1), eugenol, limonene (L.2.2), geraniol (L.2.3), thymol (L.2.4); Reynoutria sachalinensis extract, aureobasidin (in particular aureobasidin A (L.2.5)), ambruticin (L.2.6), bafilomycin (L.2.7) (in particular bafilomycin A1, B1 and C1), chlorflavonin (L.2.8), cinnamaldehyde (L.2.9), natamycin (L.2.10; F8);
L3) Microbial pesticides with insecticidal, acaricidal, molluscidal and/or nematicidal activity: *Agrobacterium radiobacter*, *Bacillus cereus*, *B. firmus*, *B. thuringiensis*, *B. thuringiensis* ssp. *aizawai*, *B. t.* ssp. *israelensis*, *B. t.* ssp. *galleriae*, *B. t.* ssp. *kurstaki*, *B. t.* ssp. *tenebrionis*, *Beauveria bassiana*, *B*. *brongniartii*, *Burkholderia* spp., *Chromobacterium subtsugae*, *Cydia pomonella* granulovirus (CpGV), *Cryptophlebia leucotreta* granulovirus (CrleGV), *Flavobacterium* spp., *Helicoverpa armigera* nucleopolyhedrovirus (HearNPV), *Helicoverpa zea* nucleopolyhedrovirus (HzNPV), *Helicoverpa* zea single capsid nucleopolyhedrovirus (HzSNPV), *Heterorhabditis bacteriophora*, *Isaria fumosorosea*, *Lecanicillium longisporum, L. muscarium*, *Metarhizium anisopliae, M. anisopliae var. anisopliae*, *M. anisopliae var. acridum*, *Nomuraea rileyi*, *Paecilomyces fumosoroseus, P. lilacinus*, *Paenibacillus popilliae*, *Pasteuria* spp., *P*. *nishizawae*, *P. penetrans*, *P*. *ramosa*, *P*. *thornea*, *P. usgae*, *Pseudomonas fluorescens*, *Spodoptera littoralis* nucleopolyhedrovirus (SpliNPV), *Steinernema carpocapsae*, *S. feltiae*, *S. kraussei, Streptomyces galbus, S. microflavus;*
L4) Biochemical pesticides with insecticidal, acaricidal, molluscidal, pheromone and/or nematicidal activity: L-carvone, citral, (*E*,*Z*)-7,9-dodecadien-1-yl acetate, ethyl formate, (*E*,*Z*)-2,4-ethyl decadienoate (pear ester), (*Z*,*Z*,*E*)-7,11,13-hexadecatrienal, heptyl butyrate, isopropyl myristate, lavanulyl senecioate, cis-jasmone, 2-methyl-1-butanol, methyl eugenol, methyl jasmonate, (*E*,*Z*)-2,13-octadecadien-1-ol, (*E*,*Z*)-2,13-octadecadien-1-ol acetate, (*E*,*Z*)-3,13-octadecadien-1-ol, (*R*)-1-octen-3-ol, pentatermanone, (*E*,*Z*,*Z*)-3,8,11-tetradecatrienyl acetate, (*Z*,*E*)-9, 12- tetradecadien-1-yl acetate, (*Z*)-7-tetradecen-2-one, (*Z*)-9-tetradecen-1-yl acetate, (*Z*)-11-tetradecenal, (*Z*)-11-tetradecen-1-ol, extract of *Chenopodium ambrosiodes*, Neem oil, Quillay extract;
L5) Microbial pesticides with plant stress reducing, plant growth regulator, plant growth promoting and/or yield enhancing activity: *Azospirillum amazonense*, *A. brasilense*, *A. lipoferum*, *A. irakense*, *A. halopraeferens*, *Bradyrhizobium* spp., *B. elkanii*, *B. japonicum*, *B. liaoningense*, *B. lupini*, *Delftia acidovorans*, *Glomus intraradices*, *Mesorhizobium* spp., *Rhizobium leguminosarum* bv. *phaseoli*, *R. I. bv. trifolii*, *R. I.* bv. *viciae*, *R. tropici, Sinorhizobium meliloti.* The biopesticides from group L1) and/or L2) may also have insecticidal, acaricidal, molluscidal, pheromone, nematicidal, plant stress reducing, plant growth regulator, plant growth promoting and/or yield enhancing activity. The biopesticides from group L3) and/or L4) may also have fungicidal, bactericidal, viricidal, plant defense activator, plant stress reducing, plant growth regulator, plant growth promoting and/or yield enhancing activity. The biopesticides from group L5) may also have fungicidal, bactericidal, viricidal, plant defense activator, insecticidal, acaricidal, molluscidal, pheromone and/or nematicidal activity.

Many of these biopesticides have been deposited under deposition numbers mentioned herein (the prefices e.g. ATCC or DSM refer to the acronym of the respective culture collection, for details see e.g. here: http://www. wfcc.info/ccinfo/collection/by_acronym/), are referred to in literature, registered and/or are commercially available: mixtures of Aureobasidium pullulans DSM 14940 and DSM 14941 isolated in 1989 in Konstanz, Germany (e.g. blastospores in BlossomProtect^{®} from bio-ferm GmbH, Austria), Azospirillum brasilense Sp245 originally isolated in wheat reagion of South Brazil (Passo Fundo) at least prior to 1980 (BR 11005; e.g. GELFIX^{®} Gramineas from BASF Agricultural Specialties Ltd., Brazil), A. brasilense strains Ab-V5 and Ab-V6 (e.g. in AzoMax from Novozymes BioAg Produtos papra Agricultura Ltda., Quattro Barras, Brazil or Simbiose-Maíz^{®} from Simbiose-Agro, Brazil; Plant Soil 331, 413-425, 2010), Bacillus amyloliquefaciens strain AP-188 (NRRL B-50615 and B-50331; US8,445,255); B. amylo-liquefaciens ssp. plantarum strains formerly also sometimes referred to as B. subtilis, recently together with B. methylotrophicus, and B. velezensis classified as B. velezensis (Int. J. Syst. Evol. Microbiol. 66, 1212-1217, 2016): B. a. ssp. plantarum or B. velezensis D747 isolated from air in Kikugawashi, Japan (US 20130236522 A1; FERM BP 8234; e.g. Double Nickel^{™} 55 WDG from Certis LLC, USA), B. a. ssp. plantarum or B. velezensis FZB24 isolated from soil in Brandenburg, Germany (also called SB3615; DSM 96-2; J. Plant Dis. Prot. 105, 181-197, 1998; e.g. Taegro^{®} from Novozyme Biologicals, Inc., USA), B. a. ssp. plantarum or B. velezensis FZB42 isolated from soil in Brandenburg, Germany (DSM 23117; J. Plant Dis. Prot. 105, 181-197, 1998; e.g. RhizoVital^{®} 42 from AbiTEP GmbH, Germany), B. a. ssp. plantarum or B. vele-zensis MBI600 isolated from faba bean in Sutton Bonington, Nottinghamshire, U.K. at least before 1988 (also called 1430; NRRL B 50595; US 2012/0149571 A1; e.g. Integral^{®} from BASF Corp., USA), B. a. ssp. plantarum or B. velezensis QST-713 isolated from peach orchard in 1995 in California, U.S.A. (NRRL B 21661; e.g. Serenade^{®} MAX from Bayer Crop Science LP, USA), B. a. ssp. plantarum or B. velezensis TJ1000 isolated in 1992 in South Dakoda, U.S.A. (also called 1BE; ATCC BAA-390; CA 2471555 A1; e.g. QuickRoots^{™} from TJ Technologies, Watertown, SD, USA); B. firmus CNCM I-1582, a variant of parental strain EIP-N1 (CNCM I-1556) isolated from soil of central plain area of Israel (WO 2009/126473, US6,406,690; e.g. Votivo^{®} from Bayer CropScience LP, USA), B. pumilus GHA 180 isolated from apple tree rhizo-sphere in Mexico (IDAC 260707-01; e.g. PRO-MIX^{®} BX from Premier Horticulture, Quebec, Canada), B. pumilus INR-7 otherwise referred to as BU F22 and BU-F33 isolated at least be-fore 1993 from cucumber infested by Erwinia tracheiphila (NRRL B-50185, NRRL B-50153; US 8,445,255), B. pumilus KFP9F isolated from the rhizosphere of grasses in South Africa at least before 2008 (NRRL B-50754; WO 2014/029697; e.g. BAC-UP or FUSION-P from BASF Agricultural Specialities (Pty) Ltd., South Africa), B. pumilus QST 2808 was isolated from soil collected in Pohnpei, Federated States of Micronesia, in 1998 (NRRL B 30087; e.g. Sonata^{®} or Ballad^{®} Plus from Bayer Crop Science LP, USA), B. simplex ABU 288 (NRRL B-50304; US8,445,255), B. subtilis FB17 also called UD 1022 or UD10-22 isolated from red beet roots in North America (ATCC PTA-11857; System. Appl. Microbiol. 27, 372-379, 2004; US2010/0260735; WO 2011/109395); B. thuringiensis ssp. aizawai ABTS-1857 isolated from soil taken from a lawn in Ephraim, Wisconsin, U.S.A., in 1987 (also called ABG 6346; ATCC SD-1372; e.g. XenTari^{®} from BioFa AG, Münsingen, Germany), B. t. ssp. kurstaki ABTS-351 identical to HD-1 isolated in 1967 from diseased Pink Bollworm black larvae in Brownsville, Texas, U.S.A. (ATCC SD-1275; e.g. Dipel^{®} DF from Valent BioSciences, IL, USA), B. t. ssp. kurstaki SB4 isolated from E. saccharina larval cadavers (NRRL B-50753; e.g. Beta Pro^{®} from BASF Agricultural Specialities (Pty) Ltd., South Africa), B. t. ssp. tenebrionis NB-176-1, a mutant of strain NB-125, a wild type strain isolated in 1982 from a dead pupa of the beetle Tenebrio molitor (DSM 5480; EP 585 215 B1; e.g. Novodor^{®} from Valent BioSciences, Switzerland), Beauveria bassiana GHA (ATCC 74250; e.g. BotaniGard^{®} 22WGP from Laverlam Int. Corp., USA), B. bassiana JW-1 (ATCC 74040; e.g. Naturalis^{®} from CBC (Europe) S.r.l., Italy), B. bassiana PPRI 5339 isolated from the larva of the tortoise beetle Conchyloctenia punctata (NRRL 50757; e.g. BroadBand^{®} from BASF Agricultural Specialities (Pty) Ltd., South Africa), Bradyrhizobium elkanii strains SEMIA 5019 (also called 29W) isolated in Rio de Janeiro, Brazil and SEMIA 587 isolated in 1967 in the State of Rio Grande do Sul, from an area previously inoculated with a North American isolate, and used in commercial inoculants since 1968 (Appl. Environ. Microbiol. 73(8), 2635, 2007; e.g. GELFIX 5 from BASF Agricultural Specialties Ltd., Brazil), B. japonicum 532c isolated from Wisconsin field in U.S.A. (Nitragin 61A152; Can. J. Plant. Sci. 70, 661-666, 1990; e.g. in Rhizoflo^{®}, Histick^{®}, Hicoat^{®} Super from BASF Agricultural Specialties Ltd., Canada), B. japonicum E-109 variant of strain USDA 138 (INTA E109, SEMIA 5085; Eur. J. Soil Biol. 45, 28-35, 2009; Biol. Fertil. Soils 47, 81-89, 2011); B. japonicum strains deposited at SEMIA known from Appl. Environ. Microbiol. 73(8), 2635, 2007: SEMIA 5079 isolated from soil in Cerrados region, Brazil by Embrapa-Cerrados used in commercial inoculants since 1992 (CPAC 15; e.g. GELFIX 5 or ADHERE 60 from BASF Agricultural Specialties Ltd., Brazil), B. japonicum SEMIA 5080 obtained under lab condtions by Embrapa-Cerrados in Brazil and used in commercial inoculants since 1992, being a natural variant of SEMIA 586 (CB1809) originally isolated in U.S.A. (CPAC 7; e.g. GELFIX 5 or ADHERE 60 from BASF Agricultural Specialties Ltd., Brazil); Burkholderia sp. A396 isolated from soil in Nikko, Japan, in 2008 (NRRL B-50319; WO 2013/032693; Marrone Bio Innovations, Inc., USA), Coniothyrium minitans CON/M/91-08 isolated from oilseed rape (WO 1996/021358; DSM 9660; e.g. Contans^{®} WG, Intercept^{®} WG from Bayer CropScience AG, Germany), harpin (alpha-beta) protein (Science 257, 85-88, 1992; e.g. Messenger^{™} or HARP-N Tek from Plant Health Care plc, U.K.), Helicoverpa armigera nucleopolyhedrovirus (HearNPV) (J. Invertebrate Pathol. 107, 112-126, 2011; e.g. Helicovex^{®} from Adermatt Biocontrol, Switzerland; Diplomata^{®} from Koppert, Brazil; Vivus^{®} Max from AgBiTech Pty Ltd., Queensland, Australia), Helicoverpa zea single capsid nucleopolyhedrovirus (HzSNPV) (e.g. Gemstar^{®} from Certis LLC, USA), Helicoverpa zea nucleopolyhedrovirus ABA-NPV-U (e.g. Heligen^{®} from AgBiTech Pty Ltd., Queensland, Australia), Heterorhabditis bacteriophora (e.g. Nemasys^{®} G from BASF Agricultural Specialities Limited, UK), Isaria fumosorosea Apopka-97 isolated from mealy bug on gynura in Apopka, Florida, U.S.A. (ATCC 20874; Biocontrol Science Technol. 22(7), 747-761, 2012; e.g. PFR-97^{™} or PreFeRal^{®} from Certis LLC, USA), Metarhizium anisopliae var. anisopliae F52 also called 275 or V275 isolated from codling moth in Austria (DSM 3884, ATCC 90448; e.g. Met52^{®} Novozymes Biologicals BioAg Group, Canada), Metschnikowia fructicola 277 isolated from grapes in the central part of Israel (US 6,994,849; NRRL Y-30752; e.g. formerly Shemer^{®} from Agrogreen, Israel), Paecilomyces ilacinus 251 isolated from infected nematode eggs in the Philippines (AGAL 89/030550; WO1991/02051; Crop Protection 27, 352-361, 2008; e.g. BioAct^{®}from Bayer CropScience AG, Germany and MeloCon^{®} from Certis, USA), Paenibacillus alvei NAS6G6 isolated from the rhizosphere of grasses in South Africa at least before 2008 (WO 2014/029697; NRRL B-50755; e.g. BAC-UP from BASF Agricultural Specialities (Pty) Ltd., South Africa), Paenibacillus strains isolated from soil samples from a variety of European locations including Germany: P. epiphyticus Lu17015 (WO 2016/020371; DSM 26971), P. polymyxa ssp. plantarum Lu16774 (WO 2016/020371; DSM 26969), P. p. ssp. plantarum strain Lu17007 (WO 2016/020371; DSM 26970); Pasteuria nishizawae Pn1 isolated from a soybean field in the mid-2000s in Illinois, U.S.A. (ATCC SD 5833; Federal Register 76(22), 5808, February 2, 2011; e.g. Clariva^{™} PN from Syngenta Crop Protection, LLC, USA), Penicillium bilaiae (also called P. bilaii) strains ATCC 18309 (= ATCC 74319), ATCC 20851 and/or ATCC 22348 (=ATCC 74318) originally isolated from soil in Alberta, Canada (Fertilizer Res. 39, 97-103, 1994; Can. J. Plant Sci. 78(1), 91-102, 1998; US 5,026,417, WO 1995/017806; e.g. Jump Start^{®}, Provide^{®} from Novozymes Biologicals BioAg Group, Canada), Reynoutria sachalinensis extract (EP 0307510 B1; e.g. Regalia^{®} SC from Marrone Biolnnovations, Davis, CA, USA or Milsana^{®} from BioFa AG, Germany), Steinernema carpocapsae (e.g. Millenium^{®} from BASF Agricultural Specialities Limited, UK), S. feltiae (e.g. Nemashield^{®} from BioWorks, Inc., USA; Nemasys^{®} from BASF Agricultural Specialities Limited, UK), Streptomyces microflavus NRRL B-50550 (WO 2014/124369; Bayer CropScience, Germany), Trichoderma asperelloides JM41R isolated in South Africa (NRRL 50759; also referred to as T. fertile; e.g. Trichoplus^{®} from BASF Agricultural Specialities (Pty) Ltd., South Africa), T. harzianum T-22 also called KRL-AG2 (ATCC 20847; BioControl 57, 687-696, 2012; e.g. Plantshield^{®} from BioWorks Inc., USA or SabrEx^{™} from Advanced Biological Marketing Inc., Van Wert, OH, USA).

According to the invention, the solid material (dry matter) of the biopesticides (with the ex-ception of oils e.g. Neem oil) are considered as active components (e.g. to be obtained after drying or evaporation of the extraction or suspension medium in case of liquid formulations of the microbial pesticides).

In accordance with the invention, the weight ratios and percentages used herein for a biological extract e.g. Quillay extract are based on the total weight of the dry content (solid material) of the respective extract(s).

The total weight ratios of compositions comprising at least one microbial pesticide in the form of viable microbial cells including dormant forms, can be determined using the amount of CFU of the respective microorganism to calculate the total weight of the respective active component with the following equation that 1x10¹⁰ CFU equals one gram of total weight of the respective active component. Colony forming unit is measure of viable microbial cells, in particular fungal and bacterial cells. In addition, here "CFU" may also be understood as the number of (juvenile) individual nematodes in case of (entomopathogenic) nematode biopesticides, e.g. Steinernema feltiae.

When mixtures comprising microbial pesticides are employed in crop protection, the application rates range from 1x10⁶ to 5x10¹⁶ (or more) CFU/ha, preferably from 1x10⁸ to 1x10¹³ CFU/ha, and even more preferably from 1x10⁹ to 5x10¹⁵ CFU/ha and in particular from 1x10¹² to 5x10¹⁴ CFU/ha. In the case of nematodes as microbial pesticides (e.g. Steinernema feltiae), the application rates regularly range from 1x10⁶ to 1x10¹² (or more), preferably from 1x10⁸ to 1x10¹¹, more preferably from 5x10⁸ to 1x10¹⁰ individuals (e.g. in the form of eggs, juvenile or any other live stages, preferably in an infetive juvenile stage) per ha.

When mixtures comprising microbial pesticides are employed in seed treatment, the application rates generally range from 1x10⁶ to 1x10¹² (or more) CFU/seed, preferably from 1x10⁶ to 1x10⁹ CFU/seed. Furthermore, the application rates with respect to seed treatment generally range from 1x10⁷ to 1x10¹⁴ (or more) CFU per 100 kg of seed, preferably from 1x10⁹ to 1x10¹² CFU per 100 kg of seed.

### Formulations

The invention also relates to agrochemical compositions comprising an auxiliary and at least one compound of the invention or a mixture thereof.

An agrochemical composition comprises a pesticidally effective amount of a compound of the invention or a mixture thereof.

The compounds of the invention or the mixtures thereof can be converted into customary types of agro-chemical compositions, e.g. solutions, emulsions, suspensions, dusts, powders, pastes, granules, pressings, capsules, and mixtures thereof. Examples for composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials e.g. seeds (e.g. GF). These and further compositions types are defined in the "Catalogue of pesticide formulation types and international coding system", Technical Monograph No. 2, 6th Ed. May 2008, CropLife International.

The compositions are prepared in a known manner, e.g. described by Mollet and Grubemann, Formulation technology, Wiley VCH, Weinheim, 2001; or Knowles, New developments in crop protection product formulation, Agrow Reports DS243, T&F Informa, London, 2005.

Examples for suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, e.g. mineral oil fractions of medium to high boiling point, e.g. kerosene, diesel oil; oils of vegetable or animal origin; hydrocarbons, e.g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, CaSO₄, MgSO₄, MgO; polysaccharide powders, e.g. cellulose, starch; fertilizers, e.g. (NH₄)₂SO₄, (NH₄)₃PO₄, NH₄NO₃, ureas; products of vegetable origin, e.g. cereal meal, tree bark meal, wood meal, nutshell meal, and mixtures thereof.

Suitable surfactants are surface-active compounds, e.g. anionic, cationic, nonionic and amphoteric surfactants, block polymers, polyelectrolytes, and mixtures thereof. Such surfactants can be used as emusifier, dispersant, solubilizer, wetter, penetration enhancer, protective col-loid, or adjuvant. Examples of surfactants are listed in McCutcheon's, Vol.1: Emulsifiers & Detergents, McCutcheon's Directories, Glen Rock, USA, 2008 (International or North American Ed.). Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates. Suitable nonionic surfactants are alkoxylates, N-subsituted fatty acid amides, amine oxides, esters, sugar-based surfactants, polymeric surfactants, and mixtures thereof. Examples of alkoxylates are compounds e.g. alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-subsititued fatty acid amides are fatty acid glucamides or fatty acid alkanola-mides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are homo- or copolymers of vinylpyrrolidone, vinylalcohols, or vinylacetate.

Suitable cationic surfactants are quaternary surfactants, e.g. quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines, or polyethyleneamines.

Suitable adjuvants are compounds, which have a neglectable or even no pesticidal activity themselves, and which improve the biological performance of the compounds of the invention on the target. Examples are surfactants, mineral or vegetable oils, and other auxilaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), inorganic clays (organically modified or unmodified), polycarboxylates, and silicates.

Suitable bactericides are bronopol and isothiazolinone derivatives e.g. alkylisothiazolinones and benzisothiazolinones.

Suitable anti-freezing agents are ethylene glycol, propylene glycol, urea, and glycerin.

Suitable anti-foaming agents are silicones, long chain alcohols, and salts of fatty acids. Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo-, and phthalocyanine colorants). Suitable tackifiers or binders are polyvinylpyrrolidons, polyvinylacetates, polyvinyl alcohols, polyacrylates, biological or synthetic waxes, and cellulose ethers.

Examples for composition types and their preparation are:
i) Water-soluble concentrates (SL, LS)
   10-60 wt% of a compound I according to the invention and 5-15 wt% wetting agent (e.g. alcohol alkoxylates) are dissolved in water and/or in a water-soluble solvent (e.g. alcohols) up to 100 wt%. The active substance dissolves upon dilution with water.
ii) Dispersible concentrates (DC)
   5-25 wt% of a compound I according to the invention and 1-10 wt% dispersant (e.g. polyvinylpyrrolidone) are dissolved in up to 100 wt% organic solvent (e.g. cyclohexanone). Dilution with water gives a dispersion.
iii) Emulsifiable concentrates (EC) 15-70 wt% of a compound I according to the invention and 5-10 wt% emulsifiers (e.g. calcium dodecylbenzenesulfonate and castor oil ethoxylate) are dissolved in up to 100 wt% water-insoluble organic solvent (e.g. aromatic hydrocarbon). Dilution with water gives an emulsion.
iv) Emulsions (EW, EO, ES)
   5-40 wt% of a compound I according to the invention and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This mixture is introduced into up to 100 wt% water by means of an emulsifying machine and made into a homogeneous emulsion. Dilution with water gives an emulsion.
v) Suspensions (SC, OD, FS)
   In an agitated ball mill, 20-60 wt% of a compound I according to the invention are comminuted with addition of 2-10 wt% dispersants and wetting agents (e.g. sodium lignosulfonate and alcohol ethoxylate), 0,1-2 wt% thickener (e.g. xanthan gum) and up to 100 wt% water to give a fine active substance suspension. Dilution with water gives a stable suspension of the active sub-stance. For FS type composition up to 40 wt% binder (e.g. polyvinylalcohol) is added.
vi) Water-dispersible granules and water-soluble granules (WG, SG)
   50-80 wt% of a compound I according to the invention are ground finely with addition of up to 100 wt% dispersants and wetting agents (e.g. sodium lignosulfonate and alcohol ethoxylate) and prepared as water-dispersible or water-soluble granules by means of technical appliances (e.g. extrusion, spray tower, fluidized bed). Dilution with water gives a stable dispersion or solution of the active substance.
vii) Water-dispersible powders and water-soluble powders (WP, SP, WS)
   50-80 wt% of a compound I according to the invention are ground in a rotor-stator mill with addition of 1-5 wt% dispersants (e.g. sodium lignosulfonate), 1-3 wt% wetting agents (e.g. alcohol ethoxylate) and up to 100 wt% solid carrier, e.g. silica gel. Dilution with water gives a stable dispersion or solution of the active substance.
viii) Gel (GW, GF)
   In an agitated ball mill, 5-25 wt% of a compound I according to the invention are comminuted with addition of 3-10 wt% dispersants (e.g. sodium lignosulfonate), 1-5 wt% thickener (e.g. carboxymethylcellulose) and up to 100 wt% water to give a fine suspension of the active substance. Dilution with water gives a stable suspension of the active substance.
ix) Microemulsion (ME)
   5-20 wt% of a compound I according to the invention are added to 5-30 wt% organic solvent blend (e.g. fatty acid dimethylamide and cyclohexanone), 10-25 wt% surfactant blend (e.g. alcohol ethoxylate and arylphenol ethoxylate), and water up to 100 %. This mixture is stirred for 1 h to produce spontaneously a thermodynamically stable microemulsion.
x) Microcapsules (CS)
   An oil phase comprising 5-50 wt% of a compound I according to the invention, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 wt% of a compound I according to the invention, 0-40 wt% water insoluble organic solvent (e.g. aromatic hydrocarbon), and an isocyanate monomer (e.g. di-phenylmethene-4,4'-diisocyanatae) are dispersed into an aqueous solution of a protective colloid (e.g. polyvinyl alcohol). The addition of a polyamine (e.g. hexamethylenediamine) results in the foration of a polyurea microcapsule. The monomers amount to 1-10 wt%. The wt% relate to the total CS composition.
xi) Dustable powders (DP, DS)
   1-10 wt% of a compound I according to the invention are ground finely and mixed intimately with up to 100 wt% solid carrier, e.g. finely divided kaolin.
xii) Granules (GR, FG)
   0.5-30 wt% of a compound I according to the invention is ground finely and associated with up to 100 wt% solid carrier (e.g. silicate). Granulation is achieved by extrusion, spray-drying or the fluidized bed.
xiii) Ultra-low volume liquids (UL)
   1-50 wt% of a compound I according to the invention are dissolved in up to 100 wt% organic solvent, e.g. aromatic hydrocarbon.

The compositions types i) to xi) may optionally comprise further auxiliaries, e.g. 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95%, preferably be-tween 0.1 and 90%, and most preferably between 0.5 and 75%, by weight of active substance. The active substances are employed in a purity of from 90% to 100%, preferably from 95% to 100% (according to NMR spectrum).

Various types of oils, wetters, adjuvants, fertilizer, or micronutrients, and other pesticides (e.g. herbicides, insecticides, fungicides, growth regulators, safeners) may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

The user applies the composition according to the invention usually from a predosage device, a knapsack sprayer, a spray tank, a spray plane, or an irrigation system. Usually, the agro-chemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition of the invention e.g. parts of a kit or parts of a binary or ternary mixture may be mixed by the user himself in a spray tank and further auxiliaries may be added, if appropriate.

In a further embodiment, either individual components of the composition according to the invention or partially premixed components, e.g. components comprising compounds of the invention and/or mixing partners as defined above, may be mixed by the user in a spray tank and further auxiliaries and additives may be added.

In a further embodiment, either individual components of the composition according to the invention or partially premixed components, e.g. components comprising compounds of the invention and/or mixing partners as defined above, can be applied jointly (e.g. after tank mix) or consecutively.

### Application methods

The compounds of the invention are suitable for use in protecting crops, plants, plant propagation materials, e.g. seeds, or soil or water, in which the plants are growing, from attack or infestation by animal pests. Therefore, the invention also relates to a plant protection method, which comprises contacting crops, plants, plant propagation materials, e.g. seeds, or soil or water, in which the plants are growing, to be protected from attack or infestation by animal pests, with a pesticidally effective amount of a compound of the invention.

The compounds of the invention are also suitable for use in combating or controlling animal pests. Therefore, the invention also relates to a method of combating or controlling animal pests, which comprises contacting the animal pests, their habitat, breeding ground, or food supply, or the crops, plants, plant propagation materials, e.g. seeds, or soil, or the area, material or environment in which the animal pests are growing or may grow, with a pesticidally effective amount of a compound of the invention.

The compounds of the invention are effective through both contact and ingestion. Further-more, the compounds of the invention can be applied to any and all developmental stages, e.g. egg, larva, pupa, and adult.

The compounds of the invention can be applied as such or in form of compositions comprising them as defined above. Furthermore, the compounds of the invention can be applied together with a mixing partner or in form of compositions comprising said mixtures. The components of said mixture can be applied simultaneously, jointly or separately, or in succession, that is immediately one after another and thereby creating the mixture "in situ" on the desired location, e.g. the plant, the sequence, in the case of separate application, generally not having any effect on the result of the control measures.

The application can be carried out both before and after the infestation of the crops, plants, plant propagation materials, e.g. seeds, soil, or the area, material or environment by the pests. Suitable application methods include i.a. soil treatment, seed treatment, in furrow application, and foliar application. Soil treatment methods include drenching the soil, drip irrigation (drip application onto the soil), dipping roots, tubers or bulbs, or soil injection. Seed treatment techniques include seed dressing, seed coating, seed dusting, seed soaking, and seed pelleting. In furrow applications typically include the steps of making a furrow in cultivated land, seeding the furrow with seeds, applying the pesticidally active compound to the furrow, and closing the furrow. Foliar application refers to the application of the pesticidally active compound to plant foliage, e.g. through spray equipment. For foliar applications, it can be advantageous to modify the behavior of the pests by use of pheromones in combination with the compounds of the invention. Suitable pheromones for specific crops and pests are known and publicly available from databases of pheromones and semiochemicals, e.g. http://www.pherobase.com.

As used herein, the term "contacting" includes both direct contact (applying the compounds/compositions directly on the animal pest or plant - typically to the foliage, stem or roots of the plant) and indirect contact (applying the compounds/compositions to the locus, i.e. habitat, breeding ground, plant, seed, soil, area, material, or environment in which a pest is growing or may grow, of the animal pest or plant).

The term "animal pest" includes arthropods, gastropods, and nematodes. Preferred animal pests according to the invention are arthropods, preferably insects and arachnids, in particular insects. Insects, which are of particular relevance for crops, are typically referred to as crop insect pests. The term "crop" refers to both, growing and harvested crops.

The term "plant" includes cereals, e.g. durum and other wheat, rye, barley, triticale, oats, rice, or maize (fodder maize and sugar maize / sweet and field corn); beet, e.g. sugar beet, or fodder beet; fruits, e.g. pomes, stone fruits, or soft fruits, e.g. apples, pears, plums, peaches, nectarines, almonds, cherries, papayas, strawberries, raspberries, blackberries or gooseberries; leguminous plants, e.g. beans, lentils, peas, alfalfa, or soybeans; oil plants, e.g. rapeseed (oilseed rape), turnip rape, mustard, olives, sunflowers, coconut, cocoa beans, castor oil plants, oil palms, ground nuts, or soybeans; cucurbits, e.g. squashes, pumpkins, cucumber or melons; fiber plants, e.g. cotton, flax, hemp, or jute; citrus fruit, e.g. oranges, lemons, grapefruits or mandarins; vegetables, e.g. eggplant, spinach, lettuce (e.g. iceberg lettuce), chicory, cabbage, asparagus, cabbages, carrots, onions, garlic, leeks, tomatoes, potatoes, cucurbits or sweet peppers; lauraceous plants, e.g. avocados, cinnamon, or camphor; energy and raw material plants, e.g. corn, soybean, rapeseed, sugar cane or oil palm; tobacco; nuts, e.g. walnuts; pistachios; coffee; tea; bananas; vines; hop; sweet leaf (Stevia); natural rubber plants or ornamental and forestry plants, shrubs, broad-leaved trees or evergreens, eucalyptus; turf; lawn; grass. Preferred plants include potatoes sugar beets, tobacco, wheat, rye, barley, oats, rice, corn, cotton, soybeans, rapeseed, legumes, sunflowers, coffee, or sugar cane; fruits; vines; ornamentals; or vegetables, e.g. cucumbers, tomatoes, beans or squashes.

The term "cultivated plants" is to be understood as including plants which have been modified by mutagenesis or genetic engineering in order to provide a new trait to a plant or to modify an already present trait.

Mutagenesis includes techniques of random mutagenesis using X-rays or mutagenic chemicals, but also techniques of targeted mutagenesis, in order to create mutations at a specific locus of a plant genome. Targeted mutagenesis techniques frequently use oligonucleotides or proteins like CRISPR/Cas, zinc-finger nucleases, TALENs or meganucleases to achieve the targeting effect. Genetic engineering usually uses recombinant DNA techniques to create modifications in a plant genome which under natural circumstances cannot readily be obtained by cross breeding, mutagenesis or natural recombination. Typically, one or more genes are integrated into the genome of a plant in order to add a trait or improve a trait. These integrated genes are also referred to as transgenes in the art, while plant comprising such transgenes are referred to as transgenic plants. The process of plant transformation usually produces several transformation events, which differ in the genomic locus in which a transgene has been integrated. Plants comprising a specific transgene on a specific genomic locus are usually described as comprising a specific "event", which is referred to by a specific event name. Traits which have been introduced in plants or have been modified include in particular herbicide tolerance, insect resistance, increased yield and tolerance to abiotic conditions, like drought.

Herbicide tolerance has been created by using mutagenesis as well as using genetic engineering. Plants which have been rendered tolerant to ALS inhibitor herbicides by conventional methods of mutagenesis and breeding comprise plant varieties commercially available under the name Clearfield^{®}.

Herbicide tolerance has been created to glyphosate, glufosinate, 2,4-D, dicamba, oxynil herbicides, like bromoxynil and ioxynil, sulfonylurea herbicides, ALS inhibitor herbicides and HPPD inhibitors, like isoxaflutole and mesotrione.

Transgenes which have been used to provide herbicide tolerance traits comprise: for tolerance to glyphosate: cp4 epsps, epsps grg23ace5, mepsps, 2mepsps, gat4601, gat4621 and goxv247, for tolerance to glufosinate: pat and bar, for tolerance to 2,4-D: aad-1 and aad-12, for tolerance to dicamba: dmo, for tolerance to oxynil herbicies: bxn, for tolerance to sulfonylurea herbicides: zm-hra, csr1-2, gm-hra, S4-HrA, for tolerance to ALS inhibitor herbicides: csr1-2, for tolerance to HPPD inhibitor herbicides: hppdPF, W336 and avhppd-03.

Transgenic corn events comprising herbicide tolerance genes are e.g., but not excluding others, DAS40278, MON801, MON802, MON809, MON810, MON832, MON87411, MON87419, MON87427, MON88017, MON89034, NK603, GA21, MZHG0JG, HCEM485, VCO-Ø1981-5, 676, 678, 680, 33121, 4114, 59122, 98140, Bt10, Bt176, CBH-351, DBT418, DLL25, MS3, MS6, MZIR098, T25, TC1507 and TC6275.

Transgenic soybean events comprising herbicide tolerance genes are e.g., but not excluding others, GTS 40-3-2, MON87705, MON87708, MON87712, MON87769, MON89788, A2704-12, A2704-21, A5547-127, A5547-35, DP356043, DAS44406-6, DAS68416-4, DAS-81419-2, GU262, SYHTØH2, W62, W98, FG72 and CV127.

Transgenic cotton events comprising herbicide tolerance genes are e.g., but not excluding others, 19-51a, 31707, 42317, 81910, 281-24-236, 3006-210-23, BXN10211, BXN10215, BXN10222, BXN10224, MON1445, MON1698, MON88701, MON88913, GHB119, GHB614, LLCotton25, T303-3 and T304-40.

Transgenic canola events comprising herbicide tolerance genes are e.g., but not excluding others, MON88302, HCR-1, HCN10, HCN28, HCN92, MS1, MS8, PHY14, PHY23, PHY35, PHY36, RF1, RF2 and RF3.

Insect resistance has mainly been created by transferring bacterial genes for insecticidal pro-teins to plants. Transgenes which have most frequently been used are toxin genes of Bacillus spec. and synthetic variants thereof, like cry1A, cry1Ab, cry1Ab-Ac, cry1Ac, cry1A.105, cry1F, cry1Fa2, cry2Ab2, cry2Ae, mcry3A, ecry3.1Ab, cry3Bb1, cry34Ab1, cry35Ab1, cry9C, vip3A(a), vip3Aa20. However, also genes of plant origin have been transferred to other plants. In particular genes coding for protease inhibitors, like CpTI and pinll. A further approach uses transgenes in order to produce double stranded RNA in plants to target and downregulate in-sect genes. An example for such a transgene is dvsnf7.

Transgenic corn events comprising genes for insecticidal proteins or double stranded RNA are e.g., but not excluding others, Bt10, Bt11, Bt176, MON801, MON802, MON809, MON810, MON863, MON87411, MON88017, MON89034, 33121, 4114, 5307, 59122, TC1507, TC6275, CBH-351, MIR162, DBT418 and MZIR098.

Transgenic soybean events comprising genes for insecticidal proteins are e.g., but not excluding others, MON87701, MON87751 and DAS-81419.

Transgenic cotton events comprising genes for insecticidal proteins are e.g., but not excluding others, SGK321, MON531, MON757, MON1076, MON15985, 31707, 31803, 31807, 31808, 42317, BNLA-601, Event1, COT67B, COT102, T303-3, T304-40, GFM Cry1A, GK12, MLS 9124, 281-24-236, 3006-210-23, GHB119 and SGK321.

Increased yield has been created by increasing ear biomass using the transgene athb17, be-ing present in corn event MON87403, or by enhancing photosynthesis using the transgene bbx32, being present in the soybean event MON87712.

Cultivated plants comprising a modified oil content have been created by using the transgenes: gm-fad2-1, Pj.D6D, Nc.Fad3, fad2-1A and fatb1-A. Soybean events comprising at least one of these genes are: 260-05, MON87705 and MON87769.

Tolerance to abiotic conditions, in particular to tolerance to drought, has been created by using the transgene cspB, comprised by the corn event MON87460 and by using the transgene Hahb-4, comprised by soybean event IND-ØØ41Ø-5.

Traits are frequently combined by combining genes in a transformation event or by combining different events during the breeding process. Preferred combination of traits are herbicide tolerance to different groups of herbicides, insect tolerance to different kind of insects, in particular tolerance to lepidopteran and coleopteran insects, herbicide tolerance with one or several types of insect resistance, herbicide tolerance with increased yield as well as a combination of herbicide tolerance and tolerance to abiotic conditions.

Plants comprising singular or stacked traits as well as the genes and events providing these traits are known (http://www.isaaa.org/gmapprovaldatabase) and (http://cera-gmc.org/GMCropDatabase).

Further information on specific events and methods to detect them can be found for canola events MS1, MS8, RF3, GT73, MON88302, KK179 in WO01/031042, WO01/041558, WO01/041558, WO02/036831, WO11/153186, WO13/003558, for cotton events MON1445, MON15985, MON531(MON15985), LLCotton25, MON88913, COT102, 281-24-236, 3006-210-23, COT67B, GHB614, T304-40, GHB119, MON88701, 81910 in WO02/034946, WO02/100163, WO02/100163, WO03/013224, WO04/072235, WO04/039986, WO05/103266, WO05/103266, WO06/128573, WO07/017186, WO08/122406, WO08/151780, WO12/134808, WO13/112527, for corn events GA21, MON810, DLL25, TC1507, MON863, MIR604, LY038, MON88017, 3272, 59122, NK603, MIR162, MON89034, 98140, 32138, MON87460, 5307, 4114, MON87427, DAS40278, MON87411, 33121, MON87403, MON87419 in WO98/044140, US02/102582, US03/126634, WO04/099447, WO04/011601, WO05/103301, WO05/061720, WO05/059103, WO06/098952, WO06/039376, US2007/292854, WO07/142840, WO07/140256, WO08/112019, WO09/103049, WO09/111263, WO10/077816, WO11/084621, WO11/062904, WO11/022469, WO13/169923, WO14/116854, WO15/053998, WO15/142571, for potato events E12, F10, J3, J55, V11, X17, Y9 in WO14/178910, WO14/178913, WO14/178941, WO14/179276, WO16/183445, WO17/062831, WO17/062825, for rice events LLRICE06, LLRICE601, LLRICE62 in WO00/026345, WO00/026356, WO00/026345 for soybean events H7-1, MON89788, A2704-12, A5547-127, DP305423, DP356043, MON87701, MON87769, CV127, MON87705, DAS68416-4, MON87708, MON87712, SYHT0H2, DAS81419, DAS81419 x DAS44406-6, MON87751 in WO04/074492, WO06/130436, WO06/108674, WO06/108675, WO08/054747, WO08/002872, WO09/064652, WO09/102873, WO10/080829, WO10/037016, WO11/066384, WO11/034704, WO12/051199, WO12/082548, WO13/016527, WO13/016516, WO14/201235.

The use of compositions according to the invention on cultivated plants may result in effects which are specific to a cultivated plant comprising a certain gene or event. These effects may comprise enhanced yield, enhanced resistance or tolerance to insects, nematodes, fungal, bacterial, mycoplasma, viral or viroid pathogens as well as early vigor, early or delayed ripening, cold or heat tolerance as well as changed amino acid or fatty acid spectrum or content.

It has been found that the pesticidal activity of the compounds of the invention may be enhanced by the insecticidal trait of a modified plant. Furthermore, it has been found that the compounds of the invention are suitable for preventing insects to become resistant to the insecticidal trait or for combating pests, which already have become resistant to the insecticidal trait of a modified plant. Moreover, the compounds of the invention are suitable for combating pests, against which the insecticidal trait is not effective, so that a complementary insecticidal activity can advantageously be used.

The term "plant propagation material" refers to all the generative parts of the plant e.g. seeds and vegetative plant material e.g. cuttings and tubers (e.g. potatoes), which can be used for the multiplication of the plant. This includes seeds, roots, fruits, tubers, bulbs, rhizomes, shoots, sprouts and other parts of plants. Seedlings and young plants, which are to be trans-planted after germination or after emergence from soil, may also be included. These plant propagation materials may be treated prophylactically with a plant protection compound either at or before planting or transplanting.

The term "seed" embraces seeds and plant propagules of all kinds including but not limited to true seeds, seed pieces, suckers, corms, bulbs, fruit, tubers, grains, cuttings, cut shoots and the like, and means in a preferred embodiment true seeds.

In general, "pesticidally effective amount" means the amount of active ingredient needed to achieve an observable effect on growth, including the effects of necrosis, death, retardation, prevention, and removal, destruction, or otherwise diminishing the occurrence and activity of the target organism. The pesticidally effective amount can vary for the various compounds/compositions used in the invention. A pesticidally effective amount of the compositions will also vary according to the prevailing conditions e.g. desired pesticidal effect and duration, weather, target species, locus, mode of application.

In the case of soil treatment, in furrow application or of application to the pests dwelling place or nest, the quantity of active ingredient ranges from 0.0001 to 500 g per 100 m², preferably from 0.001 to 20 g per 100 m².

For use in treating crop plants, e.g. by foliar application, the rate of application of the active ingredients of this invention may be in the range of 0.0001 g to 4000 g per hectare, e.g. from 1 g to 2 kg per hectare or from 1 g to 750 g per hectare, desirably from 1 g to 100 g per hectare, more desirably from 10 g to 50 g per hectare, e.g., 10 to 20 g per hectare, 20 to 30 g per hec-tare, 30 to 40 g per hectare, or 40 to 50 g per hectare.

The compounds of the invention are particularly suitable for use in the treatment of seeds in order to protect the seeds from insect pests, in particular from soil-living insect pests, and the resulting seedling's roots and shoots against soil pests and foliar insects. The invention therefore also relates to a method for the protection of seeds from insects, in particular from soil insects, and of the seedling's roots and shoots from insects, in particular from soil and foliar insects, said method comprising treating the seeds before sowing and/or after pregermination with a compound of the invention. The protection of the seedling's roots and shoots is preferred. More preferred is the protection of seedling's shoots from piercing and sucking insects, chewing insects and nematodes.

The term "seed treatment" comprises e.g. seed dressing, seed coating, seed dusting, seed soaking, seed pelleting, and in-furrow application methods. Preferably, the seed treatment application of the active compound is carried out by spraying or by dusting the seeds before sowing of the plants and before emergence of the plants.

The invention also comprises seeds coated with or containing the active compound. The term "coated with and/or containing" generally signifies that the active ingredient is for the most part on the surface of the propagation product at the time of application, although a greater or lesser part of the ingredient may penetrate into the propagation product, depending on the method of application. When the said propagation product is (re)planted, it may absorb the active ingredient. Suitable seed is e.g. seed of cereals, root crops, oil crops, vegetables, spices, ornamentals, e.g. seed of durum and other wheat, barley, oats, rye, maize (fodder maize and sugar maize / sweet and field corn), soybeans, oil crops, crucifers, cotton, sunflowers, bananas, rice, oilseed rape, turnip rape, sugarbeet, fodder beet, eggplants, potatoes, grass, lawn, turf, fodder grass, tomatoes, leeks, pumpkin/squash, cabbage, iceberg lettuce, pepper, cucumbers, melons, Brassica species, melons, beans, peas, garlic, onions, carrots, tuberous plants e.g. potatoes, sugar cane, tobacco, grapes, petunias, geranium/pelargoniums, pansies and impatiens.

In addition, the active compound may also be used for the treatment of seeds from plants, which have been modified by mutagenisis or genetic engineering, and which e.g. tolerate the action of herbicides or fungicides or insecticides.

Conventional seed treatment formulations include e.g. flowable concentrates FS, solutions LS, suspoemulsions (SE), powders for dry treatment DS, water dispersible powders for slurry treatment WS, water-soluble powders SS and emulsion ES and EC and gel formulation GF. These formulations can be applied to the seed diluted or undiluted. Application to the seeds is carried out before sowing, either directly on the seeds or after having pregerminated the latter. Preferably, the formulations are applied such that germination is not included.

The active substance concentrations in ready-to-use formulations, which may be obtained after two-to-tenfold dilution, are preferably from 0.01 to 60% by weight, more preferably from 0.1 to 40% by weight.

In a preferred embodiment a FS formulation is used for seed treatment. Typically, a FS formulation may comprise 1-800 g/l of active ingredient, 1-200 g/l Surfactant, 0 to 200 g/l anti-freezing agent, 0 to 400 g/l of binder, 0 to 200 g/l of a pigment and up to 1 liter of a solvent, preferably water.

Especially preferred FS formulations of the compounds of the invention for seed treatment usually comprise from 0.1 to 80% by weight (1 to 800 g/l) of the active ingredient, from 0.1 to 20% by weight (1 to 200 g/l) of at least one surfactant, e.g. 0.05 to 5% by weight of a wetter and from 0.5 to 15% by weight of a dispersing agent, up to 20% by weight, e.g. from 5 to 20% of an anti-freeze agent, from 0 to 15% by weight, e.g. 1 to 15% by weight of a pigment and/or a dye, from 0 to 40% by weight, e.g. 1 to 40% by weight of a binder (sticker/adhesion agent), optionally up to 5% by weight, e.g. from 0.1 to 5% by weight of a thickener, optionally from 0.1 to 2% of an anti-foam agent, and optionally a preservative e.g. a biocide, antioxidant or the like, e.g. in an amount from 0.01 to 1% by weight and a filler/vehicle up to 100% by weight.

In the treatment of seed, the application rates of the compounds of the invention are generally from 0.1 g to 10 kg per 100 kg of seed, preferably from 1 g to 5 kg per 100 kg of seed, more preferably from 1 g to 1000 g per 100 kg of seed and in particular from 1 g to 200 g per 100 kg of seed, e.g. from 1 g to 100 g or from 5 g to 100 g per 100 kg of seed.

The invention therefore also relates to seed comprising a compound of the invention, or an agriculturally useful salt thereof, as defined herein. The amount of the compound of the invention or the agriculturally useful salt thereof will in general vary from 0.1 g to 10 kg per 100 kg of seed, preferably from 1 g to 5 kg per 100 kg of seed, in particular from 1 g to 1000 g per 100 kg of seed. For specific crops e.g. lettuce the rate can be higher.

The compounds of the invention may also be used for improving the health of a plant. Therefore, the invention also relates to a method for improving plant health by treating a plant, plant propagation material and/or the locus where the plant is growing or is to grow with an effective and non-phytotoxic amount of a compound of the invention.

As used herein "an effective and non-phytotoxic amount" means that the compound is used in a quantity which allows to obtain the desired effect but which does not give rise to any phytotoxic symptom on the treated plant or on the plant grown from the treated propagule or treated soil.

"Plant health" is defined as a condition of the plant and/or its products which is determined by several aspects alone or in combination with each other e.g. yield (e.g. increased biomass and/or increased content of valuable ingredients), quality (e.g. improved content or composition of certain ingredients or shelf life), plant vigour (e.g. improved plant growth and/or greener leaves ("greening effect"), tolerance to abiotic (e.g. drought) and/or biotic stress (e.g. disease) and production efficiency (e.g., harvesting efficiency, processability).

The above identified indicators for the health condition of a plant may be interdependent and may result from each other. Each indicator is defined in the art and can be determined by methods known to a skilled person.

The compounds of the invention are also suitable for use against non-crop insect pests. For use against said non-crop pests, compounds of the invention can be used as bait composition, gel, general insect spray, aerosol, as ultra-low volume application and bed net (impregnated or surface applied). Furthermore, drenching and rodding methods can be used.

As used herein, the term "non-crop insect pest" refers to pests, which are particularly relevant for non-crop targets, e.g. ants, termites, wasps, flies, ticks, mosquitoes, bed bugs, crickets, or cockroaches.

The bait can be a liquid, a solid or a semisolid preparation (e.g. a gel). The bait employed in the composition is a product, which is sufficiently attractive to incite insects e.g. ants, termites, wasps, flies, mosquitoes, crickets etc. or cockroaches to eat it. The attractiveness can be manipulated by using feeding stimulants or sex pheromones. Food stimulants are preferably chosen from animal and/or plant proteins (meat-, fish- or blood meal, insect parts, egg yolk), from fats and oils of animal and/or plant origin, or mono-, oligo- or polyorganosaccharides, especially from sucrose, lactose, fructose, dextrose, glucose, starch, pectin or even molasses or honey. Fresh or decaying parts of fruits, crops, plants, animals, insects or specific parts thereof can also serve as a feeding stimulant. Sex pheromones are known to be more insect specific. Specific pheromones are known (http://www.pherobase.com).

For use in bait compositions, the typical content of active ingredient is from 0.001 wt% to 15 wt%, desirably from 0.001 wt% to 5 wt% of active compound.

Formulations of the compounds of the invention as aerosols (e.g in spray cans), oil sprays or pump sprays are highly suitable for professional or non-professional users for controlling pests e.g. flies, fleas, ticks, bed bugs, mosquitoes or cockroaches. Aerosol recipes are preferably composed of the active compound, solvents, furthermore auxiliaries e.g. emulsifiers, perfume oils, if appropriate stabilizers, and, if required, propellants.

The oil spray formulations differ from the aerosol recipes in that no propellants are used.

For use in spray compositions, the content of active ingredient is from 0.001 to 80 wt%, preferably from 0.01 to 50 wt% and most preferably from 0.01 to 15 wt%.

The compounds of the invention and its respective compositions can also be used in mosquito and fumigating coils, smoke cartridges, vaporizer plates or long-term vaporizers and also in moth papers, moth pads or other heat-independent vaporizer systems.

Methods to control infectious diseases transmitted by insects (e.g. malaria, dengue and yellow fever, lymphatic filariasis, and leishmaniasis) with compounds of the invention and its re-spective compositions also comprise treating surfaces of huts and houses, air spraying and impregnation of curtains, tents, clothing items, bed nets, tsetse-fly trap. Insecticidal compositions for application to fibers, fabric, knitgoods, nonwovens, netting material or foils and tarpaulins preferably comprise a mixture including the insecticide, optionally a repellent and at least one binder.

The compounds of the invention and its compositions can be used for protecting wooden materials e.g. trees, board fences, sleepers, frames, artistic artifacts, etc. and buildings, but also construction materials, furniture, leathers, fibers, vinyl articles, electric wires and cables etc. from ants, termites and/or wood or textile destroying beetles, and for controlling ants and termites from doing harm to crops or human beings (e.g. when the pests invade into houses and public facilities or nest in yards, orchards or parks).

Customary application rates in the protection of materials are, e.g., from 0.001 g to 2000 g or from 0.01 g to 1000 g of active compound per m² treated material, desirably from 0.1 g to 50 g per m². Insecticidal compositions for use in the impregnation of materials typically contain from 0.001 to 95 wt%, preferably from 0.1 to 45 wt%, and more preferably from 1 to 25 wt% of at least one repellent and/or insecticide.

### Digital application

The compounds of the invention and the compositions containing them may be applied in combination with, or by utilizing smart agricultural technologies, such as precision agriculture, remote and proximate imaging and image recognition, or smart agricultural site management programs. These smart agricultural technologies typically include models, e.g. computer programs, that support the user by considering information from a wide variety of sources to increase the quality and yield of harvested material, reduce damage by pests including the prediction of pest pressure and smart application of crop protection products, secure environ-mental protection, support quick and reliable agronomic decision making, reduce usage of fertilizers and crop protection products, reduce product residues in consumables increase spatial and temporal precision of agronomical measures, automate processes, and enable traceability of measures. Commercially available systems which include agronomic models are e.g. FieldScripts^{™} from The Climate Corporation, Xarvio^{™} from BASF, AGLogic^{™} from John Deere, etc.

Information input for these models include but is not limited to soil data, information on the plants that are currently growing or that may grow at the area of interest including crop plants and/or unwanted vegetation, weather information, information on the location of the area and directly derivable information thereof, information on pest pressure, information on beneficial organisms, and / or historic information of any of the aforementioned.

The information usable for precision agriculture may be based on input by at least one user, be accessible from external data sources and databases, or be based on sensor data. Data sources typically includes proximate-detection systems like soil-borne sensors and remote sensing as may be achieved by imaging with unmanned airborne vehicles like drones, or satellites. Sensors may be included in an Internet-of-Things system and may be directly or indirectly connected to the processing unit, e.g. via a wireless network and/or cloud applications. The information is typically taken into account by at least one processing unit and used to provide recommendations and generate control signals.

Typical technologies that are used in smart agricultural technologies include self-steering ro-bots (such as tractors, harvesters, drones), artificial intelligence (e.g. machine learning), imaging technologies (e.g. image segmentation technologies), big data analysis, and model generation, cloud computing, and machine-to-machine communication.

Precision agriculture such as precision farming is characterized by spatially and/or temporally resolved, targeted application of active ingredients like pesticides, plant-growth-regulators, fertilizers, and/or water including the variation of application rates over the agronomic site, zone or spot application, and of the spatially and/or temporally resolved, targeted planting or seeding of desired plant propagation material to a agronomic site. Precision farming typically includes the use of geo-positioning technologies like GPS for gaining information on the location and boundaries of the area of interest, the utilized application equipment, sensing equipment and recorded data, and to control the actions of farm vehicles such as spraying. By combining geo-positioning data with (digital) maps, it is possible to (semi)-automate agricultural measures at the site of interest, e.g. by using (semi)-autonomous spraying or seeding equipment.

Precision farming may typically include the application of smart spraying equipment, e.g. spot spraying, and precision spraying at a farm, e.g. by irrigation systems, tractors, robots, helicopters, airplanes, unmanned aerial vehicles, such as drones. Such equipment usually includes input sensors (such as e.g. a camera) and a processing unit configured to analyze the input data and configured to provide a recommendation or decision based on the analysis of the input data to apply the compounds of the invention or compositions comprising them to the agronomic site, e.g. the soil, the crop plants, or to control pests in a specific and precise manner. For example, pests may be detected, identified, and/or classified from imagery acquired by a camera. Such identification and/ classification can make use of image processing algorithms, which may utilize artificial intelligence (e.g. machine learning algorithms), or decision trees. In this manner, the compounds or compositions described herein can be applied only at the required location, point in time and dose rate.

### Pests

The compounds of the invention are especially suitable for efficiently combating animal pests e.g. arthropods, gastropods and nematodes including:
insects from the order of Lepidoptera, e.g. Achroia grisella, Acleris spp. e.g. A. fimbriana, A. gloverana, A. variana; Acrolepiopsis assectella, Acronicta major, Adoxophyes spp. e.g. A. cyrtosema, A. orana; Aedia leucomelas, Agrotis spp. e.g. A. exclamationis, A. fucosa, A. ipsilon, A. orthogoma, A. segetum, A. subterranea; Alabama argillacea, Aleurodicus dispersus, Alsophila pometaria, Ampelophaga rubiginosa, Amyelois transitella, Anacampsis sarcitella, Anagasta kuehniella, Anarsia lineatella, Anisota senatoria, Antheraea pernyi, Anticarsia (=Thermesia) spp. e.g. A. gemmatalis; Apamea spp., Aproaerema modicella, Archips spp. e.g. A. argyrospila, A. fuscocupreanus, A. rosana, A. xyloseanus; Argyresthia conjugella, Argyroploce spp., Argyrotaenia spp. e.g. A. velutinana; Athetis mindara, Austroasca viridigrisea, Autographa gamma, Autographa nigrisigna, Barathra brassicae, Bedellia spp., Bonagota salubricola, Borbo cinnara, Bucculatrix thurberiella, Bupalus piniarius, Busseola spp., Cacoecia spp. e.g. C. murinana, C. podana; Cactoblastis cactorum, Cadra cautella, Calingo braziliensis, Caloptilis theivora, Capua reticulana, Carposina spp. e.g. C. niponensis, C. sasakii; Cephus spp., Chaetocnema aridula, Cheimatobia brumata, Chilo spp. e.g. C. Indicus, C. suppressalis, C. partellus; Choreutis pariana, Choristoneura spp. e.g. C. conflictana, C. fumiferana, C. longicellana, C. murinana, C. occidentalis, C. rosaceana; Chrysodeixis (=Pseudoplusia) spp., e.g. C. eriosoma, C. includens; Cirphis unipuncta, Clysia ambiguella, Cnaphalocerus spp., Cnaphalocrocis medinalis, Cnephasia spp., Cochylis hospes, Coleophora spp., Colias eurytheme, Conopomorpha spp., Conotrachelus spp., Copitarsia spp., Corcyra cephalonica, Crambus caliginosellus, Crambus teterrellus, Crocidosema (=Epinotia) aporema, Cydalima (=Diaphania) perspectalis, Cydia (=Carpocapsa) spp., e.g. C. pomonella, C. latiferreana; Dalaca noctuides, Datana integerrima, Dasychira pinicola, Dendrolimus spp., e.g. D. pini, D. spectabilis, D. sibiricus; Desmia funeralis, Diaphania spp., e.g. D. nitidalis, D. hyalinata; Diatraea grandiosella, Diatraea saccharalis, Diphthera festiva, Earias spp. e.g. E. insulana, E. vittella; Ecdytolopha aurantianu, Egira (=Xylomyges) curialis, Elasmopalpus lignosellus, Eldana saccharina, Endopiza viteana, Ennomos subsignaria, Eoreuma loftini, Ephestia spp., e.g. E. cautella, E. elutella, E. kuehniella; Epinotia aporema, Epiphyas postvittana, Erannis tiliaria, Erionota thrax, Etiella spp., Eulia spp., Eupoecilia ambiguella, Euproctis chrysorrhoea, Euxoa spp., Evetria bouliana, Faronta albilinea, Feltia spp. e.g. F. subterranean; Galleria mellonella, Gracillaria spp., Grapholita spp. e.g. G. funebrana, G. molesta, G. inopinata; Halysidota spp., Harrisina americana, Hedylepta spp., Helicoverpa spp. e.g. H. armigera (=Heliothis armigera), H. zea (=Heliothis zea); Heliothis spp. e.g. H. assulta, H. subflexa, H. virescens; Hellula spp. e.g. H. undalis, H. rogatalis; Helocoverpa gelotopoeon, Hemileuca oliviae, Herpetogramma licarsisalis, Hibernia defoliaria, Hofmannophila pseudospretella, Homoeosoma electellum, Homona magnanima, Hypena scabra, Hyphantria cunea, Hyponomeuta padella, Hyponomeuta malinellus, Kakivoria flavofasciata, Keiferia lycopersicella, Lambdina fiscellaria fiscellaria, Lambdina fiscellaria lugubrosa, Lamprosema indicata, Laspeyresia molesta, Leguminivora glycinivorella, Lerodea eufala, Leucinodes orbonalis, Leucoma salicis, Leucoptera spp. e.g. L. coffeella, L. scitella; Leuminivora lycinivorella, Lithocolletis blancardella, Lithophane antennata, Llattia octo (=Amyna axis), Lobesia botrana, Lophocampa spp., Loxagrotis albicosta, Loxostege spp. e.g. L. sticticalis, L. cereralis; Lymantria spp., e.g. L. dispar, L. monacha; Lyonetia clerkella, Lyonetia prunifoliella, Malacosoma spp., e.g. M. americanum, M. californicum, M. constrictum, M. neustria; Mamestra spp., e.g. M. brassicae, M. configurata; Mamstra brassicae, Manduca spp. e.g. M. quinquemaculata, M. sexta; Marasmia spp, Marmara spp., Maruca testulalis, Megalopyge lanata, Melanchra picta, Melanitis leda, Mocis spp., e.g. M. lapites, M. repanda; Mocis latipes, Monochroa fragariae, Mythimna separata, Nemapogon cloacella, Neoleucinodes elegantalis, Nepytia spp., Nymphula spp., Oiketicus spp., Omiodes indicata, Omphisa anastomosalis, Operophtera brumata, Orgyia pseudotsugata, Oria spp., Orthaga thyrisalis, Ostrinia spp. e.g. O. nubilalis; Oulema oryzae, Paleacrita vernata, Panolis flammea, Parnara spp., Papaipema nebris, Papilio cresphontes, Paramyelois transitella, Paranthrene regalis, Paysandisia archon, Pectinophora spp. e.g. P. gossypiella; Peridroma saucia, Perileucoptera spp., e.g. P. coffeella; Phalera bucephala, Phryganidia californica, Phthorimaea spp. e.g. P. operculella; Phyllocnistis citrella, Phyllonorycter spp. e.g. P. blancardella, P. crataegella, P. issikii, P. ringoniella; Pieris spp. e.g. P. brassicae, P. rapae, P. napi; Pilocrocis tripunctata, Plathypena scabra, Platynota spp. e.g. P. flavedana, P. idaeusalis, P. stultana; Platyptilia carduidactyla, Plebejus argus, Plo-dia interpunctella, Plusia spp, Plutella maculipennis, Plutella xylostella, Pontia protodica, Prays spp., Prodenia spp., Proxenus lepigone, Pseudaletia spp. e.g. P. sequax, P. unipuncta; Pyrausta nubilalis, Rachiplusia nu, Richia albicosta, Rhizobius ventralis, Rhyacionia frustrana, Sabulodes aegrotata, Schizura concinna, Schoenobius spp., Schreckensteinia festaliella, Scirpophaga spp. e.g. S. incertulas, S. innotata; Scotia segetum, Sesamia spp. e.g. S. inferens, Seudyra subflava, Sitotroga cerealella, Sparganothis pilleriana, Spilonota lechriaspis, S. ocelli-na, Spodoptera (=Lamphygma) spp. e.g. S. cosmoides, S. eridania, S. exigua, S. frugiperda, S. latisfascia, S. littoralis, S. litura, S. omithogalli; Stigmella spp., Stomopteryx subsecivella, Strymon bazochii, Sylepta derogata, Synanthedon spp. e.g. S. exitiosa, Tecia solanivora, Telehin licus, Thaumatopoea pityocampa, Thaumatotibia (=Cryptophlebia) leucotreta, Thaumetopoea pityocampa, Thecla spp., Theresimima ampelophaga, Thyrinteina spp, Tildenia inconspicuella, Tinea spp. e.g. T. cloacella, T. pellionella; Tineola bisselliella, Tortrix spp. e.g. T. viridana; Trichophaga tapetzella, Trichoplusia spp. e.g. T. ni; Tuta (=Scrobipalpula) absoluta, Udea spp. e.g. U. rubigalis, U. rubigalis; Virachola spp., Yponomeuta padella, and Zeiraphera canadensis; insects from the order of Coleoptera, e.g. Acalymma vittatum, Acanthoscehdes obtectus, Adoretus spp., Agelastica alni, Agrilus spp. e.g. A. anxius, A. planipennis, A. sinuatus; Agriotes spp. e.g. A. fuscicollis, A. lineatus, A. obscurus; Alphitobius diaperinus, Amphimallus solstitialis, Anisandrus dispar, Anisoplia austriaca, Anobium punctatum, Anomala corpulenta, Anomala rufocuprea, Anoplophora spp. e.g. A. glabripennis; Anthonomus spp. e.g. A. eugenii, A. grandis, A. pomorum; Anthrenus spp., Aphthona euphoridae, Apion spp., Apogonia spp., Athous haemorrhoidalis, Atomaria spp. e.g. A. linearis; Attagenus spp., Aulacophora femoralis, Blastophagus piniperda, Blitophaga undata, Bruchidius obtectus, Bruchus spp. e.g. B. lentis, B. pisorum, B. rufimanus; Byctiscus betulae, Callidiellum rufipenne, Callopistria floridensis, Callosobruchus chinensis, Cameraria ohridella, Cassida nebulosa, Cerotoma trifurcata, Cetonia aurata, Ceuthorhynchus spp. e.g. C. assimilis, C. napi; Chaetocnema tibialis, Cleonus mendicus, Conoderus spp. e.g. C. vespertinus; Conotrachelus nenuphar, Cosmopolites spp., Costelytra zealandica, Crioceris asparagi, Cryptolestes ferrugineus, Cryptorhynchus lapathi, Ctenicera spp. e.g. C. destructor; Curculio spp., Cylindrocopturus spp., Cyclocephala spp., Dac-tylispa balyi, Dectes texanus, Dermestes spp., Diabrotica spp. e.g. D. undecimpunctata, D. speciosa, D. longicornis, D. semipunctata, D. virgifera; Diaprepes abbreviates, Dichocrocis spp., Dicladispa armigera, Diloboderus abderus, Diocalandra frumenti (Diocalandra stigmaticollis), Enaphalodes rufulus, Epilachna spp. e.g. E. varivestis, E. vigintioctomaculata; Epitrix spp. e.g. E. hirtipennis, E. similaris; Eutheola humilis, Eutinobothrus brasiliensis, Faustinus cubae, Gibbium psylloides, Gnathocerus cornutus, Hellula undalis, Heteronychus arator, Hylamorpha elegans, Hylobius abietis, Hylotrupes bajulus, Hypera spp., e.g. H. brunneipennis, H. postica; Hypomeces squamosus, Hypothenemus spp., Ips typographus, Lachnosterna consanguinea, Lasioderma serricorne, Latheticus oryzae, Lathridius spp., Lema spp. e.g. L. bilineata, L. melanopus; Leptinotarsa spp. e.g. L. decemlineata; Leptispa pygmaea, Limonius californi-cus, Lissorhoptrus oryzophilus, Lixus spp., Luperodes spp., Lyctus spp. e.g. L. bruneus; Liogenys fuscus, Macrodactylus spp. e.g. M. subspinosus; Maladera matrida, Megaplatypus mutates, Megascelis spp., Melanotus communis, Meligethes spp. e.g. M. aeneus; Melolontha spp. e.g. M. hippocastani, M. melolontha; Metamasius hemipterus, Microtheca spp., Migdolus spp. e.g. M. fryanus, Monochamus spp. e.g. M. alternatus; Naupactus xanthographus, Niptus hololeucus, Oberia brevis, Oemona hirta, Oryctes rhinoceros, Oryzaephilus surinamensis, Oryzaphagus oryzae, Otiorrhynchus sulcatus, Otiorrhynchus ovatus, Otiorrhynchus sulcatus, Oulema melanopus, Oulema oryzae, Oxycetoniajucunda, Phaedon spp. e.g. P. brassicae, P. cochleariae; Phoracantha recurva, Phyllobius pyri, Phyllopertha horticola, Phyllophaga spp. e.g. P. helleri; Phyllotreta spp. e.g. P. chrysocephala, P. nemorum, P. striolata, P. vittula; Phyllopertha horticola, Popilliajaponica, Premnotrypes spp., Psacothea hilaris, Psylliodes chrysocephala, Prostephanus truncates, Psylliodes spp., Ptinus spp., Pulga saltona, Rhizopertha dominica, Rhynchophorus spp. e.g. R. billineatus, R. ferrugineus, R. palmarum, R. phoenicis, R. vulneratus; Saperda candida, Scolytus schevyrewi, Scyphophorus acupunctatus, Sitona lineatus, Sitophilus spp. e.g. S. granaria, S. oryzae, S. zeamais; Sphenophorus spp. e.g. S. levis; Stegobium paniceum, Sternechus spp. e.g. S. subsignatus; Strophomorphus ctenotus, Symphyletes spp., Tanymecus spp., Tenebrio molitor, Tenebrioides mauretanicus, Tribolium spp. e.g. T. castaneum; Trogoderma spp., Tychius spp., Xylotrechus spp. e.g. X. pyrrhoderus; and, Zabrus spp. e.g. Z. tenebrioides;
insects from the order of Diptera e.g. Aedes spp. e.g. A. aegypti, A. albopictus, A. vexans; Anastrepha ludens, Anopheles spp. e.g. A. albimanus, A. crucians, A. freeborni, A. gambiae, A. leucosphyrus, A. maculipennis, A. minimus, A. quadrimaculatus, A. sinensis; Bactrocera invadens, Bibio hortulanus, Calliphora erythrocephala, Calliphora vicina, Ceratitis capitata, Chrysomyia spp. e.g. C. bezziana, C. hominivorax, C. macellaria; Chrysops atlanticus, Chrysops discalis, Chrysops silacea, Cochliomyia spp. e.g. C. hominivorax; Contarinia spp. e.g. C. sorghicola; Cordylobia anthropophaga, Culex spp. e.g. C. nigripalpus, C. pipiens, C. quinquefasciatus, C. tarsalis, C. tritaeniorhynchus; Culicoides furens, Culiseta inornata, Culiseta melanura, Cuterebra spp., Dacus cucurbitae, Dacus oleae, Dasineura brassicae, Dasineura oxycoccana, Delia spp. e.g. D. antique, D. coarctata, D. platura, D. radicum; Dermatobia hominis, Drosophila spp. e.g. D. suzukii, Fannia spp. e.g. F. canicularis; Gastraphilus spp. e.g. G. intestinalis; Geomyza tipunctata, Glossina spp. e.g. G. fuscipes, G. morsitans, G. palpalis, G. tachinoides; Haematobia irritans, Haplodiplosis equestris, Hippelates spp., Hylemyia spp. e.g. H. platura; Hypoderma spp. e.g. H. lineata; Hyppobosca spp., Hydrellia philippina, Leptoconops torrens, Liriomyza spp. e.g. L. sativae, L. trifolii; Lucilia spp. e.g. L. caprina, L. cuprina, L. sericata; Lycoria pectoralis, Mansonia titillanus, Mayetiola spp. e.g. M. destructor; Musca spp. e.g. M. autumnalis, M. domestica; Muscina stabulans, Oestrus spp. e.g. O. ovis; Opomyza florum, Oscinella spp. e.g. O. frit; Orseolia oryzae, Pegomya hysocyami, Phlebotomus argentipes, Phorbia spp. e.g. P. antiqua, P. brassicae, P. coarctata; Phytomyza gymnostoma, Prosimulium mixtum, Psila rosae, Psorophora columbiae, Psorophora discolor, Rhagoletis spp. e.g. R. cerasi, R. cingulate, R. indifferens, R. mendax, R. pomonella; Rivellia quadrifasciata, Sarcophaga spp. e.g. S. haemorrhoidalis; Simulium vittatum, Sitodiplosis mosellana, Stomoxys spp. e.g. S. calcitrans; Tabanus spp. e.g. T. atratus, T. bovinus, T. lineola, T. similis; Tannia spp., Thecodiplosis japonensis, Tipula oleracea, Tipula paludosa, Wohlfahrtia spp, and Zaprionus indianus; insects from the order of Thysanoptera e.g., Baliothrips biformis, Dichromothrips corbetti, Dichromothrips ssp., Echinothrips americanus, Enneothrips flavens, Frankliniella spp. e.g. F. fusca, F. occidentalis, F. tritici; Heliothrips spp., Hercinothrips femoralis, Kakothrips spp., Microcephalothrips abdominal is, Neohydatothrips samayunkur, Pezothrips kellyanus, Rhipiphorothrips cruentatus, Scirtothrips spp. e.g. S. citri, S. dorsalis, S. perseae; Stenchaetothrips spp, Taeniothrips cardamoni, Taeniothrips inconsequens, Thrips spp. e.g. T. imagines, T. hawaiiensis, T. oryzae, T. palmi, T. parvispinus, T. tabaci;
insects from the order of Hemiptera e.g., Acizzia jamatonica, Acrosternum spp., e.g. A. hilare; Acyrthosipon spp., e.g. A. onobrychis, A. pisum; Adelges laricis, Adelges tsugae, Adelphocoris spp., e.g. A. rapidus, A. superbus; Aeneolamia spp., Agonoscena spp., Aulacorthum solani, Aleurocanthus woglumi, Aleurodes spp., Aleurodicus disperses, Aleurolobus barodensis, Aleurothrixus spp., Amrasca spp., Anasa tristis, Antestiopsis spp., Anuraphis cardui, Aonidiella spp., Aphanostigma piri, Aphidula nasturtii, Aphis spp. e.g. A. craccivora, A. fabae, A. forbesi, A. gossypii, A. grossulariae, A. maidiradicis, A. pomi, A. sambuci, A. schneideri, A. spiraecola; Arboridia apicalis, Arilus critatus, Aspidiella spp., Aspidiotus spp., Atanus spp., Aulacaspis yasumatsui, Aulacorthum solani, Bactericera cockerelli (Paratrioza cockerelli), Bemisia spp. e.g. B. argentifolii, B. tabaci (Aleurodes tabaci); Blissus spp. e.g. B. leucopterus; Brachycaudus spp. e.g. B. cardui, B. helichrysi, B. persicae, B. prunicola; Brachycolus spp., Brachycorynella asparagi, Brevicoryne brassicae, Cacopsylla spp. e.g. C. fulguralis, C. pyricola (Psylla piri); Calligypona marginata, Calocoris spp., Campylomma livida, Capitophorus horni, Carneocephala fulgida, Cavelerius spp., Ceraplastes spp., Ceratovacuna lanigera, Ceroplastes ceriferus, Cerosipha gossypii, Chaetosiphon fragaefolii, Chionaspis tegalensis, Chlorita onukii, Chromaphis juglandicola, Chrysomphalus ficus, Cicadulina mbila, Cimex spp. e.g. C. hemipterus, C. lectularius; Circulifer tenellus, Coccomytilus halli, Coccus spp. e.g. C. hesperidum, C. pseudomagnoliarum; Corythucha arcuata, Creontiades dilutus, Cryptomyzus ribis, Chrysomphalus aonidum, Cryptomyzus ribis, Ctenarytaina spatulata, Cyrtopeltis notatus, Dalbulus spp., Dasynus piperis, Dialeurodes spp. e.g. D. citrifolii; Dalbulus maidis, Diaphorina spp. e.g. D. citri; Diaspis spp. e.g. D. bromeliae; Dichelops furcatus, Diconocoris hewetti, Doralis spp., Dreyfusia nordmannianae, Dreyfusia piceae, Drosicha spp., Dysaphis spp. e.g. D. plantaginea, D. pyri, D. radicola; Dysaulacorthum pseudosolani, Dysdercus spp. e.g. D. cingulatus, D. intermedius; Dysmicoccus spp., Edessa spp., Geocoris spp., Empoasca spp. e.g. E. fabae, E. solana; Epidiaspis leperii, Eriosoma spp. e.g. E. lanigerum, E. pyricola; Erythroneura spp., Eurygaster spp. e.g. E. integriceps; Euscelis bilobatus, Euschistus spp. e.g. E. heros, E. impictiventris, E. servus; Fiorinia theae, Geococcus coffeae, Glycaspis brimblecombei, Halyomorpha spp. e.g. H. halys; Heliopeltis spp., Homalodisca vitripennis (=H. coagulata), Horcias nobilellus, Hyalopterus pruni, Hyperomyzus lactucae, Icerya spp. e.g. I. purchase; Idiocerus spp., Idioscopus spp., Laodelphax striatellus, Lecanium spp., Lecanoideus floccissimus, Lepidosaphes spp. e.g. L. ulmi; Leptocorisa spp., Leptoglossus phyllopus, Lipaphis erysimi, Lygus spp. e.g. L. hesperus, L. lineolaris, L. praten-sis; Maconellicoccus hirsutus, Marchalina hellenica, Macropes excavatus, Macrosiphum spp. e.g. M. rosae, M. avenae, M. euphorbiae; Macrosteles quadrilineatus, Mahanarva fimbriolata, Megacopta cribraria, Megoura viciae, Melanaphis pyrarius, Melanaphis sacchari, Melanocallis (=Tinocallis) caryaefoliae, Metcafiella spp., Metopolophium dirhodum, Monellia costalis, Mo-nelliopsis pecanis, Myzocallis coryli, Murgantia spp., Myzus spp. e.g. M. ascalonicus, M. cerasi, M. nicotianae, M. persicae, M. varians; Nasonovia ribisnigri, Neotoxoptera formosana, Neomegalotomus spp, Nephotettix spp. e.g. N. malayanus, N. nigropictus, N. parvus, N. vires-cens; Nezara spp. e.g. N. viridula; Nilaparvata lugens, Nysius huttoni, Oebalus spp. e.g. O. pugnax; Oncometopia spp., Orthezia praelonga, Oxycaraenus hyalinipennis, Parabemisia myricae, Parlatoria spp., Parthenolecanium spp. e.g. P. corni, P. persicae; Pemphigus spp. e.g. P. bursarius, P. populivenae; Peregrinus maidis, Perkinsiella saccharicida, Phenacoccus spp. e.g. P. aceris, P. gossypii; Phloeomyzus passerinii, Phorodon humuli, Phylloxera spp. e.g. P. devastatrix, Piesma quadrata, Piezodorus spp. e.g. P. guildinii; Pinnaspis aspidistrae, Planococcus spp. e.g. P. citri, P. ficus; Prosapia bicincta, Protopulvinaria pyriformis, Psallus seriatus, Pseudacysta persea, Pseudaulacaspis pentagona, Pseudococcus spp. e.g. P. comstocki; Psylla spp. e.g. P. mali; Pteromalus spp., Pulvinaria amygdali, Pyrilla spp., Quadraspidiotus spp., e.g. Q. perniciosus; Quesada gigas, Rastrococcus spp., Reduvius senilis, Rhizoecus americanus, Rhodnius spp., Rhopalomyzus ascalonicus, Rhopalosiphum spp. e.g. R. pseudobrassicas, R. insertum, R. maidis, R. padi; Sagatodes spp., Sahlbergella singularis, Saissetia spp., Sappaphis mala, Sappaphis mali, Scaptocoris spp., Scaphoideus titanus, Schizaphis graminum, Schizoneura lanuginosa, Scotinophora spp., Selenaspidus articulatus, Sitobion avenae, Sogata spp., Sogatella furcifera, Solubea insularis, Spissistilus festinus (=Stictocephala festina), Stephanitis nashi, Stephanitis pyrioides, Stephanitis takeyai, Tenalaphara malayensis, Tetraleurodes perseae, Therioaphis maculate, Thyanta spp. e.g. T. accerra, T. perditor; Tibraca spp., Tomaspis spp., Toxoptera spp. e.g. T. aurantii; Trialeurodes spp. e.g. T. abutilonea, T. ricini, T. vaporariorum; Triatoma spp., Trioza spp., Typhlocyba spp., Unaspis spp. e.g. U. citri, U. yanonensis; and Viteus vitifolii, Insects from the order Hymenoptera e.g. Acanthomyops interjectus, Athalia rosae, Atta spp. e.g. A. capiguara, A. cephalotes, A. cephalotes, A. laevigata, A. robusta, A. sexdens, A. texana, Bombus spp., Brachymyrmex spp., Camponotus spp. e.g. C. floridanus, C. pennsylvanicus, C. modoc; Cardiocondyla nuda, Chalibion sp, Crematogaster spp., Dasymutilla occidentalis, Diprion spp., Dolichovespula maculata, Dorymyrmex spp., Dryocosmus kuriphilus, Formica spp., Hoplocampa spp. e.g. H. minuta, H. testudinea; Iridomyrmex humilis, Lasius spp. e.g. L. niger, Linepithema humile, Liometopum spp., Leptocybe invasa, Monomorium spp. e.g. M. pharaonis, Monomorium, Nylandria fulva, Pachycondyla chinensis, Paratrechina longicornis, Paravespula spp., e.g. P. germanica, P. pennsylvanica, P. vulgaris; Pheidole spp. e.g. P. megacephala; Pogonomyrmex spp. e.g. P. barbatus, P. californicus, Polistes rubiginosa, Prenolepis impairs, Pseudomyrmex gracilis, Schelipron spp., Sirex cyaneus, Solenopsis spp. e.g. S. geminata, S.invicta, S. molesta, S. richteri, S. xyloni, Sphecius speciosus, Sphex spp., Tapinoma spp. e.g. T. melanocephalum, T. sessile; Tetramorium spp., e.g. T. caespitum, T. bicarinatum, Vespa spp., e.g. V. crabro; Vespula spp., e.g. V. squamosal; Wasmannia auropunctata, Xylocopa sp; Insects from the order Orthoptera e.g. Acheta domesticus, Calliptamus italicus, Chortoicetes terminifera, Ceuthophilus spp., Diastrammena asynamora, Dociostaurus maroccanus, Gryllotalpa spp. e.g. G. africana, G. gryllotalpa; Gryllus spp., Hieroglyphus daganensis, Kraussaria angulifera, Locusta spp. e.g. L. migratoria, L. pardalina; Melanoplus spp. e.g. M. bivittatus, M. femurrubrum, M. mexicanus, M. sanguinipes, M. spretus; Nomadacris septemfasciata, Oedaleus senegalensis, Scapteriscus spp., Schistocerca spp. e.g. S. americana, S. gregaria, Stemopelmatus spp., Tachycines asynamorus, and Zonozerus variegatus;
Pests from the Class Arachnida e.g. Acari,e.g. of the families Argasidae, Ixodidae and Sarcoptidae, e.g. Amblyomma spp. (e.g. A. americanum, A. variegatum, A. maculatum), Argas spp. e.g. A. persicu), Boophilus spp. e.g. B. annulatus, B. decoloratus, B. microplus, Dermacentor spp. e.g. D.silvarum, D. andersoni, D. variabilis, Hyalomma spp. e.g. H. truncatum, Ixodes spp. e.g. I. ricinus, I. rubicundus, I. scapularis, I. holocyclus, I. pacificus, Rhipicephalus sanguineus, Ornithodorus spp. e.g. O. moubata, O. hermsi, O. turicata, Ornithonyssus bacoti, Otobius megnini, Dermanyssus gallinae, Psoroptes spp. e.g. P. ovis, Rhipicephalus spp. e.g. R. sanguineus, R. appendiculatus, Rhipicephalus evertsi, Rhizoglyphus spp., Sarcoptes spp. e.g. S. Scabiei; and Family Eriophyidae including Aceria spp. e.g. A. sheldoni, A. anthocoptes, Acallitus spp., Aculops spp. e.g. A. lycopersici, A. pelekassi; Aculus spp. e.g. A. schlechtendali; Colomerus vitis, Epitrimerus pyri, Phyllocoptruta oleivora; Eriophytes ribis and Eriophyes spp. e.g. Eriophyes sheldoni; Family Tarsonemidae including Hemitarsonemus spp., Phytonemus pallidus and Polyphagotarsonemus latus, Stenotarsonemus spp. Steneotarsonemus spinki; Family Tenuipalpidae including Brevipalpus spp. e.g. B. phoenicis; Family Tetranychidae including Eotetranychus spp., Eutetranychus spp., Oligonychus spp., Petrobia latens, Tetranychus spp. e.g. T. cinnabarinus, T. evansi, T. kanzawai, T, pacificus, T. phaseulus, T. telarius and T. urticae; Bryobia praetiosa; Panonychus spp. e.g. P. ulmi, P. citri; Metatetranychus spp. and Oligonychus spp. e.g. O. pratensis, O. perseae, Vasates lycopersici; Raoiella indica, Family Carpoglyphidae including Carpoglyphus spp.; Penthaleidae spp. e.g. Halotydeus destructor; Family Demodicidae with species e.g. Demodex spp.; Family Trombicidea including Trombicula spp.; Family Macronyssidae including Ornothonyssus spp.; Family Pyemotidae including Pyemotes tritici; Tyrophagus putrescentiae; Family Acaridae includ-ing Acarus siro; Family Araneida including Latrodectus mactans, Eratigena agrestis, Cheiracanthium sp, Lycosa sp Achaearanea tepidariorum and Loxosceles reclusa;
Pests from the Phylum Nematoda, e.g. plant parasitic nematodes e.g. root-knot nematodes, Meloidogyne spp. e.g. M. hapla, M. incognita, M. javanica; cyst-forming nematodes, Globodera spp. e.g. G. rostochiensis; Heterodera spp. e.g. H. avenae, H. glycines, H. schachtii, H. trifolii; Seed gall nematodes, Anguina spp.; Stem and foliar nematodes, Aphelenchoides spp. e.g. A. besseyi; Sting nematodes, Belonolaimus spp. e.g. B. longicaudatus; Pine nematodes, Bursaphelenchus spp. e.g. B. lignicolus, B. xylophilus; Ring nematodes, Criconema spp., Criconemella spp. e.g. C. xenoplax and C. ornata; and, Criconemoides spp. e.g. Criconemoides informis; Mesocriconema spp.; Stem and bulb nematodes, Ditylenchus spp. e.g. D. destructor, D. dipsaci; Awl nematodes, Dolichodorus spp.; Spiral nematodes, Heliocotylenchus multicinctus; Sheath and sheathoid nematodes, Hemicycliophora spp. and Hemicriconemoides spp.; Hirshmanniella spp.; Lance nematodes, Hoploaimus spp.; False rootknot nematodes, Nacobbus spp.; Needle nematodes, Longidorus spp. e.g. L. elongatus; Lesion nematodes, Pratylenchus spp. e.g. P. brachyurus, P. neglectus, P. penetrans, P. curvitatus, P. goodeyi; Burrowing nematodes, Radopholus spp. e.g. R. similis; Rhadopholus spp.; Rhodopholus spp.; Reniform nematodes, Rotylenchus spp. e.g. R. robustus, R. reniformis; Scutellonema spp.; Stubby-root nematode, Trichodorus spp. e.g. T. obtusus, T. primitivus; Paratrichodorus spp. e.g. P. minor; Stunt nematodes, Tylenchorhynchus spp. e.g. T. claytoni, T. dubius; Citrus nematodes, Tylenchulus spp. e.g. T. semipenetrans; Dagger nematodes, Xiphinema spp.; and other plant parasitic nematode species;
Insects from the order Blattodea e.g. Macrotermes spp. e.g. M. natalensis; Cornitermes cumulans, Procornitermes spp., Globitermes sulfureus, Neocapritermes spp. e.g. N. opacus, N. parvus; Odontotermes spp., Nasutitermes spp. e.g. N. corniger; Coptotermes spp. e.g. C. formosanus, C. gestroi, C. acinaciformis; Reticulitermes spp. e.g. R. hesperus, R. tibialis, R. speratus, R. flavipes, R. grassei, R. lucifugus, R. virginicus; Heterotermes spp. e.g. H. aureus, H. longiceps, H. tenuis; Cryptotermes spp. e.g. C. brevis, C. cavifrons; Incisitermes spp. e.g. I. minor, I. snyderi; Marginitermes hubbardi, Kalotermes flavicollis, Neotermes spp. e.g. N. cas-taneus, Zootermopsis spp. e.g. Z. angusticollis, Z. nevadensis, Mastotermes spp. e.g. M. dar-winiensis; Blatta spp. e.g. B. orientalis, B. lateralis; Blattella spp. e.g. B. asahinae, B. germanica; Rhyparobia maderae, Panchlora nivea, Periplaneta spp. e.g. P. americana, P. australasiae, P. brunnea, P. fuliginosa, P. japonica; Supella longipalpa, Parcoblatta pennsylvanica, Eurycotis floridana, Pycnoscelus surinamensis,
Insects from the order Siphonoptera e.g. Cediopsylla simples, Ceratophyllus spp., Ctenocephalides spp. e.g. C. felis, C. canis, Xenopsylla cheopis, Pulex irritans, Trichodectes canis, Tunga penetrans, and Nosopsyllus fasciatus,
Insects from the order Thysanura e.g. Lepisma saccharina, Ctenolepisma urbana, and Thermobia domestica,
Pests from the class Chilopoda e.g. Geophilus spp., Scutigera spp. e.g. Scutigera coleoptrata; Pests from the class Diplopoda e.g. Blaniulus guttulatus, Julus spp., Narceus spp.,
Pests from the class Symphyla e.g. Scutigerella immaculata,
Insects from the order Dermaptera, e.g. Forficula auricularia,
Insects from the order Collembola, e.g. Onychiurus spp., e.g. Onychiurus armatus,
Pests from the order Isopoda, e.g. Armadillidium vulgare, Oniscus asellus, Porcellio scaber,
Insects from the order Phthiraptera, e.g. Damalinia spp., Pediculus spp. e.g. Pediculus hu-manus capitis, Pediculus humanus corporis, Pediculus humanus humanus; Pthirus pubis, Haematopinus spp. e.g. Haematopinus eurysternus, Haematopinus suis; Linognathus spp. e.g. Linognathus vituli; Bovicola bovis, Menopon gallinae, Menacanthus stramineus and Solenopotes capillatus, Trichodectes spp.,

Further pest species which may be controlled by compounds I include: from the Phylum Mollusca, class Bivalvia, e.g., Dreissena spp.; class Gastropoda, e.g., Arion spp., Biomphalaria spp., Bulinus spp., Deroceras spp., Galba spp., Lymnaea spp., Oncomelania spp., Pomacea canaliclata, Succinea spp.; from the class of the helminths, e.g., Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp. e.g. Haemonchus contortus; Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp., Strongyloides fuelleborni, Strongyloides stercora lis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

The compounds of the invention are particularly suitable for efficiently combating
insects from the sub-order of Auchenorrhyncha, e.g. Amrasca biguttula, Empoasca spp., Nephotettix virescens, Sogatella furcifera, Mahanarva spp., Laodelphax striatellus, Nilapar-vata lugens, Diaphorina citri, Lycorma delicatula, Pentastiridus leporinus;
Lepidoptera, e.g. Helicoverpa spp., Heliothis virescens, Lobesia botrana, Ostrinia nubilalis, Plutella xylostella, Pseudoplusia includens, Scirpophaga incertulas, Spodoptera spp., Trichoplusia ni, Tuta absoluta, Cnaphalocrocis medialis, Cydia pomonella, Chilo suppressalis, Anticarsia gemmatalis, Agrotis ipsilon, Chrysodeixis includens;
True bugs, e.g. Lygus spp., Stink bugs such as Euschistus spp., Halyomorpha halys, Nezara viridula, Piezodorus guildinii, Dichelops furcatus;
Thrips, e.g. Frankliniella spp., Thrips spp., Dichromothrips corbettii;
Aphids, e.g. Acyrthosiphon pisum, Aphis spp., Myzus persicae, Rhopalosiphum spp., Schi-zaphis graminum, Megoura viciae;
Whiteflies, e.g. Trialeurodes vaporariorum, Bemisia spp.;
Coleoptera, e.g. Phyllotreta spp., Melanotus spp., Meligethes aeneus, Leptinotarsa decimlineata, Ceutorhynchus spp., Diabrotica spp., Anthonomus grandis, Atomaria linearia, Agriotes spp., Epilachna spp.;
Flies, e.g. Delia spp., Ceratitis capitate, Bactrocera spp., Liriomyza spp.;
Coccoidea, e.g. Aonidiella aurantia, Ferrisia virgate;
Anthropods of class Arachnida (Mites), e.g. Penthaleus major, Tetranychus spp.;
Nematodes, e.g. Heterodera glycines, Meloidogyne sp., Pratylenchus spp., Caenorhabditis elegans.

### Animal health

The compounds of the invention are suitable for use in treating or protecting animals against infestation or infection by parasites. Therefore, the invention also relates to the use of a compound of the invention for the manufacture of a medicament for the treatment or protection of animals against infestation or infection by parasites. Furthermore, the invention relates to a method of treating or protecting animals against infestation and infection by parasites, which comprises orally, topically or parenterally administering or applying to the animals a parasiticidally effective amount of a compound of the invention.

The invention also relates to the non-therapeutic use of compounds of the invention for treating or protecting animals against infestation and infection by parasites. Moreover, the invention relates to a non-therapeutic method of treating or protecting animals against infestation and infection by parasites, which comprises applying to a locus a parasiticidally effective amount of a compound of the invention.

The compounds of the invention are further suitable for use in combating or controlling parasites in and on animals. Furthermore, the invention relates to a method of combating or con-trolling parasites in and on animals, which comprises contacting the parasites with a parasitically effective amount of a compound of the invention.

The invention also relates to the non-therapeutic use of compounds of the invention for controlling or combating parasites. Moreover, the invention relates to a non-therapeutic method of combating or controlling parasites, which comprises applying to a locus a parasiticidally effective amount of a compound of the invention.

The compounds of the invention can be effective through both contact (via soil, glass, wall, bed net, carpet, blankets or animal parts) and ingestion (e.g. baits). Furthermore, the compounds of the invention can be applied to any and all developmental stages.

The compounds of the invention can be applied as such or in form of compositions comprising the compounds of the invention.

The compounds of the invention can also be applied together with a mixing partner, which acts against pathogenic parasites, e.g. with synthetic coccidiosis compounds, polyetherantibiotics e.g. Amprolium, Robenidin, Toltrazuril, Monensin, Salinomycin, Maduramicin, Lasalocid, Narasin or Semduramicin, or with other mixing partners as defined above, or in form of compositions comprising said mixtures.

The compounds of the invention and compositions comprising them can be applied orally, parenterally or topically, e.g. dermally. The compounds of the invention can be systemically or non-systemically effective.

The application can be carried out prophylactically, therapeutically or non-therapeutically. Furthermore, the application can be carried out preventively to places at which occurrence of the parasites is expected.

As used herein, the term "contacting" includes both direct contact (applying the compounds/compositions directly on the parasite, including the application directly on the animal or excluding the application directly on the animal, e.g. at its locus for the latter) and indirect contact (applying the compounds/compositions to the locus of the parasite). The contact of the parasite through application to its locus is an example of a non-therapeutic use of the compounds of the invention.

The term "locus" means the habitat, food supply, breeding ground, area, material or environment in which a parasite is growing or may grow outside of the animal.

As used herein, the term "parasites" includes endo- and ectoparasites. In some embodiments of the invention, endoparasites can be preferred. In other embodiments, ectoparasites can be preferred. Infestations in warm-blooded animals and fish include lice, biting lice, ticks, nasal bots, keds, biting flies, muscoid flies, flies, myiasitic fly larvae, chiggers, gnats, mosquitoes and fleas. The compounds of the invention are especially useful for combating parasites of the following orders and species, respectively:
fleas (Siphonaptera), e.g. Ctenocephalides felis, C. canis, Xenopsylla cheopis, Pulex irri-tans, Tunga penetrans, and Nosopsyllus fasciatus; cockroaches (Blattaria - Blattodea), e.g. Blattella germanica, B. asahinae, Periplaneta americana, P. japonica, P. brunnea, P. fuligginosa, P. australasiae, and Blatta orientalis; flies, mosquitoes (Diptera), e.g. Aedes aegypti, A. albopictus, A. vexans, Anastrepha ludens, Anopheles maculipennis, A. crucians, A. albimanus, A. gambiae, A. freeborni, A. leucosphyrus, A. minimus, A. quadrimaculatus, Calliphora vicina, Chrysomya bezziana, C. hominivorax, C. macellaria, Chrysops discalis, C. silacea, C. atlanticus, Cochliomyia hominivorax, Cordylobia anthropophaga, Culicoides furens, Culex pipiens, C. nigripalpus, C. quinquefasciatus, C. tarsalis, Culiseta inornata, C. melanura, Dermatobia hominis, Fannia canicularis, Gasterophilus intestinalis, Glossina morsitans, G. palpalis, G. fuscipes, G. tachinoides, Haematobia irritans, Haplodiplosis equestris, Hippelates spp., Hypoderma line-ata, Leptoconops torrens, Lucilia caprina, L. cuprina, L. sericata, Lycoria pectoralis, Mansonia spp., Musca domestica, M. stabulans, Oestrus ovis, Phlebotomus argentipes, Psorophora columbiae, P. discolor, Prosimulium mixtum, Sarcophaga spp., S. haemorrhoidalis, Simulium vittatum, Stomoxys calcitrans, Tabanus bovinus, T. atratus, T. lineola, and T. similis; lice (Phthiraptera), e.g. Pediculus humanus capitis, P. humanus humanus, Pthirus pubis, Haematopinus eurysternus, H. suis, Linognathus vituli, Bovicola bovis, Menopon gallinae, Menacanthus stramineus, and Solenopotes capillatus; ticks and parasitic mites (Parasitiformes): ticks (Ixodida), e.g. Ixodes scapularis, I. holocyclus, I. pacificus, Rhiphicephalus sanguineus, Dermacentor andersoni, D. variabilis, Amblyomma americanum, A. maculatum, Ornithodorus hermsi, O. turicata and parasitic mites (Mesostigmata), e.g. Ornithonyssus bacoti, Dermanyssus gallinae; Actinedida (Prostigmata) and Acaridida (Astigmata), e.g. Acarapis spp., Cheyletiella spp., Ornithocheyletia spp., Myobia spp., Psorergates spp., Demo-dex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., and Laminosioptes spp; Bugs (Het-eropterida): Cimex lectularius, C. hemipterus, Reduvius senilis, Triatoma spp., Rhodnius ssp., Panstrongylus ssp., and Arilus critatus; Anoplurida, e.g. Haematopinus spp., Linognathus spp., Pediculus spp., Phtirus spp., and Solenopotes spp.; Mallophagida (suborders Arnblycerina and Ischnocerina), e.g. Trimenopon spp., Menopon spp., Trinoton spp., Bovicola spp., Werneckiella spp., Lepikentron spp., Trichodectes spp., and Felicola spp.; Roundworms Nematoda: Wipeworms and Trichinosis (Trichosyringida), e.g. Trichinellidae (Trichinella spp.), (Trichuridae) Trichuris spp., Capillaria spp.; Rhabditida, e.g. Rhabditis spp., Strongyloides spp., Helicephalobus spp.; Strongylida, e.g. Strongylus spp., Ancylostoma spp., Necator americanus, Bunostomum spp. (Hookworm), Trichostrongylus spp., Haemonchus contortus, Ostertagia spp., Cooperia spp., Nematodirus spp., Dictyocaulus spp., Cyathostoma spp., Oesophagostomum spp., Stephanurus dentatus, Ollulanus spp., Chabertia spp., Stephanurus dentatus, Syngamus trachea, Ancylostoma spp., Uncinaria spp., Globocephalus spp., Necator spp., Metastrongylus spp., Muellerius capillaris, Protostrongylus spp., Angiostrongylus spp., Parelaphostrongylus spp., Aleurostrongylus abstrusus, and Dioctophyma renale; Intestinal roundworms (Ascaridida), e.g. Ascaris lumbricoides, Ascaris suum, Ascaridia galli, Parascaris equorum, Enterobius vermicularis (Threadworm), Toxocara canis, Toxascaris leonine, Skrjabinema spp., and Oxyuris equi; Camallanida, e.g. Dracunculus medinensis (guinea worm); Spirurida, e.g. Thelazia spp., Wuchereria spp., Brugia spp., Onchocerca spp., Dirofilari spp.a, Dipetalonema spp., Setaria spp., Elaeophora spp., Spirocerca lupi, and Habronema spp.; Thorny headed worms (Acanthocephala), e.g. Acanthocephalus spp., Macracanthorhynchus hirudinaceus and Oncicola spp.; Planarians (Plathelminthes): Flukes (Trematoda), e.g. Faciola spp., Fascioloides magna, Paragonimus spp., Dicrocoelium spp., Fasciolopsis buski, Clonorchis sinensis, Schistosoma spp., Trichobilharzia spp., Alaria alata, Paragonimus spp., and Nanocyetes spp.; Cercomeromorpha, in particular Cestoda (Tapeworms), e.g. Diphyllo-bothrium spp., Tenia spp., Echinococcus spp., Dipylidium caninum, Multiceps spp., Hymenolepis spp., Mesocestoides spp., Vampirolepis spp., Moniezia spp., Anoplocephala spp., Sirometra spp., Anoplocephala spp., and Hymenolepis spp..

The term "animal" includes warm-blooded animals (including humans) and fish. Preferred are mammals, e.g. cattle, sheep, swine, camels, deer, horses, pigs, poultry, rabbits, goats, dogs and cats, water buffalo, donkeys, fallow deer and reindeer, and also in fur-bearing animals e.g. mink, chinchilla and raccoon, birds e.g. hens, geese, turkeys and ducks and fish e.g. fresh- and saltwater fish e.g. trout, carp and eels. Particularly preferred are domestic animals, e.g. dogs or cats. Generally, "parasiticidally effective amount" means the amount of active ingredient needed to achieve an observable effect on growth, including the effects of necrosis, death, retardation, prevention, and removal, destruction, or otherwise diminishing the occurrence and activity of the target organism. The parasiticidally effective amount can vary for the various compounds/compositions used in the invention. A parasiticidally effective amount of the compositions will also vary according to the prevailing conditions e.g. desired parasiticidal effect and duration, target species, mode of application.

Generally, it is favorable to apply the compounds of the invention in total amounts of 0.5 mg/kg to 100 mg/kg per day, preferably 1 mg/kg to 50 mg/kg per day.

For oral administration to warm-blooded animals, the compounds I may be formulated as animal feeds, animal feed premixes, animal feed concentrates, pills, solutions, pastes, suspensions, drenches, gels, tablets, boluses and capsules. In addition, the compounds I may be ad-ministered to the animals in their drinking water. For oral administration, the dosage form chosen should provide the animal with 0.01 mg/kg to 100 mg/kg of animal body weight per day of the compounds I, preferably with 0.5 mg/kg to 100 mg/kg of animal body weight per day.

Alternatively, the compounds I may be administered to animals parenterally, e.g., by intraruminal, intramuscular, intravenous or subcutaneous injection. The compounds I may be dispersed or dissolved in a physiologically acceptable carrier for subcutaneous injection. Alternatively, the compounds I may be formulated into an implant for subcutaneous administration. In addition the compounds I may be transdermally administered to animals. For parenteral administration, the dosage form chosen should provide the animal with 0.01 mg/kg to 100 mg/kg of animal body weight per day of the compounds I.

The compounds I may also be applied topically to the animals in the form of dips, dusts, powders, collars, medallions, sprays, shampoos, spot-on and pour-on formulations and in ointments or oil-in-water or water-in-oil emulsions. For topical application, dips and sprays usually contain 0.5 ppm to 5,000 ppm and preferably 1 ppm to 3,000 ppm of the compounds I. In addition, the compounds I may be formulated as ear tags for animals, particularly quadrupeds e.g. cattle and sheep.

Suitable preparations are:
- Solutions e.g. oral solutions, concentrates for oral administration after dilution, solutions for use on the skin or in body cavities, pouring-on formulations, gels;
- Emulsions and suspensions for oral or dermal administration; semi-solid preparations;
- Formulations in which the active compound is processed in an ointment base or in an oil-in-water or water-in-oil emulsion base;
- Solid preparations e.g. powders, premixes or concentrates, granules, pellets, tablets, boluses, capsules; aerosols and inhalants, and active compound-containing shaped articles.

Compositions suitable for injection are prepared by dissolving the active ingredient in a suit-able solvent and optionally adding further auxiliaries e.g. acids, bases, buffer salts, preservatives, and solubilizers. Suitable auxiliaries for injection solutions are known in the art. The solutions are filtered and filled sterile.

Oral solutions are administered directly. Concentrates are administered orally after prior dilution to the use concentration. Oral solutions and concentrates are prepared according to the state of the art and as described above for injection solutions, sterile procedures not being necessary. Solutions for use on the skin are trickled on, spread on, rubbed in, sprinkled on or sprayed on. Solutions for use on the skin are prepared according to the state of the art and according to what is described above for injection solutions, sterile procedures not being necessary.

Gels are applied to or spread on the skin or introduced into body cavities. Gels are prepared by treating solutions which have been prepared as described in the case of the injection solutions with sufficient thickener that a clear material having an ointment-like consistency results. Suitable thickeners are known in the art.

Pour-on formulations are poured or sprayed onto limited areas of the skin, the active compound penetrating the skin and acting systemically. Pour-on formulations are prepared by dis-solving, suspending or emulsifying the active compound in suitable skin-compatible solvents or solvent mixtures. If appropriate, other auxiliaries e.g. colorants, bioabsorption-promoting substances, antioxidants, light stabilizers, adhesives are added. Suitable such auxiliaries are known in the art. Emulsions can be administered orally, dermally or as injections. Emulsions are either of the water-in-oil type or of the oil-in-water type. They are prepared by dissolving the active com-pound either in the hydrophobic or in the hydrophilic phase and homogenizing this with the solvent of the other phase with the aid of suitable emulsifiers and, if appropriate, other auxiliaries e.g. colorants, absorption-promoting substances, preservatives, antioxidants, light stabilizers, viscosity-enhancing substances. Suitable hydrophobic phases (oils), suitable hydrophilic phases, suitable emulsifiers, and suitable further auxiliaries for emulsions are known in the art.

Suspensions can be administered orally or topically/dermally. They are prepared by suspending the active compound in a suspending agent, if appropriate with addition of other auxiliaries e.g. wetting agents, colorants, bioabsorption-promoting substances, preservatives, antioxidants, light stabilizers. Suitable suspending agents, and suitable other auxiliaries for suspensions including wetting agents are known in the art.

Semi-solid preparations can be administered orally or topically/dermally. They differ from the suspensions and emulsions described above only by their higher viscosity.

For the production of solid preparations, the active compound is mixed with suitable excipients, if appropriate with addition of auxiliaries, and brought into the desired form. Suitable auxiliaries for this purpose are known in the art.

The compositions which can be used in the invention can comprise generally from about 0.001 to 95% of the compound of the invention.

Ready-to-use preparations contain the compounds acting against parasites, preferably ectoparasites, in concentrations of 10 ppm to 80% by weight, preferably from 0.1 to 65% by weight, more preferably from 1 to 50% by weight, most preferably from 5 to 40% by weight. Preparations which are diluted before use contain the compounds acting against ectoparasites in concentrations of 0.5 to 90% by weight, preferably of 1 to 50% by weight.

Furthermore, the preparations comprise the compounds of formula I against endoparasites in concentrations of 10 ppm to 2% by weight, preferably of 0.05 to 0.9% by weight, very particularly preferably of 0.005 to 0.25% by weight.

Topical application may be conducted with compound-containing shaped articles e.g. collars, medallions, ear tags, bands for fixing at body parts, and adhesive strips and foils.

Generally it is favorable to apply solid formulations which release compounds of the invention in total amounts of 10 mg/kg to 300 mg/kg, preferably 20 mg/kg to 200 mg/kg, most preferably 25 mg/kg to 160 mg/kg body weight of the treated animal in the course of three weeks.

### A. Preparation examples

The compounds were characterized by melting point determination, by NMR spectroscopy or by the mass-to-charge ratio ([m/z]) and retention time (RT; [min.]), as determined by mass spectrometry (MS) coupled with HPLC analysis (HPLC-MS = high performance liquid chromatography-coupled mass spectrometry),LC analysis (LC-MS = liquid chromatography-coupled mass spectrometry) or chiral SFC (SFC = supercritical fluid chromatography).

Method A: Shimadzu Nexera LC-20 LCMS-2020, ESI; Column: Kinetex XB C18 1.7µm, 50 x 2.1mm; Mobile phase: A: water + 0.1% TFA; B: ACN; 5% B auf 100% B in 1.5 min; Temperature: 60 °C; Flow 0.8 mL/min to 1.0 mL/min in 1.5 min.

Method B: Shimadzu Nexera LC-40 LCMS-2050, ESI; Column: Kinetex XB C18 1.7µm, 50 x 2.1mm; Mobile phase: A: water + 0.1% TFA; B: ACN; 5% B auf 100% B in 1.5 min; Temperature: 60 °C; Flow 0.8 mL/min to 1.0 mL/min in 1.5 min.

### Synthesis of the intermediats:

### Amine intermediats:

Amine intermediats can be synthesized in the following way:
Due to the potential for degradation of the amine intermediates, these intermediates can be stored in the form of Boc-protected or sulfinamide-protected intermediates. Prior to their use in the subsequent nucleophilic substitution reaction, they can be deprotected in the following, generally applicible, way:
To a solution of 2-methyl-N-[1-[3-(4-methyl-5-oxo-1,3,4-oxadiazin-2-yl)pyrazin-2-yl]ethyl]propane-2-sulfinamide (0.05 g, 147.31 µmol, 1 eq) in dioxane (0.5 mL) was added HCl/dioxane (8 M, 184.14 µL, 10 eq) at 0°C The mixture was stirred at 10°C for 0.5hr. LC-MS showed that the starting material was consumed completely and one main peak with desired mass was detected. The reaction mixture was concentrated under reduced pressure to give a residue. The crude product was used into the next step without further purification. 2-[3-(1-aminoethyl)pyrazin-2-yl]-4-methyl-1,3,4-oxadiazin-5-one (0.03 g, 110.41 µmol, 74.95% yield, HCl) was obtained as a yellow solid. LC-MS : desired mass : 235.1, observed mass : 235.9.

The solution of tert-butyl N-[1-[3-(6,6-dimethyl-5-oxo-4H-1,3,4-oxadiazin-2-yl)pyrazin-2-yl]ethyl]-N-methyl-carbamate (4.0 g, 11.01 mmol, 1 eq) in 4M HCl in dioxane (100 mL) was stirred at 25 °C for 1h. LC-MS showed that the starting material was consumed completely and one main peak with desired mass was detected. The reaction mixture was concentrated under reduced pressure to give 1-[3-(6,6-dimethyl-5-oxo-4H-1,3,4-oxadiazin-2-yl)pyrazin-2-yl]ethyl-methyl-ammonium chloride was obtained as a yellow solid The crude product was used in the next step without further purification. LC-MS : desired mass : 264.3 , observed mass : 264.2.

### Intermediate 1 : Synthesis of 2-methyl-N-[1-[3-(4-methyl-5-oxo-1,3,4-oxadiazin-2-yl)pyrazin-2-yl]ethyl]propane-2-sulfinamide (Int-1) :

Step 1 : To a solution of 3-chloropyrazine-2-carboxylic acid (50 g, 315.37 mmol, 1 eq) in DCM (500 mL) was added DIEA (122.28 g, 946.12 mmol, 164.79 mL, 3 eq) at 0°C, and then added tert-butyl N-amino-N-methyl-carbamate (69.16 g, 473.06 mmol, 1.5 eq) in DCM (200 mL) and PyBOP (213.35 g, 409.98 mmol, 1.3 eq) in DCM (400 mL) at the same time at 0°C dropwise over 4 hr. The mixture was stirred at 0-20 °C for 1hr. LCMS showed the reaction was completed. The reaction mixture was quenched with H₂O (300 mL), and then extracted with DCM (300 mL x 3). The combined organic layers were washed with aqueous NaCl (100 mL), dried over Na₂SO₄, filtered and concentrated, purified by column (Petroleum ether/Ethyl acetate=10/1 to 2/1) to give tert-butyl N-[(3-chloropyrazine-2-carbonyl)amino]-N-methyl-carbamate (162 g, 565.02 mmol, 89.58% yield) as yellow oil. 1H NMR (400 MHz, CDCl₃) δ [ppm] = 8.89 - 9.45 (m, 1 H) 8.59 (d, J=2.13 Hz, 1 H) 8.51 (d, J=2.13 Hz, 1 H) 3.27 (s, 3 H) 1.48 (br s, 9 H)
Step 2 : To a solution of tert-butyl N-[(3-chloropyrazine-2-carbonyl)amino]-N-methyl-carbamate (54 g, 188.34 mmol, 1 eq) in EtOAC/HCl (350 mL) .The mixture was stirred at 20°C for 2hr. LCMS showed the reaction was completed. The reaction mixture was filtrated and the filter cake was concentrated to give 3-chloro-N'-methyl-pyrazine-2-carbohydrazide (104 g, 466.24 mmol, 82.52% yield, HCl) as a white solid. 1H NMR (400 MHz, DMSO-d₆) δ [ppm] = 8.79 (d, J=2.50 Hz, 1 H) 8.76 (d, J=2.50 Hz, 1 H) 2.84 (s, 3 H)
Step 3 : To a solution of tert-butyl N-[(3-chloropyrazine-2-carbonyl)amino]-N-methyl-carbamate (31 g, 138.98 mmol, 1 eq, HCl) in DMF (500 mL) was added K₂CO₃ (48.02 g, 347.44 mmol, 2.5 eq) and 2-chloroacetyl chloride (18.84 g, 166.77 mmol, 13.28 mL, 1.2eq) .The mixture was stirred at 20°C for 2.5hr, and then stirred at 45°C for 3hr. TLC (PE: EtOAc = 3:1) showed the reaction was completed. The reaction mixture was quenched with H₂O (2000 mL), and then extracted with EtOAc (500 mL x 4). The combined organic layers were dried over Na₂SO₄, filtered and concentrated to give 2-(3-chloropyrazin-2-yl)-4-methyl-1,3,4-oxadiazin-5-one (total 104 g, 413.03 mmol, 99.06% yield, 90% purity) as a yellow solid. 1H NMR (400 MHz, DMSO-d₆) δ [ppm] = 8.77 (d, J=2.47 Hz, 1 H) 8.68 (d, J=2.35 Hz, 1 H) 4.92 (s, 2 H) 3.28 (s, 3 H)
Step 4: To a solution of 2-(3-chloropyrazin-2-yl)-4-methyl-1,3,4-oxadiazin-5-one (25 g, 110.32 mmol, 1 eq) in Tol. (1000 mL) was added Pd(PPh₃)₄ (6.37 g, 5.52 mmol, 0.05 eq) and tributyl(1-ethoxyvinyl)stannane (47.81 g, 132.38 mmol, 44.72 mL, 1.2 eq). The mixture was stirred at 120°C for 12hr under N₂. LCMS showed the reaction was completed. The reaction mixture was quenched by addition aq.KF (1000 mL), and then filtered, and the filter cake was washed with EtOAc (300 mL x 4) and the filtrate was extracted with EtOAc (1200 mL x 2). The combined organic layers were dried over Na₂SO₄, filtered and concentrated to give 2-[3-(1-ethoxyvinyl)pyrazin-2-yl]-4-methyl-1,3,4-oxadiazin-5-one (110 g, 419.42 mmol, 95.05% yield) as a brown oil and used directly.
Step 5: To a solution of 2-[3-(1-ethoxyvinyl)pyrazin-2-yl]-4-methyl-1,3,4-oxadiazin-5-one (110 g, 419.42 mmol, 1 eq) in MeCN (850 mL) was added HCl (2 M, 419.42 mL, 2 eq) at 0°C.The mixture was stirred at 0 °C for 1hr . TLC (PE: EtOAc = 0:1) showed the reaction was completed. The reaction mixture was quenched by addition H₂O (1000 mL), and then extracted with EtOAc (1000 mL x 4). The combined organic layers were dried over Na₂SO₄, filtered and concentrated, triturating with MTBE (30 mL) to give 2-(3-acetylpyrazin-2-yl)-4-methyl-1,3,4-oxadiazin-5-one (90 g, 384.27 mmol, 91.62% yield) as a yellow solid. 1H NMR (400 MHz, CDCl₃) δ [ppm] = 8.77 (d, J=2.47 Hz, 1 H) 8.68 (d, J=2.35 Hz, 1 H) 4.92 (s, 2 H) 3.28 (s, 3 H)
Step 6: To a solution of 2-(3-acetylpyrazin-2-yl)-4-methyl-1,3,4-oxadiazin-5-one (10 g, 42.70 mmol, 1 eq) in THF (120 mL) was added Ti(i-PrO)₄ (30.34 g, 106.74 mmol, 31.50 mL, 2.5 eq) and 2-methylpropane-2-sulfinamide (10.35 g, 85.39 mmol, 2 eq) The mixture was stirred at 80°C for 16hr. TLC (EtOAc: MeOH = 10:1) showed the reaction was completed. The reaction mixture was quenched with H₂O (100 mL), and then filtrated, washed with EtOAc (50 mL x 6) and extracted with EtOAc (300 mL), DCM: i-PrOH=3:1 (100 mL x 3). The combined organic layers were dried over Na₂SO₄, filtered and concentrated, the crude was triturated with EtOAc (100 mL), filtered to give (NE)-2-methyl-N-[1-[3-(4-methyl-5-oxo-1,3,4-oxadiazin-2-yl)pyrazin-2-yl]ethylidene]propane-2-sulfinamide (40 g, 118.55 mmol, 92.56% yield) as a yellow solid. 1H NMR (400 MHz, CDCl₃) δ [ppm] = 8.67 (dd, J=12.88, 1.88 Hz, 1 H) 8.58 (br d, J=5.00 Hz, 1 H) 4.68 - 5.02 (m, 2 H) 3.39 (s, 3 H) 2.51 - 2.87 (m, 3 H) 1.22 (s, 9 H).
Step 7: To a solution of (NE)-2-methyl-N-[1-[3-(4-methyl-5-oxo-1,3,4-oxadiazin-2-yl)pyrazin-2-yl]ethylidene]propane-2-sulfinamide (5 g, 14.82 mmol, 1 eq) in THF (1000 mL) and MeOH (500 mL) was added NaBH₄ (560.65 mg, 14.82 mmol, 1 eq) at 0°C under N₂ The mixture was stirred 0°C for 0.5hr under N₂ . TLC (EtOAc: MeOH = 10:1) showed the reaction was completed. 8 reactions were combined for workup. The reaction mixture was quenched by addition H₂O 1000 mL, and then extracted with EtOAc (1000 mL), DCM: i-PrOH=3:1 (300 mL x 3). The combined organic layers were dried over Na₂SO₄, filtered and concentrated, purified by prep-HPLC (neutral) to give 2-methyl-N-[1-[3-(4-methyl-5-oxo-1,3,4-oxadiazin-2-yl)pyrazin-2-yl]ethyl]propane-2-sulfinamide (13 g, 38.30 mmol, 32.31% yield) as a yellow solid. 1H NMR (400 MHz, CDCl₃) δ [ppm] = 8.61 (d, J=2.25 Hz, 1 H) 8.56 (d, J=2.38 Hz, 1 H) 5.38 (dd, J=8.94, 6.69 Hz, 1 H) 4.93 (br d, J=9.01 Hz, 1 H) 4.87 (s, 2 H) 3.46 (s, 3 H) 1.53 (d, J=6.63 Hz, 3 H) 1.24 (s, 9 H) ; LC-MS : desired mass : 339.1, observed mass: 340.1.

### Intermediate 2 : Synthesis of 2-methyl-N-[1-[3-(5-oxo-4H-1,3,4-oxadiazin-2-yl)pyrazin-2-yl]ethyl]propane-2-sulfinamide (Int-2):

Step 1 : To a solution of 3-chloropyrazine-2-carboxylic acid (35 g, 220.76 mmol, 1 eq) in DCM (350 mL) was added TEA (67.02 g, 662.28 mmol, 92.18 mL, 3 eq), T₄P (238.59 g, 331.14 mmol, 50% purity, 1.5 eq), and tert-butyl N-aminocarbamate (32.09 g, 242.84 mmol, 1.1 eq) at 20°C and then stirred for 2 h at 20°C, the reaction mixture was quenched with aq.NaHCO₃ (200 mL), extracted with DCM (500 mL x 2), washed with aqueous NaCl (200 mL), dried over Na₂SO₄, filtrated and concentrated to give product. The residue was purified by column chromatography (SiO₂, Petroleum ether/Ethyl acetate=3/1 to 1/1) to give tert-butyl N-[(3-chloropyrazine-2-carbonyl)amino]carbamate (98 g, 359.39 mmol, 81.40% yield) as a yellow solid. 1H NMR (400 MHz, CDCl₃) δ [ppm] = 9.18 (br s, 1 H) 8.59 (d, J=2.25 Hz, 1 H) 8.51 (d, J=2.25 Hz, 1 H) 6.71 (br s, 1 H) 1.51 (s, 9 H)
Step 2 : To a solution of tert-butyl N-[(3-chloropyrazine-2-carbonyl)amino]carbamate (86 g, 315.38 mmol, 1 eq) in EtOAC/HCl (600 mL) was stirred at 25°C for 2hr. LCMS showed the reaction was completed. The reaction mixture was filtrated and the filter cake was concentrated to give 3-chloropyrazine-2-carbohydrazide (63 g, 301.39 mmol, 95.56% yield, HCl) as a yellow solid. 1H NMR (400 MHz MeOD) δ [ppm] = 8.67 - 8.70 (m, 2 H)
Step 3: To a solution of 3-chloropyrazine-2-carbohydrazide (20 g, 95.68 mmol, 1 eq, HCl) in MeCN (300 mL) was added K₂CO₃ (39.67 g, 287.04 mmol, 3 eq) and 2-chloroacetyl chloride (11.89 g, 105.25 mmol, 8.38 mL, 1.1 eq) at 25°C. The mixture was stirred at 25°C for 2hr. LCMS showed the reaction was completed. The reaction mixture was filtrated and the filter cake was washed with MeCN (50 mL x 3), the filtrate was concentrated to give 3-chloro-N'-(2-chloroacetyl)pyrazine-2-carbohydrazide (49.7 g, crude) as a yellow solid. 1H NMR (400 MHz, DMSO-d₆) δ [ppm] = 10.92 (s, 1 H) 10.71 (d, J=1.00 Hz, 1 H) 8.75 (d, J=2.50 Hz, 1 H) 8.69 (d, J=2.38 Hz, 1 H) 4.21 (s, 2 H)
Step 4: To a solution of 3-chloro-N'-(2-chloroacetyl)pyrazine-2-carbohydrazide (12 g, 48.18 mmol, 1 eq) in DMF (120 mL) was added NaHCO₃ (40.47 g, 481.82 mmol, 18.75 mL, 10 eq) at 25°C. The mixture was stirred at 100°C for 1.5hr. LCMS showed the reaction was completed. The reaction mixture was quenched with H₂O (300 mL), and then extracted with EtOAc (200 mL x 2) and DCM:i-PrOH=3:1 (100 mL x 2). The combined organic layers were dried over Na₂SO₄, filtered and concentrated under reduced pressure to give a residue. The crude product was triturated with MTBE (25 mL) to give 2-(3-chloropyrazin-2-yl)-4H-1,3,4-oxadiazin-5-one (19 g, 89.37 mmol, 46.37% yield) as a yellow solid. 1H NMR (400 MHz, DMSO-d₆) δ [ppm] = 11.32 (s, 1 H) 8.76 (d, J=2.38 Hz, 1 H) 8.66 (d, J=2.38 Hz, 1 H) 4.86 (s, 2 H)
Step 5 : To a solution of 2-(3-chloropyrazin-2-yl)-4H-1,3,4-oxadiazin-5-one (2.9 g, 13.64 mmol, 1 eq) in dioxane (300 mL) was added Pd(PPh₃)₂Cl₂ (957.46 mg, 1.36 mmol, 0.1 eq) and tributyl(1-ethoxyvinyl)stannane (6.40 g, 17.73 mmol, 5.99 mL, 1.3 eq). The mixture was stirred at 120°C for 36hr under N₂. LCMS showed the reaction was completed. The reaction mixture was quenched by addition aq. KF (500 mL), extracted with EtOAc (300 mL x 4). The combined organic layers were dried over Na₂SO₄, filtered and concentrated under reduced pressure to give a residue. The residue was purified by flash silica gel chromatography (ISCO^{®}; 120 g SepaFlash^{®} Silica Flash Column, Eluent of 0~60% Ethyl acetate/Petroleum ethergradient @ 150 mL/min) to give 2-[3-(1-ethoxyvinyl)pyrazin-2-yl]-4H-1,3,4-oxadiazin-5-one (16 g crude) as a yellow solid and used directly.
Step 6 : To a solution of 2-[3-(1-ethoxyvinyl)pyrazin-2-yl]-4H-1,3,4-oxadiazin-5-one (8 g, 32.23 mmol, 1 eq) in MeCN (80 mL) was added HCl (2 M, 32.23 mL, 2 eq) at 0°C. The mixture was stirred at 0 °C for 1hr under N₂. TLC (PE: EtOAc = 1:1) showed the reaction was completed. The reaction mixture was quenched by addition H₂O (100 mL), and extracted with EtOAc (100 mL * 4). The combined organic layers were dried over Na₂SO₄, filtered and concentrated under reduced pressure to give a residue.The residue was purified by triturated and flash silica gel chromatography (ISCO^{®}; 40 g SepaFlash^{®} Silica Flash Column, Eluent of 0~60% Ethyl acetate/Petroleum ethergradient @ 80 mL/min) to give 2-(3-acetylpyrazin-2-yl)-4H-1,3,4-oxadiazin-5-one (9 g, 40.87 mmol, 63.42% yield) was obtained as a yellow solid. 1H NMR (400 MHz, CDCl₃) δ [ppm] = 8.77 (d, J=2.38 Hz, 1 H) 8.70 (d, J=2.38 Hz, 1 H) 8.50 (br s, 1 H) 4.80 (s, 2 H) 2.74 (s, 3 H)
Step 7 : To a solution of 2-(3-acetylpyrazin-2-yl)-4H-1,3,4-oxadiazin-5-one (2 g, 9.08 mmol, 1 eq) in THF (30 mL) and 2-methylpropane-2-sulfinamide (1.65 g, 13.62 mmol, 1.5 eq) was added Ti(i-PrO)₄ (6.45 g, 22.71 mmol, 6.70 mL, 2.5 eq) at 20°C under N₂. The mixture was stirred 90°C for 12hr under N₂. TLC (PE: EtOAc = 0:1) showed the reaction was completed. The reaction mixture was quenched with H₂O (100 mL), and then filtrated, washed with EtOAc (100 mL x 3) and extracted with EtOAc (300 mL), DCM: i-PrOH=3:1 (100 mL x 2). The combined organic layers were dried over Na₂SO₄, filtered and concentrated under reduced pressure to give a residue. The residue was purified by flash silica gel chromatography (ISCO^{®}; 40 g SepaFlash^{®} Silica Flash Column, Eluent of 0~80% Ethyl acetate/Petroleum ethergradient @ 80 mL/min) to give (NE)-2-methyl-N-[1-[3-(5-oxo-4H-1,3,4-oxadiazin-2-yl)pyrazin-2-yl]ethylidene]propane-2-sulfinamide (4 g, 12.37 mmol, 68.09% yield) was obtained as a yellow solid. 1H NMR (400 MHz, CDCl₃) δ [ppm] = 8.68 (dd, J=11.88, 2.00 Hz, 1 H) 8.60 (br d, J=4.50 Hz, 1 H) 8.41 - 8.50 (m, 1 H) 4.74 - 5.08 (m, 2 H) 2.52 - 2.86 (m, 3 H) 1.23 - 1.24 (m, 9 H).
Step 8: To a solution of (NE)-2-methyl-N-[1-[3-(5-oxo-4H-1,3,4-oxadiazin-2-yl)pyrazin-2-yl]ethylidene]propane-2-sulfinamide (2 g, 6.18 mmol, 1 eq) in THF (36 mL) and MeOH (12 mL) was added NaBH₄ (163.78 mg, 4.33 mmol, 0.7 eq) at 0°C under N₂. The mixture was stirred at 0 °C for 0.5hr. LCMS showed the reaction was completed. The reaction mixture was quenched by addition H₂O (40 mL) at 0 °C, extracted with EtOAc (30 mL x 2) and DCM:iPrOH=3:1 (30 mL x 2). The combined organic layers were dried over Na₂SO₄, filtered and concentrated under reduced pressure to give a residue. The residue was purified by triturated with EtOAc (3 mL) and prep-HPLC (neutral condition) to give 2-methyl-N-[1-[3-(5-oxo-4H-1,3,4-oxadiazin-2-yl)pyrazin-2-yl]ethyl]propane-2-sulfinamide (1.3 g, 4.00 mmol, 32.30% yield) as a white solid. 1H NMR (400 MHz, DMSO-d₆) δ [ppm] = 11.26 (s, 1 H) 8.74 (d, J=2.38 Hz, 1 H) 8.63 (d, J=2.38 Hz, 1 H) 5.56 (d, J=8.88 Hz, 1 H) 5.17 - 5.27 (m, 1 H) 4.85 (s, 2 H) 1.45 (d, J=6.75 Hz, 3 H) 1.07 (s, 9 H) LC-MS : desired mass : 325.1, observed mass : 326.2.

### Intermediate 3: Synthesis of 2-methyl-N-[1-[3-(4,6,6-trimethyl-5-oxo-1,3,4-oxadiazin-2-yl)pyrazin-2-yl]ethyl]propane-2-sulfinamide (Int-3) :

Step 1 : To a solution of 3-chloro-N'-methyl-pyrazine-2-carbohydrazide (20 g, 89.66 mmol, 1 eq, HCl) in MeCN (600 mL) was added K₂CO₃ (30.98 g, 224.16 mmol, 2.5 eq) and 2-chloro-2-methylpropanoyl chloride (15.17 g, 107.59 mmol, 1.2 eq) at 0°C. The mixture was stirred at 0~30 °C for 2hr. TLC (PE: EA= 1:1) showed that the starting material was consumed completely. The reaction mixture was quenched by addition H₂O 500 mL at 0 °C, and then extracted with EA 600 mL (200 mL x 3). The combined organic layers were dried over Na₂SO₄, filtered and concentrated under reduced pressure to give 3-chloro-N'-(2-chloro-2-methyl-propanoyl)-N'-methyl-pyrazine-2-carbohydrazide (27 g, Yield: crude) as yellow oil. 1H NMR (400 MHz, CDCl₃) δ [ppm] = 9.77 (br s, 1 H) 8.64 (d, J=2.13 Hz, 1 H) 8.56 (d, J=2.25 Hz, 1 H) 3.40 (br s, 3 H) 1.79 (s, 6 H)
Step 2: To a solution of 3-chloro-N'-(2-chloro-2-methyl-propanoyl)-N'-methyl-pyrazine-2-carbohydrazide (27 g, 92.74 mmol, 1 eq) in DMSO (270 mL) was added NaHCO₃ (77.91 g, 927.41 mmol, 36.09 mL, 10 eq) The mixture was stirred at 100°C for 1hr. TLC (PE: EA= 1:1) showed 3-chloro-N'-(2-chloro-2-methyl-propanoyl)-N'-methyl-pyrazine-2-carbohydrazide was consumed completely. The reaction mixture was quenched by addition H₂O 1000 mL at 0 °C, and then extracted with EA 800 mL (400 mL x 2). The combined organic layers were washed with aqueous NaCl 500 mL, dried over Na₂SO₄, filtered and concentrated under reduced pressure to give a residue. The residue was purified by flash silica gel chromatography (ISCO^{®}; 220 g SepaFlash^{®} Silica Flash Column,
   Eluent of 0~40% ethyl acetate/petroleum ethergradient @ 120 mL/min) to give 2-(3-chloropyrazin-2-yl)-4,6,6-trimethyl-1,3,4-oxadiazin-5-one (20.6 g, 87.22%) as yellow oil. 1H NMR (400 MHz, CDCl₃) δ [ppm] = 8.60 (d, J=2.38 Hz, 1 H) 8.47 (d, J=2.38 Hz, 1 H) 3.43 (s, 3 H) 1.69 (s, 6 H)
Step 3 : To a solution of 2-(3-chloropyrazin-2-yl)-4,6,6-trimethyl-1,3,4-oxadiazin-5-one (10.3 g, 40.44 mmol, 1 eq) in toluene (200 mL) was added Pd(PPh₃)₄ (2.34 g, 2.02 mmol, 0.05 eq) and tributyl(1-ethoxyvinyl)stannane (17.53 g, 48.53 mmol, 16.40 mL, 1.2 eq). The mixture was stirred at 110 °C for 16hr. TLC (PE: EA= 1:1) showed 2-(3-chloropyrazin-2-yl)-4,6,6-trimethyl-1,3,4-oxadiazin-5-one was consumed completely. The reaction mixture was quenched by addition KF aq 1000 mL at 0 °C, and then extracted with EA 700 mL (350 mL x 2). The combined organic layers were dried over Na₂SO₄, filtered and concentrated under reduced pressure to give 2-[3-(1-ethoxyvinyl)pyrazin-2-yl]-4,6,6-trimethyl-1,3,4-oxadiazin-5-one (23 g, Yield: crude) as yellow oil.
Step 4 : To a solution of 2-[3-(1-ethoxyvinyl)pyrazin-2-yl]-4,6,6-trimethyl-1,3,4-oxadiazin-5-one (23 g, 79.22 mmol, 1 eq) in MeCN (200 mL) was added HCl (2 M, 79.22 mL, 2 eq) at 0°C. The mixture was stirred at 0~25 °C for 2hr. TLC (PE: EA= 1:1) showed 2-[3-(1-ethoxyvinyl)pyrazin-2-yl]-4,6,6-trimethyl-1,3,4-oxadiazin-5-one was consumed completely. The reaction mixture was quenched by addition H₂O 400 mL at 0 °C, and then extracted with EA 500 mL (250 mL x 2). The combined organic layers were washed with aqueous NaCl 300 mL, dried over Na₂SO₄, filtered and concentrated under reduced pressure to give a residue. The residue was purified by flash silica gel chromatography (ISCO^{®}; 220 g SepaFlash^{®} Silica Flash Column, Eluent of 0-45% Ethyl acetate/Petroleum ethergradient @ 120 mL/min) to give 2-(3-acetylpyrazin-2-yl)-4,6,6-trimethyl-1,3,4-oxadiazin-5-one (19.7 g, 75.12 mmol, 94.81% yield) as a yellow oil. 1H NMR (400 MHz, CDCl₃) δ [ppm] = 8.73 (d, J=2.50 Hz, 1 H) 8.62 (d, J=2.38 Hz, 1 H) 3.37 (s, 3 H) 2.71 (s, 3 H) 1.66 (s, 6 H)
Step 5: To a solution of 2-(3-acetylpyrazin-2-yl)-4,6,6-trimethyl-1,3,4-oxadiazin-5-one (4 g, 15.25 mmol, 1 eq) and 2-methylpropane-2-sulfinamide (2.77 g, 22.88 mmol, 1.5 eq) in THF (80 mL) was added Ti(i-PrO)₄ (10.84 g, 38.13 mmol, 11.25 mL, 2.5 eq). The mixture was stirred at 80 °C for 16hr. TLC (PE: EA= 0:1) showed 2-(3-acetylpyrazin-2-yl)-4,6,6-trimethyl-1,3,4-oxadiazin-5-one was consumed completely. The reaction mixture was quenched by addition H₂O 200 mL at 0 °C, and then extracted with EA 300 mL (100 mL x 3). The combined organic layers were dried over Na₂SO₄, filtered and concentrated under reduced pressure to give a residue. The crude product was triturated with MeCN at 25°C for 30 min, then triturated with EA at 25°C for 60 min to give (NE)-2-methyl-N-[1-[3-(4,6,6-trimethyl-5-oxo-1,3,4-oxadiazin-2-yl)pyrazin-2-yl]ethylidene]propane-2-sulfinamide (13 g, Yield: 77.75%) as yellow solid. 1H NMR (400 MHz, CDCl₃) δ [ppm] = 8.46 - 8.76 (m, 2 H) 3.39 (s, 3 H) 2.49 - 2.89 (m, 3 H) 1.65 (br d, J=4.95 Hz, 3 H) 1.61 (d, J=7.05 Hz, 3 H) 1.23 (d, J=4.45 Hz, 9 H)
Step 6: To a solution of (NE)-2-methyl-N-[1-[3-(4,6,6-trimethyl-5-oxo-1,3,4-oxadiazin-2-yl)pyrazin-2-yl]ethylidene]propane-2-sulfinamide (3 g, 8.21 mmol, 1 eq) in THF (32 mL) and MeOH (8 mL) was added NaBH₄ (310.55 mg, 8.21 mmol, 1 eq) at 0°C. The mixture was stirred at 0 °C for 1hr. TLC (DCM: MeOH= 20:1) showed (NE)-2-methyl-N-[1-[3-(4,6,6-trimethyl-5-oxo-1,3,4-oxadiazin-2-yl)pyrazin-2-yl]ethylidene]propane-2-sulfinamide was consumed completely. The reaction mixture was quenched by addition H₂O 300 mL at 0 °C, and then extracted with EA 600 mL (200 mL x 3). The combined organic layers were dried over Na₂SO₄, filtered and concentrated under reduced pressure to give a residue. The residue was purified by prep-HPLC (neutral condition) give two isomers:
   The peak 1 give 2 g crude and then purified by prep-HPLC give 2-methyl-N-[1-[3-(4,6,6-trimethyl-5-oxo-1,3,4-oxadiazin-2-yl)pyrazin-2-yl]ethyl]propane-2-sulfinamide (P1) (1 g, Yield: 16.6%) as yellow solid.

The peak 2 give 2 g crude and then purified by column (PE in (EA :MeOH= 50:1)= 0-70%) to give 2-methyl-N-[1-[3-(4,6,6-trimethyl-5-oxo-1,3,4-oxadiazin-2-yl)pyrazin-2-yl]ethyl]propane-2-sulfinamide (P2) (1.6 g, Yield: 26.5%) as a white solid.

P1 : 1H NMR (400 MHz, DMSO-d₆) δ [ppm] = 8.77 (d, J=2.25 Hz, 1 H) 8.67 (d, J=2.38 Hz, 1 H) 5.49 (d, J=8.25 Hz, 1 H) 5.26 - 5.39 (m, 1 H) 3.31 (s, 3 H) 1.57 (d, J=6.75 Hz, 3 H) 1.53 (d, J=10.38 Hz, 6 H) 1.03 (s, 9 H); LC-MS : desired mass: 367.1, observed mass: 368.1.

P2 : 1H NMR (400 MHz, DMSO-d₆) δ [ppm] = 8.77 (d, J=2.25 Hz, 1 H) 8.67 (d, J=2.25 Hz, 1 H) 5.53 (d, J=8.38 Hz, 1 H) 5.20 (dd, J=8.07, 6.94 Hz, 1 H) 3.32 (s, 3 H) 1.55 (d, J=10.13 Hz, 6 H) 1.50 (d, J=6.75 Hz, 3 H) 1.07 (s, 9 H); LC-MS : desired mass: 367.1, observed mass: 368.1.

The stereocenters of the two diastereomers could not be assigned to the respective molecular structures.

### Intermediate 4: Synthesis of N-[1-[3-(6,6-dimethyl-5-oxo-4H-1,3,4-oxadiazin-2-yl)pyrazin-2-yl]ethyl]-2-methyl-propane-2-sulfinamide (Int-4):

Step 1: To a solution of 3-chloropyrazine-2-carboxylic acid (70 g, 441.52 mmol, 1 eq) and tert-butyl N-aminocarbamate (89.34 g, 529.83 mmol, 1.2 eq, HCl) in DCM (700 mL) was added T₄P (477.19 g, 662.28 mmol, 50% purity, 1.5 eq) and TEA (134.03 g, 1.32 mol, 184.36 mL, 3 eq). The mixture was stirred at 25 °C for 12 h. LCMS showed 3-chloropyrazine-2-carboxylic acid was consumed completely and one main peak with desired mass was detected. After the completion of the reaction, it was poured into sat. aq. NaHCO₃ (1000 mL), extracted with DCM (50 mL x 3). The combined organic layers were washed with brine (100 mL), dried over Na₂SO₄, filtered and concentrated in vacuum to afford the title compound tert-butyl N-[(3-chloropyrazine-2-carbonyl)amino]carbamate (117 g, 399.03 mmol, 90.38% yield, 93% purity) as a brown oil, which was used to the next step without purification.
¹H NMR: (400 MHz, DMSO-d₆)
δ [ppm] = 10.61 - 10.30 (m, 1H), 9.15 (br s, 1H), 8.78 - 8.62 (m, 2H), 1.43 (s, 9H)

Step 2: A solution of tert-butyl N-[(3-chloropyrazine-2-carbonyl)amino]carbamate (117 g, 429.06 mmol, 1 eq) in HCl/EtOAc (1 L) was stirred at 25 °C for 1 h. LCMS showed tert-butyl N-[(3-chloropyrazine-2-carbonyl)amino]carbamate was consumed completely and one major peak with desired mass was detected. After the completion of the reaction, it was concentrated in vacuum. The mixture was triturated with EtOAc (1000 mL) for 30 min, filtered. The filter cake was washed with EtOAc, and then concentrated in vacuum to afford the title compound 3-chloropyrazine-2-carbohydrazide (110 g, crude, HCl) as a gray solid.

Step 3: To a solution of 3-chloropyrazine-2-carbohydrazide (108 g, 516.66 mmol, 1 eq, HCI), NaOAc (63.57 g, 775.00 mmol, 1.5 eq), TsOH (133.45 g, 775.00 mmol, 1.5 eq) and 4-methoxybenzaldehyde (105.51 g, 775.00 mmol, 94.29 mL, 1.5 eq) in THF (1 L) was stirred at 25 °C for 1 h. Then NaBH₃CN (64.93 g, 1.03 mol, 2 eq) was added at 0 °C. The mixture was stirred at 25 °C for another 11 h. LCMS showed 3-chloropyrazine-2-carbohydrazide was consumed completely and one peak with desired mass was detected. After the completion of the reaction, it was filtered and the filtrate was concentrated in vacuum. The residue was purified by flash silica gel chromatography (ISCO^{®}; 330 g SepaFlash^{®} Silica Flash Column, Eluent of 0-43% Ethylacetate/Petroleum ethergradient @ 150 mL/min). The residue was triturated with EtOAc (200 mL) for 30 min, filtered. The filter cake was washed with EtOAc (100 mL). Then the filtration was concentrated in vacuum. The product was dissolved with EtOAc, then it was washed with sat. aq. NaHCO₃ (50 mL x 3). The combined organic layers were washed with brine (100 mL), dried over Na₂SO₄, concentrated in vacuum to afford 3-chloro-N'-[(4-methoxyphenyl)methyl]pyrazine-2-carbohydrazide (89 g, 234.11 mmol, 65.89% yield, 77% purity) as a yellow oil.
1H NMR: (400 MHz, DMSO-d₆)
δ [ppm] = 10.08 (d, J = 6.3 Hz, 1H), 8.68 (d, J = 2.5 Hz, 1H), 8.61 (d, J = 2.5 Hz, 1H), 7.33 - 7.28 (m, 2H), 6.91 - 6.87 (m, 2H), 5.63 - 5.54 (m, 1H), 3.93 (d, J = 5.1 Hz, 2H), 3.73 (s, 3H)

Step 4: To a solution of 3-chloro-N'-[(4-methoxyphenyl)methyl]pyrazine-2-carbohydrazide (88 g, 231.48 mmol, 1 eq) and Pyridine (54.93 g, 694.45 mmol, 56.05 mL, 3 eq) in DCM (1 L) was added 2-chloro-2-methyl-propanoyl chloride (39.17 g, 277.78 mmol, 1.2 eq) at -40 °C. The mixture was stirred at -40 °C for 1 h. LCMS showed 3-chloro-N'-[(4-methoxyphenyl)methyl]pyrazine-2-carbohydrazide was consumed completely and one main peak with desired mass was detected. After the completion of the reaction, it was combined with another batch (1 g scale). The mixture was poured into H₂O (1 L), and extracted with DCM (50 mL x 3). The combined organic layers were washed with brine (100 mL x 2), dried over Na₂SO₄, filtered and concentrated in vacuum. The residue was purified by flash silica gel chromatography (ISCO^{®}; 120 g SepaFlash^{®} Silica Flash Column, Eluent of 0~20% Ethylacetate/Petroleum ethergradient @ 150 mL/min) to afford 3-chloro-N'-(2-chloro-2-methyl-propanoyl)-N'-[(4-methoxyphenyl)methyl]pyrazine-2-carbohydrazide (61.8 g, 129.12 mmol, 55.78% yield, 83% purity) as a yellow solid.
¹H NMR: (400 MHz, DMSO-d₆)
δ [ppm] = 11.26 (s, 1H), 8.76 (d, *J* = 2.5 Hz, 1H), 8.69 (d, *J* = 2.5 Hz, 1H), 7.25 (d, *J* = 8.6 Hz, 2H), 6.91 (d, *J* = 8.6 Hz, 2H), 5.51 - 4.10 (m, 2H), 3.73 (s, 3H), 1.80 (s, 6H)

Step 5: To a solution of 3-chloro-N'-(2-chloro-2-methyl-propanoyl)-N'-[(4-methoxyphenyl)methyl]pyrazine-2-carbohydrazide (60.8 g, 127.03 mmol, 1 eq) in EtOH (610 mL) was added K₂CO₃ (35.11 g, 254.06 mmol, 2 eq). The mixture was stirred at 50 °C for 12 h. LCMS showed 3-chloro-N'-(2-chloro-2-methyl-propanoyl)-N'-[(4-methoxyphenyl)methyl]pyrazine-2-carbohydrazide was consumed completely and one main peak with desired mass was detected. After the completion of the reaction, it was combined with another batch (1.0 g scale). The mixture was filtered and concentrated in vacuum. The residue was purified by flash silica gel chromatography (ISCO^{®}; 220 g SepaFlash^{®} Silica Flash Column, Eluent of 0~30% Ethyl acetate/Petroleum ethergradient @ 150 mL/min) to afford 2-(3-chloropyrazin-2-yl)-4-[(4-methoxyphenyl)methyl]-6,6-dimethyl-1,3,4-oxadiazin-5-one (44.7 g, 93% purity) as a yellow oil.
¹H NMR: (400 MHz, DMSO-d₆)
δ [ppm] = 8.77 (d, *J* = 2.4 Hz, 1H), 8.67 (d, *J* = 2.4 Hz, 1H), 7.28 - 7.23 (m, 2H), 6.94 - 6.88 (m, 2H), 4.80 (s, 2H), 3.73 (s, 3H), 1.56 (s, 6H).

Step 6. To a solution of 2-(3-chloropyrazin-2-yl)-4-[(4-methoxyphenyl)methyl]-6,6-dimethyl-1,3,4-oxadiazin-5-one (44 g, 121.95 mmol, 1 eq) in Tol. (500 mL) was added Pd(PPh₃)₄ (7.05 g, 6.10 mmol, 0.05 eq) and tributyl(1-ethoxyvinyl)stannane (60.99 g, 168.88 mmol, 57.05 mL, 1.38 eq). The mixture was stirred at 120 °C for 12 h under N₂. LCMS showed 2-(3-chloropyrazin-2-yl)-4-[(4-methoxyphenyl)methyl]-6,6-dimethyl-1,3,4-oxadiazin-5-one was consumed completely and one main peak with desired mass was detected. After the completion of the reaction, the reaction mixture was quenched by addition aq.KF (1 L), and then filtrated. The filter cake was washed with EtOAc (100 mL x 2) and the filtrate was extracted with EtOAc (200 mL x 3). The combined organic layers were dried over Na₂SO₄, filtered and concentrated in vacuum to afford 2-[3-(1-ethoxyvinyl)pyrazin-2-yl]-4-[(4-methoxyphenyl)methyl]-6,6-dimethyl-1,3,4-oxadiazin-5-one (48.35 g, crude) as a brown oil.

Step 7: To a solution of 2-[3-(1-ethoxyvinyl)pyrazin-2-yl]-4-[(4-methoxyphenyl)methyl]-6,6-dimethyl-1,3,4-oxadiazin-5-one (48.35 g, 121.96 mmol, 1 eq) in MeCN (250 mL) was added HCl (2 M, 243.92 mL, 4 eq). The mixture was stirred at 25 °C for 2 h. LCMS showed 2-[3-(1-ethoxyvinyl)pyrazin-2-yl]-4-[(4-methoxyphenyl)methyl]-6,6-dimethyl-1,3,4-oxadiazin-5-one was consumed completely and one main peak with desired mass was detected. After the completion of the reaction, the reaction mixture was poured into H₂O (500 mL), and then extracted with EtOAc (100 mL x 3). The combined organic layers were washed with brine (100 mL x 2), dried over Na₂SO₄, filtered and concentrated in vacuum. The residue was purified by flash silica gel chromatography (ISCO^{®}; 220 g SepaFlash^{®} Silica Flash Column, Eluent of 0-33% Ethylacetate/Petroleum ethergradient @ 60 mL/min) to afford 2-(3-acetylpyrazin-2-yl)-4-[(4-methoxyphenyl)methyl]-6,6-dimethyl-1,3,4-oxadiazin-5-one (39 g, 100.36 mmol, 82.29% yield, 94.8% purity) as a yellow oil.
1H NMR: (400 MHz, DMSO-d₆)
δ [ppm] = 8.85 (d, J = 2.5 Hz, 1H), 8.80 (d, J = 2.5 Hz, 1H), 7.23 - 7.17 (m, 2H), 6.93 - 6.88 (m, 2H), 5.75 (s, 1H), 4.72 (s, 2H), 3.73 (s, 3H), 2.52 (d, J = 1.9 Hz, 3H), 1.53 (s, 6H)

Step 8: A solution of 2-(3-acetylpyrazin-2-yl)-4-[(4-methoxyphenyl)methyl]-6,6-dimethyl-1,3,4-oxadiazin-5-one (36 g, 97.72 mmol, 1 eq) in TFA (100 mL) and CF₃SO₃H (20 mL) was stirred at 25 °C for 1 h. LCMS showed 2-(3-acetylpyrazin-2-yl)-4-[(4-methoxyphenyl)methyl]-6,6-dimethyl-1,3,4-oxadiazin-5-one was remained and one main peak with desired mass was detected. After the completion of the reaction, the reaction mixture was added dropwise to sat. aq. NaHCO₃ (1.5 L), and then extracted with EtOAc (100 mL x 4). The combined organic layers were washed with brine (100 mL x 2), dried over Na₂SO₄, filtered and concentrated in vacuum. The residue was purified by flash silica gel chromatography (ISCO^{®}; 120 g SepaFlash^{®} Silica Flash Column, Eluent of 0-41% Ethylacetate/Petroleum ethergradient @ 150 mL/min). Then the product was triturated with MTBE (100 mL) for 10 min, filtered. The filter cake was washed with MTBE (80 mL), concentrated in vacuum to afford 2-(3-acetylpyrazin-2-yl)-6,6-dimethyl-4H-1,3,4-oxadiazin-5-one (6.2 g, 24.79 mmol, 99.24% purity, yield: 25.3%) as a white solid.
LCMS: Desired MS: 248.1, Observed MS: 249.1
1H NMR: (400 MHz, DMSO-d₆)
δ [ppm] = 11.20 (s, 1H), 8.87 (d, J = 2.4 Hz, 1H), 8.81 (d, J = 2.4 Hz, 1H), 2.60 (s, 3H), 1.51 (s, 6H))

Step 9: To a solution of 2-(3-acetylpyrazin-2-yl)-6,6-dimethyl-4H-1,3,4-oxadiazin-5-one (1.7 g, 6.85 mmol, 1 eq) in dioxane (20 mL) was added Ti(i-PrO)₄ (4.87 g, 17.12 mmol, 5.05 mL, 2.5 eq) and 2-methylpropane-2-sulfinamide (1.66 g, 13.70 mmol, 2 eq). The mixture was stirred at 100 °C for 12 h. LCMS showed 2-(3-acetylpyrazin-2-yl)-6,6-dimethyl-4H-1,3,4-oxadiazin-5-one was consumed completely and one main peak with desired mass was detected. After the completion of the reaction, it was poured into H₂O (30 mL), and then filtered. The filtration was extracted with EtOAc (10 mL x 2). The combined organic layers were washed with brine (20 mL), dried over Na₂SO₄, filtered and concentrated in vacuum. The residue was purified by flash silica gel chromatography (ISCO^{®}; 12 g SepaFlash^{®} Silica Flash Column, Eluent of 0-39% Ethylacetate/Petroleum ethergradient @ 60 mL/min). It was combined with another batch (7.0 g of product) to afford the (NE)-N-[1-[3-(6,6-dimethyl-5-oxo-4H-1,3,4-oxadiazin-2-yl)pyrazin-2-yl]ethylidene]-2-methyl-propane-2-sulfinamide (8.6 g) as a brown oil.
¹H NMR (400 MHz, DMSO-d₆)
δ [ppm] = 11.31 - 11.13 (m, 1H), 8.82 (dd, *J* = 2.3, 11.0 Hz, 1H), 8.67 (s, 1H), 2.71 (s, 1H), 2.47 (s, 2H), 1.49 (d, *J* = 2.1 Hz, 6H), 1.13 (d, *J =* 16.3 Hz, 9H)

Step 10: To a solution of (NE)-N-[1-[3-(6,6-dimethyl-5-oxo-4H-1,3,4-oxadiazin-2-yl)pyrazin-2-yl]ethylidene]-2-methyl-propane-2-sulfinamide (8.1 g, 23.05 mmol, 1 eq) in THF (100 mL) was added 9-BBN (0.5 M, 101.42 mL, 2.2 eq) at 25 °C. The mixture was stirred at 25 °C for 3 h. LCMS showed (NE)-N-[1-[3-(6,6-dimethyl-5-oxo-4H-1,3,4-oxadiazin-2-yl)pyrazin-2-yl]ethylidene]-2-methyl-propane-2-sulfinamide was remained and one peak with desired mass was detected. MeOH (300 mL), acetone (20 mL) was added slowly to the reaction solution, and then stirred for 1 h. Then it was poured into H₂O (1 L) and extracted with DCM (50 mL x 3). The combined organic layers were added 2% aq. NaClO. Then the organic was washed with H₂O (100 mL x 3), brine (100 mL), dried over Na₂SO₄, filtered and concentrated. The residue was purified by flash silica gel chromatography (ISCO^{®}; 80 g SepaFlash^{®} Silica Flash Column, Eluent of 0~100% Ethylacetate/Petroleum ethergradient (added 5% MeOH)@ 150 mL/min). Then 4 g of residue was further purified by prep-HPLC (neutral condition; column: Welch Xtimate C18 180*70mm#10um;mobile phase: [H₂O(10mM NH₄HCO₃)-ACN];gradient:10%-40% B over 17.0 min). It was combined with another batch (0.15 g of the product) to afford N-[1-[3-(6,6-dimethyl-5-oxo-4H-1,3,4-oxadiazin-2-yl)pyrazin-2-yl]ethyl]-2-methyl-propane-2-sulfinamide (1.7763 g, 5.03 mmol, 21.80% yield, 100% purity) as a white solid.
LCMS: Desired MS: 353.1, Observed MS: 354.1
¹H NMR: (400 MHz, DMSO-d₆)
δ [ppm] = 11.26 (br s, 1H), 8.76 (d, *J* = 2.3 Hz, 1H), 8.66 (d, *J* = 2.4 Hz, 1H), 5.54 (d, *J* = 8.8 Hz, 1H), 5.26 - 5.17 (m, 1H), 1.53 (d, *J =* 10.9 Hz, 6H), 1.46 (d, *J =* 6.6 Hz, 3H), 1.07 (s, 9H)

### Intermediate 5: Synthesis of N,2-dimethyl-N-[1-[3-(5-oxo-4H-1,3,4-oxadiazin-2-yl)pyrazin-2-yl]ethyl]propane-2-sulfinamide (Int-5) :

Step 1 : To a solution of 2,3-dichloropyrazine (165 g, 1.11 mol, 1 eq) in toluene (1650 mL) was added Pd(PPh₃)₄ (63.99 g, 55.38 mmol, 0.05 eq) and tributyl(1-ethoxyvinyl)stannane (479.99 g, 1.33 mol, 449.01 mL, 1.2 eq) at 25°C.The mixture was stirred at 120°C for 12h. TLC (petroleum ether/ethyl acetate = 5/1, Rf= 0.45) indicated the starting material was consumed completely and one new spot with large polarity was formed. The reaction mixture was quenched by KF aqueous (3000 mL) at 0°C, filtered and the filtrate was extracted with EtOAc (2000 mL x 2).The combined organic layers were washed with brine (1000 mL), dried over Na₂SO₄, filtered and concentrated under reduced pressure to give a residue. Compound 2-chloro-3-(1-ethoxyvinyl)pyrazine (230 g, crude) was obtained as black oil.

Step 2 : To a solution of 2-chloro-3-(1-ethoxyvinyl)pyrazine (230 g, 1.25 mol, 1 eq) in MeCN (1050 mL) was added HCl (2 M, 1.25 L, 2 eq) at 25°C. The mixture was stirred at 25°C for 2h . TLC (petroleum ether/ethyl acetate = 5/1, Rf = 0.40) indicated the starting material was consumed completely and one new spot with large polarity was formed. The reaction mixture was quenched by addition H₂O (2000 mL), and then extracted with EtOAc (1000 mL x 4). The combined organic layers were dried over Na₂SO₄, filtered and concentrated with the crude product. The residue was purified by flash silica gel chromatography (ISCO^{®}; 330 g SepaFlash^{®} Silica Flash Column, Eluent of 0~50% ethyl acetate/petroleum ethergradient @ 200 mL/min). Compound 1-(3-chloropyrazin-2-yl)ethanone (169 g, 1.08 mol, 86.64% yield) was obtained as a yellow solid.
¹H NMR (400 MHz, DMSO-d₆) δ [ppm] = 8.77 (d, J = 2.4 Hz, 1H), 8.71 (d, J = 2.5 Hz, 1H), 2.64 (s, 3H)

Step 3 : To a solution of 1-(3-chloropyrazin-2-yl)ethanone (149 g, 951.65 mmol, 1 eq) in THF (1500 mL) was added Ti(OEt)₄ (540.95 g, 1.90 mol, 561.73 mL, 2 eq) and 2-methylpropane-2-sulfinamide (161.48 g, 1.33 mol, 1.4 eq) The mixture was stirred at 50°C for 12h . TLC (petroleum ether/ethyl acetate = 5/1, Rf = 0.30) indicated the starting material was consumed completely and one new spot with large polarity was formed. The reaction mixture was quenched with H₂O (2000 mL), and then filtrated, the aueous layer was extracted with EtOAc (700 mL), the filter cake was washed with EtOAc (1000 mL x 6). The combined organic layers were dried over Na₂SO₄, filtered and concentrated to give the crude product. The residue was purified by flash silica gel chromatography (ISCO^{®}; 2 Kg SepaFlash^{®} Silica Flash Column, eluent of 0~50% ethyl acetate/petroleum ethergradient @ 200 mL/min). Compound (NE)-N-[1-(3-chloropyrazin-2-yl)ethylidene]-2-methyl-propane-2-sulfinamide (60 g, 162.61 mmol, 17.09% yield, 70.4% purity) was obtained as yellow oil. Desired MS: 260.3 Observed MS: 260.3

Step 4: To a solution of (NE)-N-[1-(3-chloropyrazin-2-yl)ethylidene]-2-methyl-propane-2-sulfinamide (60 g, 230.99 mmol, 1 eq) in THF (400 mL) and MeOH (200 mL) was added NaBH₄ (6.12 g, 161.69 mmol, 0.7 eq) at 0°C. The mixture was stirred at 0 °C for 0.5h. LCMS showed the starting material was consumed completely and one main peak with desired Ms was detected. The reaction mixture was quenched by water (1000 mL) at 0°C, extracted with EtOAc (500 mL x 2). The combined organic layers were washed with brine (500 mL), dried over Na₂SO₄, filtered and concentrated under reduced pressure to give a residue. The residue was purified by flash silica gel chromatography (ISCO^{®}; 1000 g SepaFlash^{®} Silica Flash Column, Eluent of 0~100% Ethyl acetate/Petroleum ether gradient @ 200 mL/min). Compound N-[1-(3-chloropyrazin-2-yl)ethyl]-2-methyl-propane-2-sulfinamide (43 g, 156.87 mmol, 67.91% yield, 95.5% purity) was obtained as yellow oil. LCMS: Desired MS: 262.3, Observed MS: 262.3, ¹H NMR : (400 MHz, DMSO-d₆) δ [ppm] = 8.68 - 8.65 (m, 1H), 8.44 (d, J = 2.5 Hz, 1H), 5.59 (d, J = 8.0 Hz, 1H), 4.93 - 4.82 (m, 1H), 1.54 (d, J = 6.8 Hz, 3H), 1.07 (s, 9H)

Step 5 : To a solution of N-[1-(3-chloropyrazin-2-yl)ethyl]-2-methyl-propane-2-sulfinamide (39 g, 148.99 mmol, 1 eq) in THF (390 mL) was added NaOH (11.92 g, 297.97 mmol, 2 eq)and Mel (63.44 g, 446.96 mmol, 27.82 mL, 3 eq) at 25°C. The mixture was stirred at 25°C for 12h. TLC (Petroleum ether/Ethyl acetate = 1/1, Rf= 0.50) indicated the starting material was consumed completely and one new spot with small polarity was formed. The reaction mixture was quenched by water (700 mL), extracted with EtOAc (350 mL x 2).The combined organic layers were washed with brine (500 mL), dried over Na₂SO₄, filtered and concentrated under reduced pressure to give a residue. The residue was purified by flash silica gel chromatography (ISCO^{®}; 330 g SepaFlash^{®} Silica Flash Column, Eluent of 0~100% Ethyl acetate/Petroleum ether gradient @ 200 mL/min). Compound N-[1-(3-chloropyrazin-2-yl)ethyl]-N,2-dimethyl-propane-2-sulfinamide (32.8 g, 118.93 mmol, 79.83% yield) was obtained as yellow oil.
¹H NMR: (400 MHz, DMSO-d₆) δ [ppm] = 8.70 (d, J = 2.3 Hz, 1H), 8.49 (d, J = 2.3 Hz, 1H), 5.03 (q, J = 6.9 Hz, 1H), 2.42 (s, 3H), 1.51 (br d, J = 6.8 Hz, 3H), 1.09 (s, 9H)

Step 6 : A mixture of N-[1-(3-chloropyrazin-2-yl)ethyl]-N,2-dimethyl-propane-2-sulfinamide (13 g, 47.14 mmol, 1 eq) and Pd(dppf)Cl₂ (6.90 g, 9.43 mmol, 0.2 eq) and Et₃N (9.54 g, 94.27 mmol, 13.12 mL, 2 eq) in MeOH (150 mL) was stirred at 50°C for 4 h under CO (3447.38 mbar). TLC (Petroleum ether/Ethyl acetate = 1/1, Rf = 0.2) indicated the starting material was consumed completely and one new spot with large polarity was formed. The reaction mixture was filtered and the filtrate was concentrated to give product. The residue was purified by flash silica gel chromatography (ISCO^{®}; 120 g SepaFlash^{®} Silica Flash Column, Eluent of 0~100% Ethyl acetate/Petroleum ethergradient @ 150 mL/min). Compound methyl 3-[1-[tert-butylsulfinyl(methyl)amino]ethyl]pyrazine-2-carboxylate (12 g, 40.08 mmol, 85.03% yield) was obtained as a black solid.
¹H NMR: (400 MHz, DMSO-d₆), δ [ppm] = 8.87 (d, J = 2.4 Hz, 1H), 8.69 (d, J = 2.4 Hz, 1H), 5.35 (q, J = 6.9 Hz, 1H), 3.92 (s, 3H), 2.42 (s, 3H), 1.52 (d, J = 6.9 Hz, 3H), 1.04 (s, 9H)

Step 7 : To a solution of methyl 3-[1-[tert-butylsulfinyl(methyl)amino]ethyl]pyrazine-2-carboxylate (12 g, 40.08 mmol, 1 eq) in EtOH (120 mL) was added NH₂NH₂.H₂O (15.65 g, 312.64 mmol, 15.17 mL, 7.8 eq) at 25°C. The mixture was stirred at 60°C for 2h. TLC (Petroleum ether/Ethyl acetate = 0/1, Rf = 0.20) indicated the starting material was consumed completely and one new spot with large polarity was formed. The reaction was concentrated to give crude product. The reaction mixture was quenched by water (200 mL), extracted with DCM/IPA = 3/1 (350 mL x 4). The combined organic layers were washed with brine (100 mL), dried over Na₂SO₄, filtered and concentrated under reduced pressure to give a residue. The residue was purified by flash silica gel chromatography (ISCO^{®}; 120 g SepaFlash^{®} Silica Flash Column, Eluent of 0~100% Ethyl acetate/Petroleum ether gradient @ 150 mL/min). Compound N-[1-[3-(hydrazinecarbonyl)pyrazin-2-yl]ethyl]-N,2-dimethyl-propane-2-sulfinamide (12 g, 34.87 mmol, 87.00% yield, 87% purity) was obtained as yellow oil. ¹H NMR: (400 MHz, DMSO-d₆)
δ [ppm] = 9.99 (s, 1H), 8.78 (d, J = 2.5 Hz, 1H), 8.60 (d, J = 2.4 Hz, 1H), 5.52 (q, J = 6.9 Hz, 1H), 4.61 (br s, 2H), 2.40 (s, 3H), 1.50 (d, J = 7.0 Hz, 3H), 1.02 (s, 9H)

Step 8 : To a solution of N-[1-[3-(hydrazinecarbonyl)pyrazin-2-yl]ethyl]-N,2-dimethyl-propane-2-sulfinamide (10 g, 33.40 mmol, 1 eq) in DCM (100 mL) was added TEA (8.11 g, 80.16 mmol, 11.16 mL, 2.4 eq) and 2-chloroacetyl chloride (4.53 g, 40.08 mmol, 3.19 mL, 1.2 eq) at 0°C. The mixture was stirred at 25°C for 1h. TLC (Petroleum ether/Ethyl acetate = 0/1, Rf= 0.25) indicated the starting material was consumed completely and one new spot with small polarity was formed. The reaction was concentrated under reduced pressure to give a residue. The crude product was used for next step directly without purification. Compound N-[1-[3-[[(2-chloroacetyl)amino]carbamoyl]pyrazin-2-yl]ethyl]-N,2-dimethyl-propane-2-sulfinamide (12.7 g, 25.04 mmol, 74.96% yield, 74.1% purity) was obtained as black oil.
¹H NMR: (400 MHz, DMSO-d₆) δ [ppm] = 10.92 - 10.31 (m, 2H), 8.88 - 8.83 (m, 1H), 8.66 (d, J = 2.3 Hz, 1H), 5.41 (q, J = 6.9 Hz, 1H), 4.22 (s, 2H), 2.46 (s, 3H), 1.51 (d, J = 7.0 Hz, 3H), 1.02 (s, 9H)

Step 9: To a solution of N-[1-[3-[[(2-chloroacetyl)amino]carbamoyl]pyrazin-2-yl]ethyl]-N,2-dimethyl-propane-2-sulfinamide (14 g, 37.25 mmol, 1 eq) in DMF (140 mL) was added NaHCO₃ (15.64 g, 186.23 mmol, 7.25 mL, 5 eq) at 25°C. The mixture was stirred at 100°C for 3h. LCMS showed the starting material was consumed completely and one main peak with desired Ms was detected. The reaction mixture was quenched by water (200 mL) at 25°C, extracted with DCM (70 mL x 4). The combined organic layers were washed with brine (100 mL), dried over Na₂SO₄, filtered and concentrated under reduced pressure to give a residue. The residue was purified by flash silica gel chromatography (ISCO^{®}; 220 g SepaFlash^{®} Silica Flash Column, Eluent of 0~100% Ethyl acetate/Petroleum ether gradient @ 150 mL/min) to give residue (21 g, 84.1% purity). The residue was purified by prep-HPLC (column: Welch Xtimate C18 180*70mm#10um; mobile phase: [H₂O (10mM NH₄HCO₃)-ACN]; gradient: 8%-38% B over 18.0 min). Compound N,2-dimethyl-N-[1-[3-(5-oxo-4H-1,3,4-oxadiazin-2-yl)pyrazin-2-yl]ethyl]propane-2-sulfinamide (2.78 g, 8.18 mmol, 21.97% yield, 99.9% purity) was obtained as a white solid.
LCMS: Desired MS: 340.1, Observed MS: 340.1, 1H NMR: (400 MHz, CDCl₃)
δ [ppm] = 8.69 (d, J = 2.4 Hz, 1H), 8.57 (d, J = 2.3 Hz, 2H), 5.57 (q, J = 6.9 Hz, 1H), 4.92 (d, J = 1.3 Hz, 2H), 2.63 (s, 3H), 1.62 (s, 3H), 1.15 (s, 9H)

### Intermediate 6: Synthesis of tert-butyl N-[1-[3-(6,6-dimethyl-5-oxo-4H-1,3,4-oxadiazin-2-yl)pyrazin-2-yl]ethyl]-N-methyl-carbamate (Int-6):

### Step 1: Two reactions were setup together.

To a solution of 1-(3-chloropyrazin-2-yl)ethanone (69 g, 440.70 mmol, 1 eq) and NH₄OAc (169.85 g, 2.20 mol, 5 eq) in MeOH (2 L) and NH₃/MeOH (300 mL), the mixture was stirred at 25 °C for 2 hrs, then NaBH₃CN (83.08 g, 1.32 mol, 3 eq) was added to the mixture. The mixture was stirred at 50 °C for 16 hrs. LCMS showed the starting material was consumed completely and one main peak with desired Ms was detected. The reaction was concentrated, and the residue was washed with THF, the organic phase was concentrated to give crude product. The crude product 1-(3-chloropyrazin-2-yl)ethanamine (total 138 g crude) as yellow oil was used into the next step without further purification.

### Step 2: Two reactions were setup together.

A mixture of 1-(3-chloropyrazin-2-yl)ethanamine (69 g, 437.82 mmol, 1 eq), TEA (88.60 g, 875.63 mmol, 121.88 mL, 2 eq) in THF (800 mL) was added CbzCl (82.16 g, 481.60 mmol, 68.75 mL, 1.1 eq), and then the mixture was stirred at 25 °C for 2 hr, under N₂ atmosphere. LCMS showed the starting material was consumed completely and one main peak with desired Ms was detected.

The reaction mixture was poured into H₂O (2 L) slowly and extracted with EA (2 L * 2). The combined organic layers were washed with brine (2 L), dried over Na₂SO₄, filtered and concentrated to give crude product. The residue was purified by column chromatography (SiO2, Petroleum ether/Ethyl acetate=0/1 to 3/1). Benzyl N-[1-(3-chloropyrazin-2-yl)ethyl]carbamate (total 200 g, 685.56 mmol, 78.29% yield, 60% purity) was obtained as yellow oil. ¹H NMR: (400 MHz, DMSO-d₆) δ = 8.65 (d, J = 2.3 Hz, 1H), 8.43 (d, J = 2.5 Hz, 1H), 7.95 (br d, J = 7.1 Hz, 1H), 7.33 (br dd, J = 2.6, 8.4 Hz, 4H), 7.27 - 7.19 (m, 1H), 5.13 - 5.05 (m, 1H), 4.99 - 4.97 (m, 2H), 1.38 (d, J = 7.0 Hz, 3H)

### Step 3: Two reactions were setup together.

To a solution of benzyl *N*-[1-(3-chloropyrazin-2-yl)ethyl]carbamate (100 g, 342.78 mmol, 1 eq) in THF (1 L) was added NaH (20.56 g, 514.17 mmol, 60% purity, 1.5 eq) in five portion at 0 °C, the reaction was stirred at 0 °C for 30 mins, then Mel (97.31 g, 685.56 mmol, 42.68 mL, 2 eq) was added to the mixture. The mixture was stirred at 25 °C for 15.5 hr. LCMS showed the starting material was consumed completely and one main peak with desired Ms was detected. The reaction mixture was quenched with H₂O (1 L), filtered, the filtrate was extracted with EtOAc (1 L * 3). The combined organic layer was dried over Na₂SO₄, filtered and concentrated. The residue was purified by column chromatography (SiO₂, Petroleum ether/Ethyl acetate=50/1 to 5/1). Benzyl *N*-[1-(3-chloropyrazin-2-yl)ethyl]-*N*-methyl-carbamate (total 147 g, 480.77 mmol, 70.13% yield) was obtained as yellow oil.

¹H NMR: (400 MHz, CDCl₃) δ = 8.74 - 8.60 (m, 1H), 8.57 - 8.36 (m, 1H), 7.37 - 7.29 (m, 4H), 7.21 (br s, 1H), 5.73 - 5.45 (m, 1H), 5.13 - 5.00 (m, 2H), 2.78 - 2.68 (m, 3H), 1.48 (br d, J = 6.9 Hz, 3H) Step 4: Three reactions were setup together. A mixture of benzyl *N*-[1-(3-chloropyrazin-2-yl)ethyl]-*N*-methyl-carbamate (49 g, 160.26 mmol, 1 eq), Pd(dppf)Cl₂ (11.73 g, 16.03 mmol, 0.1 eq), TEA (48.65 g, 480.77 mmol, 66.92 mL, 3 eq) in MeOH (500 mL) and then the mixture was stirred at 80 °C for 16 hr under CO atmosphere (3447 mbar). The reaction mixture was quenched with H₂O (800 mL), filtered, the filtrate was extracted with EtOAc (200 mL * 3). The combined organic layer was dried over Na₂SO₄, filtered and concentrated. The residue was purified by column chromatography (SiO2, Petroleum ether/Ethyl acetate=40/1 to 5/1). Methyl 3-[1 [benzyloxycarbonyl(methyl)amino]ethyl]pyrazine-2-carboxylate (total 134 g, 406.86 mmol, 84.36% yield) was obtained as yellow oil.
¹H NMR: (400 MHz, DMSO-d₆) δ = 8.80 (d, J = 2.5 Hz, 1H), 8.67 - 8.59 (m, 1H), 7.41 - 7.23 (m, 4H), 7.13 (br s, 1H), 5.67 (br s, 1H), 5.09 - 4.86 (m, 2H), 3.85 - 3.73 (m, 3H), 2.88 - 2.74 (m, 3H), 1.51 (d, J = 7.0 Hz, 3H)

### Step 5: Two reactions were setup together.

A mixture of methyl 3-[1 [benzyloxycarbonyl(methyl)amino]ethyl]pyrazine-2-carboxylate (67 g, 203.43 mmol, 1 eq), LiOH.H₂O (25.61 g, 610.29 mmol, 3 eq) in H₂O (200 mL) and MeOH (200 mL) then the mixture was stirred at 25 °C for 1hr. LCMS showed the starting material was consumed completely and one main peak with desired Ms was detected. The reaction mixture was poured into iced water (500 mL) slowly and extracted with EtOAc (500 mL). The water layer was adjusted to pH=3 with 1N HCl and extracted with EtOAc (500 mL * 2). The combined organic layers were washed with brine (500 mL), dried over Na₂SO₄, filtered and concentrated to give crude product. The crude product 3-[1-[benzyloxycarbonyl(methyl)amino]ethyl]pyrazine-2-carboxylic acid (total 108 g crude) as yellow oil was used into the next step without further purification.

¹H NMR: (400 MHz, DMSO-d₆) δ = 8.78 - 8.68 (m, 1H), 8.61 (d, J = 1.9 Hz, 1H), 7.36 - 7.24 (m, 4H), 7.12 (br s, 1H), 5.75 (q, J = 6.9 Hz, 1H), 5.10 - 4.87 (m, 2H), 2.90 (br s, 3H), 1.51 (d, J = 7.1 Hz, 3H)

### Step 6: Two reactions were setup together.

A mixture of 3-[1-[benzyloxycarbonyl(methyl)amino]ethyl]pyrazine-2-carboxylic acid (54 g, 171.25 mmol, 1 *eq*), HATU (97.67 g, 256.88 mmol, 1.5 *eq*), DIEA (44.27 g, 342.51 mmol, 59.66 mL, 2 *eq*) in THF (500 mL), the mixture was stirred at 25 °C for 30 min, then *tert-*butyl N-aminocarbamate (24.90 g, 188.38 mmol, 1.1 eq) was added to the mixture, the mixture was stirred at 25 °C for 15.5 hr under N₂ atmosphere. LCMS showed the starting material was consumed completely and one main peak with desired Ms was detected. The reaction mixture was quenched with H₂O (1 L), filtered, the filtrate was extracted with EtOAc (500 mL * 3). The combined organic layer was dried over Na₂SO₄, filtered and concentrated. The residue was purified by column chromatography (SiO2, Commercial hexanes: Ethyl acetate=50/1 to 1/1). Benzyl *N*-[1-[3-[(*tert-*butoxycarbonylamino)carbamoyl]pyrazin-2-yl]ethyl]-N-methyl-carbamate (total 120 g, 279.42 mmol, 81.58% yield) was obtained as yellow oil.
¹H NMR: (400 MHz, DMSO-d₆) δ = 10.38 (s, 1H), 9.01 (s, 1H), 8.76 - 8.70 (m, 1H), 8.59 ( s, 1H), 7.35 - 7.18 (m, 4H), 7.09 (s, 1H), 6.13 - 5.88 (m, 1H), 4.99 (dd, J = 7.0, 13.1 Hz, 2H), 3.01 (d, J = 13.1 Hz, 3H), 1.51 (d, J = 7.1 Hz, 3H), 1.44 - 1.39 (m, 9H)

### Step 7: Two reactions were setup together.

A mixture of benzyl *N*-[1-[3-[(*tert*-butoxycarbonylamino)carbamoyl]pyrazin-2-yl]ethyl]-*N*-methyl-carbamate (45 g, 104.78 mmol, 1 eq) in EtOAc (100 mL) and HCl/EtOAc (400 mL), then the mixture was stirred at 25 °C for 1hr. LCMS showed the starting material was consumed completely and one main peak with desired Ms was detected. The reaction was concentrated. The crude product benzyl N-[1-[3-(hydrazinecarbonyl)pyrazin-2-yl]ethyl]-*N*-methyl-carbamate (total 105 g, crude) as a yellow soild was used into the next step without further purification.

¹H NMR: (400 MHz, DMSO-d₆) δ = 10.38 (s, 1H), 9.01 (s, 1H), 8.76 - 8.70 (m, 1H), 8.59 (s, 1H), 7.35 - 7.18 (m, 4H), 7.09 (s, 1H), 6.13 - 5.88 (m, 1H), 4.99 (dd, J = 7.0, 13.1 Hz, 2H), 3.01 (d, J = 13.1 Hz, 3H), 1.51 (d, J = 7.1 Hz, 3H), 1.44 - 1.39 (m, 9H).

### Step 8: Two reactions were setup together.

A mixture of benzyl N-[1-[3-(hydrazinecarbonyl)pyrazin-2-yl]ethyl]-N-methyl-carbamate (50 g, 151.81 mmol, 1 eq), PMBCHO (31.00 g, 227.72 mmol, 27.71 mL, 1.5 eq), TsOH (26.14 g, 151.81 mmol, 1 eq) and NaOAc (14.94 g, 182.18 mmol, 1.2 eq) in THF (500 mL) the reaction was stirred at 50°C for 1hr, NaBH₃CN (19.08 g, 303.63 mmol, 2 eq) was added to the mixture, then the mixture was stirred at 50 °C for 11 hrs under N₂ atmosphere. LCMS showed the starting material was consumed completely and one main peak with desired Ms was detected. The reaction mixture was poured into H₂O (1 L), and extracted with EA (500 mL x 2). The combined organic layers were washed with brine (500 mL), dried over Na₂SO₄, filtered and concentrated to give crude product. The residue was purified by column chromatography (SiO₂, Commercial hexanes: Ethyl acetate=40/1 to 1/1). Benzyl *N*-[1-[3-[[bis[(4-methoxyphenyl)methyl]amino]carbamoyl]pyrazin-2-yl]ethyl]-*N*-methyl-carbamate (total 60 g, 105.33 mmol, 34.69% yield) was obtained as yellow oil.

Step 9: To a mixture of benzyl *N*-[1-[3-[[bis[(4-methoxyphenyl)methyl]amino]carbamoyl]pyrazin-2-yl]ethyl]-*N*-methyl-carbamate (60 g, 105.33 mmol, 1 eq) in TFA (10 mL) and DCM (50 mL) at 25°C under nitrogen. The mixture was stirred at 40°C for 16 h. LCMS showed 19.5% of starting material was remained and ~49.8% with desired Ms was detected. The reaction mixture was poured into H₂O (200 mL), filtered and extracted with DCM (100 mL * 3). The combined organic layer was dried over Na₂SO₄, filtered and concentrated. The residue was purified by flash silica gel chromatography (ISCO^{®}; 330 g SepaFlash^{®} Silica Flash Column, Eluent of 0~40% Ethyl acetate/Petroleum ether gradient @ 200 mL/min). benzyl *N*-[1-[3-[[(4-methoxyphenyl)methylamino]carbamoyl]pyrazin-2-yl]ethyl]-N-methyl-carbamate
(23 g, 51.17 mmol, 48.58% yield) was obtained as yellow oil. ¹H NMR: (400 MHz, DMSO-d₆) δ = 10.23 (br s, 1H), 8.71 (d, J = 10.3 Hz, 1H), 8.61 - 8.48 (m, 1H), 7.30 (d, J = 7.6 Hz, 6H), 7.11 (s, 1H), 6.87 (d, J = 8.4 Hz, 2H), 6.03 - 5.84 (m, 1H), 5.07 - 4.86 (m, 2H), 3.99 - 3.84 (m, 2H), 3.72 (s, 3H), 2.90 (s, 3H), 1.46 (d, J = 7.0 Hz, 3H)

Step 10: A mixture of benzyl *N*-[1-[3-[[(4-methoxyphenyl)methylamino]carbamoyl]pyrazin-2-yl]ethyl]-*N*-methyl-carbamate (23 g, 51.17 mmol, 1 eq), TEA (10.36 g, 102.34 mmol, 14.24 mL, 2 eq) in THF (300 mL) was added 2-chloro-2-methyl-propanoyl chloride (7.21 g, 51.17 mmol, 1 eq) at 0°C and then the mixture was stirred at 25°C for 1hr under N₂ atmosphere. LCMS showed the starting material was consumed completely and one main peak with desired Ms was detected. The reaction was filtered and the filtrate was concentrated. The crude product benzyl *N*-[1-[3-[[(2-chloro-2-methyl-propanoyl)-[(4-methoxyphenyl)methyl]amino]carbamoyl]pyrazin-2-yl]ethyl]-*N-*methyl-carbamate (28 g, crude) as yellow oil was used into the next step without further purification.
¹H NMR: (400 MHz, DMSO-d₆) δ = 11.28 - 10.97 (m, 1H), 8.88 - 8.73 (m, 1H), 8.66 (s, 1H), 7.36 - 7.15 (m, 6H), 7.04 ( d, J = 2.1 Hz, 1H), 6.83 (d, J = 7.6 Hz, 2H), 6.07 - 5.86 (m, 1H), 5.04 - 4.89 (m, 2H), 3.70 (s, 3H), 3.60 (s, 2H), 3.01 - 2.90 (m, 3H), 1.76 (d, J = 2.3 Hz, 9H), 1.50 (d, J = 6.4 Hz, 3H)

Step 11: A mixture of product benzyl *N*-[1-[3-[[(2-chloro-2-methyl-propanoyl)-[(4-methoxyphenyl)methyl]amino]carbamoyl]pyrazin-2-yl]ethyl]-*N*-methyl-carbamate (28 g, 50.54 mmol, 1 eq) in EtOH (300 mL) was added K₂CO₃ (13.97 g, 101.08 mmol, 2 eq) and then the mixture was stirred at 50 °C for 16hr under N₂ atmosphere. LCMS showed the starting material was consumed completely and one main peak with desired Ms was detected. The reaction mixture was quenched with H₂O (500 mL), filtered, the filtrate was extracted with EtOAc (500 mL * 3). The combined organic layer was dried over Na₂SO₄, filtered and concentrated. The residue was purified by flash silica gel chromatography (ISCO^{®}; 220 g SepaFlash^{®} Silica Flash Column, Eluent of 0~100% Ethyl acetate/Petroleum ether gradient @ 200 mL/min). Benzyl *N*-[1-[3-[4-[(4-methoxyphenyl)methyl]-6,6-dimethyl-5-oxo-1,3,4-oxadiazin-2-yl]pyrazin-2-yl]ethyl]-*N*-methyl-carbamate (20 g, 38.64 mmol, 76.46% yield) was obtained as a white solid.

¹H NMR: (400 MHz, DMSO-d₆) δ = 8.71 (d, J = 16.0 Hz, 1H), 8.64 (s, 1H), 7.39 - 7.13 (m, 6H), 6.96 (s, 1H), 6.86 (d, J = 6.1 Hz, 2H), 5.81 - 5.45 (m, 1H), 5.10 - 4.68 (m, 4H), 3.70 (s, 3H), 3.04 - 2.71 (m, 3H), 1.56 - 1.43 (m, 6H), 1.39 (d, J = 5.5 Hz, 3H).

### Step 12: Two reactions were setup together.

A mixture of benzyl *N*-[1-[3-[4-[(4-methoxyphenyl)methyl]-6,6-dimethyl-5-oxo-1,3,4-oxadiazin-2-yl]pyrazin-2-yl]ethyl]-*N*-methyl-carbamate (10 g, 19.32 mmol, 1 *eq*) in TFA (100 mL) and CF₃SO₃H (50 mL) and then the mixture was stirred at 40 °C for 2hr under N₂ atmosphere. LCMS showed the starting material was consumed completely and one main peak with desired Ms was detected. The reaction was filtered and the filtrate was concentrated. The crude product 6,6-dimethyl-2-[3-[1-(methylamino)ethyl]pyrazin-2-yl]-4*H*-1,3,4-oxadiazin-5-one (total 100 g, crude) as yellow oil was used into the next step without further purification.

### Step 13: Two reactions were setup together.

A mixture of 6,6-dimethyl-2-[3-[1-(methylamino)ethyl]pyrazin-2-yl]-4*H*-1,3,4-oxadiazin-5-one (50 g crude) in THF (500 mL), adjusted to pH > 7 with TEA at 0 °C, then TEA (38.43 g, 379.80 mmol, 52.86 mL, 2 eq) and Boc₂O (62.17 g, 284.85 mmol, 65.44 mL, 1.5 eq) was added, the mixture was stirred at 25 °C for 1hr under N₂ atmosphere. LCMS showed the starting material was consumed completely and one main peak with desired Ms was detected. The reaction mixture was quenched with H₂O (1 L), filtered, the filtrate was extracted with EtOAc (500 mL * 3). The combined organic layer was dried over Na₂SO₄, filtered and concentrated. The residue was purified by flash silica gel chromatography (ISCO^{®}; 330 g SepaFlash^{®} Silica Flash Column, Eluent of 0~40% Ethyl acetate/Petroleum ether gradient @ 200 mL/min). Compound *tert-butyl N-*[1-[3-(6,6-dimethyl-5-oxo-4*H*-1,3,4-oxadiazin-2-yl)pyrazin-2-yl]ethyl]-*N*-methyl-carbamate (total 9.43 g, 25.42 mmol, 67.3% yield over two steps) was obtained as a yellow solid.
¹H NMR (400 MHz, DMSO-d₆) δ = 11.39 - 11.09 (m, 1H), 8.77 (d, *J* = 2.4 Hz, 1H), 8.71 (d, *J* = 1.9 Hz, 1H), 5.98 - 5.60 (m, 1H), 2.95 - 2.68 (m, 3H), 1.56 (s, 9H), 1.41 - 1.16 (m, 9H); LCMS: (Desired MS: 363.1, Observed MS: 364.2)

### Intermediate 7: Synthesis of 2-methyl-N-[1-[3-(8-oxo-4-oxa-6,7-diazaspiro[2.5]oct-5-en-5-yl)pyrazin-2-yl]ethyl]propane-2-sulfinamide (Int-7):

Step 1: To a solution of 4-methoxyphenyl)methylhydrazine (100 g, 657 mmol, 1 eq, HCl) in DCM (1000 mL) was added DIEA (254.76 g, 1.97 mol, 3 eq) and Boc₂O (129.06 g, 591.35 mmol, 0.9 eq). The mixture was stirred at 20 °C for 2 h. After the completion of the reaction, it was washed with 10% aq. citric acid (100 mL), 0.5 M HCl (100 mL), sat.aq. NaHCO₃ (500 mL), brine (500 mL), dried over Na₂SO₄, filtered and concentrated under reduced pressure to give a residue to give *tert-*butyl *N*-amino*-N*-[(4-methoxyphenyl)methyl]carbamate
(148 g, 65.17% yield, 73% purity) as a yellow oil, which was used to the next step without purification.

¹H NMR: (400 MHz, CDCl₃) δ = 7.23 (2 H, d, *J*=8.66 Hz) 6.87 (2 H, d, *J*=8.66 Hz) 4.49 (2 H, s) 3.91 - 4.01 (2 H, m) 3.81 (3 H, s) 1.50 (9 H, s).

Step 2: To a solution of 3-chloropyrazine-2-carboxylic acid (148 g, 428.21 mmol, 1.1 eq), *tert-butyl N*-amino-*N*-[(4-methoxyphenyl)methyl]carbamate (74.68 g, 471.03 mmol, 1 eq) in DCM (1000 mL) was added TEA (86.66 g, 856.41 mmol, 48.55 mL, 2 eq). Then T₄P (462.80 g, 642.31 mmol, 50% purity, 1.5 eq) was added at 0 °C. The mixture was stirred at 15 °C for 2 h. The mixture was poured into sat. aq. NaHCO₃ (1 L), then washed with brine (300 mL), dried over Na₂SO₄, filtered and concentrated in vacuum. The residue was triturated with hexane/EA=3:1 (300 mL) for 30 min and filtered. The filter cake was dried in vacuum to afford *tert-*butyl N-[(3-chloropyrazine-2-carbonyl)amino]-*N*-[(4-methoxyphenyl)methyl]carbamate (114 g, 67.77% yield) as a white solid. ¹H NMR: (400 MHz, DMSO-d₆) δ = 10.86 (1 H, s) 8.63 - 8.76 (2 H, m) 7.25 (2 H, d, *J*=8.53 Hz) 6.90 (2 H, d, *J*=8.66 Hz) 4.56 (2 H, br s) 3.73 (3 H, s) 1.43 (9 H, s)

Step 3: A solution of *tert-*butyl *N*-[(3-chloropyrazine-2-carbonyl)amino]-*N*-[(4-methoxyphenyl)methyl]carbamate (114 g, 290.20 mmol, 1 eq) in HCl/EtOAc (4 M, 1.2 L, 16.54 eq) was stirred at 15 °C for 1 h. LCMS showed the reaction was completed, the mixture was filtered. The filter cake was washed with EtOAc (100 mL x 3) and dried in vacuum to give 3-chloro-*N*'-[(4-methoxyphenyl)methyl]pyrazine-2-carbohydrazide (93 g, 97.35% yield, HCl) as a white solid.

¹H NMR: (400 MHz, DMSO-d₆) δ = 10.94 - 11.29 (1 H, m) 8.73 (1 H, d, *J*=2.38 Hz) 8.68 (1 H, d, *J*=2.51 Hz) 7.37 (2 H, d, *J*=8.53 Hz) 6.89 - 6.98 (2 H, m) 4.13 (2 H, s) 3.74 (3 H, s) 3.69 (1 H, s).

### Step 4: 4 Batches:

To a solution of 4-chlorobutanoyl chloride (100 g, 709.24 mmol, 79.37 mL, 1 eq) in DCM (500 mL) was added NBS (189.35 g, 1.06 mol, 1.5 eq) and SOCl₂ (8.44 g, 70.92 mmol, 5.15 mL, 0.1 *eq*), HBr (7.17 g, 35.46 mmol, 4.81 mL, 40% purity, 0.05 *eq*) The mixture was stirred at 60 °C for 2hr under N₂. The reaction mixture was filtrated and concentrated under reduced pressure to give a residue. The crude product was used into the next step without further purification to give 2-bromo-4-chloro-butanoyl chloride (660 g, crude) was obtained as a green oil.
¹H NMR: (400 MHz, CDCl₃) δ ppm 4.83 (dd, *J*=9.13, 4.88 Hz, 1 H) 3.71 - 3.76 (m, 2 H) 2.54 - 2.65 (m, 1 H) 2.40 - 2.51 (m, 1 H)

### Step 5: 4 Batches:

To a solution of 3-chloro-*N*'-[(4-methoxyphenyl)methyl]pyrazine-2-carbohydrazide (65 g, 197.46 mmol, 1 eq, HCl) in MeCN (1300 mL) was added K₂CO₃ (109.16 g, 789.84 mmol, 4 eq) and 2-bromo-4-chloro-butanoyl chloride (73.81 g, 335.68 mmol, 1.7 *eq)* at 0°C .The mixture was stirred at 0°C for 1 hr. LC-MS showed 3-chloro-*N*'-[(4-methoxyphenyl)methyl]pyrazine-2-carbohydrazide was consumed and *N*'-(2-bromo-4-chloro-butanoyl)-3-chloro-*N*'-[(4-methoxyphenyl)methyl]pyrazine-2-carbohydrazide was detected. Then K₂CO₃ (27.29 g, 197.46 mmol, 1 *eq*) was added and stirred at 60°C for 4 hr. LC-MS showed *N*'-(2-bromo-4-chlorobutanoyl)-3-chloro-*N'*-[(4-methoxyphenyl)methyl]pyrazine-2-carbohydrazide was consumed, and ~76% of desired compound was detected. The reaction mixture was filtrated and the filtrate was concentrated and quenched by addition H₂O (1.5 L) at 0 °C, and then extracted with EtOAc (1000 mL * 3). The combined organic layers were washed with aqueous NaCl (500 mL * 2), dried over Na₂SO₄, filtered and concentrated under reduced pressure to give a residue. The residue was triturated with EtOAc: PE=1:1 (300 mL) to give 6-(2-chloroethyl)-2-(3-chloropyrazin-2-yl)-4-[(4-methoxyphenyl)methyl]-1,3,4-oxadiazin-5-one (total 240 g, 607.23 mmol, 76.88% yield) as a yellow solid.
¹H NMR: (400 MHz, CDCl₃) δ ppm 8.58 (d, *J*=2.00 Hz, 1 H) 8.46 (d, *J*=1.88 Hz, 1 H) 7.37 (d, *J*=8.38 Hz, 2 H) 6.87 (d, *J*=8.38 Hz, 2 H) 5.06 (dd, *J*=9.01, 4.00 Hz, 1 H) 4.81 - 4.98 (m, 2 H) 3.73 - 3.87 (m, 5 H) 2.48 - 2.60 (m, 1 H) 2.32 - 2.45 (m, 1 H)

### Step 6: 7 Batches:

To a solution of 6-(2-chloroethyl)-2-(3-chloropyrazin-2-yl)-4-[(4-methoxyphenyl)methyl]-1,3,4-oxadiazin-5-one (30 g, 75.90 mmol, 1 *eq*) in DMSO (500 mL) was added Cs₂CO₃ (98.92 g, 303.61 mmol, 4 *eq*).The mixture was stirred at 90 °C for 5hr. LC-MS showed ~6% of 6-(2-chloroethyl)-2-(3-chloropyrazin-2-yl)-4-[(4-methoxyphenyl)methyl]-1,3,4-oxadiazin-5-one remained and ~36% of desired compound was detected. The reaction mixture was filtrated and the filtrate was quenched by addition aqueous NaCl (4 L) at 0°C, and then filtrated and the filtrate was extracted with EtOAc (2000 mL x 3). The combined organic layers were washed with aqueous NaCl (500 mL), dried over Na₂SO₄, filtered and concentrated under reduced pressure to give a residue. The residue was purified by column chromatography (SiO2, Commercial hexanes: Ethyl acetate=5/1 to 3/1) to give 5-(3-chloropyrazin-2-yl)-7-[(4-methoxyphenyl)methyl]-4-oxa-6,7-diazaspiro[2.5]oct-5-en-8-one (113 g, 314.96 mmol, 59.28% yield) as a yellow oil.
¹H NMR: (400 MHz, CDCl₃) δ ppm 8.55 (d, *J*=2.38 Hz, 1 H) 8.43 (d, *J*=2.38 Hz, 1 H) 7.36 - 7.41 (m, 2 H) 6.86 - 6.91 (m, 2 H) 4.90 (s, 2 H) 3.80 (s, 3 H) 1.56 - 1.63 (m, 2 H) 1.31 - 1.36 (m, 2 H)

### Step 7: 3 Batches:

To a solution of 5-(3-chloropyrazin-2-yl)-7-[(4-methoxyphenyl)methyl]-4-oxa-6,7-diazaspiro[2.5]oct-5-en-8-one (53.3 g, 148.56 mmol, 1 *eq*) in Tol. (530 mL) was added Pd(PPh₃)₄ (8.58 g, 7.43 mmol, 0.05 *eq*) and tributyl(1-ethoxyvinyl)stannane (59.02 g, 163.42 mmol, 55.21 mL, 1.1 eq) .The mixture was stirred at 120 °C for 12hr . LC-MS showed 5-(3-chloropyrazin-2-yl)-7-[(4-methoxyphenyl)methyl]-4-oxa-6,7-diazaspiro[2.5]oct-5-en-8-one was consumed and ~66% of desired compound was detected. The reaction mixture was quenched by addition aqueous KF (1.5 L) at 0 °C, and then filtrated and washed with EtOAc (500 mL x 4), and extracted with EtOAc (2 L x 2). The combined organic layers were washed with aqueous NaCl (500 mL), dried over Na₂SO₄, filtered and concentrated under reduced pressure to give a residue. The residue was purified by column chromatography (SiO2, hexanes: Ethyl acetate=4/1 to 2/1) to give 5-[3-(1-ethoxyvinyl)pyrazin-2-yl]-7-[(4-methoxyphenyl)methyl]-4-oxa-6,7-diazaspiro[2.5]oct-5-en-8-one (200 g, crude) as a brown oil.

Step 8: To a solution of 5-[3-(1-ethoxyvinyl)pyrazin-2-yl]-7-[(4-methoxyphenyl)methyl]-4-oxa-6,7-diazaspiro[2.5]oct-5-en-8-one (200 g, 507.07 mmol, 1 eq) in MeCN (600 mL) was added HCl (2 M, 507.07 mL, 2 *eq*) at 0°C .The mixture was stirred at 0 °C for 1hr . LC-MS showed Reactant 1 was consumed completely and one main peak with desired mass was detected. The reaction mixture was quenched by addition H₂O (1 L) at 0 °C, and then extracted with EtOAc (1 Lx 3). The combined organic layers were washed with aqueous NaCl (500 mL), dried over Na₂SO₄, filtered and concentrated under reduced pressure to give a residue. The residue was purified by column chromatography (SiO2, Commercial hexanes: Ethyl acetate=4/1 to 2/1) to give 5-(3-acetylpyrazin-2-yl)-7-[(4-methoxyphenyl)methyl]-4-oxa-6,7-diazaspiro[2.5]oct-5-en-8-one (118 g, 322.08 mmol, 63.52% yield) as a white solid. ¹H NMR: (400 MHz, CDCl₃) δ ppm 8.68 (d, *J*=2.38 Hz, 1 H) 8.59 (d, *J*=2.38 Hz, 1 H) 7.31 (d, *J*=8.63 Hz, 2 H) 6.88 (d, *J*=8.50 Hz, 2 H) 4.84 (s, 2 H) 3.80 (s, 3 H) 2.57 (s, 3 H) 1.57 - 1.63 (m, 2 H) 1.29 - 1.34 (m, 2 H).

### Step 9: 2 Batch:

To a solution of 5-(3-acetylpyrazin-2-yl)-7-[(4-methoxyphenyl)methyl]-4-oxa-6,7-diazaspiro[2.5]oct-5-en-8-one (10 g, 27.29 mmol, 1 eq) in TFA (70 mL) was added TFMSA (8.19 g, 54.59 mmol, 4.83 mL, 2 eq). The mixture was stirred at 40°C for 1h. LC-MS showed 5-(3-acetylpyrazin-2-yl)-7-[(4-methoxyphenyl)methyl]-4-oxa-6,7-diazaspiro[2.5]oct-5-en-8-one was consumed completely and one main peak with desired mass was detected. The reaction mixture was quenched with cooled H₂O (100 mL), extracted with EtOAc (70 mL x 4). The combined organic layer was washed with brine (150 mL x 3), dried over Na₂SO₄, filtered and concentrated. The residue was dissolved in EtOAc and poured into column chromatography and standing for 0.5h, then purified by column chromatography (SiO₂, Commercial hexanes : Ethyl acetate=1/0 to 1/1) to give 5-(3-acetylpyrazin-2-yl)-4-oxa-6,7-diazaspiro[2.5]oct-5-en-8-one (8.6 g, 34.93 mmol, 63.98% yield) as a yellow solid.
¹H NMR: (400 MHz, DMSO-d₆) δ = 11.30 (br s, 1H), 8.88 (d, J = 2.5 Hz, 1H), 8.83 (d, J = 2.4 Hz, 1H), 2.61 (s, 3H), 1.44 - 1.38 (m, 2H), 1.26 - 1.21 (m, 2H); LCMS: (Desired MS: 247, Observed MS: 247)

Step 10: To a solution of 5-(3-acetylpyrazin-2-yl)-4-oxa-6,7-diazaspiro[2.5]oct-5-en-8-one (8.5 g, 34.52 mmol, 1 eq) in THF (85 mL) was added Ti(i-PrO)₄ (24.53 g, 86.30 mmol, 25.47 mL, 2.5 eq) and 2-methylpropane-2-sulfinamide (8.37 g, 69.04 mmol, 2 eq) .The mixture was stirred at 15 °C for 0.5 hr and stirred at 80°C for 11.5 hr. LC-MS showed 5-(3-acetylpyrazin-2-yl)-4-oxa-6,7-diazaspiro[2.5]oct-5-en-8-one was consumed completely and one main peak with desired mass was detected. The reaction mixture was quenched by addition H₂O (300 mL) at 0 °C, and then filtrated and the filter cake was washed with EtOAc (200 mL x 3), and the filtrate was extracted with EtOAc (600 mL x 2). The combined organic layers were washed with aqueous NaCl (100 mL), dried over Na₂SO₄, filtered and concentrated under reduced pressure to give a residue. The residue was purified by flash silica gel chromatography (ISCO^{®}; 120 g SepaFlash^{®} Silica Flash Column, Eluent of 0~60% Ethyl acetate/Petroleum ether gradient @ 120 mL/min) and triturated with EtOAc (20 mL) to give (*NE*)-2-methyl-*N*-[1-[3-(8-oxo-4-oxa-6,7-diazaspiro[2.5]oct-5-en-5-yl)pyrazin-2-yl]ethylidene]propane-2-sulfinamide (9 g, 25.76 mmol, 74.61% yield) as a yellow solid.
¹H NMR: (400 MHz, CDCl₃) δ ppm 8.65 (dd, *J*=11.19, 2.19 Hz, 1 H) 8.56 (dd, *J*=9.44, 2.19 Hz, 1 H) 8.45 - 8.54 (m, 1 H) 2.52 - 2.85 (m, 3 H) 1.56 - 1.65 (m, 2 H) 1.33 - 1.48 (m, 2 H) 1.22 - 1.31 (m, 9 H)

Step 11: To a solution of (*NE*)-2-methyl-*N*-[1-[3-(8-oxo-4-oxa-6,7-diazaspiro[2.5]oct-5-en-5-yl)pyrazin-2-yl]ethylidene]propane-2-sulfinamide (6.2 g, 17.74 mmol, 1 *eq*) in THF (48 mL) and MeOH (12 mL) was added NaBH₄ (671.27 mg, 17.74 mmol, 1 eq) at 0°C under N₂. The mixture was stirred at 0 °C for 1hr under N₂. The reaction mixture was quenched by addition H₂O (100 mL) at 0 °C, and then extracted with EtOAc (200 mL x 7). The combined organic layers were dried over Na₂SO₄, filtered and concentrated under reduced pressure to give a residue. The residue was purified by flash silica gel chromatography (ISCO^{®}; 40 g SepaFlash^{®} Silica Flash Column, Eluent of 0~100% Ethyl acetate/Petroleum ether gradient @ 100 mL/min) and prep-HPLC (neutral) to give 2-methyl-*N*-[1-[3-(8-oxo-4-oxa-6,7-diazaspiro[2.5]oct-5-en-5-yl)pyrazin-2-yl]ethyl]propane-2-sulfinamide (3 g, 8.54 mmol, 48.11% yield) as a yellow solid.
¹H NMR: (400 MHz, CDCl₃) δ ppm 8.83 (br d, *J*=3.75 Hz, 1 H) 8.58 - 8.63 (m, 1 H) 8.52 - 8.57 (m, 1 H) 5.31 - 5.46 (m, 1 H) 4.38 - 5.02 (m, 1 H) 1.48 - 1.66 (m, 5 H) 1.39 - 1.46 (m, 2 H) 1.17 - 1.26 (m, 9 H)
LCMS: (Desired mass: 351.1; Observed mass: 352.1)

### Intermediate 8: Synthesis of 2-methyl-N-[1-[3-(7-methyl-8-oxo-4-oxa-6,7-diazaspiro[2.5]oct-5-en-5-yl)pyrazin-2-yl]ethyl]propane-2-sulfinamide (Int-8):

### Step 1: Two batches

To a solution of 3-chloro-*N*'-methyl-pyrazine-2-carbohydrazide (50 g, 224.16 mmol, 1 *eq,* HCl) in MeCN (600 mL) was added K₂CO₃ (123.92 g, 896.62 mmol, 4 *eq*) and 2-bromo-4-chlorobutanoyl chloride (98.58 g, 448.31 mmol, 2 eq) dropwise at 0°C .The mixture was stirred at 0-10°C for 1.5hr. LC-MS showed 3-chloro-*N*'-methyl-pyrazine-2-carbohydrazide was consumed completely. The reaction solution was used directly without work up. Then K₂CO₃ (30.98 g, 224.16 mmol, 1 *eq*) was added to the mixture and stirred at 60°C for 2hr LC-MS showed *N*'-(2-bromo-4-chloro-butanoyl)-3-chloro-*N'*-methyl-pyrazine-2-carbohydrazide was consumed completely. The reaction solution was filtered and the filtrate was concentrated and the residue was poured into ice H₂O (1000 mL), extracted with EtOAc (500 mL *2), the organics washed with brine, dried over Na₂SO₄, filtered and concentrated. The residue was purified by column chromatography (SiO₂, hexanes: ethyl acetate=3/1 to 3/2) to give 6-(2-chloroethyl)-2-(3-chloropyrazin-2-yl)-4-methyl-1,3,4-oxadiazin-5-one (total 100 g, 345.88 mmol, 77.15% yield) was obtained as a yellow solid.
¹H NMR: (400 MHz, CDCl₃) δ = 8.61 (d, *J =* 2.0 Hz, 1H), 8.50 (d, *J* = 2.0 Hz, 1H), 5.09 (dd, *J* = 3.9, 9.0 Hz, 1H), 3.87 - 3.75 (m, 2H), 3.45 (s, 3H), 2.66 - 2.55 (m, 1H), 2.49 - 2.32 (m, 1H)

### Step 2: Four batches

To a solution of 6-(2-chloroethyl)-2-(3-chloropyrazin-2-yl)-4-methyl-1,3,4-oxadiazin-5-one (32.5 g, 112.41 mmol, 1 eq) in DMSO (500 mL) was added Cs₂CO₃ (146.50 g, 449.64 mmol, 4 eq) at 10°C .The mixture was stirred at 90°C for 5hr. LC-MS showed 6-(2-chloroethyl)-2-(3-chloropyrazin-2-yl)-4-methyl-1,3,4-oxadiazin-5-one was consumed completely. The reaction solution was filtered, and the filtrate was poured into H₂O (2000 mL), extracted with EtOAc (800 mL *2), the organics washed with brine, dried over Na₂SO₄, filtered and concentrated. The residue was purified by column chromatography (SiO2, Commercial hexanes: Ethyl acetate=3/1 to 1/1) to give 5-(3-chloropyrazin-2-yl)-7-methyl-4-oxa-6,7-diazaspiro[2.5]oct-5-en-8-one (total 46 g, 182.07 mmol, 40.49% yield) was obtained as a yellow solid. ¹H NMR: (400 MHz, CDCl₃) δ = 8.58 (d, *J* = 2.4 Hz, 1H), 8.46 (d, *J* = 2.3 Hz, 1H), 3.44 (s, 3H), 1.64 - 1.57 (m, 2H), 1.37 - 1.32 (m, 2H).

### Step 3: Two batchs

A mixture of 5-(3-chloropyrazin-2-yl)-7-methyl-4-oxa-6,7-diazaspiro[2.5]oct-5-en-8-one (34.8 g, 137.74 mmol, 1 eq), tributyl(1-ethoxyvinyl)stannane (54.72 g, 151.51 mmol, 51.19 mL, *1.1 eq*) , Pd(PPh₃)₄ (7.96 g, 6.89 mmol, 0.05 *eq*) in toluene (350 mL) was degassed and purged with N₂ for 3 times, and then the mixture was stirred at 10-120 °C for 16hr under N₂ atmosphere. LC-MS showed 5-(3-chloropyrazin-2-yl)-7-methyl-4-oxa-6,7-diazaspiro[2.5]oct-5-en-8-one was consumed completely. The reaction mixture was concentrated and the residue was quenched by addition KF aq (2000 mL) at 0°C, and stirred at 10°C for 0.5hr, then mixture was filtered and the filtrate was extracted with EtOAc (100 mL x 3). The combined organic layers was concentrated to give 5-[3-(1-ethoxyvinyl)pyrazin-2-yl]-7-methyl-4-oxa-6,7-diazaspiro[2.5]oct-5-en-8-one (total 160 g crude) was obtained as brown oil.

Step 4: To a solution of 5-[3-(1-ethoxyvinyl)pyrazin-2-yl]-7-methyl-4-oxa-6,7-diazaspiro[2.5]oct-5-en-8-one (160 g, crude) in MeCN(100 mL) was aaded HCl (2N, 80mL) and stirred at 10°C for 1hr. LC-MS showed 5-[3-(1-ethoxyvinyl)pyrazin-2-yl]-7-methyl-4-oxa-6,7-diazaspiro[2.5]oct-5-en-8-one was consumed completely. The mixture was poured into H₂O (400 mL), extracted with EtOAc (200 mL x 3). The combined organic layers were dried over Na₂SO₄, filtered and concentrated. The residue was purified by column chromatography (SiO2, Commercial hexanes: Ethyl acetate=3/1 to 1/1) and further purified by column chromatography to give 5-(3-acetylpyrazin-2-yl)-7-methyl-4-oxa-6,7-diazaspiro[2.5]oct-5-en-8-one (60 g, 230.55 mmol, 83.69% yield) was obtained as a yellow solid. ¹H NMR: (400 MHz, CDCl₃) δ = 8.73 (d, *J* = 2.4 Hz, 1H), 8.64 (d, *J* = 2.4 Hz, 1H), 3.39 (s, 3H), 2.70 (s, 3H), 1.63 - 1.56 (m, 2H), 1.30 - 1.24 (m, 3H).

### Step 5: Three batches

To a solution of 5-(3-acetylpyrazin-2-yl)-7-methyl-4-oxa-6,7-diazaspiro[2.5]oct-5-en-8-one (16.3 g, 62.63 mmol, 1 eq) and 2-methylpropane-2-sulfinamide (15.18 g, 125.27 mmol, 2 *eq*) in THF (180 mL) was added Ti(i-PrO)₄ (44.50 g, 156.58 mmol, 46.21 mL, 2.5 eq) and stirred at 10°C for 0.5hr. Then the mixture was stirred at 80°Cfor 16hr. LC-MS showed 5-(3-acetylpyrazin-2-yl)-7-methyl-4-oxa-6,7-diazaspiro[2.5]oct-5-en-8-onewas consumed completely. The reaction solution was poured into H₂O (600 mL), and stirred at 10°C for 0.5hr, then filtered and the filtered cake washed with EtOAc(500 mL*3),the filtrate was extracted with EtOAc(500 mL *3), the organics dried over Na₂SO₄, filtered and concentrated. The residue was purified by column chromatography (SiO2, Commercial hexanes: Ethyl acetate=1/1 to 0/1) to give (*NE*)-2-methyl-*N-*[1-[3-(7-methyl-8-oxo-4-oxa-6,7-diazaspiro[2.5]oct-5-en-5-yl)pyrazin-2-yl]ethylidene]propane-2-sulfinamide (total 55 g, 151.33 mmol, 80.54% yield) was obtained as yellow oil.

¹H NMR: (400 MHz, CDCl₃) δ = 8.64 (dd, *J* = 2.3, 12.6 Hz, 1H), 8.60 - 8.50 (m, 1H), 3.39 (d, *J =* 2.6 Hz, 3H), 2.93 - 2.46 (m, 3H), 1.63 - 1.54 (m, 2H), 1.32 - 1.21 (m, 11H).

### Step 6: Six batchs

To a solution of (*NE*)-2-methyl-*N*-[1-[3-(7-methyl-8-oxo-4-oxa-6,7-diazaspiro[2.5]oct-5-en-5-yl)pyrazin-2-yl]ethylidene]propane-2-sulfinamide (11.6 g, 31.92 mmol, 1 eq) in THF (96 mL) and MeOH (24 mL) was added NaBH₄ (1.21 g, 31.92 mmol, 1 eq) at 0°C. The mixture was stirred at 0°C for 1.5 hr. LC-MS showed (*NE*)-2-methyl-*N*-[1-[3-(7-methyl-8-oxo-4-oxa-6,7-diazaspiro[2.5]oct-5-en-5-yl)pyrazin-2-yl]ethylidene]propane-2-sulfinamide was consumed completely. The reaction solution was poured into ice H₂O (1000 mL), extracted with EtOAc (800 mL x 4), the organics dried over Na₂SO₄, filtered and concentrated. The residue was purified by column chromatography (SiO₂, hexanes: ethyl acetate=1/9 to 0/1) to give 2-methyl-*N*-[1-[3-(7-methyl-8-oxo-4-oxa-6,7-diazaspiro[2.5]oct-5-en-5-yl)pyrazin-2-yl]ethyl]propane-2-sulfinamide (50 g, 136.82 mmol, 71.44% yield) was obtained as a red solid.
¹H NMR: (400 MHz, DMSO-d₆) δ = 8.77 (t, *J* = 2.2 Hz, 1H), 8.64 (t, *J* = 2.2 Hz, 1H), 6.62 (s, 1H), 5.62 - 5.46 (m, 1H), 5.36 - 5.13 (m, 1H), 3.32 (d, *J =* 5.4 Hz, 3H), 1.59 - 1.47 (m, 3H), 1.47 - 1.26 (m, 4H), 1.06 (d, *J* = 9.3 Hz, 9H)

### Intermediate 9: Synthesis of N-[1-[3-(6-isopropyl-4-methyl-5-oxo-1,3,4-oxadiazin-2-yl)pyrazin-2-yl]ethyl]-2-methyl-propane-2-sulfinamide (Int-9):

Step 1: To a solution of 3-chloro-*N*'-methyl-pyrazine-2-carbohydrazide (97.5 g, 522.51 mmol, 1 eq) *2 in MeCN (1500 mL) was added K₂CO₃ (144.43 g, 1.05 mol, 2 eq) and 2-bromo-3-methyl-butanoyl chloride (125.07 g, 627.01 mmol, 1.2 eq). The mixture was stirred at 25 °C for 2hr. LCMS showed the reaction was completed. The crude product *N*'-(2-bromo-3-methyl-butanoyl)-3-chloro-*N*'-methyl-pyrazine-2-carbohydrazide (366 g, crude) solution and used into the next step without further purification.

Step 2: To a solution of *N*'-(2-bromo-3-methyl-butanoyl)-3-chloro-N'-methyl-pyrazine-2-carbohydrazide (183 g, 523.44 mmol, 1 eq) in MeCN (2 L) was added K₂CO₃ (144.68 g, 1.05 mol, 2 eq). The mixture was stirred at 50 °C for 2hr. LCMS showed the reaction was completed. The reaction mixture was quenched by addition H₂O 1L at 20 °C,and extracted with EtOAc (1.5 L * 3). The combined organic layers were washed with brine (1L), dried over Na₂SO₄, filtered and concentrated under reduced pressure to give a residue. The residue was purified by column chromatography (SiO₂, petroleum ether/ethyl acetate=3/1) to give 2-(3-chloropyrazin-2-yl)-6-isopropyl-4-methyl-1,3,4-oxadiazin-5-one as a white soild.
¹H NMR: (400 MHz, DMSO-*d*₆) δ = 8.80 (d, J = 2.4 Hz, 1H), 8.72 - 8.65 (m, 1H), 4.91 (d, J = 3.6 Hz, 1H), 3.29 (s, 3H), 2.41 - 2.28 (m, 1H), 1.04 (d, J = 7.0 Hz, 3H), 0.99 (d, J = 6.8 Hz, 3H)

Step 3: A mixture of 2-(3-chloropyrazin-2-yl)-6-isopropyl-4-methyl-1,3,4-oxadiazin-5-one (60 g, 223.30 mmol, 1 *eq*) , tributyl(1-ethoxyvinyl)stannane (96.77 g, 267.96 mmol, 90.53 mL, 1.2 *eq*) , Pd(PPh₃)₄ (12.90 g, 11.16 mmol, 0.05 eq) in Tol (500 mL) was degassed and purged with N₂ for 3 times, and then the mixture was stirred at 110 °C for 16hr under N₂ atmosphere. LCMS showed the reaction was completed. The reaction mixturewas quenched by KF aqueous (1L) at 0°C, filtered and thefiltrate was extracted with EtOAc(1L x 2). The combined organic layers were washed with brine (60 mL), dried over Na₂SO₄, filtered andconcentrated under reduced pressure to give a residue. The crude product 2-[3-(1-ethoxyvinyl)pyrazin-2-yl]-6-isopropyl-4-methyl-1,3,4-oxadiazin-5-one (70 g, crude) was obtained as black oil and used into the next step without further purification.

Step 4: To a solution of 2-[3-(1-ethoxyvinyl)pyrazin-2-yl]-6-isopropyl-4-methyl-1,3,4-oxadiazin-5-one (70 g, 230.00 mmol, 1 eq) in MeCN (500 mL) was added HCl (6 M, 76.67 mL, 2 eq). The mixture was stirred at 20 °C for 2hr. LCMS showed the reaction was completed. The reaction mixture was quenched by addition H₂O 1L at 20 °C,and extracted with EtOAc (1L *3) The combined organic layers were washed with brine (1L), dried over Na₂SO₄, filtered and concentrated under reduced pressure to give a residue. The residue was purified by column chromatography (SiO₂, petroleum ether/ethyl acetate=1/1) to give 2-(3-acetylpyrazin-2-yl)-6-isopropyl-4-methyl-1,3,4-oxadiazin-5-one (80g) as yellow oil.

¹H NMR: (400 MHz, DMSO-d₆) δ = 8.90 (d, J = 2.5 Hz, 1H), 8.84 (d, J = 2.4 Hz, 1H), 4.80 (d, J = 3.8 Hz, 1H), 3.23 (s, 3H), 2.64 (s, 3H), 2.42 - 2.27 (m, 1H), 1.01 (d, J = 7.0 Hz, 3H), 0.94 (d, J = 6.8 Hz, 3H).

Step 5: A mixture of 2-(3-acetylpyrazin-2-yl)-6-isopropyl-4-methyl-1,3,4-oxadiazin-5-one (80 g, 289.55 mmol, 1 eq) , 2-methylpropane-2-sulfinamide (49.13 g, 405.37 mmol, 1.4 eq) , Ti(i-PrO)₄ (132.10 g, 579.10 mmol, 120.09 mL, 2 eq), in THF (1 L) was degassed and purged with N₂ for 3 times, and then the mixture was stirred at 80 °C for 16hr under N₂ atmosphere. LCMS showed the reaction was completed. The reaction mixture was quenched by addition H₂O 1L at 20 °C,and extracted with EtOAc (1L *3) The combined organic layers were washed with brine (1L), dried over Na₂SO₄, filtered and concentrated under reduced pressure to give a residue. The residue was purified by column chromatography (SiO₂, petroleum ether/ethyl acetate=0/1) to give (*NE*)-*N*-[1-[3-(6-isopropyl-4-methyl-5-oxo-1,3,4-oxadiazin-2-yl)pyrazin-2-yl]ethylidene]-2-methylpropane-2-sulfinamide (60 g) as a yellow solid.
¹H NMR: (400 MHz, DMSO-*d*₆) δ = 8.90 - 8.80 (m, 1H), 8.70 (s, 1H), 4.95 - 4.77 (m, 1H), 3.23 (d, J = 7.6 Hz, 3H), 2.38 - 2.22 (m, 1H), 1.27 (s, 2H), 1.20 - 0.92 (m, 16H) (400 MHz, DMSO-d6) δ = 8.90 - 8.80 (m, 1H), 8.70 (s, 1H), 4.95 - 4.77 (m, 1H), 3.23 (d, J = 7.6 Hz, 3H), 2.38 - 2.22 (m, 1H), 1.27 (s, 2H), 1.20 - 0.92 (m, 16H)

Step 6: A mixture of (*NE*)-*N*-[1-[3-(6-isopropyl-4-methyl-5-oxo-1,3,4-oxadiazin-2-yl)pyrazin-2-yl]ethylidene]-2-methyl-propane-2-sulfinamide (10 g, 21.08 mmol, 1 eq), in THF (80 mL) /MeOH (20 mL) was added NaBH₄ (558.30 mg, 14.76 mmol, 0.7 eq) at 0°C and then the mixture was stirred at 25 °Cfor 1hr under N₂ atmosphere. LCMS showed the reaction was completed. The reaction mixture was quenched by addition NH₄Cl 100mL at 20°C, and extracted with EtOAc (100mL * 3). The combined organic layers were washed with brine (100mL *3), dried over Na₂SO₄, filtered and concentrated under reduced pressure to give a residue. The residue was purified by column chromatography (SiO₂, Petroleum ether/Ethyl acetate=1/1) to give *N*-[1-[3-(6-isopropyl-4-methyl-5-oxo-1,3,4-oxadiazin-2-yl)pyrazin-2-yl]ethyl]-2-methyl-propane-2-sulfinamide (35 g) as brown oil.

¹H NMR: (400 MHz, DMSO-d*₆*) δ = 8.79 - 8.76 (m, 1H), 8.70 - 8.65 (m, 1H), 5.59 - 5.44 (m, 1H), 5.33 - 5.09 (m, 1H), 4.93 - 4.79 (m, 1H), 3.31 - 3.29 (m, 3H), 1.10 - 1.05 (m, 10H), 1.04 - 1.01 (m, 6H), 0.99 - 0.95 (m, 3H).

Step 7: To a solution of *N*-[1-[3-(6-isopropyl-4-methyl-5-oxo-1,3,4-oxadiazin-2-yl)pyrazin-2-yl]ethyl]-2-methyl-propane-2-sulfinamide (32 g, 67.10 mmol, 1 eq) in dioxane (300 mL) was added HCl/MeOH (4 M, 167.76 mL, 10 eq). The mixture was stirred at 20 °C for 2hr. LCMS showed the reaction was completed. The reaction mixture was concentrated under reduced pressure to remove solvent. The crude product 2-[3-(1-aminoethyl)pyrazin-2-yl]-6-isopropyl-4-methyl-1,3,4-oxadiazin-5-one (19 g, crude) was obtained as yellow oil and used into the next step without further purification.

Step 8: To a solution of 2-[3-(1-aminoethyl)pyrazin-2-yl]-6-isopropyl-4-methyl-1,3,4-oxadiazin-5-one (14 g, 50.48 mmol, 1 eq) in THF (7 mL) was added TEA (15.32 g, 151.45 mmol, 21.08 mL, 3 eq) and Boc₂O (16.53 g, 75.72 mmol, 17.40 mL, 1.5 eq). The mixture was stirred at 25 °C for 1hr. LCMS showed the reaction was completed. The reaction mixture was quenched by addition H₂O 100 mL at 20 °C, and extracted with EtOAc (100 mL * 3). The combined organic layers were washed with brine (100 mL * 3), dried over Na₂SO₄, filtered and concentrated under reduced pressure to give a residue. The residue was purified by flash silica gel chromatography (ISCO^{®}; 120 g SepaFlash^{®} Silica Flash Column, Eluent of 0-15% ethyl acetate/petroleum ether gradient @ 120 mL/min) to give *tert-*butyl *N*-[1-[3-(6-isopropyl-4-methyl-5-oxo-1,3,4-oxadiazin-2-yl)pyrazin-2-yl]ethyl]carbamate (6.3 g, yield 32.79 %) as white solid.
¹H NMR: (400 MHz, DMSO-*d*₆) δ = 8.72 (br s, 1H), 8.63 (dd, J = 2.3, 7.4 Hz, 1H), 7.38 - 7.28 (m, 1H), 5.31 - 5.19 (m, 1H), 4.90 - 4.80 (m, 1H), 3.28 (d, J = 4.4 Hz, 3H), 2.44 - 2.22 (m, 1H), 1.40 (br d, J = 6.9 Hz, 3H), 1.30 (br d, J = 1.8 Hz, 7H), 1.15 - 0.90 (m, 8H), LCMS: (Desired MS: 377.2, Observed MS: 378.2)

### Intermediate 14: Synthesis of (S)-2-methyl-N-[(1S)-1-[3-(8-oxo-4-oxa-6,7-diazaspiro[2.5]oct-5-en-5-yl)pyrazin-2-yl]ethyl]propane-2-sulfinamide (Int-14):

Step 1: To a solution of 5-(3-acetylpyrazin-2-yl)-4-oxa-6,7-diazaspiro[2.5]oct-5-en-8-one (22 g, 89.35 mmol, 1 eq) in THF (110 mL) was added (*S*)-2-methylpropane-2-sulfinamide (21.66 g, 178.70 mmol, 2 eq) and Ti(i-PrO)₄ (63.49 g, 223.38 mmol, 65.92 mL, 2.5 eq). The mixture was stirred at 15°C for 0.5h. Then stirred at 80°C for 12h. LC-MS showed 5-(3-acetylpyrazin-2-yl)-4-oxa-6,7-diazaspiro[2.5]oct-5-en-8-one was consumed completely and one main peak with desired mass was detected. The reaction mixture was quenched with ice-H₂O (100 mL), extracted with EtOAc (70 mL x 3). The combined organic layer was washed with brine (150 mL), dried over Na₂SO₄, filtered and concentrated. The residue was purified by column chromatography (SiO₂, Commercial hexanes : Ethyl acetate=1/0 to 2/1) and prep-HPLC (column: Welch Xtimate C18 250*70mm*10um; mobile phase: [H₂O(10mM NH₄HCO₃)-ACN]; gradient:15%-45% B over 19.0 min) to give (*NE*,*S*)-2-methyl-*N*-[1-[3-(8-oxo-4-oxa-6,7-diazaspiro[2.5]oct-5-en-5-yl)pyrazin-2-yl]ethylidene]propane-2-sulfinamide (15 g, 42.93 mmol, yield : 48.05%) as a yellow solid.
¹H NMR: (400 MHz, CDCl₃)
δ [ppm] = 8.65 (dd, J = 2.2, 9.7 Hz, 1H), 8.59 - 8.54 (m, 1H), 8.28 (br d, J = 5.2 Hz, 1H), 2.84 - 2.51 (m, 3H), 1.64 - 1.59 (m, 2H), 1.49 - 1.33 (m, 2H), 1.32 - 1.21 (m, 9H).

### Step 2: 4 Batch:

To a solution of (*NE*,*S*)-2-methyl-*N*-[1-[3-(8-oxo-4-oxa-6,7-diazaspiro[2.5]oct-5-en-5-yl)pyrazin-2-yl]ethylidene]propane-2-sulfinamide (2.1 g, 6.01 mmol, 1 eq) in THF (35 mL) was added 9-BBN (0.5 M, 28.25 mL, 2.35 eq) at 0°C under N₂. The mixture was stirred at 0°C for 2h. TLC indicated (*NE,S*)*-*2-methyl-*N*-[1-[3-(8-oxo-4-oxa-6,7-diazaspiro[2.5]oct-5-en-5-yl)pyrazin-2-yl]ethylidene]propane-2-sulfinamide was consumed completely and one new spot formed. The reaction was clean according to TLC. The reaction mixture was quenched with saturated NaHCO₃ aq. (35 mL), extracted with EtOAc (15 mL x 5). The combined organic layer was concentrated. The crude was purified by prep-HPLC (column: Welch Xtimate C18 250*100mm#10um;mobile phase: [H₂O(10mM NH₄HCO₃)-ACN];gradient:10%-40% B over 20.0 min) to give (*S*)-2-methyl-*N-*[(1*S*)-1-[3-(8-oxo-4-oxa-6,7-diazaspiro[2.5]oct-5-en-5-yl)pyrazin-2-yl]ethyl]propane-2-sulfinamide (total 4.5 g, 12.80 mmol, yield: 53.3%) as a yellow solid.
¹H NMR: (400 MHz, DMSO-*d*₆)
δ [ppm] = 11.37 (br d, J = 4.4 Hz, 1H), 8.76 (d, J = 2.4 Hz, 1H), 8.63 (d, J = 2.3 Hz, 1H), 5.57 (d, J = 8.9 Hz, 1H), 5.20 (dd, J = 6.8, 8.6 Hz, 1H), 1.51 - 1.36 (m, 5H), 1.35 - 1.22 (m, 2H), 1.07 (s, 9H).
LCMS: (Desired MS: 352, Observed MS: 352)

### Summary amine intermediats :

2-[3-(1-aminoethyl)pyrazin-2-yl]-4-methyl-1,3,4-oxadiazin-5-one (Int-1a)
2-[3-(1-aminoethyl)pyrazin-2-yl]-4*H*-1,3,4-oxadiazin-5-one (Int-2a)
2-[3-(1-aminoethyl)pyrazin-2-yl]-4,6,6-trimethyl-1,3,4-oxadiazin-5-one (Int-3a)
2-[3-(1-aminoethyl)pyrazin-2-yl]-6,6-dimethyl-4*H*-1,3,4-oxadiazin-5-one (Int-4a)
2-[3-[1-(methylamino)ethyl]pyrazin-2-yl]-4*H*-1,3,4-oxadiazin-5-one (Int-5a)
6,6-dimethyl-2-[3-[1-(methylamino)ethyl]pyrazin-2-yl]-4*H*-1,3,4-oxadiazin-5-one (Int-6a)
5-[3-(1-aminoethyl)pyrazin-2-yl]-4-oxa-6,7-diazaspiro[2.5]oct-5-en-8-one (Int-7a)
5-[3-(1-aminoethyl)pyrazin-2-yl]-7-methyl-4-oxa-6,7-diazaspiro[2.5]oct-5-en-8-one (Int-8a)
2-[3-(1-aminoethyl)pyrazin-2-yl]-6-isopropyl-4-methyl-1,3,4-oxadiazin-5-one (Int-9a)
5-[3-[(1*S*)-1-aminoethyl]pyrazin-2-yl]-4-oxa-6,7-diazaspiro[2.5]oct-5-en-8-one (Int-14a)
6,6-dimethyl-2-[3-[(1S)-1-(methylamino)ethyl]pyrazin-2-yl]-4*H*-1,3,4-oxadiazin-5-one (Int-15a)

### Bicyclic-chlorid intermediats:

Some of the bicyclic-chloride intermediats can be synthesized as described in the literature. Synthetic procedures of the bicyclic chlorid precursors 4,6-dichloro-8-(trifluoromethyl)quinazoline, 4,8-dichloro-6-(trifluoromethyl)quinazoline and 4-chloro-6,8-bis(trifluoromethyl)quinazoline were reported in WO2023104714. Synthetic procedures of 2,6-dichloro-5-(trifluoromethyl)-1,3-benzoxazole and 2,7-dichloro-5-(trifluoromethyl)-1,3-benzoxazole were reported in WO2022101265. Synthetic procedures of 4,8-dichloro-1-methyl-6-(trifluoromethyl)quinazolin-2-one were reported in WO2021148639.

Other bicyclic-chlorid intermediats can be synthesized in the following way:
Due to the potential for hydrolysis of the bicyclic chloride intermediates, these intermediates can be stored in the form of bicyclic-OH derivatives. Prior to their use in the subsequent reaction, they can be halogenated as detailed below.

Generally the bicyclic-OH intermediats can be halogenated in the following, generally applicible, way:
To a solution of compound 6-chloro-8-(trifluoromethylsulfonyl)quinazolin-4-ol (8.00 g, 25.6 mmol, *1.00* eq) in SOCl₂ (80.0 mL) was added DMF (0.4 mL). The reaction mixture was stirred at 100 °C for 0.5 hrs. LCMS showed ~96% of desired compound was detected. The reaction mixture was concentrated under reduced pressure to give a residue. The crude product was triturated with MTBE (15 mL) at 25 °C for 2 hrs and then was further purified by flash silica gel chromatography (ISCO^{®}; 80g SepaFlash^{®} Silica Flash Column, Eluent of 0-15% THF/Petroleum ether @100 mL/min). Compound 4,6-dichloro-8-(trifluoromethylsulfonyl)quinazoline (5.24 g, 15.8 mmol, 61.9% yield) was obtained as a white solid. LCMS: (Rt = 1.578 min, [M+H]⁺ = 330.8)

### Intermediate 10: Synthesis of 6-chloro-8-(trifluoromethylsulfanyl)quinazolin-4-ol (Int-10) :

Step 1 : To a solution of compound 2-amino-5-chloro-benzoic acid (30.0 g, 174 mmol, 1.00 *eq)* in DMF (300 mL) was added TFA (19.9 g, 174 mmol, 1.00 *eq*) and NIS (51.1 g, 227 mmol, 1.30 eq). The reaction mixture was stirred at 25 °C for 16 hrs. LCMS showed ~92% of desired compound was detected. The reaction mixture was poured into H₂O (400 mL) and the resulting mixture was filtered. The filter cake was dried under reduced pressure. The crude product was triturated with THF (20 mL) at 25 °C for 2 hrs. The mixture was filtered, and the cake was dried under reduced pressure. 2-Amino-5-chloro-3-iodo-benzoic acid (50.0 g, 168 mmol, 96.1% yield) was obtained as a gray solid. LCMS: (Rt = 1.409 min, [M+H]⁺ = 297.8) ¹H NMR: (400 MHz, DMSO-*d*₆) δ 13.73 - 12.55 (m, 1H), 7.89 (d, *J* = 2.5 Hz, 1H), 7.75 (d, *J* = 2.5 Hz, 1H), 7.02 - 6.53 (m, 2H).

Step 2: A solution of 2-amino-5-chloro-3-iodo-benzoic acid (50.0 g, 168 mmol, 1.00 eq) in formamide (500 mL) was stirred at 140 °C for 16 hrs. LCMS showed ~90% of desired compound was detected. The reaction mixture was poured into H₂O (300 mL) and filtered. The filter cake was dried under reduced pressure. The crude product was triturated with H₂O (50 mL) at 25 °C for 2 hrs. The mixture was filtered, and the cake was dried under reduced pressure. 6-chloro-8-iodo-quinazolin-4-ol (50.0 g, 163 mmol, 97.1% yield) was obtained as an off-white solid. LCMS: (Rt = 1.435 min, [M+H]⁺ = 306.9) ¹H NMR: (400 MHz, CDCl₃) δ 13.16 (d, *J =* 2.4 Hz, 1H), 12.99 (s, 1H), 12.82 (d, *J* = 2.4 Hz, 1H).

Step 3: To a solution of 6-chloro-8-iodo-quinazolin-4-ol (40.0 g, 131 mmol, 1.00 eq) in DMF (500 mL) was added NaH (10.4 g, 261 mmol, 60% purity, 2.00 eq) at 0 °C under N₂. The reaction mixture was stirred at 0 °C for 30 min. Then SEM-Cl (32.6 g, 196 mmol, 34.6 mL, 1.50 eq) was added into the solution. The reaction mixture was stirred at 25 °C for 16 hrs. TLC (Petroleum ether/Ethyl acetate = 3/1, compound 2-[(6-chloro-8-iodo-quinazolin-4-yl)oxymethoxy]ethyl-trimethyl-silane: R_{f} = 0.4) indicated 6-chloro-8-iodo-quinazolin-4-ol was consumed completely. The reaction mixture was quenched by addition of NH₄Cl solution (500 mL) at 0 °C under N₂, and then diluted with brine (200 mL) and extracted with EtOAc (300 mL x 2). The combined organic layers were washed with brine (200 mL), dried over Na₂SO₄, filtered and concentrated under reduced pressure to give a residue. The residue was purified by flash silica gel chromatography (ISCO^{®}; 330g SepaFlash^{®} Silica Flash Column, Eluent of 0-13% THF/Petroleum ether @100 mL/min). 2-[(6-chloro-8-iodo-quinazolin-4-yl)oxymethoxy]ethyl-trimethyl-silane (40.0 g, 91.6 mmol, 70.2% yield) was obtained as a yellow solid. ¹H NMR: (400 MHz, DMSO-*d*₆) δ 8.59 (s, 1H), 8.48 (d, *J* = 2.4 Hz, 1H), 8.17 (d, *J* = 2.4 Hz, 1H), 5.40 (s, 2H), 3.75 - 3.57 (m, 2H), 0.96 - 0.87 (m, 2H), 0.00 (s, 9H).

Step 4: To a solution of compound 2-[(6-chloro-8-iodo-quinazolin-4-yl)oxymethoxy]ethyl-trimethyl-silane (39.0 g, 89.3 mmol, 1.00 eq) in DMF (400 mL) was added trifluoromethylsulfanylsilver (26.1 g, 125 mmol, 1.40 *eq*) and Cul (17.0 g, 89.3 mmol, 1.00 *eq*)*.* The reaction mixture was stirred at 130 °C for 16 hrs under N₂. LCMS showed ~52% of desired compound was detected. The reaction mixture was quenched with H₂O (500 mL) at 0 °C, and extracted with EtOAc (300 mL x 3). The combined organic layers were washed with brine (300 mL), dried over Na₂SO₄, filtered and concentrated under reduced pressure to give a residue. The residue was purified by flash silica gel chromatography (ISCO^{®}; 330g SepaFlash^{®} Silica Flash Column, Eluent of 0~9% THF/Petroleum ether @100 mL/min). 2-[[6-chloro-8-(trifluoromethylsulfanyl)quinazolin-4-yl]oxymethoxy]ethyl-trimethyl-silane (14.0 g, 34.1 mmol, 38.2% yield) was obtained as a white solid. LCMS: (Rt = 2.210 min, [M+H]⁺ = 411.0).

Step 5: A solution of compound 2-[[6-chloro-8-(trifluoromethylsulfanyl)quinazolin-4-yl]oxymethoxy]ethyl-trimethyl-silane (10.0 g, 24.3 mmol, 1.00 *eq*) in HCl/MeOH (2 M, 100 mL) was stirred at 35 °C for 16 hrs. LCMS showed ~79% of desired compound was detected. The reaction mixture was filtered and concentrated under reduced pressure to give a residue. The crude product was triturated with DCM (5 mL) at 25 °C for 1 hr. The mixture was filtered, and the cake was dried under reduced pressure. 6-Chloro-8-(trifluoromethylsulfanyl)quinazolin-4-ol (Int-10) (5.13 g, 18.3 mmol, 75.1% yield) was obtained as an off-white solid. LCMS: (Rt = 1.425 min, [M+H]⁺ = 280.9).

### Intermediate 11 : Synthesis of 6-chloro-8-(trifluoromethylsulfinyl)quinazolin-4-ol (Int-11):

To a solution of 6-chloro-8-(trifluoromethylsulfanyl)quinazolin-4-ol (Int-3) (6.50 g, 23.2 mmol, *1.00 eq*) in DCM (70.0 mL) was added m-CPBA (11.8 g, 57.9 mmol, 85% purity, 2.50 *eq*) at 0 °C. The reaction mixture was stirred at 25 °C for 3 hrs. LCMS showed ~34% of desired compound was detected. The reaction mixture was quenched with aq. Na₂SO₃ (150 mL), and extracted with DCM (100 mL x 3). The combined organic extracts were washed with brine (100 mL), dried over Na₂SO₄, filtered and concentrated under reduced pressure to give a residue. The crude product was recrystallized with DMSO (10 mL) at 110 °C. The mixture was filtered, and the cake was dried under reduced pressure. 6-chloro-8-(trifluoromethylsulfinyl)quinazolin-4-ol (Int-11) (5.03 g, 17.0 mmol, 73.2% yield) was obtained as a white solid. LCMS: (Rt = 1.343 min, [M+H]⁺ = 296.9) ¹H NMR: (400 MHz, DMSO-*d*₆) δ 13.13 - 12.60 (m, 1H), 8.37 (d, *J* = 2.5 Hz, 1H), 8.33 (s, 1H), 8.16 (d, *J* = 2.5 Hz, 1H) LCMS: (Rt = 1.133 min, [M+H]⁺ = 296.8).

### Intermediate 12 : Synthesis of 6-chloro-8-(trifluoromethylsulfonyl)quinazolin-4-ol (Int-12):

To a solution of 6-chloro-8-(trifluoromethylsulfanyl)quinazolin-4-ol (Int-3) (7.00 g, 24.9 mmol, 1.00 *eq*) in CCl₄ (50 mL), ACN (50 mL) and H₂O (100 mL) was added NalO₄ (10.7 g, 49.9 mmol, 2.00 *eq*) and RuCl₃ (517 mg, 2.49 mmol, 0.10 *eq*)*.* The reaction mixture was stirred at 25 °C for 3 hrs. LCMS showed ~86% of desired compound was detected. The reaction mixture was quenched with aq. Na₂SO₃ (150 mL), and extracted with EtOAc (100 mL x 3). The combined organic extracts were washed with brine (100 mL), dried over Na₂SO₄, filtered and concentrated under reduced pressure to give a residue. The crude product was purified by reversed-phase flash chromatography (H₂O/ACN = 1/0 to 0/1). 6-Chloro-8-(trifluoromethylsulfonyl)quinazolin-4-ol (Int-12) (5.00 g, 16.0 mmol, 64.1% yield) was obtained as a white solid. LCMS: (Rt = 1.527 min, [M+H]⁺ = 313.0).

### Intermediate 13: Synthesis of 6,8-bis(trifluoromethoxy)quinazolin-4-ol (Int-13):

Step 1: To a solution of 2-amino-5-(trifluoromethoxy)benzoic acid (30.0 g, 136 mmol, 1.00 *eq*) in AcOH (300 mL) was added NBS (25.4 g, 142 mmol, 1.05 *eq*) at 0 °C. The reaction mixture was stirred at 25 °C for 16 hr. TLC (Petroleum ether/Ethyl acetate = 5/1, 2-amino-3-bromo-5-(trifluoromethoxy)benzoic acid: R_{f} = 0.3) indicated 2-amino-5-(trifluoromethoxy)benzoic acid was consumed completely. The reaction mixture was poured into H₂O (400 mL) and filtered. The filter cake was dried under reduced pressure. The crude product was triturated with THF (30 mL) at 25 °C for 2 hrs. The mixture was filtered, and the cake was dried under reduced pressure. 2-amino-3-bromo-5-(trifluoromethoxy)benzoic acid (39.5 g, 132 mmol, 97.0% yield) was obtained as a yellow solid. ¹H NMR: (400 MHz, DMSO-*d*₆) δ 13.81 - 12.88 (m, 1H), 7.80 (d, *J* = 2.4 Hz, 1H), 7.70 (dd, *J* = 0.8, 2.8 Hz, 1H), 7.08 - 6.75 (m, 1H)

Step 2: A solution of 2-amino-3-bromo-5-(trifluoromethoxy)benzoic acid (39.0 g, 130 mmol, 1.00 eq) in formamide (400 mL) was stirred at 140 °C for 16 hrs. LCMS, product: RT = 1.338 min) showed ~94% of desired compound was detected. The reaction mixture is poured into H₂O (300 mL) and filtered. The filter cake was dried under reduced pressure. The crude product was triturated with H₂O (50 mL) at 25 °C for 2 hr. The mixture was filtered, and the cake was dried under reduced pressure. 8-Bromo-6-(trifluoromethoxy)quinazolin-4-ol (36.0 g, 116 mmol, 89.6% yield) was obtained as a gray solid. LCMS: (Rt = 1.338 min, [M+H]⁺ =308.9/310.9) ¹H NMR: (400 MHz, DMSO-*d*₆) δ 12.81 (br s, 1H), 8.44 - 8.25 (m, 2H), 8.05 (dd, *J* = 1.3, 2.6 Hz, 1H).

Step 3: To a solution of 8-bromo-6-(trifluoromethoxy)quinazolin-4-ol (18.0 g, 58.3 mmol, 1.00 *eq*) in toluene (660 mL) was added TFA (664 mg, 5.82 mmol, 0.10 *eq*) and DHP (49.0 g, 582 mmol, 10.0 *eq*). The reaction mixture was stirred at 115 °C for 16 hrs. TLC (Petroleum ether/Ethyl acetate = 4/1, 8-bromo-4-tetrahydropyran-2-yloxy-6-(trifluoromethoxy)quinazoline: R_{f} = 0.4) indicated 8-bromo-6-(trifluoromethoxy)quinazolin-4-ol was consumed completely. The reaction mixture was concentrated under reduced pressure to give a residue. The crude product was triturated with MTBE (20 mL) at 25 °C for 16 hr. The mixture was filtered, and the cake was dried under reduced pressure. 8-bromo-4-tetrahydropyran-2-yloxy-6-(trifluoromethoxy)quinazoline (35.0 g, 89.0 mmol, 76.4% yield) was obtained as a white solid. ¹H NMR: (400 MHz, DMSO-*d*₆) δ 8.62 (s, 1H), 8.31 (d, *J* = 2.4 Hz, 1H), 8.03 (dd, *J* = 1.3, 2.6 Hz, 1H), 5.90 - 5.75 (m, 1H), 4.20 - 4.01 (m, 1H), 3.70 (dt, *J* = 3.0, 11.4 Hz, 1H), 2.01 - 1.50 (m, 6H).

Step 4: To a solution of 8-bromo-4-tetrahydropyran-2-yloxy-6-(trifluoromethoxy)quinazoline (17.5 g, 44.5 mmol, 1.00 eq) in dioxane (180 mL) and H₂O (50 mL) was added CsOH * H₂O (22.4 g, 134 mmol, 3.00 *eq*) and t-BuBrettphos Pd G3 (3.80 g, 4.45 mmol, 0.10 *eq*)*.* The reaction mixture was stirred at 115 °C for 16 hrs under N₂. LCMS (product: RT = 1.627 min) showed ~84% of desired compound was detected. The reaction mixture was quenched with H₂O (100 mL) and diluted hydrochloric acid was added to adjust pH=4, and the resulting mixture was extracted with EtOAc (200 mL * 3). The combined organic extracts were washed with brine (150 mL), dried over Na₂SO₄, filtered and concentrated under reduced pressure to give a residue. The crude product was used into the next step without further purification. 4-Tetrahydropyran-2-yloxy-6-(trifluoromethoxy)quinazolin-8-ol (21.0 g, 63.6 mmol, 71.4% yield) was obtained as a white solid. LCMS: (Rt= 1.627 min, [M-THP+H]⁺ = 246.9) ¹H NMR: (400 MHz, DMSO-*d*₆) δ 10.89 - 10.53 (m, 1H), 8.40 (s, 1H), 7.42 (dd, *J* = 1.3, 2.5 Hz, 1H), 7.17 (d, *J =* 2.1 Hz, 1H), 5.84 (dd, *J* = 5.7, 7.3 Hz, 1H), 4.20 - 4.07 (m, 1H), 3.70 (dt, *J* = 2.6, 11.5 Hz, 1H), 2.00 - 1.52 (m, 6H).

Step 5: To a solution of 4-tetrahydropyran-2-yloxy-6-(trifluoromethoxy)quinazolin-8-ol (17.0 g, 51.5 mmol, 1.00 *eq*) in DMF (160 mL) was added NaH (4.12 g, 103 mmol, 60% purity, 2.00 *eq*) at 0 °C under N₂. The mixture was stirred at 0 °C for 60 mins. Then dibromo(difluoro)methane (32.4 g, 154 mmol, 3.00 eq) was added into the solution. The reaction mixture was stirred at 25 °C for 16 hrs under N₂. LCMS showed ~55% of desired compound was detected. The reaction mixture was quenched by addition of H₂O (300 mL) at 0 °C under N₂, and then extracted with EtOAc (300 mL x 2). The combined organic layers were washed with brine (200 mL), dried over Na₂SO₄, filtered and concentrated under reduced pressure to give a residue. The crude product was triturated with MTBE/EtOAc (V/V=10:1, 20 mL) at 25 °C for 1 hr. The mixture was filtered, and the cake was dried under reduced pressure. 8-[bromo(difluoro)methoxy]-4-tetrahydropyran-2-yloxy-6-(trifluoromethoxy)quinazoline (16.0 g, 34.9 mmol, 67.7% yield) was obtained as a white solid. LCMS: (Rₜ = 1.989 min, [M-THP+H]⁺ = 376.9/374.9).

Step 6: To a solution of 8-[bromo(difluoro)methoxy]-4-tetrahydropyran-2-yloxy-6-(trifluoromethoxy)quinazoline (15.0 g, 32.7 mmol, 1.00 eq) in DCM (160 mL) was added AgBF₄ (9.54 g, 49.0 mmol, 1.50 *eq*). The reaction mixture was stirred at 25°C for 16 hrs. LCMS, product: RT = 1.500 min) showed ~85% of desired compound was detected. The reaction mixture was quenched by addition of H₂O (200 mL) at 0 °C, and then extracted with DCM (300 mL * 2). The combined organic layers were washed with brine (200 mL), dried over Na₂SO₄, filtered and concentrated under reduced pressure to give a residue. The crude product was purified by reversed-phase flash chromatography (H₂O/ACN = 1:0 - 0:1). 6,8-Bis(trifluoromethoxy)quinazolin-4-ol (Int-13) (6.62 g, 21.1 mmol, 64.5% yield) was obtained as a yellow solid. LCMS: (Rt = 1.500 min, [M+H]⁺ = 315.0) ¹H NMR: (400 MHz, CDCl₃) δ [ppm] 10.85 - 10.28 (m, 1H), 8.20 (s, 1H), 8.14 (dd, *J* = 1.1, 2.5 Hz, 1H), 7.61 (s, 1H) LCMS: (Rt = 1.381 min, [M+H]⁺ = 314.8).

### Bicyclic-chlorid intermediats :

4,6-dichloro-8-(trifluoromethylsulfanyl)quinazoline (Int-10c)
4,6-dichloro-8-(trifluoromethylsulfinyl)quinazoline (Int-11c)
4,6-dichloro-8-(trifluoromethylsulfonyl)quinazoline (Int-12c)
4-chloro-6,8-bis(trifluoromethoxy)quinazoline (Int-13c)

### Synthesis of the final compounds:

### Final compounds:

### Example 1: Synthesis of 2-[3-[1-[[6,8-bis(trifluoromethoxy)quinazolin-4-yl]amino]ethyl]pyrazin-2-yl]-4-methyl-1,3,4-oxadiazin-5-one (I-9):

Step 1: To a solution of 6-chloro-8-(trifluoromethylsulfonyl)quinazolin-4-ol (8.00 g, 25.6 mmol, 1.00 eq) in SOCl₂ (80.0 mL) was added DMF (0.4 mL). The reaction mixture was stirred at 100 °C for 0.5 hrs. LCMS showed ~96% of desired compound was detected. The reaction mixture was concentrated under reduced pressure to give a residue. The crude product was triturated with MTBE (15 mL) at 25 °C for 2 hrs and then was further purified by flash silica gel chromatography (ISCO^{®}; 80g SepaFlash^{®} Silica Flash Column, Eluent of 0-15% THF/Petroleum ether @100 mL/min). Compound 4,6-dichloro-8-(trifluoromethylsulfonyl)quinazoline (5.24 g, 15.8 mmol, 61.9% yield) was obtained as a white solid. LCMS: (Rt = 1.578 min, [M+H]⁺ = 330.8)

Step 2: To a solution of 1-[3-(4-methyl-5-oxo-1,3,4-oxadiazin-2-yl)pyrazin-2-yl]ethylammonium chloride (85 mg, 0.313 mmol, 1 eq.) and 4,6-dichloro-8-(trifluoromethylsulfonyl)quinazoline (155 mg, 0.469 mmol, 1.5 eq.) in 2-propanol (6 mL), DIPEA (0.54 mL, 129 mg, 3.13 mmol, 10 eq.) was added. The reaction mixture was stirred at 50 °C for 4 hrs. The mixture was concentrated under reduced pressure purified by flash-column chromatography on silica gel to afford the compound 2-[3-[1-[[6-chloro-8-(trifluoromethylsulfonyl)quinazolin-4-yl]amino]ethyl]pyrazin-2-yl]-4-methyl-1,3,4-oxadiazin-5-one (26 mg, 16% yield) as a white solid. LC-MS (ESI) m/z = 532.0 (M+H)⁺. ¹H NMR: (400 MHz, MeOD) δ [ppm] 8.65 (d, J = 2.4 Hz, 1H), 8.54 (d, J = 2.4 Hz, 1H), 8.46 - 8.43 (m, 1H), 8.40 (s, 1H), 7.75 - 7.72 (m, 1H), 6.22 (q, J = 6.9, 1H), 4.92 (s, 2H), 3.37 (s, 3H), 1.81 (d, J = 6.9, 3H).

### Example 2: Synthesis of 5-[3-[(1S)-1-[[6,8-bis(trifluoromethoxy)quinazolin-4-yl]amino]ethyl]pyrazin-2-yl]-4-oxa-6,7-diazaspiro[2.5]oct-5-en-8-one (I-17):

Step 1: To a solution of 6,8-bis(trifluoromethoxy)quinazolin-4-ol (800 mg, 2.55 mmol, 1.00 eq) in SOCl₂ (16 mL) was added DMF (18.6 mg). The reaction mixture was stirred at 100 °C for 3 hrs. The reaction mixture was concentrated under reduced pressure to give a residue. The crude product 4-chloro-6,8-bis(trifluoromethoxy)quinazoline was obtained without further purification.

Step 2: To a solution of [(1S)-1-[3-(8-oxo-4-oxa-6,7-diazaspiro[2.5]oct-5-en-5-yl)pyrazin-2-yl]ethyl]ammonium chloride (794.9 mg, 2.80 mmol, 1.1 eq.) and 4-chloro-6,8-bis(trifluoromethoxy)quinazoline (847 mg, 2.55 mmol, 1.0 eq.) in 2-propanol (30 mL), DIPEA (4.36 mL, 3292 mg, 25.5 mmol, 10 eq.) was added. The reaction mixture was stirred at 50 °C for 4 hrs. The mixture was concentrated under reduced pressure purified by flash-column chromatography on silica gel to afford the compound 5-[3-[(1S)-1-[[6,8-bis(trifluoromethoxy)quinazolin-4-yl]amino]ethyl]pyrazin-2-yl]-4-oxa-6,7-diazaspiro[2.5]oct-5-en-8-one (656 mg, 47% yield) as a white solid. LC-MS (ESI) m/z = 544.1 (M+H)⁺. ¹H NMR: (400MHz, DMSO-d₆) δ [ppm] 11.34 (s, 1H), 9.12 (d, J = 6.5 Hz, 1H), 8.70 (dd, J = 2.6, 1.1 HZ, 1H), 8.60 (d, J= 2.4 Hz, 1H), 8.59 (d, J = 2.4 Hz, 1H), 8.41 (s, 1H), 7.99 (d, J = 2.5 Hz, 1H), 6.12 - 6.02 (m, 1H), 1.69 (d, J = 6.9, 3H), 1.44 - 1.32 (m, 2H), 1.32 - 1.21 (m, 2H).

### Example 3: Synthesis of 7-methyl-5-[3-[rac-(1S)-1-[[6,8-bis(trifluoromethoxy)quinazolin-4-yl]amino]ethyl]pyrazin-2-yl]-4-oxa-6,7-diazaspiro[2.5]oct-5-en-8-one (I-18):

To a solution of 5-[3-[(1S)-1-[[6,8-bis(trifluoromethoxy)quinazolin-4-yl]amino]ethyl]pyrazin-2-yl]-4-oxa-6,7-diazaspiro[2.5]oct-5-en-8-one (313 mg, 0.576 mmol, 1 eq.) in DMF (6 mL), iodomethane (98.1, 0.691 mmol, 1.2 eq.) and Cs₂CO₃ (375 mg, 1.15 mmol, 2 eq.) were added. The reaction mixture was stirred at 25 °C for 10 min. The mixture was concentrated under reduced pressure, washed with water and brine, extracted with EtOAc and dried over MgSO₄. Afterwards it was purified by flash-column chromatography on silica gel to afford 5-[3-[(1S)-1-[[6,8-bis(trifluoromethoxy)quinazolin-4-yl]amino]ethyl]pyrazin-2-yl]-7-methyl-4-oxa-6,7-diazaspiro[2.5]oct-5-en-8-one (277 mg, 86%) as a white solid. LC-MS (ESI) m/z = 558.1 (M+H)⁺. ¹H NMR: (400MHz, DMSO-d₆) δ [ppm] 9.12 (d, J = 6.6 Hz, 1H), 8.72 (d, J = 2.4 Hz, 1H), 8.67 (s, 1H), 8.62 (d, J = 2.4 Hz, 1H), 8.44 (s, 1H), 8.00 (d, J = 2.5 Hz, 1H), 6.07 (p, J = 6.8 Hz), 3.33 (s, 3H), 3.22 (s, 3H), 1.72 (d, J = 6.9 Hz, 3H), 1.39 - 1.31 (m, 2H), 1.23 - 1.18 (m, 2H).

With appropriate modification of the starting materials, the procedures given in the synthesis descriptions were used to obtain further compounds I, in particular further compounds IIa with Z as listed below. The compounds obtained in this manner are listed in the table that follows (Table I), together with physical data.

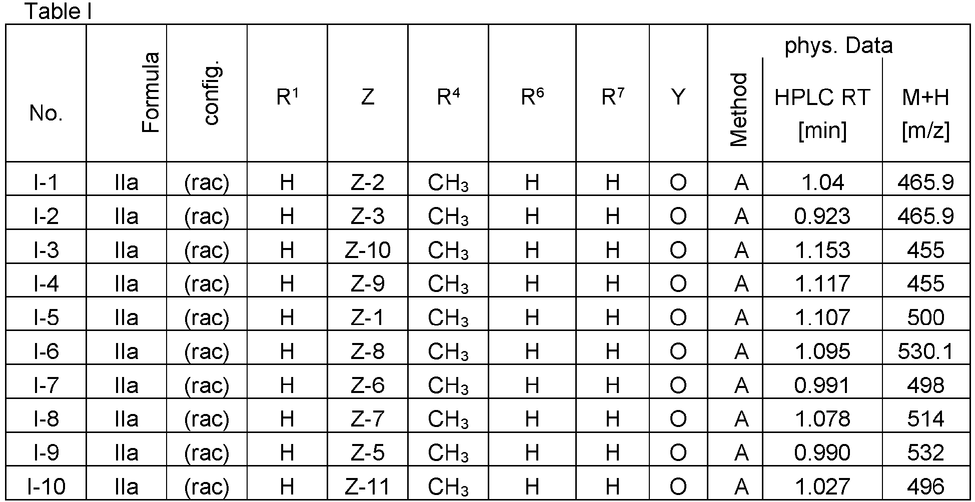

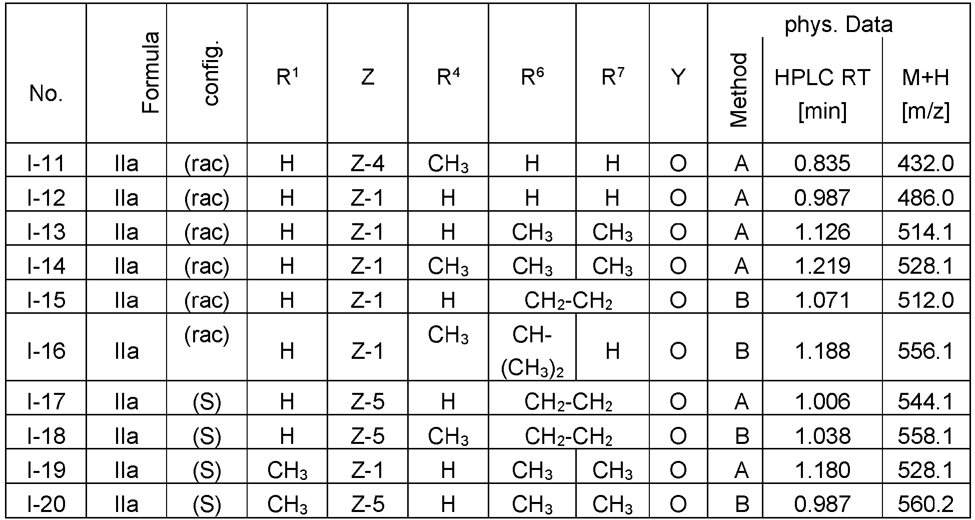

In I-15, I-17 and I-18, R⁶ and R⁷ together with the carbon atom to which they are bound, form a cyclopropyl ring.

### Biological examples

If not otherwise specified, the test solutions are prepared as follow:
The active compound is dissolved at the desired concentration in a mixture of 1:1 (vol:vol) distilled water : acetone. The test solution is prepared on the day of use.

The activity of the compounds of formula I of the present invention can be demonstrated and evaluated by the following biological tests.

### B.1 Tobacco budworm (Heliothis virescens)

For evaluating control of tobacco budworm (Heliothis virescens) the test unit consists of 96-well-microtiter plates containing an insect diet and 15-25 H. virescens eggs.

The compounds are formulated using a solution containing 75% v/v water and 25% v/v DMSO. Different concentrations of formulated compounds are sprayed onto the insect diet at 10µl, using a custom-built micro atomizer, at two replications.

After application, microtiter plates are incubated at about 28 ± 1°C and about 80 ± 5 % relative humidity for 5 days. Egg and larval mortality is then visually assessed.

In this test, compounds I-1, I-2, I-3, I-4, I-6, I-7, I-9 I-10, I-11, I-12, I-13, I-14, I-15 and I-16, resp., at 800 ppm showed at least 75% mortality in comparison with untreated controls.

### B.2 Boll weevil (Anthonomus grandis)

For evaluating control of boll weevil (Anthonomus grandis) the test unit consists of 96-well-microtiter plates containing an insect diet and 5-10 A. grandis eggs.

The compounds are formulated using a solution containing 75% v/v water and 25% v/v DMSO. Different concentrations of formulated compounds are sprayed onto the insect diet at 5µl, using a custom-built micro atomizer, at two replications.

After application, microtiter plates are incubated at about 25 ± 1°C and about 75 ± 5 % relative humidity for 5 days. Egg and larval mortality is then visually assessed.

In this test, compounds I-1, I-2, I-3, I-4, I-6, I-7, I-9, I-10, I-11, I-12, I-13, I-14, I-15 and I-16, resp., at 800 ppm showed at least 75% mortality in comparison with untreated controls.

### B.3 Diamond back moth (Plutella xylostella)

The active compound was dissolved at the desired concentration in a mixture of 1:1 (vol:vol) distilled water : acetone. Surfactant (Kinetic HV) was added at a rate of 0.01% (vol/vol). The test solution was prepared on the day of use.

Leaves of cabbage were dipped in test solution and air-dried. Treated leaves were placed in petri dishes lined with moist filter paper and inoculated with ten 3rd instar larvae. Mortality was recorded 72 hours after treatment. Feeding damages were also recorded using a scale of 0-100%.

In this test, compounds I-1, I-2, I-3, I-4, I-6, I-7, I-9, I-10, I-11, I-12, I-13, I-14, I-15 and I-16, resp., at 800 ppm showed at least 75% mortality in comparison with untreated controls.

### B.4 Yellow fever mosquito (Aedes aegypti)

For evaluating control of yellow fever mosquito (Aedes aegypti) the test unit consists of 96-well-microtiter plates containing 200µl of tap water per well and 5-15 freshly hatched A. aegypti larvae.

The active compounds are formulated using a solution containing 75% (v/v) water and 25% (v/v) DMSO. Different concentrations of formulated compounds or mixtures are sprayed onto the insect diet at 2.5µl, using a custom-built micro atomizer, at two replications.

After application, microtiter plates are incubated at 28±1°C, 80±5 % RH for 2 days. Larval mortality is then visually assessed.

In this test, compounds I-1, I-2, I-3, I-4, I-6, I-7, I-9, I-10, I-11, I-12, I-13, I-14, I-15 and I-16, resp., at 800 ppm showed at least 75% mortality in comparison with untreated controls.

### B.5 Green peach aphid (Myzus persicae)

For evaluating control of green peach aphid (Myzus persicae) through systemic means, the test unit consisted of 96-well-microtiter plates containing liquid artificial diet under an artificial membrane.

The compounds were formulated using a solution containing 75% v/v water and 25% v/v DMSO. Different concentrations of formulated compounds were pipetted into the aphid diet, using a custom built pipetter, at two replications.

After application, 5 - 8 adult aphids were placed on the artificial membrane inside the microtiter plate wells. The aphids were then allowed to suck on the treated aphid diet and incubated at about 23 ± 1°C and about 50 ± 5 % relative humidity for 3 days. Aphid mortality and fecundity was then visually assessed.

In this test, compounds I-1, I-2, I-4, I-5, I-6, I-7, I-9 I-10, I-11, I-12, I-13, I-14, I-15 and I-16, resp., at 800 ppm showed at least 75% mortality in comparison with untreated controls.

### Example B.6: Action on Southern armyworm (Spodoptera eridania), 2nd instar larvae

The active compounds were formulated by a Tecan liquid handler in 100% cyclohexanone as a 10,000 ppm solution supplied in tubes. The 10,000 ppm solution was serially diluted in 100% cyclohexanone to make interim solutions. These served as stock solutions for which final dilutions were made by the Tecan in 50% acetone:50% water (v/v) into 10 or 20 ml glass vials. A nonionic surfactant (Kinetic^{®}) was included in the solution at a volume of 0.01% (v/v). The vials were then inserted into an automated electrostatic sprayer equipped with an atomizing nozzle for application to plants/insects.

Lima bean plants (variety Sieva) were grown 2 plants to a pot and selected for treatment at the 1st true leaf stage. Test solutions were sprayed onto the foliage by an automated electrostatic plant sprayer equipped with an atomizing spray nozzle. The plants were dried in the sprayer fume hood and then removed from the sprayer. Each pot was placed into perforated plastic bags with a zip closure. About 10 to 11 armyworm larvae were placed into the bag and the bags
zipped closed. Test plants were maintained in a growth room at about 25°C and about 20-40% relative humidity for 4 days, avoiding direct exposure to fluorescent light (24 hour photoperiod) to prevent trapping of heat inside the bags. Mortality and reduced feeding were assessed 4 days after treatment, compared to untreated control plants
I-1, I-2, I-3, I-4, I-5, I-6, I-7, I-8, I-9, I-11, I-12, I-13, I-14, I-15 and I-16 at 100 ppm showed at least 75 % mortality in comparison with untreated controls.

## Claims

1. Compounds of formula I 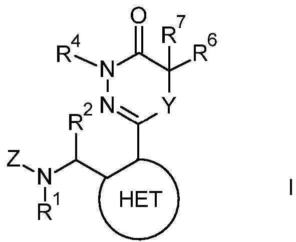 wherein
R¹ is H, OR^{10a}, NR¹²R¹³, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₆-alkoxy, C₂-C₆-alkenyl, C₂-C₆-alkynyl, C₁-C₆-alkyl-C₁-C₆-alkoxy, C₁-C₆-alkoxy-C₃-C₆-cycloalkyl, C₃-C₆-cycloalkyl-C₁-C₆-alkoxy, C₂-C₆-alkenyl-Cₐ-C₆-cycloalkyl, C₂-C₆-alkynyl-C₃-C₆-cycloalkyl, C₃-C₆-cycloalkyl-C₂-C₆-alkenyl, C₃-C₆-cycloalkyl-C₂-C₆-alkynyl, wherein the alkyl, alkoxy, alkenyl, alkynyl, and cycloalkyl moieties are unsubstituted or substituted with R¹¹; or C(O)R^{11a};
R² is H, C₁-C₃-alkyl, C₂-C₃-alkenyl, C₃-C₆-cycloalkyl, or C₂-C₃-alkynyl, wherein the alkyl, alkenyl, alkynyl and cycloalkyl moieties are unsubstituted or substituted with one or more halogen;
R³ is halogen, CN, NO₂, SF₅, C₁-C₆-alkyl, C₁-C₆-alkoxy, C₃-C₆-cycloalkyl, C₂-C₆-alkenyl, C₂-C₆-alkynyl, C₃-C₆-cycloalkyl-C₁-C₆-alkyl, C₁-C₆-alkyl-C₃-C₆-cycloalkyl, 3- to 6-membered heterocyclyl, wherein the alkyl, alkenyl, alkynyl, alkoxy, heterocyclyl and cycloalkyl moieties are unsubstituted or substituted with R¹¹; OR^{10a}, NR¹²R¹³, C(O)NR¹²R¹³, C(O)OR¹⁰, C(O)R¹⁴, OS(O)₂-R¹⁴, S(O)ₘ-R¹⁴, -N=S(O)R^{12a}R^{13a};
R⁴ H, C₁-C₃-alkyl, C₂-C₄-alkenyl, C₂-C₄-alkynyl, C₃-C₆-cycloalkyl-C₁-C₂-alkyl, C₁-C₂-alkyl-C₃-C₆-cycloalky, C₃-C₆-cycloalkyl, wherein the alkyl, alkenyl, alkynyl or cycloalkyl moieties are unsubstituted or substituted with one or more halogen, CN, or C(O)NH₂; phenyl or 5- or 6-membered hetaryl, wherein the phenyl or hetaryl moiety are unsubstituted or substituted with one or more halogen, CN, C(O)NH₂, C₁-C₃-alkyl, C₁-C₃-haloalkyl, C₁-C₃-alkoxy, or C₁-C₃-haloalkoxy;
HET is a group HA or HB 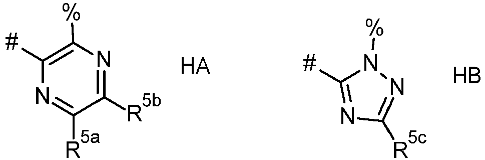 wherein # is the bond to the CH(R²)N(R¹) spacer, and % is the bond to the diazinone ring;
R^{5a}, R5b are independently from each other H, halogen, CN, C₁-C₃-alkyl, C₁-C₃-haloalkyl, C₃-C₆-cycloalkyl, C₃-C₆-cycloalkyl-C₁-C₃-alkyl, C₁-C₃-alkyl-C₃-C₆-cycloalkyl, C₁-C₃-alkoxy, C₁-C₃-haloalkoxy, C₂-C₃-alkenyl, or C₂-C₃-alkynyl;
R^{5c} is H, halogen, CN, OR^{10a}, NR¹²R¹³, C(O)NR¹²R¹³, C(O)OR^{10a}, C(O)R¹⁴, S(O)ₘ-R¹⁴, C₁-C₃-alkyl, C₃-C₆-cycloalkyl, C₃-C₆-cycloalkyl-C₁-C₃-alkyl, C₁-C₃-alkyl-C₃-C₆-cycloalkyl, C₁-C₃-alkoxy, C₂-C₃-alkenyl, C₂-C₃-alkynyl, -C(=NOC₁-C₄-alkyl)H, or -C(=NOC₁-C₄-alkyl)-C₁-C₄-alkyl, wherein alkyl, alkenyl, alkynyl, cycloalkyl and alkoxy are unsubstituted or substituted with one or more halogen and/or CN;
R⁶, R⁷ are independently from each other H, halogen, CN, C(O)NH₂, C₁-C₃-alkyl, C₁-C₃-haloalkyl, or C₃-C₆-cycloalkyl; or
R⁶ and R⁷ together with the carbon atom to which they are bound, form a 3-, 4-, 5-, or 6-membered saturated or partially unsaturated carbocycle, wherein the carbocycle is unsubstituted or substituted with one or more halogen, cyano, C₁-C₃-alkyl, or C₁-C₃-haloalkyl; or form a 3-, 4-, 5-, or 6-membered saturated or partially unsaturated heterocycle, which may contain 1 or 2 heteroatoms or heteroatom-containing groups selected from N, O, S(O)ₘ, and optionally one or two C(O) groups as ring members, wherein the heterocycle is unsubstituted or substituted with one or more halogen, cyano, C₁-C₃-alkyl, C₁-C₃-haloalkyl;
R¹⁰ is H, C₁-C₄-alkyl, C₁-C₄-haloalkyl, C₃-C₆-cycloalkyl, C₃-C₆-halocycloalkyl, C₃-C₄-cycloalkyl-C₁-C₂-alkyl, C₃-C₄-halocycloalkyl-C₁-C₂-alkyl, C(O)-C₁-C₄-alkyl, C(O)-C₁-C₄-haloalkyl, C(O)-C₃-C₄-cycloalkyl, C(O)-C₃-C₄-halocycloalkyl, SOₘ-C₁-C₄-alkyl, SOₘ-C₁-C₄-haloalkyl, SOₘ₋C₃-C₆-cycloalkyl, or phenyl which is unsubstituted or partially or fully substituted with R^{a};
R^{10a} is H, C₃-C₆-cycloalkyl, C₃-C₆-halocycloalkyl, C₃-C₄-cycloalkyl-C₁-C₂-alkyl, C₃-C₄-halocycloalkyl-C₁-C₂-alkyl, C(O)-C₁-C₄-alkyl, C(O)-C₁-C₄-haloalkyl, C(O)-C₃-C₄-cycloalkyl, C(O)-C₃-C₄-halocycloalkyl, SOₘ-C₁-C₄-alkyl, SOₘ-C₁-C₄-haloalkyl, SOₘ-C₃-C₆-cycloalkyl, or phenyl which is unsubstituted or partially or fully substituted with R^{a};
R¹¹ is halogen, CN, NO₂, NR¹²R¹³, C(O)NH₂, C(S)NH₂, C(O)OH, OR¹⁰; 3- to 6-membered heterocyclyl, 5- or 6-membered hetaryl, or phenyl, wherein the heterocyclyl, hetaryl and phenyl moieties are unsubstituted or substituted with one or more halogen, C₁-C₃-haloalkyl, and/or CN;
R^{11a} is NR¹²R¹³, C₁-C₆-alkyl, C₁-C₆-alkoxy, C₂-C₆-alkenyl, C₂-C₆-alkynyl, C₃-C₄-cycloalkyl-C₁-C₂-alkyl, wherein the alkyl, alkoxy, alkenyl, alkynyl or cycloalkyl moiety is unsubstituted or substituted with one or more halogen; or 3- to 6-membered heterocyclyl, wherein the heterocyclyl moiety is unsubstituted or substituted with one or more halogen, C₁-C₃-haloalkyl, and/or CN;
R¹², R¹³ are independently from each other H, C₁-C₄-alkyl, C₁-C₄-alkoxy, C₁-C₄-halo-alkoxy, C₁-C₄-haloalkyl, C₃-C₆-cycloalkyl, C(O)-C₁-C₄-alkyl, C(O)-C₁-C₄-haloalkyl, C(O)-C₃-C₄-cycloalkyl, C(O)-C₃-C₄-halocycloalkyl, C(O)NH-C₁-C₄-alkyl, C(O)NH-C₁-C₄-haloalkyl, C(O)N(C₁-C₄-alkyl)-C₁-C₄-alkyl, C(O)N(C₁-C₄-haloalkyl)-C₁-C₄-alkyl, C(O)N(C₁-C₄-haloalkyl)-C₁-C₄-haloalkyl, C(O)NH-C₁-C₄-alkoxy, C(O)NH-C₁-C₄-halo-alkoxy, C(O)NH-C₁-C₄-alkoxy-C₁-C₄-alkyl, C(O)NH-C₁-C₄-alkoxy-C₁-C₄-haloalkyl; C(O)NH-3-6-membered heterocyclyl, C(O)NH-phenyl, C(O)NH-5-6-membered hetaryl, C(O)NH-C₁-C₄-alkyl-3-6-membered heterocyclyl, C(O)NH-C₁-C₄-alkylphenyl, C(O)NH-C₁-C₄-alkyl-5-6-membered hetaryl, wherein the phenyl, heterocyclyl and hetaryl moieties are unsubstituted or substituted with halogen, C₁-C₃-haloalkyl, and/or CN; S(O)ₘ-C₁-C₄-haloalkyl, S(O)ₘ-C₃-C₄-cycloalkyl, S(O)ₘ-C₃-C₄-halocycloalkyl; 3- to 6-membered heterocyclyl, 5- or 6-membered hetaryl, or phenyl, wherein the phenyl, heterocyclyl and hetaryl moieties are unsubstituted or substituted with halogen, C₁-C₃-haloalkyl, and/or CN; or
R¹² and R¹³ together with the atom/atoms to which they are bound, form a 3-, 4-, 5-, 6-, or 7-membered saturated or partially unsaturated heterocycle, which heterocycle may additionally contain 1 or 2 heteroatoms or heteroatom-containing groups selected from N, O, S(O)ₘ, and optionally one or two groups C(O) as ring members, and wherein the heterocycle moiety is unsubstituted or substituted with one or more R^{a};
R12a, R13a are independently from each other C₁-C₄-alkyl, C₁-C₄-haloalkyl, C₃-C₆-cycloalkyl, unsubstituted or substituted with halogen, C₁-C₃-haloalkyl, and/or CN; or R^{12a} and R^{13a} together with the atom to which they are bound, form a 3-, 4-, 5-, 6-, or 7-membered saturated or partially unsaturated heterocycle, wherein the heterocycle moiety may additionally contain 1 or 2 heteroatoms or heteroatom-containing groups selected from N, O, S(O)ₘ, and optionally one or two groups C(O) as ring members, and wherein the heterocycle moiety is unsubstituted or substituted with one or more R^{a};
R¹⁴ is H, C₁-C₆-alkyl, C₃-C₆-cycloalkyl, wherein the alkyl and cycloalkyl moieties are unsubstituted or partially or fully substituted with R¹¹; or 3- to 6-membered heterocyclyl, 5- or 6-membered hetaryl, or phenyl, wherein the phenyl, heterocyclyl and hetaryl moieties are unsubstituted or substituted with R^{a};
R^{a} is halogen, CN, NO₂, OH, C₁-C₄-alkyl, C₁-C₄-alkoxy, C₁-C₄-haloalkyl, C₁-C₄-alkyl-C₁-C₄-alkoxy, C₁-C₄-haloalkoxy, C₃-C₄-cycloalkyl, C₃-C₄-halocycloalkyl, S(O)ₘ-C₁-C₄-alkyl, S(O)ₘ-C₁-C₄-haloalkyl, S(O)ₘ-C₃-C₄-cycloalkyl, or S(O)ₘ-C₃-C₄-halocycloalkyl;
m is 0, 1, or 2;
n is 0, 1, 2, 3 or 4;
Y is O, S, or N-R^{N};
R^{N} is as defined for R⁴;
Z is a 9- or 10-membered saturated, partially or fully unsaturated bicyclic heterocyclic ring which contains 1-4 heteroatoms or heteroatom-containing groups selected from N, O, S(O)ₘ, wherein the bicyclic heterocyclic ring is unsubstituted or substituted with 1-4 R³;
and the N-oxides, stereoisomers, tautomers, and agriculturally or veterinarily acceptable salts thereof.

2. Compounds of formula I according to claim 1, wherein R¹ is H, C₁-C₆-alkyl, C₁-C₆-haloalkyl, C₃-C₆-cycloalkyl, C₃-C₆-halocycloalkyl, C₃-C₆-cycloalkyl-C₁-C₄-alkyl, C₁-C₄-alkyl-C₃-C₆-cycloalkyl, C₂-C₆-alkenyl, C₂-C₆-alkynyl, or C₁-C₆-alkyl-C₁-C₆-alkoxy.

3. Compounds of formula I according to any one of claims 1 or 2, wherein R² is CH₃.

4. Compounds of formula I according to any one of claims 1 to 3, wherein Z is selected from BC1, BC2 or BC3, 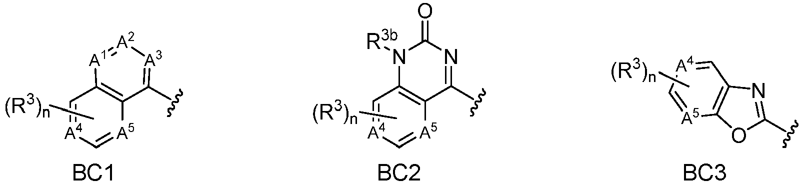 wherein
A¹, A², A³, A⁴ and A⁵ are independently selected from N, CH or CR³; and
R^{3b} is H, C₁-C₃-alkyl, or C₁-C₃-haloalkyl.

5. Compounds of formula I according to any one of claims 1 to 4, wherein Z is selected from BC1a, BC2a, BC3a or BC3b 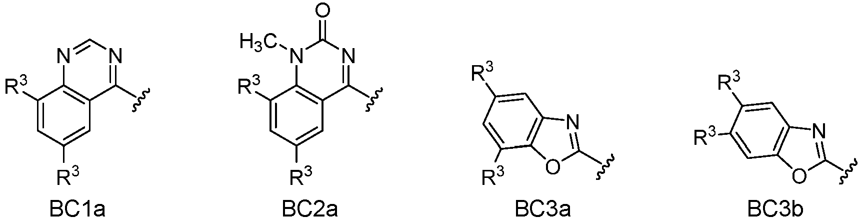 or, wherein Z is selected from Z1 to Z11

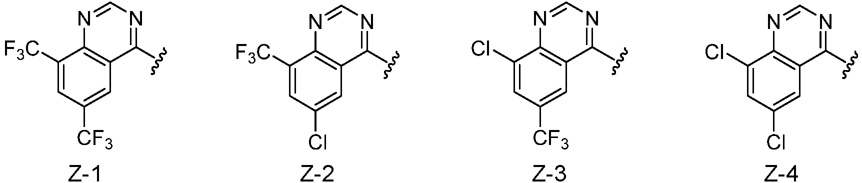

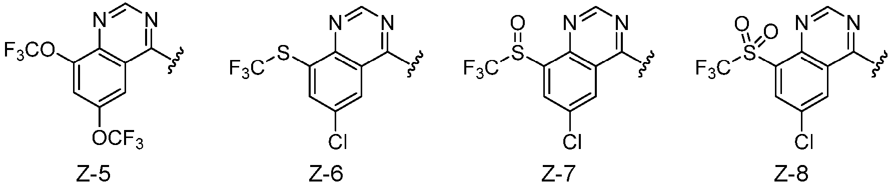

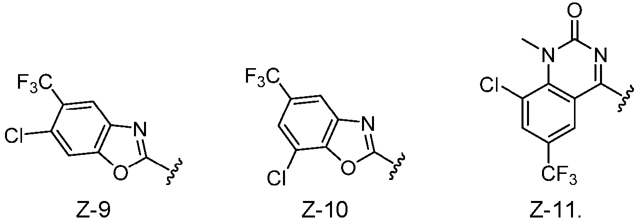

6. Compounds of formula I according to any one of claims 1 to 5, wherein
a) R⁶ and R⁷ are independently from each other H, CH₃, CH₂CH₃, CH(CH₃)₂ or CH₂CH₂CH₃; and/or
b) R₆ and R₇ together with the carbon atom to which they are bound, form a cyclopropyl ring or a cyclobutyl ring.

7. Compounds of formula I according to any one of the claims 1 to 6, wherein the compound is of formula IIa, IIb, Ilc or Ild.

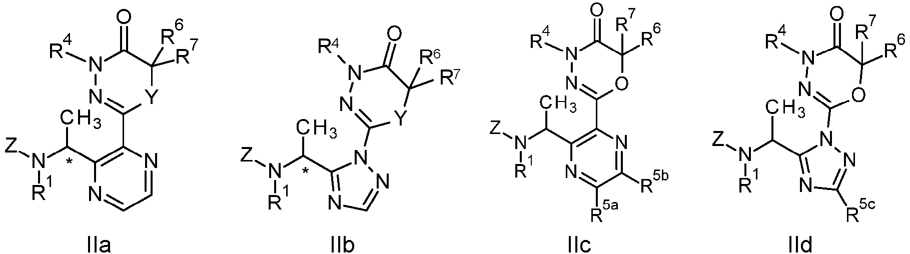

8. Compounds of formula I according to any one of the preceding claims, which consist mainly of the isomer I.S.

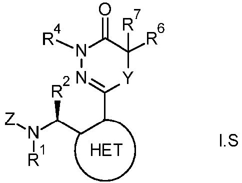

9. The compound of any one of the preceding claims, wherein the compound is selected from the group consisting of:
| | | | | | | | | |
|---|---|---|---|---|---|---|---|---|
| No. | Formula | Config. (*) | R¹ | Z | R⁴ | R⁶ | R⁷ | Y |
| I-1 | Ila | (rac) | H | Z-2 | CH₃ | H | H | O |
| I-2 | Ila | (rac) | H | Z-3 | CH₃ | H | H | O |
| I-3 | Ila | (rac) | H | Z-10 | CH₃ | H | H | O |
| I-4 | Ila | (rac) | H | Z-9 | CH₃ | H | H | O |
| I-5 | Ila | (rac) | H | Z-1 | CH₃ | H | H | O |
| I-6 | Ila | (rac) | H | Z-8 | CH₃ | H | H | O |
| I-7 | Ila | (rac) | H | Z-6 | CH₃ | H | H | O |
| I-8 | Ila | (rac) | H | Z-7 | CH₃ | H | H | O |
| I-9 | IIa | (rac) | H | Z-5 | CH₃ | H | H | O |
| I-10 | IIa | (rac) | H | Z-11 | CH₃ | H | H | O |
| I-11 | IIa | (rac) | H | Z-4 | CH₃ | H | H | O |
| I-12 | Ila | (rac) | H | Z-1 | H | H | H | O |
| I-13 | Ila | (rac) | H | Z-1 | H | CH₃ | CH₃ | O |
| I-14 | Ila | (rac) | H | Z-1 | CH₃ | CH₃ | CH₃ | O |
| I-15 | Ila | (rac) | H | Z-1 | H | CH₂-CH₂ | | O |
| I-16 | Ila | (rac) | H | Z-1 | CH₃ | CH-(CH₃)₂ | H | O |
| I-17 | Ila | (S) | H | Z-5 | H | CH₂-CH₂ | | O |
| I-18 | Ila | (S) | H | Z-5 | CH₃ | CH₂-CH₂ | | O |
|---|---|---|---|---|---|---|---|---|
| I-19 | Ila | (S) | CH₃ | Z-1 | H | CH₃ | CH₃ | O |
| I-20 | Ila | (S) | CH₃ | Z-5 | H | CH₃ | CH₃ | O |
and the N-oxides, stereoisomers, and agriculturally or veterinarily acceptable salts thereof.

10. Intermediate compounds of formula Int-6a, Int-7a, Int-8a or Int-9a:
6,6-dimethyl-2-[3-[1-(methylamino)ethyl]pyrazin-2-yl]-4H-1,3,4-oxadiazin-5-one (Int-6a),
5-[3-(1-aminoethyl)pyrazin-2-yl]-4-oxa-6,7-diazaspiro[2.5]oct-5-en-8-one (Int-7a),
5-[3-(1-aminoethyl)pyrazin-2-yl]-7-methyl-4-oxa-6,7-diazaspiro[2.5]oct-5-en-8-one (Int-8a),
2-[3-(1-aminoethyl)pyrazin-2-yl]-6-isopropyl-4-methyl-1,3,4-oxadiazin-5-one (Int-9a),
5-[3-[(1S)-1-aminoethyl]pyrazin-2-yl]-4-oxa-6,7-diazaspiro[2.5]oct-5-en-8-one (Int-14a)
6,6-dimethyl-2-[3-[(1S)-1-(methylamino)ethyl]pyrazin-2-yl]-4H-1,3,4-oxadiazin-5-one (Int-15a), or
the N-oxides, stereoisomers, and salts thereof, wherein the variables are as defined according to any of the preceding claims.

11. An agricultural or veterinary composition comprising at least one compound according to any one of claims 1 to 9 and/or at least one agriculturally or veterinarily acceptable salt thereof, and at least one inert liquid and/or solid agriculturally or veterinarily acceptable carrier.

12. An agricultural composition for combating animal pests comprising at least one compound as defined in any of claims 1 to 9 and at least one inert liquid and/or solid acceptable carrier and, if desired, at least one surfactant.

13. A method for combating or controlling invertebrate pests, which method comprises contacting said pest or its food supply, habitat or breeding grounds with a pesticidally effective amount of at least one compound as defined in any one of claims 1 to 9.

14. A method for protecting growing plants from attack or infestation by invertebrate pests, which method comprises contacting a plant, or soil or water in which the plant is growing, with a pesticidally effective amount of at least one compound as defined in any of claims 1 to 9.

15. Seed comprising a compound as defined in any of claims 1 to 9, or the enantiomers, diastereomers or salts thereof, in an amount of from 0.1 g to 10 kg per 100 kg of seed.

16. A method for treating or protecting an animal from infestation or infection by invertebrate pests which comprises bringing the animal in contact with a pesticidally effective amount of at least one compound of the formula I as defined in any of claims 1 to 9, a stereoisomer thereof and/or at least one veterinarily acceptable salt thereof.
